# EUROPEAN PATENT SPECIFICATION

(11) **EP 2 051 962 B1**
(45) Date of publication and mention of the grant of the patent: **26.10.2011**
(21) Application number: 07805287.5
(22) Date of filing: 02.08.2007
(51) Int. Cl.: C07D 209/88

(54) **(3-AMINO-1,2,3,4-TETRAHYDRO-9H-CARBAZOL-9-YL)-ACETIC ACID DERIVATIVES**
(3-AMINO-1,2,3,4-TETRAHYDRO-9H-CARBAZOL-9-YL)-ESSIGSÄUREDERIVATE
DÉRIVÉS DE L'ACIDE (3-AMINO-1,2,3,4-TÉTRAHYDRO-9H-CARBAZOL-9-YL)-ACÉTIQUE

(30) Priority: 07.08.2006 WO PCT/IB2006/052723
(43) Date of publication of application: 29.04.2009
(60) Divisional application: 11000641.8
(73) Proprietor: Actelion Pharmaceuticals Ltd., 4123 Allschwil (CH)
(72) Inventor: FRETZ, Heinz, CH-4125 Riehen (CH); POTHIER, Julien, F-68300 Saint-louis (FR); RISCH, Philippe, F-68130 Zaessingue (FR)
(74) Representative: Gschwend, Thomas Peter
(86) International application number: PCT/IB2007/053046
(87) International publication number: WO 2008/017989

(56) References cited:
- EP-A- 1 505 061
- WO-A-2006/070325
- GB-A- 2 388 540

## Description

### Field of the invention:

The present invention relates to (3-amino-1,2,3,4-tetrahydro-9*H*-carbazol-9-yl)-acetic acid derivatives of Formula **I** and their use as prostaglandin receptor modulators, most particularly as prostaglandin D₂ receptor ("DP receptor") modulators, in the treatment of various prostaglandin-mediated diseases and disorders, to pharmaceutical compositions containing these compounds and to processes for their preparation. In particular, such derivatives may be used alone or in pharmaceutical compositions for the treatment of both, chronic and acute allergic/immune diseases/disorders such as allergic asthma, rhinitis, allergic rhinitis, chronic obstructive pulmonary disease (COPD), dermatitis, inflammatory bowel disease, rheumatoid arthritis, allergic nephritis, conjunctivitis, atopic dermatitis, bronchial asthma, food allergy, systemic mast cell disorders, anaphylactic shock, urticaria, eczema, itching, inflammation, ischemia-reperfusion injury, cerebrovascular disorders, pleuritis, ulcerative colitis, eosinophil-related diseases, such as Churg-Strauss syndrome and sinusitis, and basophil-related diseases, such as basophilic leukemia and basophilic leukocytosis, in humans and other mammals.

### Background of the invention:

As a response to allergen exposure in allergic conditions, mast cells are activated and release chemotactic key mediators like histamine, thromboxane A2 (TxA2), cysteinyl leukotrienes (CysLTs) and prostaglandin D₂ (PGD₂). These mediators interact with their respective receptors and cause physiological effects such as increased vascular permeability, edema, pruritus, nasal and pulmonary congestion, bronchoconstriction, and mucus secretion. An increased vascular permeability for example, allows excessive infiltration of eosinophilic and basophilic leukocytes into the tissue and thus amplifies the allergic response.

Current treatments of allergic diseases comprise agents that can block or otherwise interrupt such interactions, e.g. anti-histamines (histamine H1 receptor antagonists), leukotriene receptor antagonists, beta-adrenergic receptor agonists, and corticosteroids. Generally, treatments with anti-histamines and leukotriene antagonists are limited in efficacy, and long-term usage of corticosteroids is associated with unwanted side effects.

PGD₂ is an agonist known to act on two G-protein-coupled receptors, the PGD₂ receptor DP1 and the recently identified CRTH2 (chemoattractant receptor-homologous molecule expressed on Th2 cells) receptor (also referred to as "DP2 receptor").

Elevated PGD₂ levels are considered to cause inflammation actions as observed in allergic diseases such as allergic rhinitis, allergic asthma, allergic conjunctivitis, atopic dermatitis and the like. Therefore, blocking the interaction of PGD₂ with its receptors is considered a useful therapeutic strategy for the treatment of such diseases.

WO 01/79169 discloses (tetrahydrocarbazol-1-yl)acetic acid derivatives as PGD₂ receptor antagonists.

GB 2388540 (Bayer AG) discloses the use of ramatroban ((3*R*)-3-(4-fluorobenzene-sulfonamido)-1,2,3,4-tetrahydrocarbazole-9-propionic acid), a TxA2 receptor (also referred to as "TP receptor") antagonist with additional antagonistic activity on CRTH2, for the prophylaxis and treatment of allergic diseases, such as asthma, allergic rhinitis or allergic conjunctivitis. In T. Ishizuka et al., Cardiovascular Drug Rev. 2004, 22(2), 71-90 effects of ramatroban on late-phase inflammation are described. Furthermore, oral bioavailability of ramatroban and its ability to inhibit prostaglandin D₂-induced eosinophil migration *in vitro* has been reported *(*Journal of Pharmacology and Experimental Therapeutics, 305(1), p.347-352 (2003)).

WO 03/097598 and WO 03/097042 disclose Ramatroban analogues with CRTH2 antagonistic activity. Ulven et al, in J. Med. Chem. 2005, 48(4), 897-900 disclose further ramatroban analogues.

The compounds of the invention are structurally different from corticosteroids, anti-histamines, leukotriene antagonists or beta-adrenergic agonists.

### Description of the invention:

i) The present invention relates to (3-amino-1,2,3,4-tetrahydro-9*H*-carbazol-9-yl)-acetic acid compounds of the Formula **I**: 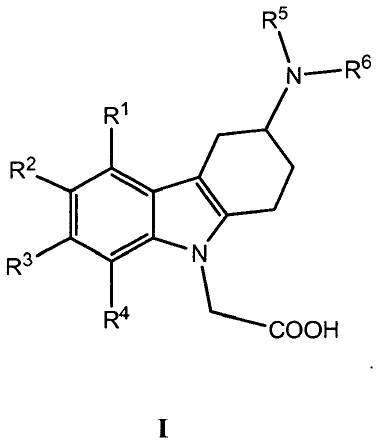 wherein
   R¹, R², R³ and R⁴ independently represent hydrogen, C₁₋₅-alkyl, C₁₋₅-alkoxy, alkenyl (especially allyl or vinyl), halogen, nitro, cyano, halo-C₁₋₆-alkoxy, halo-C₁₋₆-alkyl, C₁₋₆-alkylsulfonyl, or formyl;
   R⁵ represents hydrogen, alkenyl (especially allyl or vinyl), C₁₋₆-alkyl, cycloalkyl-C₁₋₄-alkyl, C₁₋₃-alkoxy-C₁₋₄-alkyl, aryl-C₁₋₄-alkyl, or aryloxy-C₁₋₄-alkyl (especially R⁵ represents hydrogen, C₁₋₆-alkyl, cycloalkyl-C₁₋₄-alkyl, C₁₋₃-alkoxy-C₁₋₄-alkyl, aryl-C₁₋₄-alkyl, or aryloxy-C₁₋₄-alkyl);
   wherein aryl is unsubstituted, mono- or di-substituted with a group independently selected from C₁₋₂-alkylendioxy, C₁₋₄alkoxy, C₁₋₄-alkyl, halogen, trifluoromethyl, and trifluoromethoxy (especially trifluoromethyl); and
   R⁶ represents C₁₋₉-alkylaminocarbonyl; C₁₋₉-alkylaminothiocarbonyl; C₁₋₉-alkylcarbonyl; C₁₋₉-alkoxycarbonyl; arylalkenylcarbonyl; arylaminocarbonyl; arylaminothiocarbonyl; aryl-C₁₋₃-alkoxy-C₁₋₃-alkoxycarbonyl; aryl-C₁₋₃-alkoxycarbonyl; aryl-C₁₋₃-alkylaminocarbonyl; aryl-C₁₋₆-alkylcarbonyl, aryl-C₁₋₃-alkoxy-C₁₋₃-alkylcarbonyl; arylcarbonyl; arylcarbonyl-C₁₋₄-alkylcarbonyl; aryloxy-C₁₋₃-alkylcarbonyl; arylsulfonylaminocarbonyl; cycloalkyl-C₁₋₃-alkylcarbonyl; diaryl-C₁₋₃-alkylcarbonyl; heterocyclylcarbonyl; heteroaryl-C₁₋₃-alkylcarbonyl; heteroarylcarbonyl; aryl-C₁₋₆-cycloalkylcarbonyl; cycloalkylcarbonyl; or R⁷-C₁₋₄-alkylcarbonyl, wherein the bridging C₁₋₄-alkyl group may additionally be mono-substituted with aryl or disubstituted with hydroxy, and R⁷ represents arylaminocarbonyl, heteroarylaminocarbonyl, C₁₋₆-alkylaminocarbonyl, or aryl-C₁₋₃-alkylaminocarbonyl; wherein aryl is unsubstituted, mono- or di-substituted with a group independently selected from C₁₋₂-alkylendioxy; C₁₋₆-alkoxy; C₁₋₆-alkyl; C₁₋₆-alkylsulfonyl; phenyl which is unsubstituted, mono- or di-substituted by substituents independently selected from halogen, trifluoromethyl, methoxy and methyl; naphthyl; phenyl-C₁₋₃-alkyl, wherein the phenyl group is unsubstituted, mono- or di-substituted with substituents independently selected from halogen, trifluoromethyl, methoxy and methyl; naphthyl-C₁₋₃-alkyl; phenoxy, wherein the phenyl group is unsubstituted, mono- or di-substituted with substituents independently selected from halogen, trifluoromethyl, methoxy and methyl; naphthyloxy; halogen; hydroxy; halo-C₁₋₆-alkyl; halo-C₁₋₆-alkoxy; C₁₋₆-alkylthio; and C₁₋₄-alkoxycarbonylamino.
ii) In another embodiment, the invention relates to compounds of Formula **I** according to embodiment i), wherein
   R⁵ represents hydrogen, alkenyl (especially allyl or vinyl), or C₁₋₆-alkyl; and
   R⁶ represents C₁₋₉-alkylaminocarbonyl, C₁₋₉-alkylaminothiocarbonyl, C₁₋₉-alkylcarbonyl, C₁₋₉-alkoxycarbonyl, arylalkenylcarbonyl, arylaminocarbonyl, arylaminothiocarbonyl, aryl-C₁₋₃-alkoxy-C₁₋₃-alkoxycarbonyl, aryl-C₁₋₃-alkoxycarbonyl, aryl-C₁₋₃-alkylaminocarbonyl, aryl-C₁₋₃-alkylcarbonyl, aryl-C₁₋₃₋alkoxy-C₁₋₃-alkylcarbonyl, arylcarbonyl, arylcarbonyl-C₁₋₄-alkylcarbonyl, aryloxy-C₁₋₃-alkylcarbonyl, arylsulfonylaminocarbonyl, cycloalkyl-C₁₋₃-alkylcarbonyl, diaryl-C₁₋₃-alkylcarbonyl, heterocyclylcarbonyl, heteroaryl-C₁₋₃-alkylcarbonyl, or heteroarylcarbonyl;
   wherein aryl is unsubstituted, mono- or di-substituted with a group independently selected from C₁₋₂-alkylendioxy; C₁₋₆-alkoxy; C₁₋₆-alkyl; C₁₋₆-alkylsulfonyl; phenyl which is unsubstituted, mono- or di-substituted with substituents independently selected from halogen, trifluoromethyl, methoxy and methyl; naphthyl; phenyl-C₁₋₃-alkyl, wherein the phenyl group is unsubstituted, mono- or di-substituted with substituents independently selected from halogen, trifluoromethyl, methoxy and methyl; naphthyl-C₁₋₃-alkyl; phenoxy, wherein the phenyl group is unsubstituted, mono- or di-substituted with substituents independently selected from halogen, trifluoromethyl, methoxy and methyl; naphthyloxy; halogen; hydroxy; halo-C₁₋₆-alkyl; and halo-C₁₋₆-alkoxy.
iii) A further embodiment of the invention relates to compounds of Formula **I** according to embodiment ii), wherein
   R¹, R², R³ and R⁴ independently represent hydrogen; C₁₋₅-alkyl, especially methyl, or isopropyl; C₁₋₅-alkoxy, especially methoxy; alkenyl, especially allyl or vinyl; halogen, especially fluoro, or chloro; halo-C₁₋₆-alkyl, especially trifluoromethyl; or C₁₋₆-alkylsulfonyl, especially methanesulfonyl;
   R⁵ represents hydrogen; alkenyl, especially ethenyl, or 2-propenyl; or C₁₋₆-alkyl, especially methyl, ethyl, or propyl; and
   R⁶ represents C₁₋₉-alkylaminocarbonyl, such as butylaminocarbonyl; C₁₋₉-alkylcarbonyl, such as propylcarbonyl, isobutylcarbonyl, hexylcarbonyl, or nonylcarbonyl; C₁₋₉-alkoxycarbonyl, such as propoxycarbonyl, *tert*-butoxycarbonyl, or isobutoxycarbonyl; arylalkenylcarbonyl, such as naphthalenylethenylcarbonyl (especially 2-naphthalen-2-yl-ethenylcarbonyl), or phenylethenylcarbonyl; arylaminocarbonyl, such as naphthalenaminocarbonyl (especially naphthalen-1-aminocarbonyl), or phenylaminocarbonyl; aryl-C₁₋₃-alkoxy-C₁₋₃-alkoxycarbonyl, such as benzyloxyethoxycarbonyl (especially 2-benzyloxy-ethoxycarbonyl); aryl-C₁₋₃-alkoxycarbonyl, such as benzyloxycarbonyl; aryl-C₁₋₃-alkylaminocarbonyl, such as benzylaminocarbonyl, or phenylethylaminocarbonyl; aryl-C₁₋₃-alkylcarbonyl, such as phenylmethylcarbonyl, phenylethylcarbonyl (especially 2-phenylethyl-carbonyl), or naphthalenylethylcarbonyl (especially 2-naphthalen-2-yl-ethylcarbonyl); aryl-C₁₋₃-alkoxy-C₁₋₃-alkylcarbonyl, such as benzyloxymethyl-carbonyl; arylcarbonyl, such as phenylcarbonyl; arylcarbonyl-C₁₋₄-alkylcarbonyl, such as phenylcarbonylethylcarbonyl (especially 2-phenylcarbonyl-ethylcarbonyl); aryloxy-C₁₋₃-alkylcarbonyl, such as phenoxymethylcarbonyl; arylsulfonylaminocarbonyl, such as phenylsulfonylaminocarbonyl; cycloalkyl-C₁₋₃-alkylcarbonyl, such as cyclopentylethylcarbonyl (especially 2-cyclopentylethylcarbonyl), or indanylmethylcarbonyl (especially indan-2-ylmethylcarbonyl); diaryl-C₁₋₃-alkylcarbonyl, such as 1,2-diphenylethylcarbonyl, or 2,2-diphenylethylcarbonyl; heterocyclylcarbonyl, such as dihydroindolylcarbonyl (especially 2,3-dihydro-1*H*-indole-2-carbonyl); heteroaryl-C₁₋₃-alkylcarbonyl, such as benzimidazolyl-C₁₋₃-alkylcarbonyl (especially 2-1*H*-benzimidazol-2-yl-ethylcarbonyl), or indolyl-C₁₋₃-alkylcarbonyl, such as indolylethylcarbonyl (especially 2-1*H*-indol-3-yl-ethylcarbonyl), or thienylmethylcarbonyl (especially 2-thienylmethylcarbonyl), or pyridinylethylcarbonyl (especially 2-(pyridin-3-yl)ethylcarbonyl); or heteroarylcarbonyl, such as indolylcarbonyl (especially 1*H*-indole-2-yl-carbonyl);
   wherein aryl (especially phenyl or naphthyl) is unsubstituted, mono- or di-substituted with (a) group(s) independently selected from C₁₋₂-alkylendioxy (especially methylendioxy), C₁₋₆-alkoxy (especially methoxy), C₁₋₆-alkyl (especially methyl, ethyl, isopropyl, or *tert-*butyl), C₁₋₆-alkylsulfonyl (especially methanesulfonyl), halogen (especially chloro, fluoro or bromo), hydroxy, and halo-C₁₋₆-alkyl (especially trifluoromethyl).
iv) A further embodiment of the invention relates to compounds of Formula **I** according to any one of embodiments i) to iii), wherein R¹ represents hydrogen, or halogen.
v) A further embodiment of the invention relates to compounds of Formula **I** according to any one of embodiments i) to iv), wherein R² represents hydrogen, trifluoromethyl, or halogen (especially hydrogen, or halogen).
vi) A further embodiment of the invention relates to compounds of Formula **I** according to any one of embodiments i) to v), wherein R³ represents hydrogen, or halogen.
vii) A further embodiment of the invention relates to compounds of Formula **I** according to any one of embodiments i) to vi), wherein R⁴ represents hydrogen, alkenyl (especially allyl or vinyl), halogen (especially chloro or bromo), C₁₋₆-alkylsulfonyl (especially methanesulfonyl); especially hydrogen or halogen.
viii) A further embodiment of the invention relates to compounds of Formula **I** according to any one of embodiments i) to vii), wherein R¹, R³ and R⁴ represent hydrogen.
ix) A further embodiment of the invention relates to compounds of Formula **I** according to any one of embodiments i) to viii), wherein R² represents fluoro.
x) A further embodiment of the invention relates to compounds of Formula **I** according to any one of embodiments i) and iv) to ix), wherein R⁵ represents hydrogen, C₁₋₆-alkyl (especially C₁₋₃-alkyl), cycloalkyl-C₁₋₄-alkyl (especially cyclopropylmethyl), C₁₋₃-alkoxy-C₁₋₄-alkyl (especially 2-methoxyethyl), aryl-C₁₋₄-alkyl (especially naphthylmethyl, or preferably phenyl-C₂₋₃-alkyl), or aryloxy-C₁₋₄-alkyl (especially phenoxyethyl); wherein aryl (especially phenyl) is unsubstituted (preferred), or mono- or di-substituted with a group independently selected from C₁₋₂-alkylendioxy, C₁₋₄-alkoxy, C₁₋₄-alkyl, halogen, trifluoromethyl, and trifluoromethoxy.
xi) A further embodiment of the invention relates to compounds of Formula **I** according to any one of embodiments i) and iv) to ix), wherein R⁵ represents hydrogen; C₁₋₃-alkyl (especially methyl); cyclopropylmethyl; 2-methoxyethyl; phenyl-C₂₋₃-alkyl; or phenoxyethyl, wherein the phenyl group is unsubstituted (preferred), or mono-substituted with a group selected from C₁₋₂-alkylendioxy, C₁₋₄-alkoxy, C₁₋₄-alkyl, halogen, trifluoromethyl, and trifluoromethoxy (especially trifluoromethyl).
xii) A further embodiment of the invention relates to compounds of Formula **I** according to any one of embodiments i) and iv) to ix), wherein R⁵ represents C₁₋₃-alkyl (especially methyl); cyclopropylmethyl; 2-methoxyethyl; phenyl-C₂₋₃-alkyl; or phenoxyethyl, wherein the phenyl group is unsubstituted (preferred), or mono-substituted with a group selected from C₁₋₂-alkylendioxy, C₁₋₄-alkoxy, C₁₋₄-alkyl, halogen, trifluoromethyl, and trifluoromethoxy (especially trifluoromethyl).
xiii) A further embodiment of the invention relates to compounds of Formula **I** according to any one of embodiments i) and iv) to ix), wherein R⁵ represents hydrogen, C₁₋₃-alkyl (especially methyl), cyclopropylmethyl, or 2-methoxyethyl; especially cyclopropylmethyl, or 2-methoxyethyl.
xiv) A further embodiment of the invention relates to compounds of Formula **I** according to any one of embodiments i) and iv) to ix), wherein R⁵ represents hydrogen, methyl, ethyl, or *n*-propyl.
xv) A further embodiment of the invention relates to compounds of Formula **I** according to any one of embodiments i) and iv) to ix), wherein R⁵ represents hydrogen.
xvi) A further embodiment of the invention relates to compounds of Formula **I** according to any one of embodiments i) and iv) to ix), wherein R⁵ represents phenyl-C₂₋₃-alkyl.
xvii) A further embodiment of the invention relates to compounds of Formula **I** according to any one of embodiments i) and iv) to xvi), wherein R⁶ represents C₁₋₉-alkylaminocarbonyl; C₁₋₉-alkylcarbonyl; C₁₋₉-alkoxycarbonyl; arylalkenylcarbonyl; arylaminocarbonyl; aryl-C₁₋₃-alkoxy-C₁₋₃-alkoxycarbonyl; aryl-C₁₋₃-alkoxycarbonyl; aryl-C₁₋₃-alkylaminocarbonyl; aryl-C₁₋₆-alkylcarbonyl; aryl-C₁₋₃-alkoxy-C₁₋₃-alkylcarbonyl; arylcarbonyl; arylcarbonyl-C₁₋₄-alkylcarbonyl; aryloxy-C₁₋₃-alkylcarbonyl; arylsulfonylaminocarbonyl; cycloalkyl-C₁₋₃-alkylcarbonyl; diaryl-C₁₋₃-alkylcarbonyl; heterocyclylcarbonyl; heteroaryl-C₁₋₃-alkylcarbonyl; heteroarylcarbonyl; aryl-C₃₋₆-cycloalkylcarbonyl; cycloalkylcarbonyl; or R⁷-C₁₋₄-alkylcarbonyl, wherein the bridging C₁₋₄-alkyl group may additionally be mono-substituted with aryl, and R⁷ represents arylaminocarbonyl, heteroarylaminocarbonyl, C₁₋₆-alkylaminocarbonyl, or aryl-C₁₋₃-alkylaminocarbonyl;
   wherein aryl is unsubstituted, mono- or di-substituted with a group independently selected from C₁₋₂-alkylendioxy, C₁₋₆-alkoxy, C₁₋₆-alkyl, C₁₋₆-alkylsulfonyl, halogen, hydroxy, halo-C₁₋₆-alkyl, halo-C₁₋₆-alkoxy, C₁₋₆-alkylthio, and C₁₋₄-alkoxycarbonylamino.
xviii) A further embodiment of the invention relates to compounds of Formula **I** according to any one of embodiments i) and iv) to xvi), wherein R⁶ represents aryl-C₁₋₃-alkoxycarbonyl; aryl-C₁₋₃-alkylaminocarbonyl; aryl-C₁₋₆-alkylcarbonyl; aryl-C₁₋₃-alkoxy-C₁₋₃-alkylcarbonyl; arylcarbonyl-C₁₋₄-alkylcarbonyl; aryloxy-C₁₋₃-alkylcarbonyl; cycloalkyl-C₁₋₃-alkylcarbonyl; diaryl-C₁₋₃-alkylcarbonyl; aryl-C₃₋₆-cycloalkylcarbonyl; or R⁷-C₁₋₄-alkylcarbonyl, wherein the bridging C₁₋₄-alkyl group may additionally be mono-substituted with aryl, and R⁷ represents arylaminocarbonyl, heteroarylaminocarbonyl,
   C₁₋₆-alkylaminocarbonyl, or aryl-C₁₋₃-alkylaminocarbonyl;
   wherein aryl is unsubstituted, mono- or di-substituted with a group independently selected from C₁₋₂-alkylendioxy, C₁₋₆-alkoxy, C₁₋₆-alkyl, C₁₋₆-alkylsulfonyl, halogen, hydroxy, halo-C₁₋₆-alkyl, halo-C₁₋₆-alkoxy, C₁₋₆-alkylthio, and C₁₋₄-alkoxycarbonylamino.
xix) A further embodiment of the invention relates to compounds of Formula **I** according to any one of embodiments i) and iv) to xvi), wherein R⁶ represents aryl-C₁₋₃-alkoxycarbonyl; aryl-C₁₋₃-alkylaminocarbonyl; aryl-C₁₋₆-alkylcarbonyl; aryloxy-C₁₋₃-alkylcarbonyl; diaryl-C₁₋₃-alkylcarbonyl; or R⁷-C₁₋₄-alkylcarbonyl, wherein the bridging C₁₋₄-alkyl group may additionally be mono-substituted with aryl, and R⁷ represents arylaminocarbonyl, or C₁₋₆-alkylaminocarbonyl;
   wherein aryl is unsubstituted, mono- or di-substituted with a group independently selected from C₁₋₂-alkylendioxy, C₁₋₆-alkoxy, C₁₋₆-alkyl, C₁₋₆-alkylsulfonyl, halogen, hydroxy, halo-C₁₋₆-alkyl, halo-C₁₋₆-alkoxy, C₁₋₆-alkylthio, and C₁₋₄-alkoxycarbonylamino.
xx) A further embodiment of the invention relates to compounds of Formula **I** according to any one of embodiments i) and iv) to xvi), wherein R⁶ represents aryl-C₁₋₂-alkoxycarbonyl; aryl-C₁₋₂alkylaminocarbonyl; aryl-C₁₋₄-alkylcarbonyl; aryloxy-C₁₋₂-alkylcarbonyl; or diaryl-C₂₋₃-alkylcarbonyl; or R⁷-C₂₋₄-alkylcarbonyl, wherein the bridging C₂₋₄-alkyl group may additionally be mono-substituted with aryl, and R⁷ represents arylaminocarbonyl, or C₁₋₄-alkylaminocarbonyl;
   wherein aryl is unsubstituted, mono- or di-substituted with a group independently selected from C₁₋₂-alkylendioxy, C₁₋₆-alkoxy, C₁₋₆-alkyl, C₁₋₆-alkylsulfonyl, halogen, hydroxy, trifluoromethyl, and trifluoromethoxy.
xxi) A further embodiment of the invention relates to compounds of Formula **I** according to any one of embodiments i) and iv) to xvi), wherein R⁶ represents aryl-C₁₋₂-alkylaminocarbonyl; wherein aryl is unsubstituted, mono- or di-substituted with a group independently selected from C₁₋₂-alkylendioxy, C₁₋₆-alkoxy, C₁₋₆-alkyl, C₁₋₆-alkylsulfonyl, halogen, hydroxy, trifluoromethyl, and trifluoromethoxy.
xxii) A further embodiment of the invention relates to compounds of Formula **I** according to any one of embodiments i) and iv) to xvi), wherein R⁶ represents aryl-C₁₋₂-alkoxycarbonyl; aryl-C₁₋₄-alkylcarbonyl; aryloxy-C₁₋₂-alkylcarbonyl; or diaryl-C₂₋₃-alkylcarbonyl;
   wherein aryl is unsubstituted, mono- or di-substituted with a group independently selected from C₁₋₂-alkylendioxy, C₁₋₆-alkoxy, C₁₋₆-alkyl, C₁₋₆-alkylsulfonyl, halogen, hydroxy, trifluoromethyl, and trifluoromethoxy.
xxiii) A further embodiment of the invention relates to compounds of Formula **I** according to any one of embodiments i) to xxii), wherein, in case R⁶ represents a group which contains aryl, the aryl group is phenyl which unsubstituted, or mono- or di-substituted with a group independently selected from C₁₋₄-alkoxy, C₁₋₄-alkyl, halogen, and trifluoromethyl.
xxiv) A further embodiment of the invention relates to compounds of Formula **I** according to any one of embodiments i) and iv) to xvi), wherein R⁶ represents C₁₋₄-alkylcarbonyl (especially acetyl); or aryl-C₂₋₄-alkylcarbonyl, wherein aryl is unsubstituted, mono- or di-substituted with a group independently selected from C₁₋₄-alkoxy, C₁₋₄-alkyl, halogen, and trifluoromethyl.
xxv) A further embodiment of the invention relates to compounds of Formula **I** according to any one of embodiments i) and iv) to xvi), wherein R⁶ represents aryl-C₂₋₄-alkylcarbonyl, wherein aryl is unsubstituted, mono- or di-substituted with a group independently selected from C₁₋₄-alkoxy, C₁₋₄-alkyl, halogen, and trifluoromethyl.
xxvi) A further embodiment of the invention relates to compounds of Formula **I** according to any one of embodiments i) to xxv), wherein, in case R⁶ represents a group which contains a carbonyl group and one or more aryl moieties, said group is such that it contains a bridging group between the carbonyl group and said aryl moiety (moieties) of said R⁶, wherein the carbonyl moiety and at least one of the aryl moieties are directly attached to different atoms of said bridging group.
xxvii) A further embodiment of the invention relates to compounds of Formula **I** according to any one of embodiments i) to xxv), wherein, in case R⁶ represents a group which contains a carbonyl group and exactly one aryl moiety, said group is such that it contains a bridging group between the carbonyl group and said aryl moiety of said R⁶, wherein the carbonyl moiety and the aryl moiety are directly attached to the same atom of said bridging group.
xxviii) A further embodiment of the invention relates to compounds of Formula **I** according to embodiment i), wherein
   R¹ represents hydrogen, or halogen;
   R² represents hydrogen, trifluoromethyl, or halogen;
   R³ represents hydrogen, or halogen;
   R⁴ represents hydrogen, halogen (especially chloro or bromo), or C₁₋₆-alkylsulfonyl (especially methanesulfonyl);
   R⁵ represents hydrogen, C₁₋₆-alkyl, cycloalkyl-C₁₋₄-alkyl, C₁₋₃-alkoxy-C₁₋₄-alkyl, aryl-C₁₋₄-alkyl, or aryloxy-C₁₋₄-alkyl;
   wherein aryl is unsubstituted, mono-substituted with a group selected from trifluoromethyl; and
   R⁶ represents C₁₋₉-alkylaminocarbonyl; C₁₋₉-alkylcarbonyl; C₁₋₉-alkoxycarbonyl; arylalkenylcarbonyl; arylaminocarbonyl; aryl-C₁₋₃-alkoxy-C₁₋₃-alkoxycarbonyl; aryl-C₁₋₃-alkoxycarbonyl; aryl-C₁₋₃-alkylaminocarbonyl; aryl-C₁₋₆-alkylcarbonyl; aryl-C₁₋₃-alkoxy-C₁₋₃-alkylcarbonyl; arylcarbonyl; arylcarbonyl-C₁₋₄-alkylcarbonyl; aryloxy-C₁₋₃-alkylcarbonyl; arylsulfonylaminocarbonyl; cycloalkyl-C₁₋₃-alkylcarbonyl; diaryl-C₁₋₃-alkylcarbonyl; heterocyclylcarbonyl; heteroaryl-C₁₋₃-alkylcarbonyl; heteroarylcarbonyl; aryl-C₃₋₆-cycloalkylcarbonyl; cycloalkylcarbonyl; or R⁷-C₁₋₄-alkylcarbonyl, wherein the bridging C₁₋₄-alkyl group may additionally be mono-substituted with aryl or disubstituted with hydroxy, and R⁷ represents arylaminocarbonyl, heteroarylaminocarbonyl, C₁₋₆-alkylaminocarbonyl, or aryl-C₁₋₃-alkylaminocarbonyl;
   wherein aryl is unsubstituted, mono- or di-substituted with a group independently selected from C₁₋₂-alkylendioxy; C₁₋₆-alkoxy; C₁₋₆-alkyl; C₁₋₆-alkylsufonyl; halogen; hydroxy; trifluoromethyl; trifluoromethoxy; C₁₋₆-alkylthio; and C₁₋₄-alkoxycarbonylamino.
xxix) A further embodiment of the invention relates to compounds of Formula **I** according to any one of embodiments i) to xxviii), wherein the position C(3) of the tetrahydrocarbazole ring of Formula **I** is (*S*)-configurated.
xxx) A further embodiment of the invention relates to compounds of Formula **I** according to any one of embodiments i) to xxviii), wherein the position C(3) of the tetrahydrocarbazole ring of Formula **I** is (*R*)-configurated.

In another embodiment preferred compounds of Formula **I** are selected from the group consisting of:
(3*S*)-[3-(3,3-diphenyl-propionylamino)-6-fluoro-1,2,3,4-tetrahydro-9*H*-carbazol-9-yl]-acetic acid;
(3*R*)- {3-[2-(3-chloro-phenoxy)-acetylamino]-6-fluoro-1,2,3,4-tetrahydro-9*H*-carbazol-9-yl}-acetic acid;
(3*S*)-[6-fluoro-3-(3-phenyl-propionylamino)-1,2,3,4-tetrahydro-9*H*-carbazol-9-yl]-acetic acid;
(3*R*)-{3-[2-(4-chloro-phenoxy)-acetylamino]-6-fluoro-1,2,3,4-tetrahydro-9*H*-carbazol-9-yl}-acetic acid;
(3*S*)-{3-[3-(2-chloro-phenyl)-propionylamino]-6-fluoro-1,2,3,4-tetrahydro-9*H*-carbazol-9-yl}-acetic acid;
(3*R*)-(3-isobutoxycarbonylamino-1,2,3,4-tetrahydro-9*H*-carbazol-9-yl)-acetic acid;
(3*R*)-[6-fluoro-3-(3-phenyl-propionylamino)-1,2,3,4-tetrahydro-9*H*-carbazol-9-yl]-acetic acid;
(3*S*)-{3-[3-(4-chloro-phenyl)-propionylamino]-6-fluoro-1,2,3,4-tetrahydro-9*H*-carbazol-9-yl}-acetic acid;
(3*R*)-(3-benzyloxycarbonylamino-6-fluoro-1,2,3,4-tetrahydro-9*H*-carbazol-9-yl)-acetic acid;
(3*S*)-(3-benzyloxycarbonylamino-6-fluoro-1,2,3,4-tetrahydro-9*H*-carbazol-9-yl)-acetic acid; and
(3*S*)-{3-[2-(4-chloro-phenoxy)-acetylamino]-6-fluoro-1,2,3,4-tetrahydro-9*H*-carbazol-9-yl}-acetic acid.

In another embodiment preferred compounds of Formula **I** are selected from the group consisting of:
(3*R*)-{3-[2-(2-chloro-phenoxy)-acetylamino]-6-fluoro-1,2,3,4-tetrahydro-9*H*-carbazol-9-yl}-acetic acid;
(3*S*)-{3-[3-(3-chloro-phenyl)-propionylamino]-6-fluoro-1,2,3,4-tetrahydro-9*H*-carbazol-9-yl}-acetic acid;
(3*S*)-[6-fluoro-3-(4-oxo-4-phenyl-butyrylamino)-1,2,3,4-tetrahydro-9*H*-carbazol-9-yl]-acetic acid;
(3*S*)-[6-fluoro-3-(2-indan-2-yl-acetylamino)-1,2,3,4-tetrahydro-9*H*-carbazol-9-yl]-acetic acid;
(3*S*)-{3-[(2,3-dihydro-1*H*-indole-2-carbonyl)-amino]-6-fluoro-1,2,3,4-tetrahydro-9*H-*carbazol-9-yl}-acetic acid;
(3*S*)-(3-{[2-(4-chloro-phenyl)-acetyl]-ethyl-amino}-6-fluoro-1,2,3,4-tetrahydro-9*H-*carbazol-9-yl)-acetic acid;
(3*R*)-(3-propoxycarbonylamino-1,2,3,4-tetrahydro-9*H*-carbazol-9-yl)-acetic acid;
(3*R*)-[6-fluoro-3-(2-*p*-tolyloxy-acetylamino)-1,2,3,4-tetrahydro-9*H*-carbazol-9-yl]-acetic acid;
(3*S*)-{6-fluoro-3-[methyl-(3-phenyl-propionyl)-amino]-1,2,3,4-tetrahydro-9*H*-carbazol-9-yl}-acetic acid;
(3S)-[6-fluoro-3-(3-1*H*-indol-3-yl-propionylamino)-1,2,3,4-tetrahydro-9*H*-carbazol-9-yl]-acetic acid;
(3*S*)-[3-(3-benzo[1,3]dioxol-5-yl-propionylamino)-6-fluoro-1,2,3,4-tetrahydro-9*H*-carbazol-9-yl]-acetic acid;
(3*S*)-{6-fluoro-3-[ethyl-(3-phenyl-propionyl)-amino]-1,2,3,4-tetrahydro-9*H*-carbazol-9-yl}-acetic acid;
(3*S*)-{3-[2-(4-chloro-phenyl)-acetylamino]-6-fluoro-1,2,3,4-tetrahydro-9*H*-carbazol-9-yl}-acetic acid;
(3*S*)-[3-(2,3-diphenyl-propionylamino)-6-fluoro-1,2,3,4-tetrahydro-9*H*-carbazol-9-yl]-acetic acid;
(3*R*)-[6-fluoro-3-(2-phenoxy-acetylamino)-1,2,3,4-tetrahydro-9*H*-carbazol-9-yl]-acetic acid;
(3*S*)-{3-[3-(3,4-difluoro-phenyl)-propionylamino]-6-fluoro-1,2,3,4-tetrahydro-9*H*-carbazol-9-yl}-acetic acid;
(3*S*)-[3-(3-phenyl-propionylamino)-1,2,3,4-tetrahydro-9*H*-carbazol-9-yl]-acetic acid;
(3*R*)-[3-(2-benzyloxy-acetylamino)-1,2,3,4-tetrahydro-9*H*-carbazol-9-yl]-acetic acid;
(3*S*)-{6-fluoro-3-[3-(2-methoxy-phenyl)-propionylamino]-1,2,3,4-tetrahydro-9*H*-carbazol-9-yl}-acetic acid;
(3*S*)-{6-fluoro-3-[propyl-(3-phenyl-propionyl)-amino]-1,2,3,4-tetrahydro-9*H*-carbazol-9-yl}-acetic acid;
(3*S*)-[3-(2-benzyloxy-acetylamino)-1,2,3,4-tetrahydro-9*H*-carbazol-9-yl]-acetic acid;
(3*R*)-(3-benzyloxycarbonylamino-1,2,3,4-tetrahydro-9*H*-carbazol-9-yl)-acetic acid;
(3*R*)-{6-fluoro-3-[2-(4-methoxy-phenyl)-acetylamino]-1,2,3,4-tetrahydro-9*H*-carbazol-9-yl}-acetic acid;
(3*R*)-{3-[3-(4-chloro-phenyl)-propionylamino]-6-fluoro-1,2,3,4-tetrahydro-9*H*-carbazol-9-yl}-acetic acid;
(3*S*)-{3-[4-(4-bromo-phenyl)-4-oxo-butyrylamino]-6-fluoro-1,2,3,4-tetrahydro-9*H-*carbazol-9-yl}-acetic acid;
(3*S*)-(3-{[2-(4-chloro-phenyl)-acetyl]-propyl-amino}-6-fluoro-1,2,3,4-tetrahydro-9*H-*carbazol-9-yl)-acetic acid;
(3*R*)-(3-phenylacetylamino-1,2,3,4-tetrahydro-9*H*-carbazol-9-yl)-acetic acid;
(3*R*)-{3-[3-(2-chloro-phenyl)-propionylamino]-6-fluoro-1,2,3,4-tetrahydro-9*H*-carbazol-9-yl}-acetic acid;
(3*S*)-{6-fluoro-3-[2-(4-trifluoromethyl-phenoxy)-acetylamino]-1,2,3,4-tetrahydro-9*H-*carbazol-9-yl}-acetic acid;
(3*R*)-(6-fluoro-3-phenylacetylamino-1,2,3,4-tetrahydro-9*H*-carbazol-9-yl)-acetic acid;
(3*S*)-{-fluoro-3-[3-(2-hydroxy-phenyl)-propionylamino]-1,2,3,4-tetrahydro-9*H*-carbazol-9-yl}-acetic acid;
(3*S*)-[3-(3-1*H*-benzoimidazol-2-yl-propionylamino)-6-fluoro-1,2,3,4-tetrahydro-9*H-*carbazol-9-yl]-acetic acid;
(3*S*)-{6-fluoro-3-[3-(4-hydroxy-phenyl)-propionylamino]-1,2,3,4-tetrahydro-9*H*-carbazol-9-yl}-acetic acid;
(3*S*)-[6-fluoro-3-(2-p-tolyloxy-acetylamino)-1,2,3,4-tetrahydro-9*H*-carbazol-9-yl]-acetic acid;
(3*R*)-[6-fluoro-3-(2-p-tolyl-acetylamino)-1,2,3,4-tetrahydro-9*H*-carbazol-9-yl]-acetic acid;
(3*R*)-{3-[3-(3-chloro-phenyl)-propionylamino]-6-fluoro-1,2,3,4-tetrahydro-9*H*-carbazol-9-yl}-acetic acid;
(3*R*)-[3-(2-phenoxy-acetylamino)-1,2,3,4-tetrahydro-9*H*-carbazol-9-yl]-acetic acid;
(3*S*)-[6-fluoro-3-(3-p-tolyl-propionylamino)-1,2,3,4-tetrahydro-9*H*-carbazol-9-yl]-acetic acid;
(3*S*)-(3-benzyloxycarbonylamino-1,2,3,4-tetrahydro-9*H*-carbazol-9-yl)-acetic acid;
(3*S*)-[6-fluoro-3-(2-p-tolyl-acetylamino)-1,2,3,4-tetrahydro-9*H*-carbazol-9-yl]-acetic acid;
(3*S*)-[3-(3-phenethyl-ureido)-1,2,3,4-tetrahydro-9*H*-carbazol-9-yl]-acetic acid;
(3*S*)-{6-fluoro-3-[3-(3-hydroxy-phenyl)-propionylamino]-1,2,3,4-tetrahydro-9*H*-carbazol-9-yl}-acetic acid;
(3*R*)-[3-(2-benzyloxy-ethoxycarbonylamino)-1,2,3,4-tetrahydro-9*H*-carbazol-9-yl]-acetic acid;
(3*S*)-[6-fluoro-3-(3-naphthalen-2-yl-propionylamino)-1,2,3,4-tetrahydro-9*H*-carbazol-9-yl]-acetic acid;
(3*S*)-{6-fluoro-3-[4-(4-methanesulfonyl-phenyl)-4-oxo-butyrylamino]-1,2,3,4-tetrahydro-9*H*-carbazol-9-yl}-acetic acid;
(3*S*)-(3-{[2-(4-chloro-phenyl)-acetyl]-methyl-amino}-6-fluoro-1,2,3,4-tetrahydro-9*H-*carbazol-9-yl)-acetic acid;
(3*S*)-[3-(3-phenylsulfonyl-ureido)-1,2,3,4-tetrahydro-9*H*-carbazol-9-yl]-acetic acid;
(3*S*)-{6-fluoro-3-[3-(4-methoxy-phenyl)-propionylamino]-1,2,3,4-tetrahydro-9*H*-carbazol-9-yl}-acetic acid;
(3*R*)-[3-(3-phenyl-propionylamino)-1,2,3,4-tetrahydro-9*H*-carbazol-9-yl]-acetic acid;
(3*R*)-{3-[2-(4-chloro-phenyl)-acetylamino]-6-fluoro-1,2,3,4-tetrahydro-9*H*-carbazol-9-yl}-acetic acid;
(3*R*)-[6-fluoro-3-(3-p-tolyl-propionylamino)-1,2,3,4-tetrahydro-9*H*-carbazol-9-yl]-acetic acid;
(3*R*)-{6-fluoro-3-[3-(4-methoxy-phenyl)-propionylamino]-1,2,3,4-tetrahydro-9*H*-carbazol-9-yl}-acetic acid;
(3*S*)-{6-fluoro-3-[3-(4-hydroxy-3-methoxy-phenyl)-propionylamino]-1,2,3,4-tetrahydro-9*H*-carbazol-9-yl}-acetic acid;
(3*S*)-{6-fluoro-3-[2-(3-trifluoromethyl-phenyl)-acetylamino]-1,2,3,4-tetrahydro-9*H-*carbazol-9-yl}-acetic acid;
(3*S*)-{6-fluoro-3-[2-(4-methoxy-phenyl)-acetylamino]-1,2,3,4-tetrahydro-9*H*-carbazol-9-yl}-acetic acid;
(3*R*)-{3-[2-(3-chloro-phenyl)-acetylamino]-6-fluoro-1,2,3,4-tetrahydro-9*H*-carbazol-9-yl}-acetic acid;
(3*S*)-{6-fluoro-3-[2-(4-trifluoromethyl-phenyl)-acetylamino]-1,2,3,4-tetrahydro-9*H-*carbazol-9-yl}-acetic acid;
(3*R*)-(3-*tert*-butoxycarbonylamino-1,2,3,4-tetrahydro-9*H*-carbazol-9-yl)-acetic acid;
(3*R*)-{3-[2-(3,4-dichloro-phenyl)-acetylamino]-6-fluoro-1,2,3,4-tetrahydro-9*H*-carbazol-9-yl}-acetic acid;
(3*S*)-(3-isobutoxycarbonylamino-1,2,3,4-tetrahydro-9*H*-carbazol-9-yl)-acetic acid;
(3*S*)-{6-fluoro-3-[3-(3-methoxy-phenyl)-propionylamino]-1,2,3,4-tetrahydro-9*H*-carbazol-9-yl}-acetic acid; and
(3*S*)-[6-fluoro-3-(2-phenoxy-acetylamino)-1,2,3,4-tetrahydro-9*H*-carbazol-9-yl]-acetic acid.

In another embodiment preferred compounds of Formula **I** are selected from the group consisting of:
(3*R*)-[3-(3-phenethyl-ureido)-1,2,3,4-tetrahydro-9*H*-carbazol-9-yl]-acetic acid;
(3*S*)-[3-(2-benzyloxy-ethoxycarbonylamino)-1,2,3,4-tetrahydro-9*H*-carbazol-9-yl]-acetic acid;
(3*S*)-[3-(2-phenoxy-acetylamino)-1,2,3,4-tetrahydro-9*H*-carbazol-9-yl]-acetic acid;
(3*S*)-(3-propoxycarbonylamino-1,2,3,4-tetrahydro-9*H*-carbazol-9-yl)-acetic acid;
(3*S*)-[3-(2-thiophen-2-yl-acetylamino)-1,2,3,4-tetrahydro-9*H*-carbazol-9-yl]-acetic acid;
(3*S*)-(3-phenylacetylamino-1,2,3,4-tetrahydro-9*H*-carbazol-9-yl)-acetic acid;
(3*R*)-[3-(3-phenylsulfonyl-ureido)-1,2,3,4-tetrahydro-9*H*-carbazol-9-yl]-acetic acid;
(3*S*)-[3-(3-benzyl-ureido)-1,2,3,4-tetrahydro-9*H*-carbazol-9-yl]-acetic acid;
(3*R*)-[3-(3-naphthalen-1-yl-ureido)-1,2,3,4-tetrahydro-9*H*-carbazol-9-yl]-acetic acid;
(3*S*)-(3-decanoylamino-1,2,3,4-tetrahydro-9*H*-carbazol-9-yl)-acetic acid;
(3*S*)-[6-fluoro-3-(3-naphthalen-2-yl-acryloylamino)-1,2,3,4-tetrahydro-9*H*-carbazol-9-yl]-acetic acid;
(3*S*)-{3-[2-(4-tert-butyl-phenyl)-acetylamino]-6-fluoro-1,2,3,4-tetrahydro-9*H*-carbazol-9-yl}-acetic acid;
(3R)-(3-benzyloxycarbonylamino-8-chloro-6-fluoro-1,2,3,4-tetrahydro-9*H*-carbazol-9-yl)-acetic acid;
(3*S*)-(3-benzyloxycarbonylamino-8-chloro-6-fluoro-1,2,3,4-tetrahydro-9*H*-carbazol-9-yl)-acetic acid;
(3*S*)-[6-fluoro-3-(3-pyridin-3-yl-propionylamino)-1,2,3,4-tetrahydro-9*H*-carbazol-9-yl]-acetic acid;
(3*R*)-[3-(3-benzyl-ureido)-1,2,3,4-tetrahydro-9*H*-carbazol-9-yl]-acetic acid;
(3*S*)-[3-(3-methyl-butyrylamino)-1,2,3,4-tetrahydro-9*H*-carbazol-9-yl]-acetic acid;
(3*R*)-{6-fluoro-3-[2-(4-trifluoromethyl-phenyl)-acetylamino]-1,2,3,4-tetrahydro-9*H-*carbazol-9-yl}-acetic acid;
(3*S*)-[3-(3-phenyl-ureido)-1,2,3,4-tetrahydro-9*H*-carbazol-9-yl]-acetic acid;
(3*S*)-[3-(3-cyclopentyl-propionylamino)-1,2,3,4-tetrahydro-9*H*-carbazol-9-yl]-acetic acid;
(3*R*)-[3-(2-thiophen-2-yl-acetylamino)-1,2,3,4-tetrahydro-9*H*-carbazol-9-yl]-acetic acid;
(3*S*)-(3-butyrylamino-1,2,3,4-tetrahydro-9*H*-carbazol-9-yl)-acetic acid;
(3*R*)-(3-benzyloxycarbonylamino-8-chloro-5-fluoro-1,2,3,4-tetrahydro-9*H*-carbazol-9-yl)-acetic acid;
(3*S*)-(3-benzyloxycarbonylamino-8-chloro-5-fluoro-1,2,3,4-tetrahydro-9*H*-carbazol-9-yl)-acetic acid;
(3*S*)-(3-heptanoylamino-1,2,3,4-tetrahydro-9*H*-carbazol-9-yl)-acetic acid;
(3*R*)-[3-(3-cyclopentyl-propionylamino)-1,2,3,4-tetrahydro-9H-carbazol-9-yl]-acetic acid;
(3*R*)-(3-decanoylamino-1,2,3,4-tetrahydro-9*H*-carbazol-9-yl)-acetic acid;
(3*R*)-(3-benzoylamino-1,2,3,4-tetrahydro-9*H*-carbazol-9-yl)-acetic acid;
(3*R*)-[3-(3-butyl-ureido)-1,2,3,4-tetrahydro-9*H*-carbazol-9-yl]-acetic acid;
(3*S*)-{6-fluoro-3-[(1H-indole-2-carbonyl)-amino]-1,2,3,4-tetrahydro-9*H*-carbazol-9-yl}-acetic acid;
(3*R*)-[3-(3-methyl-butyrylamino)-1,2,3,4-tetrahydro-9*H*-carbazol-9-yl]-acetic acid; and
(3*S*)-[3-(3-butyl-ureido)-1,2,3,4-tetrahydro-9*H*-carbazol-9-yl]-acetic acid.

In another embodiment preferred compounds of Formula **I** are selected from the group consisting of:
(3*R*)-[3-(3-Benzyl-ureido)-6-fluoro-1,2,3,4-tetrahydro-9*H*-carbazol-9-yl]-acetic acid;
(3*S*)-[3-(3-Benzyl-ureido)-6-fluoro-1,2,3,4-tetrahydro-9*H*-carbazol-9-yl]-acetic acid;
(3*S*)-[6-Fluoro-3-(3-phenethyl-ureido)-1,2,3,4-tetrahydro-9*H*-carbazol-9-yl]-acetic acid;
(3*R*)-[3-(3-Benzyl-ureido)-8-chloro-6-fluoro-1,2,3,4-tetrahydro-9*H*-carbazol-9-yl]-acetic acid;
(3*S*)-[3-(3-Benzyl-ureido)-8-chloro-6-fluoro-1,2,3,4-tetrahydro-9*H*-carbazol-9-yl]-acetic acid;
(3*R*)-[8-Chloro-6-fluoro-3-(3-phenethyl-ureido)-1,2,3,4-tetrahydro-9*H*-carbazol-9-yl]-acetic acid;
(3*S*)-[8-Chloro-6-fluoro-3-(3-phenethyl-ureido)-1,2,3,4-tetrahydro-9*H*-carbazol-9-yl]-acetic acid;
(3*R*)-(3-Benzyloxycarbonylamino-6-trifluoromethyl-1,2,3,4-tetrahydro-9*H*-carbazol-9-yl)-acetic acid;
(3*S*)-(3-Benzyloxycarbonylamino-6-trifluoromethyl-1,2,3,4-tetrahydro-9*H*-carbazol-9-yl)-acetic acid;
(3*R*)-(3-Benzyloxycarbonylamino-8-bromo-6-fluoro-1,2,3,4-tetrahydro-9*H*-carbazol-9-yl)-acetic acid;
(3*S*)-(3-Benzyloxycarbonylamino-8-bromo-6-fluoro-1,2,3,4-tetrahydro-9*H*-carbazol-9-yl)-acetic acid;
(3*R*)-(3-Benzyloxycarbonylamino-6-fluoro-8-vinyl-1,2,3,4-tetrahydro-9*H*-carbazol-9-yl)-acetic acid;
(3*S*)-(3-Benzyloxycarbonylamino-6-fluoro-8-vinyl-1,2,3,4-tetrahydro-9*H*-carbazol-9-yl)-acetic acid;
(3*R*)-(3-Benzyloxycarbonylamino-6-fluoro-8-methanesulfonyl-1,2,3,4-tetrahydro-9*H-*carbazol-9-yl)-acetic acid;
(3*S*)-(3-Benzyloxycarbonylamino-6-fluoro-8-methanesulfonyl-1,2,3,4-tetrahydro-9*H-*carbazol-9-yl)-acetic acid;
(3*S*)-(3-Benzyloxycarbonylamino-6-fluoro-8-methyl-1,2,3,4-tetrahydro-9*H*-carbazol-9-yl)-acetic acid;
(3*S*)-(3-Benzyloxycarbonylamino-7-chloro-6-fluoro-1,2,3,4-tetrahydro-9*H*-carbazol-9-yl)-acetic acid;
(3*S*)-(8-Allyl-3-benzyloxycarbonylamino-6-fluoro-1,2,3,4-tetrahydro-9*H*-carbazol-9-yl)-acetic acid;
(3*R*)-(3-Benzyloxycarbonylamino-8-chloro-1,2,3,4-tetrahydro-9*H*-carbazol-9-yl)-acetic acid;
(3*S*)-{3-[3-(2,4-Dimethoxy-phenyl)-propionylamino]-6-fluoro-1,2,3,4-tetrahydro-9*H-*carbazol-9-yl}-acetic acid;
(3*S*)-[6-Fluoro-3-(3-naphthalen-1-yl-propionylamino)-1,2,3,4-tetrahydro-9*H*-carbazol-9-yl]-acetic acid;
(3*R*)-{6-Fluoro-3-[2-(2-methoxy-phenoxy)-acetylamino]-1,2,3,4-tetrahydro-9*H*-carbazol-9-yl}-acetic acid;
(3*S*)-{6-Fluoro-3-[2-(2-methoxy-phenoxy)-acetylamino]-1,2,3,4-tetrahydro-9*H*-carbazol-9-yl}-acetic acid;
(3*R*)-{6-Fluoro-3-[3-(2-methylphenyl)-propionylamino]-1,2,3,4-tetrahydro-9*H*-carbazol-9-yl}-acetic acid;
(3*S*)-{6-Fluoro-3-[3-(2-methylphenyl)-propionylamino]-1,2,3,4-tetrahydro-9*H*-carbazol-9-yl}-acetic acid;
(3*R*)-{6-Fluoro-3-[3-(3-methylphenyl)-propionylamino]-1,2,3,4-tetrahydro-9*H*-carbazol-9-yl}-acetic acid;
(3*S*)-{6-Fluoro-3-[3-(3-methylphenyl)-propionylamino]-1,2,3,4-tetrahydro-9*H*-carbazol-9-yl}-acetic acid;
(3*R*)- {6-Fluoro-3-[3-(3-methoxy-phenyl)-propionylamino]-1,2,3,4-tetrahydro-9*H*-carbazol-9-yl}-acetic acid;
(3*S*)-{6-Fluoro-3-[3-(3-methoxy-phenyl)-propionylamino]-1,2,3,4-tetrahydro-9*H*-carbazol-9-yl}-acetic acid;
(3*R*)-{6-Fluoro-3-[2-(3-methoxy-phenoxy)-acetylamino]-1,2,3,4-tetrahydro-9*H*-carbazol-9-yl}-acetic acid;
(3*S*)-{6-Fluoro-3-[2-(3-methoxy-phenoxy)-acetylamino]-1,2,3,4-tetrahydro-9*H*-carbazol-9-yl}-acetic acid;
(3*R*)-{6-Fluoro-3-[2-(2-methylphenoxy)-acetylamino]-1,2,3,4-tetrahydro-9*H*-carbazol-9-yl}-acetic acid;
(3*S*)-{6-Fluoro-3-[2-(2-methylphenoxy)-acetylamino]-1,2,3,4-tetrahydro-9*H*-carbazol-9-yl}-acetic acid;
(3*S*)-{3-[3-(2,5-Dimethoxy-phenyl)-propionylamino]-6-fluoro-1,2,3,4-tetrahydro-9*H-*carbazol-9-yl}-acetic acid;
(3*S*)-{6-Fluoro-3-[3-(4-trifluoromethyl-phenyl)-propionylamino]-1,2,3,4-tetrahydro-9*H-*carbazol-9-yl}-acetic acid;
(3*S*)-{3-[3-(2,6-Dichloro-phenyl)-propionylamino]-6-fluoro-1,2,3,4-tetrahydro-9*H-*carbazol-9-yl}-acetic acid;
(3*S*)-{3-[3-(2,5-Bis-trifluoromethyl-phenyl)-propionylamino]-6-fluoro-1,2,3,4-tetrahydro-9*H*-carbazol-9-yl}-acetic acid;
(3*S*)-{6-Fluoro-3-[3-(4-methylsulfanyl-phenyl)-propionylamino]-1,2,3,4-tetrahydro-9*H-*carbazol-9-yl}-acetic acid;
(3*S*)-{6-Fluoro-3-[3-(4-iodo-phenyl)-propionylamino]-1,2,3,4-tetrahydro-9*H*-carbazol-9-yl}-acetic acid;
(3*S*)-{6-Fluoro-3-[3-(4-isopropyl-phenyl)-propionylamino]-1,2,3,4-tetrahydro-9*H*-carbazol-9-yl}-acetic acid;
(3*S*)-{6-Fluoro-3-[3-(3-trifluoromethyl-phenyl)-propionylamino]-1,2,3,4-tetrahydro-9*H-*carbazol-9-yl}-acetic acid;
(3*S*)-{3-[3-(2,4-Dichloro-phenyl)-propionylamino]-6-fluoro-1,2,3,4-tetrahydro-9*H-*carbazol-9-yl}-acetic acid;
(3*S*)-{6-Fluoro-3-[3-(4-fluoro-phenyl)-propionylamino]-1,2,3,4-tetrahydro-9*H*-carbazol-9-yl}-acetic acid;
(3*S*)-{3-[3-(3,5-Bis-trifluoromethyl-phenyl)-propionylamino]-6-fluoro-1,2,3,4-tetrahydro-9*H*-carbazol-9-yl }-acetic acid;
(3*S*)-{3-[3-(4-Ethyl-phenyl)-propionylamino]-6-fluoro-1,2,3,4-tetrahydro-9*H*-carbazol-9-yl}-acetic acid;
(3*S*)-{6-Fluoro-3-[3-(3-iodo-phenyl)-propionylamino]-1,2,3,4-tetrahydro-9*H*-carbazol-9-yl}-acetic acid;
(3*S*)-{6-Fluoro-3-[3-(4-methanesulfonyl-phenyl)-propionylamino]-1,2,3,4-tetrahydro-9*H-*carbazol-9-yl}-acetic acid;
(3*S*)-{3-[3-(2,3-Dimethoxy-phenyl)-propionylamino]-6-fluoro-1,2,3,4-tetrahydro-9*H-*carbazol-9-yl}-acetic acid;
(3*S*)-{3-[3-(2-Bromo-phenyl)-propionylamino]-6-fluoro-1,2,3,4-tetrahydro-9*H*-carbazol-9-yl}-acetic acid;
(3*S*)-{6-Fluoro-3-[3-(3-trifluoromethoxy-phenyl)-propionylamino]-1,2,3,4-tetrahydro-9*H-*carbazol-9-yl}-acetic acid;
(3*S*)-{3-[3-(2,4-Dimethyl-phenyl)-propionylamino]-6-fluoro-1,2,3,4-tetrahydro-9*H-*carbazol-9-yl}-acetic acid;
(3*S*)-{3-[3-(3-Bromo-phenyl)-propionylamino]-6-fluoro-1,2,3,4-tetrahydro-9*H*-carbazol-9-yl}-acetic acid;
(3*S*)-{3-[3-(3-*tert*-Butoxycarbonylamino-phenyl)-propionylamino]-6-fluoro-1,2,3,4-tetrahydro-9*H*-carbazol-9-yl}-acetic acid;
(3*S*)-{6-Fluoro-3-[(S)-3-(4-fluoro-phenyl)-2-phenyl-propionylamino]-1,2,3,4-tetrahydro-9*H*-carbazol-9-yl}-acetic acid;
(3*S*)-{6-Fluoro-3-[(*S*)-3-(4-methoxy-phenyl)-2-phenyl-propionylamino]-1,2,3,4-tetrahydro-9*H*-carbazol-9-yl}-acetic acid;
(3*S*)-{6-Fluoro-3-[3-(2-fluoro-phenyl)-propionylamino]-1,2,3,4-tetrahydro-9*H*-carbazol-9-yl}-acetic acid;
(3*S*)-(6-Fluoro-3-{[(2*RS*)-1,2,3,4-tetrahydro-naphthalene-2-carbonyl]-amino}-1,2,3,4-tetrahydro-9*H*-carbazol-9-yl)-acetic acid;
(3*S*)-(6-Fluoro-3-{[(2*RS*)-8-methoxy-1,2,3,4-tetrahydro-naphthalene-2-carbonyl]-amino}-1,2,3,4-tetrahydro-9*H*-carbazol-9-yl)-acetic acid;
(3*S*)-(6-Fluoro-3-{(2*RS*)-2-[(4-fluoro-phenylcarbamoyl)-methyl]-3-phenyl-propionylamino}-1,2,3,4-tetrahydro-9*H*-carbazol-9-yl)-acetic acid;
(3*S*)-{3-[(2*RS*)-2-Benzyl-3,3-dimethyl-butyrylamino]-6-fluoro-1,2,3,4-tetrahydro-9*H-*carbazol-9-yl}-acetic acid;
(3*S*)-(6-Fluoro-3-{[(2*RS*)-8-methoxy-1,2,3,4-tetrahydro-naphthalene-2-carbonyl]-amino}-1,2,3,4-tetrahydro-9*H*-carbazol-9-yl)-acetic acid;
(3*S*)-{6-Fluoro-3-[3-(3-fluoro-phenyl)-propionylamino]-1,2,3,4-tetrahydro-9*H*-carbazol-9-yl}-acetic acid;
(3*S*)-(6-Fluoro-3-{[(2*RS*)-8-methoxy-1,2,3,4-tetrahydro-naphthalene-2-carbonyl]-amino}-1,2,3,4-tetrahydro-9*H*-carbazol-9-yl)-acetic acid;
(3*S*)-{6-Fluoro-3-[(2*R*)-2-methyl-3-phenyl-propionylamino]-1,2,3,4-tetrahydro-9*H-*carbazol-9-yl}-acetic acid;
(3*S*)-[3-(2,2-Dimethyl-3-phenyl-propionylamino)-6-fluoro-1,2,3,4-tetrahydro-9*H*-carbazol-9-yl]-acetic acid;
(3*S*)-[6-Fluoro-3-(3-methyl-3-phenyl-butyrylamino)-1,2,3,4-tetrahydro-9*H*-carbazol-9-yl]-acetic acid;
(3*S*)-{6-Fluoro-3-[(3*S*)-3-phenyl-butyrylamino]-1,2,3,4-tetrahydro-9*H*-carbazol-9-yl}-acetic acid;
(3*S*)-[3-(2-Benzyloxy-acetylamino)-6-fluoro-1,2,3,4-tetrahydro-9*H*-carbazol-9-yl]-acetic acid:
(3*S*)-[6-Fluoro-3-(4-phenyl-butyrylamino)-1,2,3,4-tetrahydro-9*H*-carbazol-9-yl]-acetic acid;
(3*S*)-{3-[(2*R*,3*R*)-2,3-Dihydroxy-3-(2-methoxy-phenylcarbamoyl)-propionylamino]-6-fluoro-1,2,3,4-tetrahydro-9*H*-carbazol-9-yl}-acetic acid;
(3*R*)-{8-Chloro-6-fluoro-3-[3-(2-methylphenyl)-propionylamino]-1,2,3,4-tetrahydro-9*H-*carbazol-9-yl}-acetic acid;
(3*S*)-{8-Chloro-6-fluoro-3-[3-(2-methylphenyl)-propionylamino]-1,2,3,4-tetrahydro-9*H-*carbazol-9-yl}-acetic acid;
(3*R*)-{8-Chloro-6-fluoro-3-[2-(2-methoxy-phenoxy)-acetylamino]-1,2,3,4-tetrahydro-9*H-*carbazol-9-yl}-acetic acid;
(3*S*)-{8-Clhloro-6-fluoro-3-[2-(2-methoxy-phenoxy)-acetylamino]-1,2,3,4-tetrahydro-9*H-*carbazol-9-yl}-acetic acid;
(3*R*)-{8-Chloro-6-fluoro-3-[3-(3-hydroxy-phenyl)-propionylamino]-1,2,3,4-tetrahydro-9*H-*carbazol-9-yl}acetic acid;
(3*S*)-{8-Chloro-6-fluoro-3-[3-(3-hydroxy-phenyl)-propionylamino]-1,2,3,4-tetrahydro-9*H-*carbazol-9-yl}-acetic acid;
(3*R*)-{8-Chloro-6-fluoro-3-[3-(3-methoxy-phenyl)-propionylamino]-1,2,3,4-tetrahydro-9*H-*carbazol-9-yl}-acetic acid;
(3*S*)-{8-Chloro-6-fluoro-3-[3-(3-methoxy-phenyl)-propionylamino]-1,2,3,4-tetrahydro-9*H-*carbazol-9-yl}-acetic acid;
(3*R*)- {8-Chloro-6-fluoro-3-[3-(3-methylphenyl)-propionylamino]-1,2,3,4-tetrahydro-9*H-*carbazol-9-yl}-acetic acid;
(3S*)*-{8-Chloro-6-fluoro-3-[3-(3-methylphenyl)-propionylamino]-1,2,3,4-tetrahydro-9*H-*carbazol-9-yl}-acetic acid;
(3*R*)-{8-Chloro-6-fluoro-3-[3-(2-hydroxy-phenyl)-propionylamino]-1,2,3,4-tetrahydro-9*H-*carbazol-9-yl}-acetic acid;
(3*S*)-{8-Chloro-6-fluoro-3-[3-(2-hydroxy-phenyl)-propionylamino]-1,2,3,4-tetrahydro-9*H-*carbazol-9-yl}-acetic acid;
(3*R*)-[8-Chloro-6-fluoro-3-(3-phenyl-propionylamino)-1,2,3,4-tetrahydro-9*H*-carbazol-9-yl]-acetic acid;
(3*S*)-[8-Chloro-6-fluoro-3-(3-phenyl-propionylamino)-1,2,3,4-tetrahydro-9*H*-carbazol-9-yl]-acetic acid;
(3*R*)-{8-Chloro-6-fluoro-3-[3-(2-methoxy-phenyl)-propionylamino]-1,2,3,4-tetrahydro-9*H-*carbazol-9-yl}-acetic acid;
(3*S*)-{8-Chloro-6-fluoro-3-[3-(2-methoxy-phenyl)-propionylamino]-1,2,3,4-tetrahydro-9*H-*carbazol-9-yl}-acetic acid;
(3*R*)-{8-Chloro-3-[3-(3-chloro-phenyl)-propionylamino]-6-fluoro-1,2,3,4-tetrahydro-9*H-*carbazol-9-yl}-acetic acid;
(3*S*)-{8-Chloro-3-[3-(3-chloro-phenyl)-propionylamino]-6-fluoro-1,2,3,4-tetrahydro-9*H-*carbazol-9-yl}-acetic acid;
(3*R*)-[8-Chloro-6-fluoro-3-(3-1*H*-indol-3-yl-propionylamino)-1,2,3,4-tetrahydro-9*H-*carbazol-9-yl]-acetic acid;
(3*S*)-[8-Chloro-6-fluoro-3-(3-1*H*-indol-3-yl-propionylamino)-1,2,3,4-tetrahydro-9*H-*carbazol-9-yl]-acetic acid;
(3*R*)-{8-Chloro-3-[2-(2-chloro-phenoxy)-acetylamino]-6-fluoro-1,2,3,4-tetrahydro-9*H-*carbazol-9-yl}-acetic acid;
(3*S*)-{8-Chloro-3-[2-(2-chloro-phenoxy)-acetylamino]-6-fluoro-1,2,3,4-tetrahydro-9*H-*carbazol-9-yl}-acetic acid;
(3*R*)-{8-Chloro-6-fluoro-3-[2-(2-methylphenyl)-oxy-acetylamino]-1,2,3,4-tetrahydro-9*H-*carbazol-9-yl}-acetic acid;
(3*S*)-{8-Chloro-6-fluoro-3-[2-(2-methylphenyl)-oxy-acetylamino]-1,2,3,4-tetrahydro-9*H-*carbazol-9-yl}-acetic acid;
(3*R*)-[3-(3-Benzo[1,3]dioxol-5-yl-propionylamino)-8-chloro-6-fluoro-1,2,3,4-tetrahydro-9*H*-carbazol-9-yl]-acetic acid;
(3*S*)-[3-(3-Benzo[1,3]dioxol-5-yl-propionylamino)-8-chloro-6-fluoro-1,2,3,4-tetrahydro-9*H*-carbazol-9-yl]-acetic acid;
(3*R*)-{8-Chloro-6-fluoro-3-[2-(3-methoxy-phenoxy)-acetylamino]-1,2,3,4-tetrahydro-9*H-*carbazol-9-yl}-acetic acid;
(3*S*)-{8-Chloro-6-fluoro-3-[2-(3-methoxy-phenoxy)-acetylamino]-1,2,3,4-tetrahydro-9*H-*carbazol-9-yl}-acetic acid;
(3*R*)-{8-Chloro-3-[2-(3-chloro-phenoxy)-acetylamino]-6-fluoro-1,2,3,4-tetrahydro-9*H-*carbazol-9-yl}-acetic acid;
(3*S*)-{8-Chloro-3-[2-(3-chloro-phenoxy)-acetylamino]-6-fluoro-1,2,3,4-tetrahydro-9*H-*carbazol-9-yl}-acetic acid;
(3*R*)-{8-Chloro-3-[3-(2-chloro-phenyl)-propionylamino]-6-fluoro-1,2,3,4-tetrahydro-9*H-*carbazol-9-yl}-acetic acid;
(3*S*)-{8-Chloro-3-[3-(2-chloro-phenyl)-propionylamino]-6-fluoro-1,2,3,4-tetrahydro-9*H-*carbazol-9-yl}-acetic acid;
(3*R*)-[8-Chloro-6-fluoro-3-(2-indan-2-yl-acetylamino)-1,2,3,4-tetrahydro-9H-carbazol-9-yl]-acetic acid;
(3*S*)-[8-Chloro-6-fluoro-3-(2-indan-2-yl-acetylamino)-1,2,3,4-tetrahydro-9*H*-carbazol-9-yl]-acetic acid;
(3*S*)-[6-Fluoro-3-(1-methyl-3-phenethyl-ureido)-1,2,3,4-tetrahydro-9*H*-carbazol-9-yl]-acetic acid;
(3*S*)-{3-[3-(2-Chloro-benzyl)-1-methyl-ureido]-6-fluoro-1,2,3,4-tetrahydro-9*H*-carbazol-9-yl}-acetic acid;
(3*S*)-[3-(3-Benzyl-1-methyl-ureido)-6-fluoro-1,2,3,4-tetrahydro-9*H*-carbazol-9-yl]-acetic acid;
(3*S*)-[3-(Benzyloxycarbonyl-methyl-amino)-6-fluoro-1,2,3,4-tetrahydro-9*H*-carbazol-9-yl]-acetic acid;
{(3*S*)-3-[(2-Chloro-benzyloxycarbonyl)-methyl-amino]-6-fluoro-1,2,3,4-tetrahydro-9*H-*carbazol-9-yl}-acetic acid;
(3*S*)-(6-Fluoro-3-{[2-(4-methoxy-phenyl)-acetyl]-methyl-amino}-1,2,3,4-tetrahydro-9*H-*carbazol-9-yl)-acetic acid;
(3*S*)-(6-Fluoro-3-{methyl-[2-(4-methylphenyl)-acetyl]-amino}-1,2,3,4-tetrahydro-9*H-*carbazol-9-yl)-acetic acid;
(3*S*)-(6-Fluoro-3-{[2-(2-methoxy-phenyl)-acetyl]-methyl-amino}-1,2,3,4-tetrahydro-9*H-*carbazol-9-yl)-acetic acid;
(3*S*)-{6-Fluoro-3-[(2-indan-2-yl-acetyl)-methyl-amino]-1,2,3,4-tetrahydro-9*H*-carbazol-9-yl}-acetic acid;
(3*S*)-(3-{[2-(3-Chloro-phenyl)-acetyl]-methyl-amino}-6-fluoro-1,2,3,4-tetrahydro-9*H-*carbazol-9-yl)-acetic acid;
(3*S*)-(6-Fluoro-3-{methyl-[2-(3-methylphenyl)-acetyl]-amino}-1,2,3,4-tetrahydro-9*H-*carbazol-9-yl)-acetic acid;
(3*S*)-(6-Fluoro-3-{[2-(3-methoxy-phenyl)-acetyl]-methyl-amino}-1,2,3,4-tetrahydro-9*H-*carbazol-9-yl)-acetic acid;
(3*S*)-(3-{[2-(2-Chloro-phenoxy)-acetyl]-methyl-amino}-6-fluoro-1,2,3,4-tetrahydro-9*H-*carbazol-9-yl)-acetic acid;
(3*S*)-(3-{[2-(4-Chloro-phenoxy)-acetyl]-methyl-amino}-6-fluoro-1,2,3,4-tetrahydro-9*H-*carbazol-9-yl)-acetic acid;
(3*S*)-(6-Fluoro-3-{[3-(3-methoxy-phenyl)-propionyl]-methyl-amino}-1,2,3,4-tetrahydro-9*H-*carbazol-9-yl)-acetic acid;
(3*S*)-(6-Fluoro-3-{methyl-[2-(2-methylphenyl)-acetyl]-amino}-1,2,3,4-tetrahydro-9*H-*carbazol-9-yl)-acetic acid;
(3*S*)-{3-[(3,3-Diphenyl-propionyl)-methyl-amino]-6-fluoro-1,2,3,4-tetrahydro-9*H*-carbazol-9-yl}-acetic acid;
(3*S*)-(6-Fluoro-3-{[3-(2-methoxy-phenyl)-propionyl]-methyl-amino}-1,2,3,4-tetrahydro-9*H-*carbazol-9-yl)-acetic acid;
(3*S*)-{6-Fluoro-3-[(3-1*H*-indol-3-yl-propionyl)-methyl-amino]-1,2,3,4-tetrahydro-9*H-*carbazol-9-yl}-acetic acid;
(3*S*)-{3-[(2-Benzyloxy-acetyl)-methyl-amino]-6-fluoro-1,2,3,4-tetrahydro-9*H*-carbazol-9-yl}-acetic acid;
(3*S*)-{3-[(2,3-Diphenyl-propionyl)-methyl-amino]-6-fluoro-1,2,3,4-tetrahydro-9*H*-carbazol-9-yl}-acetic acid;
(3*S*)-{6-Fluoro-3-[[3-(2-methoxy-phenyl)-propionyl]-(3-phenyl-propyl)-amino]-1,2,3,4-tetrahydro-9*H*-carbazol-9-yl} -acetic acid;
(3*S*)-{3-[Acetyl-(3-phenyl-propyl)-amino]-6-fluoro-1,2,3,4-tetrahydro-9*H*-carbazol-9-yl}-acetic acid;
(3*S*)-{3-[3-Benzyl-(1-cyclopropylmethyl)-ureido]-6-fluoro-1,2,3,4-tetrahydro-9H-carbazol-9-yl}-acetic acid;
(3*S*)-[3-(Benzyloxycarbonyl-cyclopropylmethyl-amino)-6-fluoro-1,2,3,4-tetrahydro-9*H-*carbazol-9-yl]-acetic acid;
(3*S*)-{3-[Cyclopropylmethyl-(3-phenyl-propionyl)-amino]-6-fluoro-1,2,3,4-tetrahydro-9*H-*carbazol-9-yl}-acetic acid;
(3*S*)-{3-[Cyclopropylmethyl-((*S*)-2-methyl-3-phenyl-propionyl)-amino]-6-fluoro-1,2,3,4-tetrahydro-9*H*-carbazol-9-yl}-acetic acid;
(3*S*)-(3-{Cyclopropylmethyl-[3-(2-methoxy-phenyl)-propionyl]-amino}-6-fluoro-1,2,3,4-tetrahydro-9*H*-carbazol-9-yl)-acetic acid;
(3*S*)-(3-{[2-(3-Chloro-phenoxy)-acetyl]-cyclopropylmethyl-amino}-6-fluoro-1,2,3,4-tetrahydro-9*H*-carbazol-9-yl)-acetic acid;
(3*S*)-{3-[Cyclopropylmethyl-(3,3-diphenyl-propionyl)-amino]-6-fluoro-1,2,3,4-tetrahydro-9*H*-carbazol-9-yl}-acetic acid;
(3*S*)-{3-[Cyclopropylmethyl-(2-naphthalen-1-yl-acetyl)-amino]-6-fluoro-1,2,3,4-tetrahydro-9*H*-carbazol-9-yl}-acetic acid;
(3*S*)-(3-{Benzyloxycarbonyl-[2-(4-trifluoromethyl-phenoxy)-ethyl]-amino}-6-fluoro-1,2,3,4-tetrahydro-9*H*-carbazol-9-yl)-acetic acid;
(3*S*)-(3-{Acetyl-[2-(4-trifluoromethyl-phenoxy)-ethyl]-amino}-6-fluoro-1,2,3,4-tetrahydro-9*H*-carbazol-9-yl)-acetic acid;
(3*S*)-(6-Fluoro-3-{propionyl-[2-(4-trifluoromethyl-phenoxy)-ethyl]-amino}-1,2,3,4-tetrahydro-9*H*-carbazol-9-yl)-acetic acid;
(3*S*)-(6-Fluoro-3-{(3-phenyl-propionyl)-[2-(4-trifluoromethyl-phenoxy)-ethyl]-amino}-1,2,3,4-tetrahydro-9*H*-carbazol-9-yl)-acetic acid;
(3*S*)-(6-Fluoro-3-{[3-(2-methoxy-phenyl)-propionyl]-[2-(4-trifluoromethyl-phenoxy)-ethyl]-amino}-1,2,3,4-tetrahydro-9*H*-carbazol-9-yl)-acetic acid;
(3*S*)-{6-Fluoro-3-[(2-phenoxy-ethyl)-(3-phenyl-propionyl)-amino]-1,2,3,4-tetrahydro-9*H-*carbazol-9-yl}-acetic acid;
(3*S*)-{6-Fluoro-3-[((S)-2-methyl-3-phenyl-propionyl)-(2-phenoxy-ethyl)-amino]-1,2,3,4-tetrahydro-9*H*-carbazol-9-yl}-acetic acid;
(3*S*)-{6-Fluoro-3-[[3-(2-methoxy-phenyl)-propionyl]-(2-phenoxy-ethyl)-amino]-1,2,3,4-tetrahydro-9*H*-carbazol-9-yl}-acetic acid;
(3*S*)-{3-[Acetyl-(2-phenoxy-ethyl)-amino]-6-fluoro-1,2,3,4-tetrahydro-9*H*-carbazol-9-yl}-acetic acid;
(3*S*)-{3-[3-Benzyl-1-(2-methoxy-ethyl)-ureido]-6-fluoro-1,2,3,4-tetrahydro-9*H*-carbazol-9-yl}-acetic acid;
(3*S*)-{3-[Benzyloxycarbonyl-(2-methoxy-ethyl)-amino]-6-fluoro-1,2,3,4-tetrahydro-9*H-*carbazol-9-yl}-acetic acid;
(3*S*)-{6-Fluoro-3-[(2-methoxy-ethyl)-(3-phenyl-propionyl)-amino]-1,2,3,4-tetrahydro-9*H-*carbazol-9-yl}-acetic acid;
(3*S*)-{6-Fluoro-3-[(2-methoxy-ethyl)-((S)-2-methyl-3-phenyl-propionyl)-amino]-1,2,3,4-tetrahydro-9*H*-carbazol-9-yl} -acetic acid;
(3*S*)-(6-Fluoro-3-{(2-methoxy-ethyl)-[3-(2-methoxy-phenyl)-propionyl]-amino}-1,2,3,4-tetrahydro-9*H*-carbazol-9-yl)-acetic acid;
(3*S*)-{3-[[2-(3-Chloro-phenoxy)-acetyl]-(2-methoxy-ethyl)-amino]-6-fluoro-1,2,3,4-tetrahydro-9*H*-carbazol-9-yl}-acetic acid;
(3*S*)-{3-[(3,3-Diphenyl-propionyl)-(2-methoxy-ethyl)-amino]-6-fluoro-1,2,3,4-tetrahydro-9*H*-carbazol-9-yl}-acetic acid;
(3*S*)-{6-Fluoro-3-[(2-methoxy-ethyl)-(2-naphthalen-1-yl-acetyl)-amino]-1,2,3,4-tetrahydro-9*H*-carbazol-9-yl}-acetic acid;
(3*S*)-(6-Fluoro-3-{[(2*S*)-2-methyl-3-phenyl-propionyl]-phenethyl-amino}-1,2,3,4-tetrahydro-9*H*-carbazol-9-yl)-acetic acid;
(3*S*)-(6-Fluoro-3-{[3-(2-methoxy-phenyl)-propionyl]-phenethyl-amino}-1,2,3,4-tetrahydro-9*H*-carbazol-9-yl)-acetic acid;
(3*S*)-[3-(Acetyl-phenethyl-amino)-6-fluoro-1,2,3,4-tetrahydro-9*H*-carbazol-9-yl]-acetic acid;
(3*S*)-{6-Fluoro-3-[(2-naphthalen-1-yl-acetyl)-phenethyl-amino]-1,2,3,4-tetrahydro-9*H-*carbazol-9-yl}-acetic acid;
(3*S*)-{6-Fluoro-3-[phenethyl-(3-phenyl-propionyl)-amino]-1,2,3,4-tetrahydro-9*H*-carbazol-9-yl}-acetic acid;
(3*S*)-[3-(3-Benzyl-1-naphthalen-1-ylmethyl-ureido)-6-fluoro-1,2,3,4-tetrahydro-9*H-*carbazol-9-yl]-acetic acid;
(3*S*)-[3-(Benzyloxycarbonyl-naphthalen-1-ylmethyl-amino)-6-fluoro-1,2,3,4-tetrahydro-9*H-*carbazol-9-yl]-acetic acid;
(3*S*)-{6-Fluoro-3-[naphthalen-1-ylmethyl-(3-phenyl-propionyl)-amino]-1,2,3,4-tetrahydro-9*H*-carbazol-9-yl}-acetic acid;
(3*S*)-{6-Fluoro-3-[((*S*)-2-methyl-3-phenyl-propionyl)-naphthalen-1-ylmethyl-amino]-1,2,3,4-tetrahydro-9*H*-carbazol-9-yl}-acetic acid;
(3*S*)-(6-Fluoro-3-{[3-(2-methoxy-phenyl)-propionyl]-naphthalen-1-ylmethyl-amino}-1,2,3,4-tetrahydro-9*H*-carbazol-9-yl)-acetic acid;
(3*S*)-{3-[(3,3-Diphenyl-propionyl)-naphthalen-1-ylmethyl-amino]-6-fluoro-1,2,3,4-tetrahydro-9*H*-carbazol-9-yl}-acetic acid;
(3*S*)-[3-(Acetyl-naphthalen-1-ylmethyl-amino)-6-fluoro-1,2,3,4-tetrahydro-9*H*-carbazol-9-yl]-acetic acid;
(3*S*)-[6-Fluoro-3-(naphthalen-1-ylmethyl-propionyl-amino)-1,2,3,4-tetrahydro-9*H*-carbazol-9-yl]-acetic acid;
(3*S*)-{3-[(*RS*)-2-Benzyl-3-(2-methylphenyl)-carbamoyl-propionylamino]-6-fluoro-1,2,3,4-tetrahydro-9*H*-carbazol-9-yl} -acetic acid;
(3*S*)-{3-[(*RS*)-2-Benzyl-3-(3-methoxy-phenylcarbamoyl)-propionylamino]-6-fluoro-1,2,3,4-tetrahydro-9*H*-carbazol-9-yl}-acetic acid;
(3*S*)-{3-[(*RS*)-2-Benzyl-3-(4-chloro-phenylcarbamoyl)-propionylamino]-6-fluoro-1,2,3,4-tetrahydro-9*H*-carbazol-9-yl}-acetic acid;
(3*S*)-{3-[(*RS*)-2-Benzyl-3-(4-fluoro-benzylcarbamoyl)-propionylamino]-6-fluoro-1,2,3,4-tetrahydro-9*H*-carbazol-9-yl} -acetic acid;
[(3*S*)-3-((*RS*)-2-Benzyl-3-propylcarbamoyl-propionylamino)-6-fluoro-1,2,3,4-tetrahydro-9*H*-carbazol-9-yl]-acetic acid;
(3*S*)-[6-Fluoro-3-(3-thiophen-2-yl-propionylamino)-1,2,3,4-tetrahydro-9*H*-carbazol-9-yl]-acetic acid;
(3*S*)-{3-[3-(3-Chloro-isoxazol-5-yl)-propionylamino]-6-fluoro-1,2,3,4-tetrahydro-9*H-*carbazol-9-yl}-acetic acid;
(3*S*)-[6-Fluoro-3-(3-pyrimidin-2-yl-propionylamino)-1,2,3,4-tetrahydro-9*H*-carbazol-9-yl]-acetic acid;
(3*S*)-{6-Fluoro-3-[3-phenyl-4-([1,3,4]thiadiazol-2-ylcarbamoyl)-butyrylamino]-1,2,3,4-tetrahydro-9*H*-carbazol-9-yl}-acetic acid;
(3*S*)-[3-(1,3-Dibenzyl-ureido)-6-fluoro-1,2,3,4-tetrahydro-9*H*-carbazol-9-yl]-acetic acid;
(3*S*)-(3-{Acetyl-[2-(2-fluoro-phenyl)-ethyl]-amino}-6-fluoro-1,2,3,4-tetrahydro-9*H-*carbazol-9-yl)-acetic acid;
(3*S*)-(3-{Acetyl-[2-(3-fluoro-phenyl)-ethyl]-amino}-6-fluoro-1,2,3,4-tetrahydro-9*H-*carbazol-9-yl)-acetic acid;
(3*S*)-[3-(3-Benzyl-1-cyclohexylmethyl-ureido)-6-fluoro-1,2,3,4-tetrahydro-9*H*-carbazol-9-yl]-acetic acid; and
(3*S*)-(3-{Cyclohexylmethyl-[3-(2-methoxy-phenyl)-propionyl]-amino}-6-fluoro-1,2,3,4-tetrahydro-9*H*-carbazol-9-yl)-acetic acid.

Unless explicitly stated otherwise, the general terms and names used hereinbefore and hereinafter preferably have within the context of this disclosure the following meanings:
Where the plural form is used for compounds, salts, pharmaceutical compositions, diseases and the like, this is intended to mean also a single compound, salt, or the like.

Any reference to a compound of Formula **I** is to be understood as referring also to salts (especially pharmaceutically acceptable salts) of a compound of Formula **I,** as appropriate and expedient.

The term "pharmaceutically acceptable salts" refers to non-toxic, inorganic or organic acid and/or base addition salts. Reference can be made to "Salt selection for basic drugs", Int. J. Pharm. (1986), 33, 201-217.

The term "bridging group" or "bridging atom" as used herein means a group or atom that is placed between two distinct moieties of the molecule.
Examples for such bridging groups are the bridging C₁₋₆-alkyl group in an aryl-C₁₋₆-alkylcarbonyl group which is placed between the aryl and the carbonyl moiety; the bridging C₁₋₄-alkyl group in an aryl-C₁₋₄-alkyl group, which is placed between the aryl moiety and the parent molecular moiety; or the bridging C₁₋₄-alkyl group in an R⁷-C₁₋₄-alkylcarbonyl group which is placed between the R⁷-group and the carbonyl moiety. An example for such bridging atoms is the bridging carbon atom of a methylene (-CH₂-) group in a benzyloxy or benzylamino group, which is placed between the phenyl ring and the oxygen atom, or the phenyl ring and the nitrogen atom, respectively.

The term "alkyl" as used herein, alone or in any combination, refers to a saturated aliphatic group including a straight or branched hydrocarbon chain containing the indicated number of carbon atoms, for example C₁₋₉-alkyl, i.e. an alkyl having 1-9 carbon atoms. Representative examples of alkyl groups include, but are not limited to, methyl, ethyl, *n*-propyl, *iso*-propyl, *n*-butyl, *tert*-butyl, *iso*-butyl (or also referred to as "2-methylpropyl"), *n*-pentyl (also referred to as "*n*-amyl"), *iso*-pentyl (also referred to as "*iso*-amyl"), *n*-hexyl, *n*-heptyl, and *n*-octyl. Preferred are methyl, ethyl, *n*-propyl, *iso*-propyl, *n*-butyl, and *iso*-butyl. Most preferred are methyl, ethyl, *n*-propyl, and *iso*-propyl.

A bridging C₁₋₆-alkyl group as used in "aryl-C₁₋₆-alkylcarbonyl" as defined for R⁶ preferably means a C₂₋₄-alkyl group, whereby the aryl moiety and the carbonyl moiety are preferably attached to two different carbon atoms of the bridging C₂₋₄-alkyl group. Preferred examples of bridging C₁₋₆-alkyl groups as used in R⁶ being aryl-C₁₋₆-alkylcarbonyl are ethane-1,2-diyl, propane-1,2-diyl, propane-1,3-diyl, and 2-methyl-propane-1,2-diyl.
In another embodiment a bridging C₁₋₆-alkyl group as used in "aryl-C₁₋₆-alkylcarbonyl" as defined for R⁶ means the respective C₁₋₆-alkyl group, whereby the aryl moiety and the carbonyl moiety preferably are attached to the same carbon atom of the bridging C₁₋₆-alkyl group. Examples of such bridging C₁₋₆-alkyl groups as used in R⁶ being aryl-C₁₋₆-alkylcarbonyl are a methylene group (preferred) and ethane-1,1-diyl.
A bridging C₁₋₃-alkyl group as used in "diaryl-C₁₋₃-alkylcarbonyl" as defined for R⁶ preferably means a C₂₋₃-alkyl group, whereby the carbonyl moiety and at least one of the aryl groups are preferably attached to two different carbon atoms of the bridging C₂₋₃-alkyl group. Preferred examples of such bridging C₁₋₃-alkyl groups as used in R⁶ being diaryl-C₁₋₃-alkylcarbonyl are ethane-1,2,2-triyl, and ethane-1,1,2-triyl.
A bridging C₁₋₄-alkyl group as used in "R⁷-C₁₋₄-alkylcarbonyl, wherein the bridging C₁₋₄-alkyl group may additionally be mono-substituted with aryl" as defined for R⁶ preferably means a C₂₋₄-alkyl group (particularly, if the additional aryl substituent is present, it means propane-1,2,3-triyl) whereby the group R⁷, the carbonyl moiety. and the aryl substituent (if present) preferably are attached to two (three, if the additional aryl substituent is present) different carbon atoms of the bridging C₂₋₄-alkyl group. Examples of bridging C₁₋₄-alkyl groups as used in R⁶ being "R⁷-C₁₋₄-alkylcarbonyl, wherein the bridging C₁₋₄-alkyl group may additionally be mono-substituted with aryl" are ethane-1,2-diyl, propane-1,2-diyl, propane-1,3-diyl, 2-phenyl-propane-1,3-diyl, and 1-phenyl-propane-2,3-diyl; preferred are 2-phenyl-propane-1,3-diyl, and 1-phenyl-propane-2,3-diyl, especially 1-phenyl-propane-2,3-diyl.
An example of a bridging C₁₋₄-alkyl group as used in R⁶ being "R⁷-C₁₋₄-alkylcarbonyl, wherein the bridging C₁₋₄-alkyl group may additionally be disubstituted with hydroxy" is 1,2-dihydroxyethane-1,2-diyl.

The term "alkenyl" as used herein, alone or in any combination, refers to a straight or branched hydrocarbon chain containing 2-7, preferably 2-4, carbon atoms with at least one carbon-carbon double bond (RₐR_{b}C=CR_{c}R_{d}). Rₐ-R_{d} refer to substituents, each individually and independently selected from hydrogen and alkyl. Representative examples of alkenyl include, but are not limited to, ethenyl (also referred to as "vinyl"), 2-propenyl (also referred to as "allyl"), 2-methyl-2-propenyl, 3-butenyl, 4-pentenyl, and 5-hexenyl, especially ethenyl or 2-propenyl.

The term "C₁₋₂-alkylendioxy" as used herein, alone or in any combination, refers to an -O(CH₂)ₙO- group, wherein n is 1 or 2, and wherein the oxygen atoms are attached to two adjacent carbon atoms of the parent molecular moiety, preferably the two adjacent carbon atoms of a phenyl ring.

The term "alkoxy" as used herein, alone or in any combination, refers to an alkyl group attached to the parent molecular moiety through an oxygen bridge. Representative examples of alkoxy include, but are not limited to, methoxy, ethoxy, propoxy, 2-propoxy, butoxy, *tert*-butoxy, pentoxy, and hexyloxy, especially methoxy.

The term "alkoxycarbonyl", as used herein, alone or in any combination, refers to an alkoxy group attached to the parent molecular moiety through a carbonyl group. Representative examples of alkoxycarbonyl include, but are not limited to, methoxycarbonyl, ethoxycarbonyl, n-propoxycarbonyl, and iso-butoxycarbonyl, especially methoxy. R⁶ representing alkoxycarbonyl preferably means *n*-propoxycarbonyl, and *iso*-butoxycarbonyl. The term "alkylcarbonyl" as used herein, alone or in any combination, refers to an alkyl group attached to the parent molecular moiety through a carbonyl group. Representative examples of alkylcarbonyl include, but are not limited to, acetyl, 1-oxopropyl, 2,2-dimethyl-1-oxopropyl, I -oxobutyl, and 1-oxopentyl. Further examples are 1-oxo-2-methyl-butyl, and 3,3-dimethyl-1-oxopropyl. Preferred are acetyl, 1-oxopropyl, 1-oxobutyl, 1-oxo-2-methylbutyl, and 3,3-dimethyl-1-oxopropyl.

The term "alkylsulfonyl", as used herein, alone or in any combination, refers to an alkyl group attached to the parent molecular moiety through a sulfonyl group. Representative examples of alkylsulfonyl include, but are not limited to, methanesulfonyl and ethanesulfonyl, preferably methanesulfonyl.

The term "aminocarbonyl" (also referred to as "carbamoyl") as used herein, alone or in any combination, refers to an amino group attached to the parent molecular moiety through a carbonyl group.

The term "aryl" or "aryl group", as used herein, alone or in any combination, refers to an aromatic carbocyclic group from 6 to 14 carbon atoms having a single ring or multiple condensed rings, and preferably refers to a phenyl or naphthyl, very preferably to a phenyl group. An aryl group is preferably unsubstituted. In another embodiment the aryl group may be substituted as specifically described in the embodiments of the present invention. If an aryl group is mono- or di-substituted, preferred but not limiting examples are 4-trifluoromethyl-phenyl, 3-trifluoromethyl-phenyl, 2,5-bis-trifluoromethyl-phenyl, 3,5-bis-trifluoromethyl-phenyl, 3-trifluoromethoxy-phenyl, 4-chlorophenyl, 3-chlorophenyl, 2-chlorophenyl, 2,6-dichlorophenyl, 2,4-dichlorophenyl, 4-methylphenyl, 3-methylphenyl, 2-methylphenyl, 2,4-dimethylphenyl, 4-ethylphenyl, 4-isopropylphenyl, 4-*tert*.-butylphenyl, 4-fluorophenyl, 3-fluorophenyl, 2-fluorophenyl, 3,4-difluorophenyl, 4-iodophenyl, 3-bromophenyl, 2-bromophenyl, 4-methoxyphenyl, 3-methoxyphenyl, 2-methoxyphenyl, 2,3-dimethoxyphenyl, 2,4-dimethoxyphenyl, 2,5-dimethoxyphenyl, 4-hydroxyphenyl, 3-hydroxyphenyl, 2-hydroxyphenyl, 4-methylthio-phenyl, 4-methanesulfonyl-phenyl, and 3-*tert*.-butoxycarbonylamino-phenyl.

The term "arylalkenyl", as used herein, alone or in any combination, refers to an aryl group attached to the parent molecular moiety through an alkenyl group. Representative examples of arylalkenyl include, but are not limited to, 2-phenylethenyl, 3-phenylpropen-2-yl, and 2-naphth-2-ylethenyl.

The term "aryloxy", as used herein, alone or in any combination, refers to an aryl group attached to the parent molecular moiety through an oxygen bridge.

The term "arylsulfonyl", as used herein, alone or in any combination, refers to an aryl group attached to the parent molecular moiety through a sulfonyl group.

The term "carbonyl", as used herein, alone or in any combination, refers to a -C(O)- group.

The term "thiocarbonyl", as used herein, alone or in any combination, refers to a -C(S)-group.

The term "carboxy", as used herein, alone or in any combination, refers to a -CO₂H group.

The term "cyano", as used herein, alone or in any combination, refers to a -C≡N group.

The term "cycloalkyl", as used herein, alone or in any combination, refers to a saturated cyclic hydrocarbon moiety containing 3-10 carbon atoms (for example C₃₋₆-cycloalkyl means a cycloalkyl having 3 to 6 carbon atoms), preferably a cyclopentyl or cyclohexyl radical, whereby said radicals, especially the cyclopentyl radical, may be substituted with an annellated benzene ring. In another embodiment said benzene ring may be mono-, or di-substituted, wherein the substituent(s) are independently selected from C₁₋₄-alkyl, C₁₋₄-alkoxy, and halogen (especially C₁₋₄-alkoxy). In case the cycloalkyl group is used as a bridging group as for example in an "aryl-C₃₋₆-cycloalkylcarbonyl" group as defined for R⁶, it is preferably a cyclopropane-diyl, cyclopentane-diyl or cyclohexane-diyl radical (especially cyclopropane-1,2-diyl) said radical being preferably unsubstituted.

The term "formyl", as used herein, alone or in any combination, refers to a -C(O)H group.

The term "halo" or "halogen", as used herein, alone or in any combination, refers to fluorine, bromine, chlorine, or iodine, and unless specifically indicated otherwise, it refers to especially fluorine or chlorine.

The term "haloalkyl", as used herein, alone or in any combination, refers to an alkyl group having at least one hydrogen atom replaced with a halogen atom. Representative examples of haloalkyl include, but are not limited to, chloromethyl, 2-fluoroethyl, trifluoromethyl, pentafluoroethyl, and 2-chloro-3-fluoropentyl, preferably trifluoromethyl.

The term "haloalkoxy", as used herein, alone or in any combination, refers to an alkoxy group having at least one hydrogen atom replaced with a halogen atom. Representative examples of haloalkoxy include, but are not limited to, chloromethoxy, 2-fluoroethoxy, trifluoromethoxy, and pentafluoroethoxy, preferably trifluoromethoxy.

The term "heterocyclyl", as used herein, alone or in any combination, refers to a monocyclic, bicyclic or polycyclic non-aromatic ring system containing up to 15 ring atoms (preferably 5 to 10 ring atoms), at least one of these being a heteroatom, preferably one to three heteroatoms, independently selected from nitrogen, oxygen or sulfur, preferably nitrogen. This ring system may be saturated, partially saturated, or unsaturated, and preferably contains one or two ring heteroatoms selected from nitrogen. Representative examples of heterocyclyl include, but are not limited to, dihydroindolyl (especially dihydroindol-2-yl) and chromane (especially chroman-3-yl).

The term "heteroaryl", as used herein, alone or in any combination, has the meaning as defined for heterocyclyl above, with the difference that the ring system is aromatic.

The term "heteroarylalkyl", as used herein, alone or in any combination, refers to a heteroaryl group attached to the parent molecular moiety through an alkyl group. Representative examples of heteroarylalkyl include, but are not limited to, thienylalkyl (especially thien-2-ylalkyl), isoxazolylalkyl (especially 3-chloro-isoxazole-5-ylalkyl), pyridylalkyl (especially pyridine-3-ylalkyl), pyrimidylalkyl (especially pyrimidine-2-ylalkyl), indolylalkyl (especially indol-3-ylalkyl), and benzoimidazolylalkyl (especially benzoimidazol-2-ylalkyl).

The term "heteroarylcarbonyl", as used herein, alone or in any combination, refers to a heteroaryl group attached to the parent molecular moiety through a carbonyl group. A representative example of heteroarylcarbonyl includes, but is not limited to, indolylcarbonyl (especially indol-2-ylcarbonyl).

The term "heteroarylamino", as used herein, alone or in any combination, refers to a heteroaryl group attached to the parent molecular moiety through an amino group. A representative example of heteroarylamino includes, thiadiazolylamino (especially 1,3,4-thiadiazol-2-yl-amino).

The term "heterocyclylcarbonyl", as used herein, alone or in any combination, refers to a heterocyclyl group attached to the parent molecular moiety through a carbonyl group. A representative example of heterocyclylcarbonyl includes, dihydroindolylcarbonyl (especially dihydroindol-2-ylcarbonyl) and chromanecarbonyl (especially chroman-3-ylcarbonyl).

The term "hydroxy" or "hydroxyl" as used herein, alone or in any combination, refers to an -OH group

The term "nitro", as used herein, alone or in any combination, refers to a -NO₂ group.

The term "oxo", as used herein, alone or in any combination, refers to an =O group.

The term "oxy", as used herein, alone or in any combination, refers to an -O- group.

The terms "sulfonyl", as used herein, alone or in any combination, refer to an -S(O)₂- group.

The term "acyl" as used herein refers to groups containing a carbonyl group that is linked to a carbon atom such as C₁₋₉-alkylcarbonyl, arylalkenylcarbonyl, aryl-C₁₋₆-alkylcarbonyl, aryl-C₁₋₃-alkoxy-C₁₋₃-alkylcarbonyl, arylcarbonyl, arylcarbonyl-C₁₋₄-alkylcarbonyl, aryloxy-C₁₋₃-alkylcarbonyl, cycloalkyl-C₁₋₃-alkylcarbonyl, diaryl-C₁₋₃-alkylcarbonyl, heterocyclylcarbonyl, heteroaryl-C₁₋₃-alkylcarbonyl, heteroarylcarbonyl, aryl-C₃₋₆-cycloalkylcarbonyl, cycloalkylcarbonyl, or R⁷-C₁₋₄-alkylcarbonyl groups as used in R⁶ of Formula **I**.
In analogy, the term "acylamino" as used herein refers to an acyl group as described before that is linked to the parent molecular moiety through a nitrogen atom.
The term "ureido" as used herein refers to groups such as C₁₋₉-alkylaminocarbonyl, arylaminocarbonyl, or aryl-C₁₋₃-alkylaminocarbonyl that are linked to the parent molecular moiety through a nitrogen atom.
The term "oxycarbonylamino" as used herein refers to groups such as C₁₋₉-alkoxycarbonyl, aryl-C₁₋₃-alkoxycarbonyl, or aryl-C₁₋₃-alkoxy-C₁₋₃-alkoxycarbonyl that are linked to the parent molecular moiety through a nitrogen atom.

The compounds of Formula **I** may contain one or more stereogenic or asymmetric centers, such as one or more asymmetric carbon atoms. Substituents at a double bond or a ring may be present in cis- (= Z-) or trans (= E-) form unless indicated otherwise. The compounds of Formula **I** may thus be present as mixtures of stereoisomers or preferably as pure stereoisomers. Mixtures of stereoisomers may be separated in a manner known to a person skilled in the art.

The compounds of the present invention have useful, in particular pharmacologically useful, properties. They bind to the CRTH2 receptor and thus modulate the effects of endogenous PGD₂. The compounds according to Formula **I** may be used for the preparation of a medicament, and are suitable, for the prevention and/or treatment of diseases selected from the group consisting of chronic and acute allergic/immune diseases/disorders, comprising allergic asthma, rhinitis, allergic rhinitis, chronic obstructive pulmonary disease (COPD), dermatitis, inflammatory bowel disease, rheumatoid arthritis, allergic nephritis, conjunctivitis, atopic dermatitis, bronchial asthma, food allergy, systemic mast cell disorders, anaphylactic shock, urticaria, eczema, itching, inflammation, ischemia-reperfusion injury, cerebrovascular disorders, pleuritis, ulcerative colitis, eosinophil-related diseases comprising Churg-Strauss syndrome and sinusitis, and basophil-related diseases, comprising basophilic leukemia and basophilic leukocytosis, in humans and other mammals.

A compound of Formula **I** or a pharmaceutical composition comprising a compound of Formula **I** may be used for the preparation of a medicament, and is suitable, for the prevention and/or treatment of diseases selected from the group consisting of both chronic and acute allergic/immune diseases/disorders such as those mentioned in the paragraph before, such as especially allergic asthma, rhinitis, allergic rhinitis, COPD, dermatitis, inflammatory bowel disease, and rheumatoid arthritis.

In another aspect, the compounds of Formula **I** may be used as standard or reference compounds in tests or assays involving the modulation of the CRTH2 receptor. Such compounds could be made commercially available for use as a reference, quality standard or control, for example in pharmaceutical research when developing new assays or protocols related to CRTH2 receptor activity.

As mentioned earlier, compounds of Formula **I** modulate the PGD₂ activation of the CRTH2 receptor. The biological effect of such compounds may be tested in a variety of *in vitro, ex vivo* and *in vivo* assays. The ability of the compounds of Formula **I** to bind to the CRTH2 receptor may be measured by methods similar to those described in the literature (Arimura A. et al., J. Pharmacol. Exp. Ther. 2001, 298(2), 411-419; and Sawyer N. et al., Br. J. Pharmacol, 2002, 137, 1163-1172, respectively) and by the assays described below in the experimental part.

A functional assay with cells expressing the human CRTH2 (hCRTH2) receptor may be used to detect changes in the levels of intracellular calcium concentration following compound treatment. After addition of the compound, the cells are challenged with PGD₂. In a Fluorescent Imaging Plate Reader (FLIPR^{™}, Molecular Devices, Sunnyvale, California), fluorescence emission is recorded during both additions, emission peak values above base level after PGD₂ addition are exported, and normalized to low controls (no PGD₂) and high controls (no active compound). The relative values of the remaining activity are used to determine IC₅₀ values by curve fitting the data to a single site to a four-parameter logistic sigmoid dose response curve of the equation (A+((B-A)/(1+((C/x)^D)))).

The ability of the compounds to modulate PGD₂ induced changes of intracellular calcium levels *via* CRTH2 receptor activation may be measured by methods known to one skilled in the art or by the assay described below in the experimental part.

The present invention relates also to pharmaceutical compositions comprising a compound of Formula **I,** or a pharmaceutically acceptable salt thereof, and a pharmaceutically acceptable carrier; to the use of such pharmaceutical compositions for the therapeutic, in a broader aspect of the invention also prophylactic, treatment of the diseases/disorders mentioned herein; to the compounds of Formula **I,** or pharmaceutically acceptable salts thereof, for use as a medicament; and to the use of a compound of Formula **I,** or a pharmaceutically acceptable salt thereof, for the preparation of a pharmaceutical composition for the prevention and/or treatment of the diseases/disorders mentioned herein.

The pharmaceutical compositions according to the invention are those for enteral administration, such as nasal, buccal, rectal, dermal or, especially oral administration, and for parenteral administration, such as intramuscular, intravenous or subcutaneous, intrasternal, intravitreal, injection or infusion, to warm-blooded animals, especially humans. Such compositions comprise an effective dose of the pharmaceutically active ingredient, alone or together with a pharmaceutically acceptable carrier. The dosage of the active ingredient depends on the species of warm-blooded animal, the body weight, the age and the individual conditions, individual pharmacokinetic data, the disease/disorder to be treated and the mode of administration.

The production of the pharmaceutical compositions can be effected in a manner which will be familiar to any person skilled in the art (see for example Mark Gibson, Editor, Pharmaceutical Preformulation and Formulation, IHS Health Group, Englewood, CO, USA, 2001; Remington, The Science and Practice of Pharmacy, 20th Edition, Philadelphia College of Pharmacy and Science) by bringing the described compounds of Formula **I** or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

In one embodiment, the invention also relates to a method for the prevention or treatment of diseases/disorders that respond to an inhibition of the CRTH2 receptor in particular to a method for the prevention or treatment of the diseases/disorders mentioned herein, said methods comprising administering to a patient a pharmaceutically active amount of a compound of Formula **I,** or a pharmaceutically acceptable salt thereof.

A further aspect of the invention is a process for the preparation of compounds of Formula **I.** Compounds according to Formula **I** of the present invention can be prepared according to the sequence of reactions outlined in the schemes below wherein R¹, R², R³, R⁴, R⁵, R⁶ and R⁷ are as defined for Formula **I.** Other abbreviations used are defined in the experimental section. In some instances the generic groups R¹, R², R³, R⁴, R⁵, R⁶ and R⁷ might be incompatible with the assembly illustrated in the schemes below and, therefore, will require the use of protecting groups (PG). For example it may be necessary to protect reactive functional groups such as hydroxy, amino, imino, thio or carboxy groups, where these are desired in the final product, to avoid their unwanted participation in the reactions. The use of protecting groups is well known in the art (see for example "Protective Groups in Organic Synthesis", T.W. Greene, P.G.M. Wuts, Wiley-Interscience, 1999). It will be assumed that such protecting groups are as necessary in place. In the following description, for example, PG, when used as amino-protecting group, preferably refers to a group such as *tert*-butoxycarbonyl, benzyloxycarbonyl, or allyloxycarbonyl, most preferably *tert-*butoxycarbonyl. Further, L refers to a leaving group, such as activated (for examples as mesylate, active ester etc.) or non-activated hydroxy, or halo, in particular chloro or bromo. Further, R and R' independently refer to a C₁₋₄-alkyl group, preferably ethyl or *tert*-butyl, whereby when R' is present R is preferably ethyl and R' is preferably *tert*.-butyl.

In general, all chemical transformations can be performed according to well-known standard methodologies as described in the literature, for example those described by Larock R. C. in "Comprehensive organic transformations: a guide to functional group preparations", VCH publishers, 1999, or as described in the procedures below. The compounds obtained may also be converted into pharmaceutically acceptable salts thereof in a manner known *per se.*

Generally, compounds of Formula **I** are obtained from an ester of Structure **1**, wherein R represents C₁₋₄-alkyl, preferably ethyl, or *tert*-butyl, by hydrolysis of the ester group using routine procedures, for example stirring an intermediate of Structure **1** with aq. lithium, sodium or potassium hydroxide in an organic co-solvent such as an alcohol, like MeOH or EtOH; THF; acetone; MeCN; or TFA, respectively.

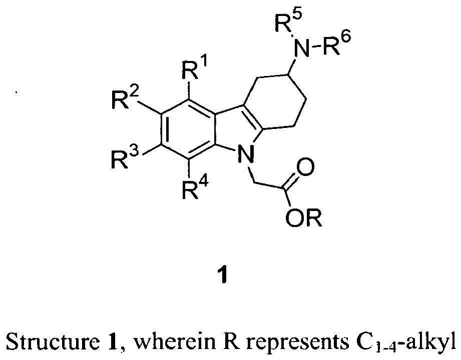

An intermediate of Structure **1** is obtained by reacting an intermediate of Structure **2a** or **2b,** or a salt thereof, such as the hydrochloride salt, with a reagent of Formula L-R⁶, wherein R⁶ is as defined for Formula **I** and L is a leaving group as defined before. R⁶ transferring reagent of Structure L-R⁶ may be a chloroformate; or an acyl halide, preferably an acid chloride, or acid bromide, used as such; or generated *in situ* from the corresponding commercially available or well known carboxylic acid with an activating reagent, such as a halogenating reagent under conditions known to a skilled person, preferably by means of oxalyl chloride or phosphorous oxychloride; or an acyl anhydride, transferring R⁶ in the presence of a base, such as Et₃N, DIEA, *N*-ethyl-morpholine, N-methylpiperidine, or pyridine, in a suitable solvent, such as THF, or DCM.

In another aspect, an intermediate of Structure **2a** or **2b** is condensed with a commercially available or well known carboxylic acid in the presence of a coupling reagent, such as DCC, diisopropylcarbodiimide, HATU and the like, in the presence of a base described hereinabove, to form an intermediate of Structure **1.**

In a further aspect, an intermediate of Structure **2a** or **2b** is reacted with a commercially available isocyanate or isothiocyanate in the presence of a base to form an intermediate of Structure **1.**

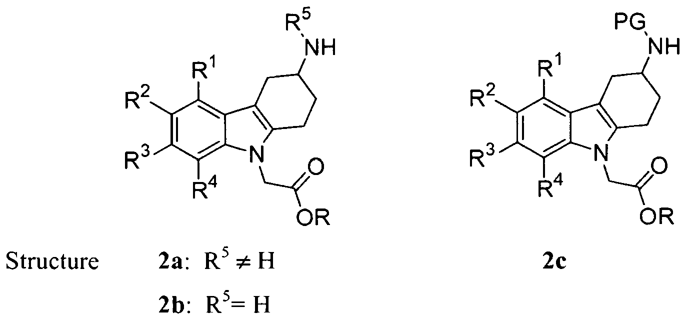

In a specific case, intermediates of Structure **1,** wherein R⁴ represents an C₁₋₅-alkyl, allyl, vinyl, or a methanesulfonyl group, is obtained by reacting intermediates of Structure **2c** wherein R⁴ represents halogen, preferably I or Br, or a methanesulfonyloxy, or a toluenesulfonyloxy group, with reagents such as tetramethyltin, allyltributyltin, a complex of vinylboronic anhydride and pyridine together with a base, such as K₂CO₃, in the presence of a palladium catalyst such as *tetrakis*(triphenylphosphine)palladium(O), or the like, or sodium methanesulfinate in the presence of copper (I) iodide, respectively, in a polar aprotic solvent such as DMF, or DME, or NMP, at a temperature between 60°C and 130°C.

A substituent R⁵ in an intermediate of Structure **2a** is obtained by reacting an intermediate of Structure **2b** with the respective aldehyde in a solvent such as DCM or the like in presence of a reducing agent, such as sodium triacetoxyborohydride, and a base, such as DIEA.

Alternatively, an intermediate of Structure **2a,** wherein R⁵ is not hydrogen, is obtained from an intermediate of Structure **2b** *via* a sulfonamide of Structure **3a.** First, an intermediate of Structure **2b** is reacted with *p*-nitrobenzenesulfonyl chloride in a solvent such as DCM, THF or another suitable organic solvent, in the presence of a base, such as DIEA, with or without a catalytic amount of *N,N*-dimethyl-aminopyridine, to afford the desired sulfonamide of Structure **3a.** In a second step, in order to afford a sulfonamide of Structure **3b,** the sulfonamide of Structure **3a** is easily alkylated with the respective commercially available or well known alkylating agent R⁵-L, with K₂CO₃ or any other suitable base, in an organic solvent, such as toluene, preferably in the presence of a phase transfer agent, such as tetrabutylammonium bromide in accordance to a procedure described in the literature (C. Peña et al., Tetrahedron Lett. 2005, 46, 2783-2787). Specifically, a methyl group is introduced by reaction of a sulfonamide of Structure **3a** either with methyl iodide or with diazomethane dissolved in diethylether.
Subsequently, the sulfonamide of Structure **3b** is treated in a typical procedure according to S.C. Miller and coworker (J. Am. Chem. Soc. 1997, 119, 2301-2302) with a thiol, such as thiophenol, or thioacetic acid, in the presence of a base, such as DBU or the like, in a suitable organic solvent, such as DMF, to remove the sulfonamido group, furnishing an intermediate of Structure **2a.**

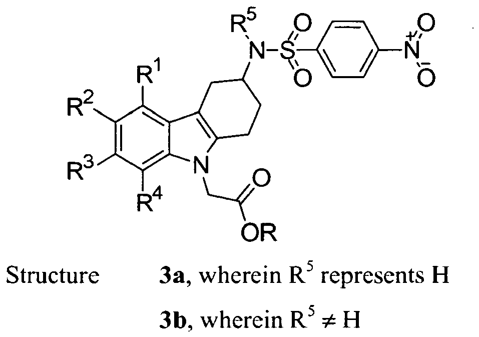

An intermediate of Structure **2b** is obtained after removal of the protective group (PG) from an intermediate of Structure **2c,** applying reaction conditions known to a skilled person. Preferably, the PG is a group such as *tert*-butoxycarbonyl, benzyloxycarbonyl, or allyloxycarbonyl, most preferably *tert*-butoxycarbonyl.

An intermediate of Structure **2c** is generated by reacting an intermediate of Structure **4** with a compound of Formula L-CH₂CO₂R wherein R and L are as defined before, in the presence of a base, such as cesium carbonate, sodium hydride, potassium *tert*-butanolate or the like, in a suitable solvent, such as acetone, MeCN, THF or dioxane. Suitable L is a leaving group such as halo, in particular bromo or chloro; mesyloxy or tosyloxy. Preferably, the compound of Formula L-CH₂CO₂R is ethyl bromoacetate.

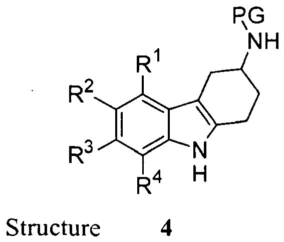

An intermediate of Structure **4,** with PG as described hereinabove, is obtained in a Fischer-type indole synthesis according to the literature (J. D. Ha et al., Bulletin of the Korean Soc. Chem. 2004, 25, 1784-1790): reaction of a commercially available or well known hydrazine of Structure **5** (either as a free base or as a salt) and a cyclohexanone of Structure **6,** which is commercially available or whose synthesis is as described in the above mentioned literature, furnishes the desired intermediate of Structure **4** as a racemate.

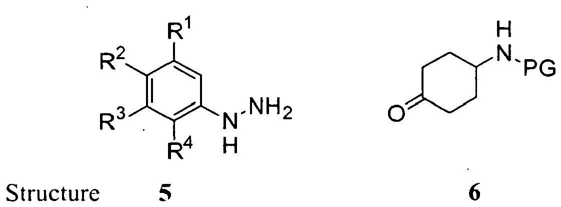

In another aspect, an intermediate of Structure **4** is obtained through protection of the amino group in a tetrahydrocarbazol-3-ylamine of Structure **7** with a hereinabove described PG applying methods known to a skilled person.

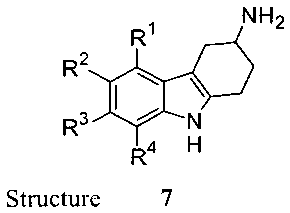

Both, the (*R*)- and the (*S*)-enantiomer of starting tetrahydrocarbazol-3-ylamine of Structure 7 arc obtained in a stereospecific reaction following a procedure described in literature (Rosentreter U. et al., Arzneim.-Forsch. 1989, 39(12), 1519-1521; and EP 0242518).

A synthesis of racemic ethyl (3*RS*)-(3-amino-1,2,3,4-tetrahydro-9*H*-carbazol-9-yl)-acetate hydrochloride is described in the literature (Ulven, T.; Kostenis, E. J. Med. Chem. 2005, 48, 897-900).

A stereoselective synthesis of methyl (3*R*)-(3-*tert*-butoxycarbonylamino-1,2,3,4-tetrahydro-9*H*-carbazol-9-yl)-acetate is described in WO 03/097598.

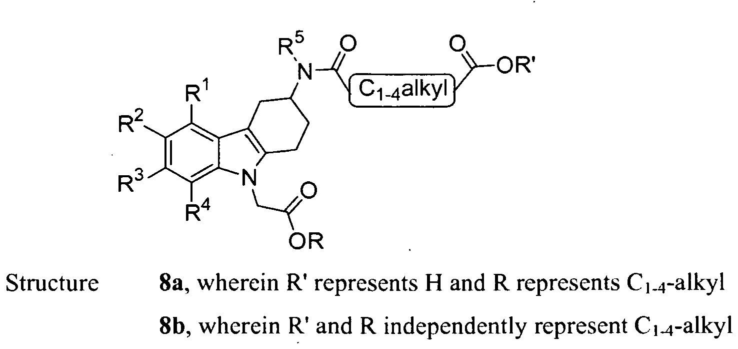

In a particular case, a compound of Structure 1, wherein R⁶ represents "R⁷-C₁₋₄-alkylcarbonyl, wherein the bridging C₁₋₄-alkyl group may additionally be mono-substituted with aryl, and R⁷ represents arylaminocarbonyl, heteroarylaminocarbonyl, C₁₋₆-alkylaminocarbonyl, or aryl-C₁₋₃-alkylaminocarbonyl", is obtained by reaction of the respective compound of Structure **8a** with the respective amine, in the presence of a coupling reagent, such as DCC, diisopropylcarbodiimide, HATU or the like, in the presence Et₃N, DIEA, or the like, in a solvent such as DCM or DMF.

A compound of Structure **8a** wherein the bridging C₁₋₄-alkyl group may additionally be mono-substituted with aryl is obtained by treating a respective compound of Structure **8b**, wherein R' represents C₁₋₄-alkyl, preferentially *tert*-butyl, as a protecting group, with reaction with TFA in DCM or hydrochloric acid in an organic solvent, such as dioxane, diethylether, AcOEt, or the like, at room temperature.

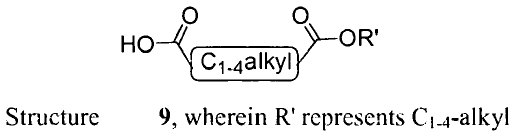

A compound of Structure **8b,** is obtained by reacting a compound of Structure **2a** or **2b** with the corresponding compound of Structure **9,** wherein the bridging C₁₋₄-alkyl group may additionally be mono-substituted with aryl, which are commercially available or synthesized according to well known methods such as enolate alkylation (see for example: J. Org. Chem. 1986, 51(6), 938-940), in the presence of a coupling reagent, such as DCC, diisopropylcarbodiimide, HATU, or the like, in the presence of a base such as Et₃N, DIEA, or the like, in a solvent such as DCM or DMF.

Whenever the compounds of Formula I are obtained in the form of mixtures of enantiomers, the enantiomers can be separated using methods known to the one skilled in the art: e.g. by formation and separation of diastereomeric salts or by HPLC over a chiral stationary phase such as a Regis Whelk-O1(R,R) (10 µm) column, a Daicel ChiralCel OD-H (5-10 µm) column, or a Daicel ChiralPak IA (10 µm) or AD-H (5 µm) column. Typical conditions of chiral HPLC are an isocratic mixture of eluent A (EtOH, in presence or absence of an amine such as Et₃N, diethylamine) and eluent B (hexane), at a flow rate of 0.8 to 150 mL/min.

### Experimental section:

### Abbreviations (as used herein):

- AcOEt: Ethyl acetate
- AcOH: Acetic acid
- aq.: aqueous
- Bdg: Binding
- BSA: Bovine Serum Albumin
- CC: Column chromatography on silica gel
- DBU: 1,8-Diazabicyclo[5.4.0]undec-7-ene
- DCC: 1,3-Dicyclohexylcarbodiimide
- DCM: Dichloromethane
- DIEA: *N*,*N*-Diisopropylethylamine
- DMAP: *N,N*-Dimethyl-4-aminopyridine
- DME: Dimethoxyethane
- DMF: Dimethylformamide
- DMSO: Dimethylsulfoxide
- EDTA: Ethylene Diamine Tetraacetic Acid
- ESI-MS: Electrospray Ionization Mass Spectroscopy
- Et₃N: Triethylamine
- FC: Flash chromatography on silica gel
- h: hour(s)
- HATU: *O*-(7-Aza-1*H*-benzotriazole-1-yl)-1,1,3,3-tetramethyluronium hexafluorophosphate
- HPLC: High Performance Liquid Chromatography
- I: liter(s)
- LC-MS: Liquid Chromatography - Mass Spectroscopy
- Me: Methyl
- MeCN: Acetonitrile
- MeI: Methyl iodide
- MeOH: Methanol
- mesyl: Methanesulfonyl
- Meth.: Method
- min: minute(s)
- MS: Mass Spectroscopy
- MW: Molecular Weight
- N: Normality of solution
- NaBH(OAc)₃: Sodium triacetoxyborohydride
- NMP: *N*-Methylpyrrolidinone
- org.: organic
- PBS: Phosphate Buffered Saline
- PG: Protecting Group
- PGD₂: Prostaglandin D₂
- PMSF: Phenylmethylsulfonyl fluoride
- rt: room temperature
- s: second(s)
- sat.: saturated
- subst.: substituted
- TFA: Trifluoroacetic acid
- THF: Tetrahydrofuran
- tlc: thin layer chromatography
- tosyl: Toluenesulfonyl
- t_{R}: retention time
- Tris: Tris-(hydroxymethyl)aminomethane buffer

### Chemistry

### General remarks

All solvents and reagents are used as obtained from commercial sources unless otherwise indicated.

Temperatures are indicated in degrees Celsius (°C). Unless otherwise indicated, the reactions take place at room temperature (rt).

In mixtures, relations of parts of solvent or eluent or reagent mixtures in liquid form are given as volume relations (v/v), unless indicated otherwise.

Analytical HPLC conditions as used in the Examples below:
HPLC/MS analyses are performed on a Waters 2795 Alliance HPLC instrument, equipped with a Waters 996 Photodiode Array Detector and a Micromass ZQ^{™} Waters mass spectrometer (electron spray ionization), detection at 200-400 nm (LC-1 and LC-2), or on a Agilent 1100 system, equipped with a Dionex P580 binary pump, a Dionex PDA-100 Photodiode Array Detector and a Finnigan AQA mass spectrometer (LC-3).

The LC retention times are obtained using the following elution conditions:
- LC-1: Analytical HPLC on a Xterra^{™} MS C18 column (4.6x50 mm, 5 µm, Waters); Linear gradient of water/ 0.06% formic acid (A) and MeCN/ 0.06% formic acid (B) from 5% to 95% B over 1 min; flow rate 3 ml/min, detection at 215 nm.
- LC-2: Analytical HPLC on a Zorbax^{®} SB-AQ column (4.6x50 mm, 5µm, Agilent); Linear gradient of water/ 0.06% formic acid (A) and MeCN/ 0.06% formic acid (B) from 5% to 95% B over 1 min; flow rate 3 ml/min, detection at 215 nm.
- LC-3: Analytical HPLC on a Zorbax^{®} SB-AQ column (4.6x50 mm, 5µm, Agilent); Linear gradient of water/ 0.05% TFA (A) and MeCN (B) from 5% to 95% B over 1 min; flow rate 4.5 ml/min, detection at 215 nm.

Preparative HPLC/MS purifications are performed on a Waters HPLC system, equipped with a Waters 600 controller, a Waters 2767 sample manager, a Waters 996 Photodiode Array Detector, and a Micromass ZQTM Waters mass spectrometer (electron spray ionization), detection at 200-400 nm, using a Zorbax® PrepHT SB.Aq (5 µm, 21.2x50 mm) or a Phenomenex® Gemini column (10 µm, 21.2x50 mm), with a linear gradient of water/0.02% formic acid (A) and MeCN/ 0.02% formic acid (B) over 5 min; flow rate 4 ml/min, detection at 215 nm.

¹H NMR spectra are recorded either on a Varian Mercury 300VX FT-NMR spectrometer or on a Bruker Advance II 400 spectometer. Chemical shifts (δ) are reported in parts per million (ppm) relative to proton resonances resulting from incomplete deuteration of the NMR solvent, *e.g*. for dimetylsulfoxide δ(H) 2.49 ppm, for chloroform δ(H) 7.24 ppm, and the abbreviations s, *d, t, q, m* and *br* refer to singlet, doublet, triplet, quartet, multiplet, and broad, respectively.

### Synthesis of Compounds of Formula I:

The following examples illustrate the preparation of pharmacologically active compounds of the invention but do not at all limit the scope thereof. First the synthesis of Example compounds is described, followed by the description of the synthesis of intermediates and starting materials. Whenever used in the experimental part, generic Structures **1** to **9** refer to the Structures described in preceeding general description of the preparation of compounds of Formula **I**.

### General method for saponification of intermediates of Structure 1:

Aq. 1N LiOH or 1N NaOH (1 ml, 1 mmol) is added to a stirred solution of the appropriate compound of Structure **1** (0.105 mmol) in THF (1 ml) and the resulting biphasic mixture is continued to stir overnight. DCM (2 ml) and AcOH (1 ml), or 2N HCl, are added to the reaction mixture. The aq. layer, obtained after phase separation, is extracted three times with DCM (1 ml). The combined org. phases are washed with brine and dried over Na₂SO₄ and the solvent is evaporated. Purification is performed by CC with a 1:1 mixture of AcOEt/heptane containing 1% AcOH, or by preparative HPLC to give the desired compound of Formula **I** in 6 to 98% yield.

Listed in Table 1 below are examples of compounds of Formula **I,** prepared according to the above-mentioned method with the corresponding compound of Structure **1** as starting material.

**Table 1**

| **Example** | **Compound of Formula I** | **Formula MW** | **t_{R} [min] LC-MS Method** | **MS Data m/z [M+H]⁺** |
|---|---|---|---|---|
| **1** | (3*R*)-[3-(3-Butyl-ureido)-1,2,3,4-tetrahydro-9*H*-carbazol-9-yl]-acetic acid | C19H25N303 | 1.01 | 344.08 |
| | | 343.426 | LC-1 | |
| **2** | (3*R*)-[3-(3-Benzyl-ureido)-1,2,3,4-tetrahydro-9*H* -carbazol-9-yl]-acetic acid | C22H23N303 | 1.15 | 378.04 |
| | | 377.443 | LC-1 | |
| **3** | (3*R*)-[3-(3-Phenethyl-ureido)-1,2,3,4-tetrahydro-9*H*-carbazol-9-yl]-acetic acid | C23H25N303 | 1.32 | 392.05 |
| | | 391.47 | LC-1 | |
| **4** | (3*R*)-[3-(3-Naphthalen-1-yl-ureido)-1,2,3,4-tetrahydro-9*H*-carbazol-9-yl]-acetic acid | C25H23N303 | 1.7 | 413.97 |
| | | 413.476 | LC-1 | |
| **5** | (3*R*)-[3-(3-Phenylsulfonyl-ureido)-1,2,3,4-tetrahydro-9*H*-carbazol-9-yl]-acetic acid | C21H21N305S | 1.13 | [M+Na]⁺ |
| | | 427.48 | LC-1 | 449.90 |
| **6** | (3*R*)-(3-*tert*-Butoxycarbonylamino-1,2,3,4-tetrahydro-9*H*-carbazol-9-yl)-acetic acid | C19H24N204 | 1.05 | [M+Na]⁺ |
| | | 344.10 | LC-1 | 367.07 |
| **7** | (3*R*)-(3-Propoxycarbonylamino-1,2,3,4-tetrahydro-9*H*-carbazol-9-yl)-acetic acid | C18H22N204 | 1.35 | 331.03 |
| | | 330.383 | LC-1 | |
| **8** | (3*R*)-(3-Isobutoxycarbonylamino-1,2,3,4-tetrahydro-9*H*-carbazol-9-yl)-acetic acid | C19H24N204 | 1.69 | 345.03 |
| | | 344.41 | LC-1 | |
| **9** | (3*R*)-(3-Benzyloxycarbonylamino-1,2,3,4-tetrahydro-9*H*-carbazol-9-yl)-acetic acid | C22H22N204 | 1.82 | [M+Na]⁺ |
| | | 378.427 | LC-1 | 400.96 |
| **10** | (3*R*)-[3-(2-Benzyloxy-ethoxycarbonylamino)-1,2,3,4-tetrahydro-9*H*-carbazol-9-yl]-acetic acid | C24H26N205 | 1.77 | [M+Na]⁺ |
| | | 422.479 | LC-1 | 444.94 |
| **11** | (3*R*)-(3-Benzoylamino-1,2,3,4-tetrahydro-9*H*-carbazol-9-yl)-acetic acid | C21H20N2O3 | 1.00 | 349.00 |
| | | 348.401 | LC-2 | |
| **12** | (3*R*)-[3-(2-Phenoxy-acetylamino)-1,2,3,4-tetrahydro-9*H*-carbazol-9-yl]-acetic acid | C22H22N204 | 1.43 | 379.04 |
| | | 378.427 | LC-1 | |
| **13** | (3*R*)-(3-Phenylacetylamino-1,2,3,4-tetrahydro-9*H*-carbazol-9-yl)-acetic acid | C22H22N203 | 1.21 | 363.02 |
| | | 362.428 | LC-1 | |
| **14** | (3*R*)-[3-(2-Thiophen-2-yl-acetylamino)-1,2,3,4-tetrahyd ro-9*H*-carbazol-9-yl]-acetic acid | C20H20N2O3S | 1.11 | 368.96 |
| | | 368.456 | LC-1 | |
| **15** | (3*R*)-[3-(3-Phenyl-propionylamino)-1,2,3,4-tetrahydro-9*H*-carbazol-9-yl]-acetic acid | C23H24N203 | 1.36 | 377.04 |
| | | 376.455 | LC-1 | |
| **16** | (3*R*)-[3-(2-Benzyloxy-acetylamino)-1,2,3,4-tetrahydro-9*H*-carbazol-9-yl]-acetic acid | C23H24N204 | 1.44 | 392.98 |
| | | 392.454 | LC-1 | |
| **17** | (3*R*)-[3-(3-Methyl-butyrylamino)-1,2,3,4-tetrahydro-9*H*-carbazol-9-yl]-acetic acid | C19H24N203 | 0.97 | 329.08 |
| | | 328.411 | LC-1 | |
| **18** | (3*R*)-[3-(3-Cyclopentyl-propionylamino)-1,2,3,4-tetrahydro-9*H*-carbazol-9-yl]-acetic acid | C22H28N203 | 1.77 | 369.07 |
| | | 368.475 | LC-1 | |
| **19** | (3*R*)-(3-Decanoytamino-1,2,3,4-tetrahydro-9*H*-carbazol-9-yl)-acetic acid | C24H34N203 | 2.77 | 399.10 |
| | | 398.545 | LC-1 | |
| **20** | (3*S*)-[3-(3-Butyl-ureido)-1,2,3,4-tetrahydro-9*H*-carbazol-9-yl]-acetic acid | C19H25N303 | 0.98 | 344.18 |
| | | 343.426 | LC-2 | |
| **21** | (3*S*)-[3-(3-Benzyl-ureido)-1,2,3,4-tetrahydro-9*H*-carbazol-9-yl]-acetic acid | C22H23N303 | 0.99 | 378.18 |
| | | 377.443 | LC-2 | |
| **22** | (3*S*)-[3-(3-Phenethyl-ureido)-1,2,3,4-tetrahydro-9*H*-carbazol-9-yl]-acetic acid | C23H25N303 | 1.01 | 392.17 |
| | | 391.47 | LC-2 | |
| **23** | (3*S*)-[3-(3-Phenylsulfonyl-ureido)-1,2,3,4-tetrahydro-9*H*-carbazol-9-yl]-acetic acid | C21H21N3O5S | 0.99 | 428.05 |
| | | 427.48 | LC-2 | |
| **24** | (3*S*)-[3-(3-Phenyl-ureido)-1,2,3,4-tetrahydro-9*H*-carbazol-9-yl]-acetic acid | C21H21N3O3 | 1.00 | 364.13 |
| | | 363.416 | LC-2 | |
| **25** | (3*S*)-(3-Propoxycarbonylamino-1,2,3,4-tetrahydro-9*H*-carbazol-9-yl)-acetic acid | C18H22N204 | 1.01 | 331.17 |
| | | 330.383 | LC-2 | |
| **26** | (3*S*)-(3-Isobutoxycarbonylamino-1,2,3,4-tetrahydro-9*H*-carbazol-9-yl)-acetic acid | C19H24N204 | 1.04 | 345.15 |
| | | 344.41 | LC-2 | |
| **27** | (3*S*)-(3-Benzyloxycarbonylamino-1,2,3,4-tetrahydro-9*H*-carbazol-9-yl)-acetic acid | C22H22N204 | 1.06 | 379.15 |
| | | 378.427 | LC-2 | |
| **28** | (3*S*)-[3-(2-Benzyloxy-ethoxycarbonylamino)-1, 2, 3,4-tetrahyd ro-9*H*-carbazol-9-yl]-acetic acid | C24H26N205 | 1.06 | 423.20 |
| | | 422.479 | LC-2 | |
| **29** | (3*S*)-[3-(2-Phenoxy-acetylamino)-1,2,3,4-tetrahydro-9*H*-carbazol-9-yl]-acetic acid | C22H22N204 | 1.02 | 379.08 |
| | | 378.427 | LC-2 | |
| **30** | (3*S*)-(3-Phenylacetylamino-1,2,3,4-tetrahydro-9*H*-carbazol-9-yl)-acetic acid | C22H22N203 | 1.00 | 363.16 |
| | | 362.428 | LC-2 | |
| **31** | (3*S*)-[3-(2-Thiophen-2-yl-acetylamino)-1,2,3,4-tetrahydro-9*H*-carbazol-9-yl]-acetic acid | C20H20N2O3S | 0.99 | 369.11 |
| | | 368.456 | LC-2 | |
| **32** | (3*S*)-[3-(3-Phenyl-propionylamino)-1,2,3,4-tetrahydro-9*H*-carbazol-9-yl]-acetic acid | C23H24N203 | 1.02 | 377.08 |
| | | 376.455 | LC-2 | |
| **33** | (3*S*)-[3-(2-Benzyloxy-acetylamino)-1,2,3,4-tetrahydro-9*H*-carbazol-9-yl]-acetic acid | C23H24N204 | 1.03 | 393.14 |
| | | 392.454 | LC-2 | |
| **34** | (3*S*)-[3-(3-Methyl-butyrylamino)-1,2,3,4-tetrahydro-9*H*-carbazol-9-yl]-acetic acid | C19H24N2O3 | 0.97 | 329.16 |
| | | 323.411 | LC-2 | |
| **35** | (3*S*)-[3-(3-Cyclopentyl-propionylamino)-1,2,3,4-tetrahydro-9*H*-carbazol-9-yl]-acetic acid | C22H28N203 | 1.01 | 369.18 |
| | | 368.475 | LC-2 | |
| **36** | (3*S*)-(3-Decanoylamino-1,2,3,4-tetrahydro-9*H*-carbazol-9-yl)-acetic acid | C24H34N203 | 1.16 | 398.82 |
| | | 398.545 | LC-2 | |
| **37** | (3*S*)-(3-Butyrylamino-1,2,3,4-tetrahydro-9*H*-carbazol-9-yl)-acetic acid | C18H22N203 | 0.94 | 315.18 |
| | | 314.384 | LC-2 | |
| **38** | (3*S*)-(3-Heptanoylamino-1,2,3,4-tetrahydro-9*H*-carbazol-9-yl)-acetic acid | C21H28N203 | 1.06 | 357.20 |
| | | 356.464 | LC-2 | |
| **39** | (3*R*)-[6-Fluoro-3-(2-phenoxy-acetylamino)-1,2,3,4-tetrahydro-9*H*-carbazol-9-yl]-acetic acid | C22H21N204F | 1.04 | 397.16 |
| | | 396.417 | LC-2 | |
| **40** | (3*R*)-(6-Fluoro-3-phenylacetylamino-1,2,3,4-tetrahydro-9*H*-carbazol-9-yl)-acetic acid | C22H21N2O3F | 1.01 | 381.16 |
| | | 380.418 | LC-2 | |
| **41** | (3*R*)-[6-Fluoro-3-(3-phenyl-propionylamino)-1,2,3,4-tetrahydro-9*H*-carbazol-9-yl]-acetic acid | C23H23N203F | 1.03 | 395.22 |
| | | 394.445 | LC-2 | |
| **42** | (3*R*)-{3-[2-(4-Chloro-phenyl)-acetylamino]-6-fluoro-1,2,3,4-tetrahydro-9*H*-carbazol-9-yl}-acetic acid | C22H20N2O3ClF | 1.05 | 415.16 |
| | | 414.863 | LC-2 | |
| **43** | (3*R*)-{6-Fluoro-3-[2-(4-methoxy-phenyl)-acetylamino]-1,2,3,4-tetrahydro-9*H*-carbazol-9-yl}-acetic acid | C23H23N204F | 1.01 | 411.22 |
| | | 410.444 | LC-2 | |
| **44** | (3*R*)-[6-Fluoro-3-(2-p-tolyl-acetylami no)-1,2,3,4-tetrahydro-9*H*-carbazol-9-yl]-acetic acid | C23H23N203F | 1.04 | 395.22 |
| | | 394.445 | LC-2 | |
| **45** | (3*R*)-{3-[2-(3,4-Dichloro-phenyl)-acetylamino]-6-fluoro-1,2,3,4-tetrahydro-9*H*-carbazol-9-yl}-acetic acid | C22H19N2O3Cl2F | 1.09 | 447.16 |
| | | 449.308 | LC-2 | |
| **46** | (3*R*)-{3-[2-(3-Chloro-phenyl)-acetylamino]-6-fluoro-1,2,3,4-tetrahydro-9*H*-carbazol-9-yl}-acetic acid | C22H20N2O3ClF | 1.06 | 415.16 |
| | | 414.863 | LC-2 | |
| **47** | (3*R*)-{6-Fluoro-3-[2-(4-trifluoromethyl-phenyl)-acetylamino]-1,2,3,4-tetrahydro-9*H*-carbazol-9-yl}-acetic acid | C23H20N2O3F4 | 1.08 | 449.17 |
| | | 448.415 | LC-2 | |
| **48** | (3*R*)-{3-[2-(4-Chloro-phenoxy)-acetylamino]-6-fluoro-1,2,3,4-tetrahydro-9*H*-carbazol-9-yl}-acetic acid | C22H20N2O4ClF | 1.08 | 431.16 |
| | | 430.862 | LC-2 | |
| **49** | (3*R*)-[6-Fluoro-3-(2-p-tolyloxy-acetylamino)-1,2,3,4-tetrahyd ro-9*H*-carbazol-9-yl]-acetic acid | C23H23N204F | 1.08 | 411.22 |
| | | 410.444 | LC-2 | |
| **50** | (3*R*)-{3-[2-(2-Chloro-phenoxy)-acetylamino]-6-fluoro-1,2,3,4-tetrahydro-9*H*-carbazol-9-yl}-acetic acid | C22H20N2O4ClF | 1.07 | 431.16 |
| | | 430.862 | LC-2 | |
| **51** | (3*R*)-{3-[2-(3-Chloro-phenoxy)-acetylamino]-6-fluoro-1,2,3,4-tetrahydro-9*H*-carbazol-9-yl}-acetic acid | C22H20N2O4ClF | 1.08 | 431.16 |
| | | 430.862 | LC-2 | |
| **52** | (3*R*)-{6-Fluoro-3-[3-(4-methoxy-phenyl)-propionylamino]-1,2,3,4-tetrahydro-9*H*-carbazol-9-yl}-acetic acid | C24H25N204F | 1.03 | 425.28 |
| | | 424.471 | LC-2 | |
| **53** | (3*R*)-[6-Fluoro-3-(3-p-tolyl-propionylamino)-1,2,3,4-tetrahydro-9*H*-carbazol-9-yl]-acetic acid | C24H25N203F | 1.06 | 409.21 |
| | | 408.472 | LC-2 | |
| **54** | (3*R*)-{3-[3-(4-Chloro-phenyl)-propionylamino]-6-fluoro-1,2,3,4-tetrahydro-9*H*-carbazol-9-yl}-acetic acid | C23H22N2O3ClF | 1.07 | 429.15 |
| | | 428.89 | LC-2 | |
| **55** | (3*R*)-{3-[3-(2-Chloro-phenyl)-propionylamino]-6-fluoro-1,2,3,4-tetrahydro-9*H*-carbazol-9-yl}-acetic acid | C23H22N2O3ClF | 1.07 | 429.15 |
| | | 428.89 | LC-2 | |
| **56** | (3*R*)-{3-[3-(3-Chloro-phenyl)-propionylamino]-6-fluoro-1,2,3,4-tetrahydro-9*H*-carbazol-9-yl}-acetic acid | C23H22N2O3ClF | 1.07 | 429.15 |
| | | 428.89 | LC-2 | |
| **57** | (3*S*)-{3-[2-(4-Chloro-phenyl)-acetylamino]-6-fluoro-1,2,3,4-tetrahydro-9*H*-carbazol-9-yl}-acetic acid | C22H20N2O3ClF | 1.05 | 415.23 |
| | | 414.863 | LC-2 | |
| **58** | (3*S*)-{6-Fluoro-3-[2-(4-methoxy-phenyl)-acetylamino]-1,2,3,4-tetrahydro-9*H*-carbazol-9-yl}-acetic acid | C23H23N204F | 1.01 | 411.22 |
| | | 410.444 | LC-2 | |
| **59** | (3*S*)-[6-Fluoro-3-(2-*p*-tolyl-acetylamino)-1,2,3,4-tetrahydro-9*H*-carbazol-9-yl]-acetic acid | C23H23N203F | 1.01 | 395.15 |
| | | 394.445 | LC-2 | |
| **60** | (3*S*)-[6-Fluoro-3-(2-phenoxy-acetylamino)-1,2,3,4-tetrahydro-9*H*-carbazol-9-yl]-acetic acid | C22H21N204F | 1.04 | 397.23 |
| | | 396.417 | LC-2 | |
| **61** | (3*S*)-[6-Fluoro-3-(3-phenyl-propionylamino)-1,2,3,4-tetrahyd ro-9*H*-carbazol-9-yl]-acetic acid | C23H23N203F | 1.03 | 395.22 |
| | | 394.445 | LC-2 | |
| **62** | (3*S*)-{3-[2-(4-Chloro-phenoxy)-acetylamino]-6-fluoro-1,2,3,4-tetrahydro-9*H*-carbazol-9-yl}-acetic acid | C22H20N2O4ClF | 1.08 | 431.16 |
| | | 430.862 | LC-2 | |
| **63** | (3*S*)-[6-Fluoro-3-(2-*p*-tolyloxy-acetylamino)-1,2,3,4-tetrahydro-9*H*-carbazol-9-yl]-acetic acid | C23H23N204F | 1.07 | 411.22 |
| | | 410.444 | LC-2 | |
| **64** | (3*S*)-{6-Fluoro-3-[3-(4-methoxy-phenyl)-propionylamino]-1,2,3,4-tetrahydro-9*H*-carbazol-9-yl}-acetic acid | C24H25N204F | 1.03 | 425.21 |
| | | 424.471 | LC-2 | |
| **65** | (3*S*)-[6-Fluoro-3-(3-*p*-tolyl-propionylamino)-1,2,3,4-tetrahydro-9*H*-carbazol-9-yl]-acetic acid | C24H25N203F | 1.06 | 409.21 |
| | | 408.472 | LC-2 | |
| **66** | (3*S*)-{3-[3-(4-Chloro-phenyl)-propionylamino]-6-fluoro-1,2,3,4-tetrahydro-9*H*-carbazol-9-yl}-acetic acid | C23H22N2O3ClF | 1.07 | 429.15 |
| | | 428.89 | LC-2 | |
| **67** | (3*S*)-{3-[3-(2-Chloro-phenyl)-propionylamino]-6-fluoro-1,2,3,4-tetrahydro-9*H*-carbazol-9-yl}-acetic acid | C23H22N2O3ClF | 1.07 | 429.15 |
| | | 428.89 | LC-2 | |
| **68** | (3*S*)-{3-[3-(3-Chloro-phenyl)-propionylamino]-6-fluoro-1,2,3,4-tetrahydro-9*H*-carbazol-9-yl}-acetic acid | C23H22N2O3ClF | 1.07 | 429.15 |
| | | 428.89 | LC-2 | |
| **69** | (3*S*)-{3-[3-(3,4-Difluoro-phenyl)-propionylamino]-6-fluoro-1,2,3,4-tetrahydro-9*H*-carbazol-9-yl}-acetic acid | C23H21N203F3 | 1.05 | 431.26 |
| | | 430.425 | LC-2 | |
| **70** | (3*S*)-{6-Fluoro-3-[3-(3-methoxy-phenyl)-propionylamino]-1,2,3,4-tetrahydro-9*H*-carbazol-9-yl}-acetic acid | C24H25N204F | 1.03 | 425.21 |
| | | 424.471 | LC-2 | |
| **71** | (3*S*)-{6-Fluoro-3-[3-(2-methoxy-phenyl)-propionylamino]-1,2,3,4-tetrahydro-9*H*-carbazol-9-yl}-acetic acid | C24H25N204F | 1.04 | 425.21 |
| | | 424.471 | LC-2 | |
| **72** | (3*S*)-[3-(2,3-Diphenyl-propionylamino)-6-fluoro-1,2,3,4-tetrahydro-9*H*-carbazol-9-yl]-acetic acid | C29H27N203F | 1.12 | 471.27 |
| | | 470.542 | LC-2 | |
| **73** | (3*S*)-[3-(3,3-Diphenyl-propionylamino)-6-fluoro-1,2,3,4-tetrahydro-9*H*-carbazol-9-yl]-acetic acid | C29H27N203F | 1.1 | 471.20 |
| | | 470.542 | LC-2 | |
| **74** | (3*S*)-{3-[4-(4-Bromo-phenyl)-4-oxo-butyrylamino]-6-fluoro-1,2,3,4-tetrahydro-9*H*-carbazol-9-yl}-acetic acid | C24H22N204BrF | 1.07 | 503.06 |
| | | 501.351 | LC-2 | |
| **75** | (3*S*)-[6-Fluoro-3-(4-oxo-4-phenyl-butyrylamino)-1,2,3,4-tetrahydro-9*H*-carbazol-9-yl]-acetic acid | C24H23N204F | 1.02 | 423.20 |
| | | 422.455 | LC-2 | |
| **76** | (3*S*)-{6-Fluoro-3-[4-(4-methanesulfonyl-phenyl)-4-oxo-butyrylamino]-1,2,3,4-tetrahydro-9*H*-carbazol-9-yl}-acetic acid | C25H25N206FS | 0.97 | 501.19 |
| | | 500.545 | LC-2 | |
| **77** | (3*S*)-[6-Fluoro-3-(2-indan-2-yl-acetylamino)-1,2,3,4-tetrahydro-9*H*-carbazol-9-yl]-acetic acid | C25H25N203F | 1.07 | 421.19 |
| | | 420.483 | LC-2 | |
| **78** | (3*S*)-{6-Fluoro-3-[3-(4-hydroxy-3-methoxy-phenyl)-propionylamino]-1,2,3,4-tetrahydro-9*H*-carbazol-9-yl}-acetic acid | C24H25N205F | 0.98 | 441.00 |
| | | 440.47 | LC-2 | |
| **79** | (3*S*)-{6-Fluoro-3-[3-(4-hydroxy-phenyl)-propionylamino]-1,2,3,4-tetrahydro-9*H*-carbazol-9-yl}-acetic acid | C23H23N204F | 0.97 | 411.02 |
| | | 410.444 | LC-2 | |
| **80** | (3*S*)-{6-Fluoro-3-[3-(3-hydroxy-phenyl)-propionylamino]-1,2,3,4-tetrahydro-9*H*-carbazol-9-yl}-acetic acid | C23H23N204F | 0.99 | 411.02 |
| | | 410.444 | LC-2 | |
| **81** | (3*S*)-{6-Fluoro-3-[3-(2-hydroxy-phenyl)-propionylamino]-1,2,3,4-tetrahydro-9*H*-carbazol-9-yl}-acetic acid | C23H23N204F | 1.02 | 411.02 |
| | | 410.444 | LC-2 | |
| **82** | (3*S*)-{3-[(2,3-Dihydro-1*H*-indole-2-carbonyl)-amino]-6-fluoro-1,2,3,4-tetrahydro-9*H*-carbazol-9-yl}-acetic acid | C23H22N303F | 1.04 | 408.04 |
| | | 407.444 | LC-2 | |
| **83** | (3*S*)-[6-Fluoro-3-(3-1*H*-indol-3-yl-propionylamino)-1,2,3,4-tetrahydro-9*H*-carbazol-9-yl]-acetic acid | C25H24N303F | 1.04 | 434.07 |
| | | 433.482 | LC-2 | |
| **84** | (3*S*)-[3-(3-1*H*-Benzoimidazol-2-yl-propionylamino)-6-fluoro-1,2,3,4-tetrahydro-9*H*-carbazol-9-yl]-acetic acid | C24H23N403F | 0.79 | 435.04 |
| | | 434.47 | LC-2 | |
| **85** | (3*S*)-[3-(3-Benzo[1,3]dioxol-5-yl-propionylamino)-6-fluoro-1,2,3,4-tetrahydro-9*H*-carbazol-9-yl]-acetic acid | C24H23N205F | 1.04 | 438.99 |
| | | 438.454 | LC-2 | |
| **86** | (3*S*)-{6-Fluoro-3-[(1*H*-indole-2-carbonyl)-amino]-1,2,3,4-tetrahydro-9*H*-carbazol-9-yl}-acetic acid | C23H20N3O3F | 1.07 | 406.03 |
| | | 405.428 | LC-2 | |
| **87** | (3*S*)-[6-Fluoro-3-(3-pyridin-3-yl-propionylamino)-1,2,3,4-tetrahydro-9*H*-carbazol-9-yl]-acetic acid | C22H22N303F | 0.7 | 396.11 |
| | | 395.433 | LC-3 | |
| **88** | (3*S*)-{3-[2-(4-*tert*-Butyl-phenyl)-acetylamino]-6-fluoro-1,2,3,4-tetrahydro-9*H*-carbazol-9-yl}-acetic acid | C26H29N203F | 1.02 | 437.17 |
| | | 436.525 | LC-3 | |
| **89** | (3*S*)-{6-Fluoro-3-[2-(4-trifluoromethyl-phenyl)-acetylamino]-1,2,3,4-tetrahydro-9*H*-carbazol-9-yl}-acetic acid | C23H20N2O3F4 | 0.98 | 449.14 |
| | | 448.415 | LC-3 | |
| **90** | (3*S*)-{6-Fluoro-3-[2-(3-trifluoromethyl-phenyl)-acetylamino]-1,2,3,4-tetrahydro-9*H*-carbazol-9-yl}-acetic acid | C23H20N2O3F4 | 0.98 | 449.09 |
| | | 448.415 | LC-3 | |
| **91** | (3*S*)-{6-Fluoro-3-[2-(4-trifluoromethyl-phenoxy)-acetylamino]-1,2,3,4-tetrahydro-9*H*-carbazol-9-yl}-acetic acid | C23H20N2O4F4 | 1.00 | 465.15 |
| | | 464.414 | LC-3 | |
| **92** | (3*S*)-[6-Fluoro-3-(3-naphthalen-2-yl-acryloylamino)-1,2,3,4-tetrahydro-9*H*-carbazol-9-yl]-acetic acid | C27H23N203F | 1.01 | 443.07 |
| | | 442.489 | LC-3 | |
| **93** | (3*S*)-[6-Fluoro-3-(3-naphthalen-2-yl-propionylamino)-1,2,3,4-tetrahydro-9*H*-carbazol-9-yl]-acetic acid | C27H25N203F | 0.99 | 445.15 |
| | | 444.505 | LC-3 | |
| **94** | (3*S*)-{6-Fluoro-3-[methyl-(3-phenyl-propionyl)-amino]-1,2,3,4-tetrahydro-9*H*-carbazol-9-yl}-acetic acid | C24H25N203F | 0.98 | 409.16 |
| | | 408.472 | LC-3 | |
| **95** | (3*S*)-(3-{[2-(4-Chloro-phenyl)-acetyl]-methyl-amino}-6-fluoro-1,2,3,4-tetrahydro-9*H*-carbazol-9-yl)-acetic acid | C23H22N2O3ClF | 0.99 | 429.10 |
| | | 428.89 | LC-3 | |
| **96** | (3*S*)-{6-Fluoro-3-[ethyl-(3-phenyl-propionyl)-amino]-1,2,3,4-tetrahydro-9*H*-carbazol-9-yl}-acetic acid | C25H27N203F | 0.99 | 423.10 |
| | | 422.498 | LC-3 | |
| **97** | (3*S*)-(3-{[2-(4-Chloro-phenyl)-acetyl]-ethyl-amino}-6-fluoro-1,2,3,4-tetrahydro-9*H*-carbazol-9-yl)-acetic acid | C24H24N2O3ClF | 1.01 | 443.06 |
| | | 442.917 | LC-3 | |
| **98** | (3*S*)-{6-Fluoro-3-[propyl-(3-phenyl-propionyl)-amino]-1,2,3,4-tetrahydro-9*H*-carbazol-9-yl}-acetic acid | C26H29N203F | 1.02 | 437.20 |
| | | 436.525 | LC-3 | |
| **99** | (3*S*)-(3-{[2-(4-Chloro-phenyl)-acetyl]-propyl-amino}-6-fluoro-1,2,3,4-tetrahydro-9*H*-carbazol-9-yl)-acetic acid | C25H26N2O3ClF | 1.03 | 457.21 |
| | | 456.943 | LC-3 | |
| **100** | (3*RS*)-(3-Benzyloxycarbonylamino-6-fluoro-1,2,3,4-tetrahydro-9*H*-carbazol-9-yl)-acetic acid | C22H21 N204F | 0.98 | 397.11 |
| | | 396.410 | LC-3 | |
| **101** | (3*RS*)-(3-Benzyloxycarbonylamino-8-chloro-6-fluoro-1,2,3,4-tetrahydro-9*H*-carbazol-9-yl)-acetic acid | C22H20N2O4ClF | 1.01 | 431.05 |
| | | 430.862 | LC-3 | |
| **102** | (3*RS*)-(3-Benzyloxycarbonylamino-8-chloro-5-fluoro-1,2,3,4-tetrahydro-9*H*-carbazol-9-yl)-acetic acid | C22H20N2O4ClF | 1.01 | 431.04 |
| | | 430.862 | LC-3 | |

Listed in Table 1a below are further compounds of Formula I, prepared according to the abovementioned general method with the corresponding compound of Structure 1 as starting material.

**Table 1a**

| **Example** | **Compound of Formula I** | **Formula MW** | **t_{R} [min] LCMS Method** | **MS Data m/z [M+H]⁺** |
|---|---|---|---|---|
| **103** | (3*RS*)-[3-(3-Benzyl-ureido)-6-fluoro-1,2,3,4-tetrahyd ro-9*H*-carbazol-9-yl]-acetic acid | C22H22N303F | 0.92 | 396.17 |
| | | 395.433 | LC-3 | |
| **104** | (3*S*)-[6-Fluoro-3-(3-phenethyl-ureido)-1,2,3,4-tetrahydro-9*H*-carbazol-9-yl]-acetic acid | C23H24N303F | 0.92 | 410.52 |
| | | 409.46 | LC-3 | |
| **105** | (3*RS*)-[3-(3-Benzyl-ureido)-8-chloro-6-fluoro-1,2,3,4-tetrahydro-9*H*-carbazol-9-yl]-acetic acid | C22H21N3O3ClF | 0.95 | 430.07 |
| | | 429.878 | LC-3 | |
| **106** | (3*RS*)-[8-Chloro-6-fluoro-3-(3-phenethyl-ureido)-1,2,3,4-tetrahydro-9*H*-carbazol-9-yl]-acetic acid | C23H23N3O3ClF | 0.97 | 444.07 |
| | | 443.905 | LC-3 | |
| **107** | (3*RS*)-(3-Benzyloxycarbonylamino-6-trifluoromethyl-1,2,3,4-tetrahydro-9*H*-carbazol-9-yl)-acetic acid | C23H21N204F3 | 1.02 | 447.25 |
| | | 446.424 | LC-3 | |
| **108** | (3*RS*)-(3-Benzyloxycarbonylamino-8-bromo-6-fluoro-1,2,3,4-tetrahydro-9*H*-carbazol-9-yl)-acetic acid | C22H20N2O4BrF | 1.02 | 474.98 |
| | | 475.313 | LC-3 | |
| **109** | (3*RS*)-(3-Benzyloxycarbonylamino-6-fluoro-8-vinyl-1,2,3,4-tetrahydro-9*H*-carbazol-9-yl)-acetic acid | C24H23N204F | 1.01 | 423.15 |
| | | 422.455 | LC-3 | |
| **110** | (3*RS*)-(3-Benzyloxycarbonylamino-6-fluoro-8-methanesulfonyl-1,2,3,4-tetrahydro-9*H*-carbazol-9-yl)-acetic acid | C23H23N206FS | 0.95 | 475.15 |
| | | 474.508 | LC-3 | |
| **111** | (3*S*)-(3-Benzyloxycarbonylamino-6-fluoro-8-methyl-1,2,3,4-tetrahydro-9*H*-carbazol-9-yl)-acetic acid | C23H23N204F | 0.99 | 411.14 |
| | | 410.444 | LC-3 | |
| **112** | (3*S*)-(3-Benzyloxycarbonylamino-7-chloro-6-fluoro-1,2,3,4-tetrahydro-9*H*-carbazol-9-yl)-acetic acid | C22H20N2O4ClF | 1.01 | 431.16 |
| | | 430.862 | LC-3 | |
| **113** | (3*S*)-(8-Allyl-3-benzyloxycarbonylamino-6-fluoro-1,2,3,4-tetrahydro-9*H*-carbazol-9-yl)-acetic acid | C25H25N204F | 1.02 | 437.1 |
| | | 436.482 | LC-3 | |
| **114** | (3*R*)-(3-Benzyloxycarbonylamino-8-chloro-1,2,3,4-tetrahydro-9*H*-carbazol-9-yl)-acetic acid | C22H21N2O4Cl | 1 | 413.03 |
| | | 412.872 | LC-3 | |
| **115** | (3*S*)-{3-[3-(2,4-Dimethoxy-phenyl)-propionylamino]-6-fluoro-1,2,3,4-tetrahydro-9*H*-carbazol-9-yl}-acetic acid | C25H27N205F | 0.95 | 455.11 |
| | | 454.496 | LC-3 | |
| **116** | (3*S*)-[6-Fluoro-3-(3-naphthalen-1-yl-propionylamino)-1,2,3,4-tetrahydro-9*H*-carbazol-9-yl]-acetic acid | C27H25N203F | 0.99 | 445.14 |
| | | 444.505 | LC-3 | |
| **117** | (3*RS*)-{6-Fluoro-3-[2-(2-methoxy-phenoxy)-acetylamino]-1,2,3,4-tetrahydro-9*H*-carbazol-9-yl}-acetic acid | C23H23N205F | 0.94 | 427.16 |
| | | 426.443 | LC-3 | |
| **118** | (3*RS*)-{6-Fluoro-3-[3-(2-methylphenyl)-propionylamino]-1,2,3,4-tetrahydro-9*H*-carbazol-9-yl}-acetic acid | C24H25N203F | 0.97 | 409.08 |
| | | 408.472 | LC-3 | |
| **119** | (3*RS*)-{6-Fluoro-3-[3-(3-methylphenyl)-propionylamino]-1,2,3,4-tetrahydro-9*H*-carbazol-9-yl}-acetic acid | C24H25N203F | 0.97 | 409.11 |
| | | 408.472 | LC-3 | |
| **120** | (3*RS*)-{6-Fluoro-3-[3-(3-methoxy-phenyl)-propionylamino]-1,2,3,4-tetrahydro-9*H*-carbazol-9-yl}-acetic acid | C24H25N204F | 0.94 | 425.1 |
| | | 424.471 | LC-3 | |
| **121** | (3*RS*)-{6-Fluoro-3-[2-(3-methoxy-phenoxy)acetylamino]-1,2,3,4-tetrahydro-9*H*-carbazol-9-yl}-acetic acid | C23H23N205F | 0.95 | 427.12 |
| | | 426.443 | LC-3 | |
| **122** | (3*RS*)-{6-Fluoro-3-[2-(2-methylphenoxy)-acetylamino]-1,2,3,4-tetrahydro-9*H*-carbazol-9-yl}-acetic acid | C23H23N204F | 0.98 | 411 |
| | | 410.444 | LC-3 | |
| **123** | (3*S*)-{3-[3-(2,5-Dimethoxy-phenyl)-propionylamino]-6-fluoro-1,2,3,4-tetrahydro-9*H*-carbazol-9-yl}-acetic acid | C25H27N205F | 0.94 | 455.19 |
| | | 454.496 | LC-3 | |
| **124** | (3*S*)-{6-Fluoro-3-[3-(4-trifluoromethyl-phenyl)-propionylamino]-1,2,3,4-tetrahydro-3*H*-carbazol-9-yl}-acetic acid | C24H22N203F4 | 0.99 | 463.15 |
| | | 462.442 | LC-3 | |
| **125** | (3*S*)-{3-[3-(2,6-Dichloro-phenyl)-propionylamino]-6-fluoro-1,2,3,4-tetrahydro-9*H*-carbazol-9-yl}-acetic acid | C23H21N2O3Cl2F | 0.99 | 463.07 |
| | | 463.335 | LC-3 | |
| **126** | (3*S*)-{3-[3-(2,5-Bis-trifluoromethyl-phenyl)-propionylamino]-6-fluoro-1,2,3,4-tetrahydro-9*H*-carbazol-9-yl}-acetic acid | C25H21 N203F7 | 1.04 | 530.97 |
| | | 530.439 | LC-3 | |
| **127** | (3*S*)-{6-Fluoro-3-[3-(4-methylsulfanyl-phenyl)-propionylamino]-1,2,3,4-tetrahydro-9*H*-carbazol-9-yl}-acetic acid | C24H25N203FS | 0.97 | 441.1 |
| | | 440.538 | LC-3 | |
| **128** | (3*S*)-{6-Fluoro-3-[3-(4-iodo-phenyl)-propionylamino]-1,2,3,4-tetrahydro-9*H*-carbazol-9-yl}-acetic acid | C23H22N2O3FI | 0.99 | 521.03 |
| | | 520.337 | LC-3 | |
| **129** | (3*S*)-{6-Fluoro-3-[3-(4-isopropyl-phenyl)-propionylamino]-1,2,3,4-tetrahydro-9*H*-carbazol-9-yl}-acetic acid | C26H29N203F | 1.01 | 437.17 |
| | | 436.525 | LC-3 | |
| **130** | (3*S*)-{6-Fluoro-3-[3-(3-trifluoromethyl-phenyl)-propionylamino]-1,2,3,4-tetra hydro-9*H*-carbazol-9-yl}-acetic acid | C24H22N203F4 | 0.99 | 463.13 |
| | | 462.442 | LC-3 | |
| **131** | (3*S*)-{3-[3-(2,4-Dichloro-phenyl)-propionylamino]-6-fluoro-1,2,3,4-tetrahydro-9*H*-carbazol-9-yl}-acetic acid | C23H21N2O3Cl2F | 1.01 | 463.08 |
| | | 463.335 | LC-3 | |
| **132** | (3*S*)-{6-Fluoro-3-[3-(4-fluoro-phenyl)-propionylamino]-1,2,3,4-tetrahydro-9*H*-carbazol-9-yl}-acetic acid | C23H22N203F2 | 0.95 | 413.08 |
| | | 412.435 | LC-3 | |
| **133** | (3*S*)-{3-[3-(3,5-Bis-trifluoromethyl-phenyl)-propionylamino]-6-fluoro-1,2,3,4-tetrahydro-9*H*-carbazol-9-yl}-acetic acid | C25H21N203F7 | 1.03 | 530.99 |
| | | 530.439 | LC-3 | |
| **134** | (3*S*)-{3-[3-(4-Ethyl-phenyl)-propionylamino]-6-fluoro-1,2,3,4-tetrahydro-9*H*-carbazol-9-yl}-acetic acid | C25H27N203F | 0.99 | 423.12 |
| | | 422.498 | LC-3 | |
| **135** | (3*S*)-{6-Fluoro-3-[3-(3-iodo-phenyl)-propionylamino]-1,2,3,4-tetrahydro-9*H*-carbazol-9-yl}-acetic acid | C23H22N2O3FI | 0.97 | 521.41 |
| | | 520.337 | LC-3 | |
| **136** | (3*S*)-{6-Fluoro-3-[3-(4-methanesulfonyl-phenyl)-propionylamino]-1,2,3,4-tetrahydro-9*H*-carbazol-9-yl}-acetic acid | C24H25N205FS | 0.87 | 473.12 |
| | | 472.536 | LC-3 | |
| **137** | (3*S*)-{3-[3-(2,3-Dimethoxy-phenyl)-propionylamino]-6-fluoro-1,2,3,4-tetrahydro-9*H*-carbazol-9-yl}-acetic acid | C25H27N205F | 0.94 | 455.16 |
| | | 454.496 | LC-3 | |
| **138** | (3*S*)-{3-[3-(2-Bromo-phenyl)-propionylamino]-6-fluoro-1,2,3,4-tetrahydro-9*H*-carbazol-9-yl}-acetic acid | C23H22N203BrF | 0.98 | 474.97 |
| | | 473.341 | LC-3 | |
| **139** | (3*S*)-{6-Fluoro-3-[3-(3-trifluoromethoxy-phenyl)-propionylamino]-1,2,3,4-tetrahydro-9*H*-carbazol-9-yl}-acetic acid | C24H22N204F4 | 1 | 479.11 |
| | | 478.441 | LC-3 | |
| **140** | (3*S*)-{3-[3-(2,4-Dimethyl-phenyl)-propionylamino]-6-fluoro-1,2,3,4-tetrahydro-9*H*-carbazol-9-yl}-acetic acid | C25H27N203F | 0.99 | 423.14 |
| | | 422.498 | LC-3 | |
| **141** | (3*S*)-{3-[3-(3-Bromo-phenyl)-propionylamino]-6-fluoro-1,2,3,4-tetrahydro-9*H*-carbazol-9-yl}-acetic acid | C23H22N203BrF | 0.98 | 474.93 |
| | | 473.341 | LC-3 | |
| **142** | (3*S*)-{3-[3-(3-*tert*-Butoxycarbonylamino-phenyl)-propionylamino]-6-fluoro-1,2,3,4-tetrahydro-9*H*-carbazol-9-yl}-acetic acid | C28H32N305F | 0.98 | 510.17 |
| | | 509.576 | LC-3 | |
| **143** | (3*S*)-{6-Fluoro-3-[(*S*)-3-(4-fluoro-phenyl)-2-phenyl-propionylamino]-1,2,3,4-tetrahydro-9*H*-carbazol-9-yl}-acetic acid | C29H26N203F2 | 1.02 | 489.15 |
| | | 488.532 | LC-3 | |
| **144** | (3*S*)-{6-Fluoro-3-[(*S*)-3-(4-methoxy-phenyl)-2-phenyl-propionylamino]-1,2,3,4-tetrahydro-9*H*-carbazol-9-yl}-acetic acid | C30H29N204F | 1.01 | 501.16 |
| | | 500.568 | LC-3 | |
| **145** | (3*S*)-{6-Fluoro-3-[3-(2-fluoro-phenyl)-propionylamino]-1,2,3,4-tetrahydro-9*H*-carbazol-9-yl}-acetic acid | C23H22N203F2 | 0.95 | 413.09 |
| | | 412.435 | LC-3 | |
| **146** | (3*S*)-(6-Fluoro-3-{[(2*RS*)-1,2,3,4-tetrahydro-naphthalene-2-carbonyl]-amino}-1,2,3,4-tetrahydro-9*H*-carbazol-9-yl)-acetic acid | C25H25N203F | 0.98 | 421.11 |
| | | 420.483 | LC-3 | |
| **147** | (3*S*)-(6-Fluoro-3-{[(2*RS*)-8-methoxy-1,2,3,4-tetrahydro-naphthalene-2-carbonyl]-amino}-1,2,3,4-tetrahydro-9*H*-carbazol-9-yl)-acetic acid | C26H27N204F | 0.98 | 451.08 |
| | | 450.508 | LC-3 | |
| **148** | (3*S*)-(6-Fluoro-3-{(2*RS*)-2-[(4-fluoro-phenylcarbamoyl)-methyl]-3-phenyl-propionylamino}-1,2,3,4-tetrahydro-9*H*-carbazol-9-yl)-acetic acid | C31H29N304F2 | 0.98 | 546.06 |
| | | 545.584 | LC-3 | |
| **149** | (3*S*)-{3-[(2*RS*)-2-Benzyl-3,3-dimethyl-butyrylamino]-6-fluoro-1,2,3,4-tetrahydro-9*H*-carbazol-9-yl}-acetic acid | C27H31N203F | 1.02 | 451.12 |
| | | 450.552 | LC-3 | |
| **150** | (3*S*)-(6-Fluoro-3-{[(2*RS*)-8-methoxy-1,2,3,4-tetrahydro-naphthalene-2-carbonyl]-amino}-1,2,3,4-tetrahydro-9*H*-carbazol-9-yl)-acetic acid | C24H23N204F | 0.95 | 423.11 |
| | | 422.455 | LC-3 | |
| **151** | (3*S*)-{6-Fluoro-3-[3-(3-fluoro-phenyl)-propionylamino]-1,2,3,4-tetrahydro-9*H*-carbazol-9-yl}-acetic acid | C23H22N203F2 | 0.94 | 413.08 |
| | | 412.435 | LC-3 | |
| **152** | (3*S*)-(6-Fluoro-3-{[(2*RS*)-8-methoxy-1,2,3,4-tetrahydro-naphthalene-2-carbonyl]-amino}-1,2,3,4-tetrahydro-9*H*-carbazol-9-yl)-acetic acid | C24H23N203F | 0.96 | 407.1 |
| | | 406.456 | LC-3 | |
| **153** | (3*S*)-{6-Fluoro-3-[(2*R*)-2-methyl-3-phenyl-propionylamino]-1,2,3,4-tetrahydro-9*H*-carbazol-9-yl}-acetic acid | C24H25N203F | 0.95 | 409.11 |
| | | 408.472 | LC-3 | |
| **154** | (3*S*)-[3-(2,2-Dimethyl-3-phenyl-propionylamino)-6-fluoro-1,2,3,4-tetrahydro-9*H*-carbazol-9-yl]-acetic acid | C25H27N203F | 0.99 | 423.19 |
| | | 422.498 | LC-3 | |
| **155** | (3*S*)-[6-Fluoro-3-(3-methyl-3-phenyl-butyrylamino)-1,2,3,4-tetrahydro-9*H*-carbazol-9-yl]-acetic acid | C25H27N203F | 1 | 423.2 |
| | | 422.498 | LC-3 | |
| **156** | (3*S*)-{6-Fluoro-3-[(3*S*)-3-phenyl-butyrylamino]-1,2,3,4-tetrahydro-9*H*-carbazol-9-yl}-acetic acid | C24H25N203F | 0.95 | 409.2 |
| | | 408.472 | LC-3 | |
| **157** | (3*S*)-[3-(2-Benzyloxy-acetylamino)-6-fluoro-1,2,3,4-tetrahydro-9*H*-carbazol-9-yl]-acetic acid | C23H23N204F | 0.94 | 411.06 |
| | | 410.444 | LC-3 | |
| **158** | (3*S*)-[6-Fluoro-3-(4-phenyl-butyrylamino)-1,2,3,4-tetrahydro-9*H*-carbazol-9-yl]-acetic acid | C24H25N203F | 0.96 | 409.16 |
| | | 408.472 | LC-3 | |
| **159** | (3*S*)-{3-[(2*R*,3*R*)-2,3-Dihydroxy-3-(2-methoxy-phenylcarbamoyl)-propionylamino]-6-fluoro-1,2,3,4-tetrahydro-9*H*-carbazol-9-yl}-acetic acid | C25H26N307F | 0.86 | 500.19 |
| | | 499.493 | LC-3 | |
| **160** | (3*RS*)-{8-Chloro-6-fluoro-3-[3-(2-methylphenyl)-propionylamino]-1,2,3,4-tetrahydro-9*H*-carbazol-9-yl}-acetic acid | C24H24N2O3ClF | 1 | 443.11 |
| | | 442.917 | LC-3 | |
| **161** | (3*RS*)-{8-Chloro-6-fluoro-3-[2-(2-methoxy-phenoxy)-acetylamino]-1,2,3,4-tetrahydro-9*H*-carbazol-9-yl}-acetic acid | C23H22N2O5ClF | 0.98 | 461.18 |
| | | 460.888 | LC-3 | |
| **162** | (3*RS*)-{8-Chloro-6-fluoro-3-[3-(3-hydroxy-phenyl)-propionylamino]-1,2,3,4-tetrahydro-9*H*-carbazol-9-yl}-acetic acid | C23H22N2O4ClF | 0.88 | 445.32 |
| | | 444.889 | LC-3 | |
| **163** | (3*RS*)-{8-Chloro-6-fluoro-3-[3-(3-methoxy-phenyl)-propionylamino]-1,2,3,4-tetrahydro-9*H*-carbazol-9-yl}-acetic acid | C24H24N2O4ClF | 0.98 | 459.04 |
| | | 458.916 | LC-3 | |
| **164** | (3*RS*)-[8-Chloro-6-fluoro-3-[3-(3-methylphenyl)-propionylamino]-1,2,3,4-tetrahydro-9*H*-carbazol-9-yl}-acetic acid | C24H24N2O3ClF | 1.01 | 443.01 |
| | | 442.917 | LC-3 | |
| **165** | (3*RS*)-{8-Chloro-6-fluoro-3-[3-(2-hydroxy-phenyl)-propionylamino]-1,2,3,4-tetrahydro-9*H*-carbazol-9-yl}-acetic acid | C23H22N2O4ClF | 0.92 | 486.38 |
| | | 444.889 | LC-3 | |
| **166** | (3*RS*)-[8-Chloro-6-fluoro-3-(3-phenyl-propionylamino)-1,2,3,4-tetrahydro-9*H*-carbazol-9-yl]-acetic acid | C23H22N2O3ClF | 0.98 | 429.13 |
| | | 428.89 | LC-3 | |
| **167** | (3*RS*)-{8-Chloro-6-fluoro-3-[3-(2-methoxy-phenyl)-propionylamino]-1,2,3,4-tetrahydro-9*H*-carbazol-9-yl}-acetic acid | C24H24N2O4ClF | 0.99 | 459.03 |
| | | 458.916 | LC-3 | |
| **168** | (3*RS*)-{8-Chloro-3-[3-(3-chloro-phenyl)-propionylamino]-6-fluoro-1,2,3,4-tetrahydro-9*H*-carbazol-9-yl}-acetic acid | C23H21N2O3Cl2F | 1.01 | 463.11 |
| | | 463.335 | LC-3 | |
| **169** | (3*RS*)-[8-Chloro-6-fluoro-3-(3-1*H*-indol-3-yl-propionylamino)-1,2,3,4-tetrahydro-9*H*-carbazol-9-yl]-acetic acid | C25H23N3O3ClF | 0.97 | 468.11 |
| | | 467.927 | LC-3 | |
| **170** | (3*RS*)-{8-Chloro-3-[2-(2-chloro-phenoxy)-acetylamino]-6-fluoro-1,2,3,4-tetrahydro-9*H*-carbazol-9-yl}-acetic acid | C22H19N2O4Cl2F | 1.01 | 465.09 |
| | | 465.307 | LC-3 | |
| **171** | (3*RS*)-{8-Chloro-6-fluoro-3-[2-(2-methylphenyl)-oxy-acetylamino]-1,2,3,4-tetrahydro-9*H*-carbazol-9-yl}-acetic acid | C23H22N2O4ClF | 1.02 | 445.11 |
| | | 444.889 | LC-3 | |
| **172** | (3*RS*)-[3-(3-Benzo[1,3]dioxol-5-yl-propionylamino)-8-chloro-6-fluoro-1,2,3,4-tetrahydro-9*H*-carbazol-9-yl]-acetic acid | C24H22N2O5ClF | 0.93 | 473.17 |
| | | 472.899 | LC-3 | |
| **173** | (3*RS*)-{8-Chloro-6-fluoro-3-[2-(3-methoxy-phenoxy)-acetylamino]-1,2,3,4-tetrahydro-9*H*-carbazol-9-yl}-acetic acid | C23H22N2O5ClF | 0.99 | 461.09 |
| | | 460.888 | LC-3 | |
| **174** | (3*RS*)-{8-Chloro-3-[2-(3-chloro-phenoxy)-acetylamino]-6-fluoro-1,2,3,4-tetrahydro-9*H*-carbazol-9-yl}-acetic acid | C22H19N2O4Cl2F | 1.02 | 465 |
| | | 465.307 | LC-3 | |
| **175** | (3*RS*)-{8-Chloro-3-[3-(2-chloro-phenyl)-propionylamino]-6-fluoro-1,2,3,4-tetrahydro-9*H*-carbazol-9-yl}-acetic acid | C23H21N2O3Cl2F | 1.01 | 463.07 |
| | | 463.335 | LC-3 | |
| **176** | (3*RS*)-[8-Chloro-6-fluoro-3-(2-indan-2-yl-acetylamino)-1,2,3,4-tetrahydro-9*H*-carbazol-9-yl]-acetic acid | C25H24N2O3ClF | 1.01 | 455.16 |
| | | 454.928 | LC-3 | |
| **177** | (3*S*)-[6-Fluoro-3-(1-methyl-3-phenethyl-ureido)-1,2,3,4-tetrahydro-9*H*-carbazol-9-yl]-acetic acid | C24H26N3O3F | 0.98 | 429.13 |
| | | 423.486 | LC-3 | |
| **178** | (3*S*)-{3-[3-(2-Chloro-benzyl)-1-methyl-ureido]-6-fluoro-1,2,3,4-tetrahydro-9*H*-carbazol-9-yl}-acetic acid | C23H23N3O3ClF | 0.97 | 444.16 |
| | | 443.905 | LC-3 | |
| **179** | (3*S*)-[3-(3-Benzyl-1-methyl-ureido)-6-fluoro-1,2,3,4-tetrahydro-9*H*-carbazol-9-yl]-acetic acid | C23H24N3O3F | 0.94 | 410.12 |
| | | 409.46 | LC-3 | |
| **180** | (3*S*)-[3-(Benzyloxycarbonyl-methyl-amino)-6-fluoro-1,2,3,4-tetrahydro-9*H*-carbazol-9-yl]-acetic acid | C23H23N2O4F | 1.01 | 411.07 |
| | | 410.444 | LC-3 | |
| **181** | {(3*S*)-3-[(2-Chloro-benzyloxycarbonyl)-methyl-amino]-6-fluoro-1,2,3,4-tetrahydro-9*H*-carbazol-9-yl}-acetic acid | C23H22N2O4ClF | 1.03 | 445.15 |
| | | 444.889 | LC-3 | |
| **182** | (3*S*)-(6-Fluoro-3-{[2-(4-methoxy-phenyl)-acetyl]-methyl-amino}-1,2,3,4-tetrahydro-9*H*-carbazol-9-yl)-acetic acid | C24H25N2O4F | 0.95 | 425.19 |
| | | 424.471 | LC-3 | |
| **183** | (3*S*)-(6-Fluoro-3-{methyl-[2-(4-methylphenyl)-acetyl]-amino}-1,2,3,4-tetrahydro-9*H*-carbazol-9-yl)-acetic acid | C24H25N2O3F | 0.98 | 409.18 |
| | | 408.472 | LC-3 | |
| **184** | (3*S*)-(6-Fluoro-3-{[2-(2-methoxy-phenyl)-acetyl]-methyl-amino}-1,2,3,4-tetrahydro-9*H*-carbazol-9-yl)-acetic acid | C24H25N2O4F | 0.96 | 425.18 |
| | | 424.471 | LC-3 | |
| **185** | (3*S*)-{6-Fluoro-3-[(2-indan-2-yl-acetyl)-methyl-amino]-1,2,3,4-tetrahydro-9*H*-carbazol-9-yl}-acetic acid | C26H27N2O3F | 1.01 | 435.16 |
| | | 434.509 | LC-3 | |
| **186** | (3*S*)-(3-{[2-(3-Chloro-phenyl)-acetyl]-methyl-amino}-6-fluoro-1,2,3,4-tetrahydro-9*H*-carbazol-9-yl)-acetic acid | C23H22N2O3ClF | 0.99 | 429.13 |
| | | 428.89 | LC-3 | |
| **187** | (3*S*)-(6-Fluoro-3-{methyl-[2-(3-methylphenyl)-acetyl]-aminol-1,2,3,4-tetrahydro-9*H*-carbazol-9-yl)-acetic acid | C24H25N2O3F | 0.98 | 409.1 |
| | | 408.472 | LC-3 | |
| **188** | (3*S*)-(6-Fluoro-3-{[2-(3-methoxy-phenyl)-acetyl]-methyl-amino}-1,2,3,4-tetrahydro-9*H*-carbazol-9-yl)-acetic acid | C24H25N2O4F | 0.95 | 425.08 |
| | | 424.471 | LC-3 | |
| **189** | (3*S*)-(3-{[2-(2-Chloro-phenoxy)-acetyl]-methyl-amino}-6-fluoro-1,2,3,4-tetrahydro-9*H*-carbazol-9-yl)-acetic acid | C23H22N2O4ClF | 0.97 | 445.38 |
| | | 444.889 | LC-3 | |
| **190** | (3*S*)-(3-{[2-(4-Chloro-phenoxy)-acetyl]-methyl-amino}-6-fluoro-1,2,3,4-tetrahydro-9*H*-carbazol-9-yl)-acetic acid | C23H22N2O4ClF | 0.98 | 445.39 |
| | | 444.889 | LC-3 | |
| **191** | (3*S*)-(6-Fluoro-3-{[3-(3-methoxy-phenyl)-propionyl]-methyl-amino}-1,2,3,4-tetrahydro-9*H*-carbazol-9-yl)-acetic acid | C25H27N2O4F | 0.96 | 439.47 |
| | | 438.497 | LC-3 | |
| **192** | (3*S*)-(6-Fluoro-3-{methyl-[2-(2-methylphenyl)-acetyl]-amino}-1,2,3,4-tetrahydro-9*H*-carbazol-9-yl)-acetic acid | C24H25N2O3F | 0.96 | 409.48 |
| | | 408.472 | LC-3 | |
| **193** | (3*S*)-{3-[(3,3-Diphenyl-propionyl)-methyl-amino]-6-fluoro-1,2,3,4-tetrahydro-9*H*-carbazol-9-yl}-acetic acid | C30H29N2O3F | 1.02 | 485.52 |
| | | 484.569 | LC-3 | |
| **194** | (3*S*)-(6-Fluoro-3-{[3-(2-methoxy-phenyl)-propionyl]-methyl-amino}-1,2,3,4-tetrahydro-9*H*-carbazol-9-yl)-acetic acid | C25H27N2O4F | 0.97 | 439.45 |
| | | 438.497 | LC-3 | |
| **195** | (3*S*)-{6-Fluoro-3-[(3-1*H*-indol-3-yl-propionyl)-methyl-amino]-1,2,3,4-tetrahydro-9*H*-carbazol-9-yl}-acetic acid | C26H26N3O3F | 0.94 | 448.44 |
| | | 447.508 | LC-3 | |
| **196** | (3*S*)-{3-[(2-Benzyloxy-acetyl)-methyl-amino]-6-fluoro-1,2,3,4-tetrahydro-9*H*-carbazol-9-yl}-acetic acid | C24H25N2O4F | 0.94 | 425.44 |
| | | 424.471 | LC-3 | |
| **197** | (3*S*)-{3-[(2,3-Diphenyl-propionyl)-methyl-amino]-6-fluoro-1,2,3,4-tetrahydro-9*H*-carbazol-9-yl}-acetic acid | C30H29N2O3F | 1.04 | 485.53 |
| | | 484.569 | LC-3 | |
| **198** | (3*S*)-{6-Fluoro-3-[[3-(2-methoxy-phenyl)-propionyl]-(3-phenyl-propyl)-amino]-1,2,3,4-tetrahydro-9*H*-carbazol-9-yl}-acetic acid | C33H35N2O4F | 1.07 | 543.18 |
| | | 542.649 | LC-3 | |
| **199** | (3*S*)-{3-[Acetyl-(3-phenyl-propyl)-amino]-6-fluoro-1,2,3,4-tetrahydro-9*H*-carbazol-9-yl}-acetic acid | C25H27N2O3F | 1.01 | 423.14 |
| | | 422.498 | LC-3 | |
| **200** | (3*S*)-{3-[3-Benzyl-(1-cyclopropylmethyl)-ureido]-6-fluoro-1,2,3,4-tetrahydro-9*H*-carbazol-9-yl}-acetic acid | C26H28N3O3F | 0.99 | 450.2 |
| | | 449.524 | LC-3 | |
| **201** | (3*S*)-[3-(Benzyloxycarbonyl-cyclopropylmethyl-amino)-6-fluoro-1,2,3,4-tetrahydro-9*H*-carbazol-9-yl]-acetic acid | C26H27N2O4F | 1.05 | 451.15 |
| | | 450.508 | LC-3 | |
| **202** | (3*S*)-{3-[Cyclopropylmethyl-(3-phenyl-propionyl)-amino]-6-fluoro-1,2,3,4-tetrahydro-9*H*-carbazol-9-yl}-acetic acid | C27H29N2O3F | 1.02 | 449.25 |
| | | 448.536 | LC-3 | |
| **203** | (3*S*)-{3-[Cyclopropylmethyl-((*S*)-2-methyl-3-phenyl-propionyl)-amino]-6-fluoro-1,2,3,4-tetrahydro-9*H*-carbazol-9-yl}-acetic acid | C28H31N2O3F | 1.03 | 463.27 |
| | | 462.563 | LC-3 | |
| **204** | (3*S*)-(3-{Cyclopropylmethyl-[3-(2-methoxy-phenyl)-propionyl]-amino}-6-fluoro-1,2,3,4-tetrahydro-9*H*-carbazol-9-yl)-acetic acid | C28H31N2O4F | 1.02 | 479.28 |
| | | 478.562 | LC-3 | |
| **205** | (3*S*)-(3-{[2-(3-Chloro-phenoxy)-acetyl]-cyclopropylmethyl-amino}-6-fluoro-1,2,3,4-tetrahydro-9H-carbazol-9-yl)-acetic acid | C26H26N2O4ClF | 1.03 | 485.2 |
| | | 484.953 | LC-3 | |
| **206** | (3*S*)-{3-[Cyclopropylmethyl-(3,3-diphenyl-propionyl)-amino]-6-fluoro-1,2,3,4-tetrahydro-9*H*-carbazol-9-yl}-acetic acid | C33H33N2O3F | 1.07 | 525.26 |
| | | 524.634 | LC-3 | |
| **207** | (3*S*)-{3-[Cyclopropylmethyl-(2-naphthalen-1-yl-acetyl)-amino]-6-fluoro-1,2,3,4-tetrahydro-9*H*-carbazol-9-yl}-acetic acid | C30H29N2O3F | 1.04 | 485.26 |
| | | 484.569 | LC-3 | |
| **208** | (3*S*)-(3-{Benzyloxycarbonyl-[2-(4-trifluoromethyl-phenoxy)-ethyl]-amino}-6-fluoro-1,2,3,4-tetrahydro-9*H*-carbazol-9-yl)-acetic acid | C31H28N2O5F4 | 1.15 | 585.14 |
| | | 584.564 | LC-3 | |
| **209** | (3*S*)-(3-{Acetyl-[2-(4-trifluoromethyl-phenoxy)-ethyl]-amino}-6-fluoro-1,2,3,4-tetrahydro-9*H*-carbazol-9-yl)-acetic acid | C25H24N2O4F4 | 1.07 | 493.17 |
| | | 492.468 | LC-3 | |
| **210** | (3*S*)-(6-Fluoro-3-{propionyl-[2-(4-trifluoromethyl-phenoxy)-ethyl]-amino}-1,2,3,4-tetrahydro-9*H*-carbazol-9-yl)-acetic acid | C26H26N2O4F4 | 1.08 | 507.18 |
| | | 506.494 | LC-3 | |
| **211** | (3*S*)-(6-Fluoro-3-{(3-phenyl-propionyl)-[2-(4-trifluoromethyl-phenoxy)-ethyl]-amino}-1,2,3,4-tetrahydro-9*H*-carbazol-9-yl)-acetic acid | C32H30N2O4F4 | 1.13 | 583.14 |
| | | 582.592 | LC-3 | |
| **212** | (3*S*)-(6-Fluoro-3-{[3-(2-methoxy-phenyl)-propionyl]-[2-(4-trifluoromethyl-phenoxy)-ethyl]-amino}-1,2,3,4-tetrahydro-9*H*-carbazol-9-yl)-acetic acid | C33H32N2O5F4 | 1.13 | 613.26 |
| | | 612.618 | LC-3 | |
| **213** | (3*S*)-{6-Fluoro-3-[(2-phenoxy-ethyl)-(3-phenyl-propionyl)-amino]-1,2,3,4-tetrahydro-9*H*-carbazol-9-yl}-acetic acid | C31H31N2O4F | 1.06 | 515.17 |
| | | 514.595 | LC-3 | |
| **214** | (3*S*)-{6-Fluoro-3-[((S)-2-methyl-3-phenyl-propionyl)-(2-phenoxy-ethyl)-amino]-1,2,3,4-tetrahydro-9*H*-carbazol-9-yl}-acetic acid | C32H33N2O4F | 1.07 | 529.26 |
| | | 528.622 | LC-3 | |
| **215** | (3*S*)-{6-Fluoro-3-[[3-(2-methoxy-phenyl)-propionyl]-(2-phenoxy-ethyl)-amino]-1,2,3,4-tetrahydro-9*H*-carbazol-9-yl}-acetic acid | C32H33N2O5F | 1.06 | 545.25 |
| | | 544.621 | LC-3 | |
| **216** | (3*S*)-{3-[Acetyl-(2-phenoxy-ethyl)-amino]-6-fluoro-1,2,3,4-tetrahydro-9*H*-carbazol-9-yl}-acetic acid | C24H25N2O4F | 0.98 | 425.19 |
| | | 424.471 | LC-3 | |
| **217** | (3*S*)-{3-[3-Benzyl-1-(2-methoxy-ethyl)-ureido]-6-fluoro-1,2,3,4-tetrahydro-9*H*-carbazol-9-yl}-acetic acid | C25H28N3O4F | 0.97 | 454.26 |
| | | 453.512 | LC-3 | |
| **218** | (3*S*)-{3-[Benzyloxycarbonyl-(2-methoxy-ethyl)-amino]-6-fluoro-1,2,3,4-tetrahydro-9*H*-carbazol-9-yl}-acetic acid | C25H27N205F | 0.97 | 454.26 |
| | | 454.496 | LC-3 | |
| **219** | (3*S*)-{6-Fluoro-3-[(2-methoxy-ethyl)-(3-phenyl-propionyl)-amino]-1,2,3,4-tetrahydro-9*H*-carbazol-9-yl}-acetic acid | C26H29N204F | 0.98 | 453.25 |
| | | 452.524 | LC-3 | |
| **220** | (3*S*)-{6-Fluoro-3-[(2-methoxy-ethyl)-((S)-2-methyl-3-phenyl-propionyl)-amino]-1,2,3,4-tetrahydro-9*H*-carbazol-9-yl}-acetic acid | C27H31N204F | 1 | 467.18 |
| | | 466.551 | LC-3 | |
| **221** | (3*S*)-(6-Fluoro-3-{(2-methoxy-ethyl)-[3-(2-methoxy-phenyl)-propionyl]-amino}-1,2,3,4-tetrahydro-9*H*-carbazol-9-yl)-acetic acid | C27H31 N205F | 0.98 | 483.19 |
| | | 482.55 | LC-3 | |
| **222** | (3*S*)-{3-[[2-(3-Chloro-phenoxy)-acetyl]-(2-methoxy-ethyl)-amino]-6-fluoro-1,2,3,4-tetrahydro-9*H*-carbazol-9-yl}-acetic acid | C25H26N2O5ClF | 1 | 489.17 |
| | | 488.941 | LC-3 | |
| **223** | (3*S*)-{3-[(3,3-Diphenyl-propionyl)-(2-methoxy-ethyl)-amino]-6-fluoro-1,2,3,4-tetrahydro-9*H*-carbazol-9-yl}-acetic acid | C32H33N204F | 1.04 | 529.28 |
| | | 528.622 | LC-3 | |
| **224** | (3*S*)-{6-Fluoro-3-[(2-methoxy-ethyl)-(2-naphthalen-1-yl-acetyl)-amino]-1,2,3,4-tetrahydro-9*H*-carbazol-9-yl}-acetic acid | C29H29N204F | 1.01 | 489.24 |
| | | 488.557 | LC-3 | |
| **225** | (3*S*)-(6-Fluoro-3-{[(2S)-2-methyl-3-phenyl-propionyl]-phenethyl-amino}-1,2,3,4-tetrahydro-9*H*-carbazol-9-yl)-acetic acid | C32H33N203F | 1.07 | 513.27 |
| | | 512.623 | LC-3 | |
| **226** | (3*S*)-(6-Fluoro-3-{[3-(2-methoxy-phenyl)-propionyl]-phenethyl-amino}-1,2,3,4-tetrahydro-9*H*-carbazol-9-yl)-acetic acid | C32H33N204F | 1.06 | 529.27 |
| | | 528.622 | LC-3 | |
| **227** | (3*S*)-[3-(Acetyl-phenethyl-amino)-6-fluoro-1,2,3,4-tetrahydro-9*H*-carbazol-9-yl]-acetic acid | C24H25N203F | 0.98 | 409.16 |
| | | 408.472 | LC-3 | |
| **228** | (3*S*)-{6-Fluoro-3-[(2-naphthalen-1-yl-acetyl)-phenethyl-amino]-1,2,3,4-tetrahydro-9*H*-carbazol-9-yl}-acetic acid | C34H31 N203F | 1.08 | 535.27 |
| | | 534.629 | LC-3 | |
| **229** | (3*S*)-{6-Fluoro-3-[phenethyl-(3-phenyl-propionyl)-amino]-1,2,3,4-tetrahydro-9*H*-carbazol-9-yl}-acetic acid | C31H31N2O3F | 1.06 | 499.2 |
| | | 498.596 | LC-3 | |
| **230** | (3*S*)-[3-(3-Benzyl-1-naphthalen-1-ylmethyl-ureido)-6-fluoro-1,2,3,4-tetrahydro-9*H*-carbazol-9-yl]-acetic acid | C33H30N303F | 1.05 | 536.25 |
| | | 535.617 | LC-3 | |
| **231** | (3*S*)-[3-(Benzyloxycarbonyl-naphthalen-1-ylmethyl-amino)-6-fluoro-1,2,3,4-tetrahydro-9*H*-carbazol-9-yl]-acetic acid | C33H29N204F | 1.09 | 537.24 |
| | | 536.601 | LC-3 | |
| **232** | (3*S*)-{6-Fluoro-3-[naphthalen-1-ylmethyl-(3-phenyl-propionyl)-amino]-1,2,3,4-tetrahydro-9*H*-carbazol-9-yl}-acetic acid | C34H31 N203F | 1.07 | 535.25 |
| | | 534.629 | LC-3 | |
| **233** | (3*S*)-{6-Fluoro-3-[((*S*)-2-methyl-3-phenyl-propionyl)-naphthalen-1-ylmethyl-amino]-1,2,3,4-tetrahydro-9*H*-carbazol-9-yl}-acetic acid | C35H33N203F | 1.09 | 549.26 |
| | | 548.656 | LC-3 | |
| **234** | (3*S*)-(6-Fluoro-3-{[3-(2-methoxy-phenyl)-propionyl]-naphthalen-1-ylmethyl-amino}-1,2,3,4-tetrahydro-9*H*-carbazol-9-yl)-acetic acid | C35H33N204F | 1.07 | 565.26 |
| | | 564.655 | LC-3 | |
| **235** | (3*S*)-{3-[(3,3-Diphenyl-propionyl)-naphthalen-1-ylmethyl-amino]-6-fluoro-1,2,3,4-tetrahydro-9*H*-carbazol-9-yl}-acetic acid | C40H35N203F | 1.11 | 611.21 |
| | | 610.727 | LC-3 | |
| **236** | (3*S*)-[3-(Acetyl-naphthalen-1-ylmethyl-amino)-6-fluoro-1,2,3,4-tetrahydro-9*H*-carbazol-9-yl]-acetic acid | C27H25N203F | 1 | 445.24 |
| | | 444.505 | LC-3 | |
| **237** | (3*S*)-[6-Fluoro-3-(naphthalen-1-ylmethyl-propionyl-amino)-1,2,3,4-tetrahydro-9*H*-carbazol-9-yl]-acetic acid | C28H27N203F | 1.01 | 459.15 |
| | | 458.531 | LC-3 | |
| **238** | (3*S*)-{3-[(*RS*)-2-Benzyl-3-(2-methylphenyl)-carbamoyl-propionylamino]-6-fluoro-1,2,3,4-tetrahydro-9*H*-carbazol-9-yl}-acetic acid | C32H32N304F | 0.97 | 542.22 |
| | | 541.621 | LC-3 | |
| **239** | (3*S*)-{3-[(*RS*)-2-Benzyl-3-(3-methoxy-phenylcarbamoyl)-propionylamino]-6-fluoro-1,2,3,4-tetrahydro-9*H*-carbazol-9-yl}-acetic acid | C32H32N305F | 0.97 | 558.24 |
| | | 557.620 | LC-3 | |
| **240** | (3*S*)-{3-[(*RS*)-2-Benzyl-3-(4-chlora-phenylcarbamoyl)-propionylamino]-6-fluoro-1,2,3,4-tetrahydro-9*H*-carbazol-9-yl)-acetic acid | C31H29N3O4ClF | 1.01 | 562.09 |
| | | 562.039 | LC-3 | |
| **241** | (3*S*)-{3-[(*RS*)-2-Benzyl-3-(4-fluoro-benzylcarbamoyl)-propionylamino]-6-fluoro-1,2,3,4-tetrahydro-9*H*-carbazol-9-yl}-acetic acid | C32H31N304F2 | 0.96 | 560.21 |
| | | 559.611 | LC-3 | |
| **242** | [(3*S*)-3-((*RS*)-2-Benzyl-3-propylcarbamoyl-propionylamino)-6-fluoro-1,2,3,4-tetrahydro-9*H*-carbazol-9-yl]-acetic acid | C28H32N304F | 0.91 | 494.25 |
| | | 493.577 | LC-3 | |
| **243** | (3*S*)-[6-Fluoro-3-(3-thiophen-2-yl-propionylamino)-1,2,3,4-tetrahydro-9*H*-carbazol-9-yl]-acetic acid | C21H21FN203S | 0.93 | 400.61 |
| | | 400.47 | LC-3 | |
| **244** | (3*S*)-{3-[3-(3-Chloro-isoxazol-5-yl)-propionylamino]-6-fluoro-1,2,3,4-tetrahydro-9*H*-carbazol-9-yl}-acetic acid | C20H19ClFN3O4 | 0.91 | 420.09 |
| | | 419.83 | LC-3 | |
| **245** | (3*S*)-[6-Fluoro-3-(3-pyrimidin-2-yl-propionylamino)-1,2,3,4-tetrahydro-9*H*-carbazol-9-yl]-acetic acid | C21H21FN4O3 | 0.8 | 397.11 |
| | | 396.41 | LC-3 | |
| **246** | (3*S*)-{6-Fluoro-3-[3-phenyl-4-([1,3,4]thiadiazol-2-ylcarbamoyl)-butyrylamino]-1,2,3,4-tetrahydro-9*H*-carbazol-9-yl}-acetic acid | C27H26FN504S | 0.87 | 536.14 |
| | | 535.59 | LC-3 | |
| **247** | (3*S*)-[3-(1,3-Dibenzyl-ureido)-6-fluoro-1,2,3,4-tetrahydro-9*H*-carbazol-9-yl]-acetic acid | C29H28FN303 | 1.02 | 486.22 |
| | | 485.55 | LC.3 | |
| **248** | (3*S*)-(3-{Acetyl-[2-(2-fluoro-phenyl)-ethyl]-amino}-6-fluoro-1,2,3,4-tetrahydro-9*H*-carbazol-9-yl)-acetic acid | C24H24F2N203 | 0.99 | 427.07 |
| | | 426.46 | LC-3 | |
| **249** | (3*S*)-(3-{Acetyl-[2-(3-fluoro-phenyl)-ethyl]-amino}-6-fluoro-1,2,3,4-tetrahydro-9*H*-carbazol-9-yl)-acetic acid | C24H24F2N203 | 0.99 | 427.07 |
| | | 426.46 | LC-3 | |
| **250** | (3*S*)-[3-(3-Benzyl-1-cyclohexylmethyl-ureido)-6-fluoro-1,2,3,4-tetrahyd ro-9*H*-carbazol-9-yl]-acetic acid | C29H34N303F | 1.06 | 492.26 |
| | | 491.605 | LC-3 | |
| **251** | (3*S*)-(3-{Cyclohexylmethyl-[3-(2-methoxy-phenyl)-propionyl]-amino}-6-fluoro-1,2,3,4-tetrahydro-9*H*-carbazol-9-yl)-acetic acid | C31H37N204F | 1.10 | 521.25 |
| | | 520.643 | LC-3 | |

### Synthesis of Precursors and Intermediates:

### General Methods for the synthesis of intermediates of Structure 1:

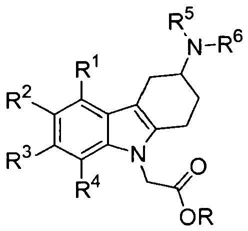

Structure **1,** wherein R represents C₁₋₄-alkyl

### 1) N-Carbamoylation of an intermediate of Structure 2a or 2b:

The appropriate isocyanate (0.132 mmol) and a catalytical amount of DMAP are added to a 0°C cold solution of a hydrochloride of the appropriate intermediate of Structure **2a** or **2b** (0.11 mmol) and Et₃N (0.034 ml, 0.242 mmol) in DCM (2 ml). The reaction mixture is stirred at rt overnight. Then, a 1:4 mixture (1 ml) of sat. NaHC₃ solution and H₂O is added. After phase separation, the aq. layer is extracted three times with DCM. The combined org. phases are washed with 10% citric acid. The solvent is evaporated and the pure [3-ureido-1,2,3,4-tetrahydro-9H-carbazol-9-yl]-acetate derivative of Structure **1** is obtained by preparative HPLC with 8 to 98% yield.

Listed in Table 2 below are ethyl [3-ureido-1,2,3,4-tetrahydro-9*H*-carbazol-9-yl]-acetate derivatives of Structure **1,** prepared according to the above mentioned method, with the corresponding compound of Structure **2a** or **2b** as starting material.

**Table 2**

| **Intermediates of Structure 1:** **Ethyl [3-ureido-1,2,3,4-tetrahydro-9*H*-carbazol-9-yl]-acetate derivatives** | **Formula MW** | **t_{R} [min] LC-MS Method** | **MS Data m/z [M₊H]⁺** |
|---|---|---|---|
| Ethyl (3*R*)-[3-(3-butyl-ureido)-1,2,3,4-tetrahydro-9*H*-carbazol-9-yl]-acetate | C21H29N303 | 1.1 | 372.05 |
| | 371.47 | LC-2 | |
| Ethyl (3*R*)-[3-(3-benzyl-ureido)-1,2,3,4-tetrahydro-9*H*-carbazol-9-yl]-acetate | C24H27N303 | 1.99 | 406.06 |
| | 405.496 | LC-1 | |
| Ethyl (3*R*)-[3-(3-phenethyl-ureido)-1,2,3,4-tetrahydro-9*H*-carbazol-9-yl]-acetate | C25H29N303 | 2.15 | 420.04 |
| | 419.523 | LC-1 | |
| Ethyl (3*R*)-[3-(3-naphthalen-1-yl-ureido)-1,2,3,4-tetrahydro-9*H*-carbazol-9-yl]-acetate | C27H27N303 | 1.19 | 441.95 |
| | 441.52 | LC-2 | |
| Ethyl (3*R*)-[3-(3-phenylsulfonyl-ureido)-1,2,3,4-tetrahydro-9*H*-carbazol-9-yl]-acetate | C23H25N305S | 1.96 | 454.1 |
| | 455.534 | LC-1 | |
| Ethyl (3*S*)-[3-(3-butyl-ureido)-1,2,3,4-tetrahydro-9*H*-carbazol-9-yl]-acetate | C21H29N303 | 1.08 | 372.16 |
| | 371.479 | LC-2 | |
| Ethyl (3*S*)-[3-(3-benzyl-ureido)-1,2,3,4-tetrahydro-9*H*-carbazol-9-yl]-acetate | C24H27N303 | 1.1 | 406.16 |
| | 405.496 | LC-2 | |
| Ethyl(3*S*)-[3-(3-phenethyl-ureido)-1,2,3,4-tetrahydro-9*H*-carbazol-9-yl]-acetate | C25H29N303 | 1.12 | 420.15 |
| | 419.523 | LC-2 | |
| Ethyl (3*S*)-[3-(3-phenylsulfonyl-ureido)-1,2,3,4-tetrahydro-9*H*-carbazol-9-yl]-acetate | C23H25N305S | 1.09 | 454.16 |
| | 455.534 | LC-2 | |
| Ethyl (3*S*)-[3-(3-phenyl-ureido)-1,2,3,4-tetrahydro-9*H*-carbazol-9-yl]-acetate | C23H25N303 | 1.11 | 392.17 |
| | 391.47 | LC-2 | |

Listed in Table 2a below are further ethyl [3-ureido-1,2,3,4-tetrahydro-9*H*-carbazol-9-yl]-acetate derivatives of Structure **1,** prepared according to the above mentioned method, with the corresponding compound of Structure **2a** or **2b** as starting material.

**Table 2a**

| **Intermediates of Structure 1:** **Ethyl [3-ureido-1,2,3,4-tetrahydro-9*H*-carbazol-9-yl]-acetate derivatives** | **Formula MW** |
|---|---|
| Ethyl (3*RS*)-[3-(3-benzyl-ureido)-6-fluoro-1,2,3,4-tetrahydro-9*H*-carbazol-9-yl]-acetate | C25H28FN303 |
| | 437.51 |
| | |
| Ethyl (3*S*)-[6-fluoro-3-(3-phenethyl-ureido)-1,2,3,4-tetrahydro-9*H*-carbazol-9-yl]-acetate | C25H28N303F |
| | 437.51 |
| Ethyl (3*RS*)-[3-(3-benzyl-ureido)-8-chloro-6-fluoro-1,2,3,4-tetrahydro-9*H*-carbazol-9-yl]-acetate | C24H25ClFN3O3 |
| | 457.93 |
| | |
| Ethyl (3*RS*)-[8-chloro-6-fluoro-3-(3-phenethyl-ureido)-1,2,3,4-tetrahydro-9*H*-carbazol-9-yl]-acetate | C25H27ClFN3O3 |
| | 471.95 |
| | |
| Ethyl (3*S*)-[6-fluoro-3-(1-methyl-3-phenethyl-ureido)-1,2,3,4-tetrahydro-9*H*-carbazol-9-yl]-acetate | C26H30FN303 |
| | 451.53 |
| | |
| Ethyl (3*S*)-{3-[3-(2-chloro-benzyl)-1-methyl-ureido]-6-fluoro-1,2,3,4-tetrahydro-9*H*-carbazol-9-yl}-acetate | C25H27ClFN3O3 |
| | 471.95 |
| | |
| Ethyl (3*S*)-[3-(3-benzyl-1-methyl-ureido)-6-fluoro-1,2,3,4-tetrahydro-9*H*-carbazol-9-yl]-acetate | C25H28FN303 |
| | 437.51 |
| | |
| Ethyl (3*S*)-{3-[3-benzyl-(1-cyclopropylmethyl)-ureido]-6-fluoro-1,2,3,4-tetrahydro-9*H*-carbazol-9-yl}-acetate | C28H32FN303 |
| | 477.57 |
| | |
| Ethyl (3*S*)-{3-[3-benzyl-1-(2-methoxy-ethyl)-ureido]-6-fluoro-1,2,3,4-tetrahydro-9*H*-carbazol-9-yl}-acetate | C27H32FN304 |
| | 481.56 |
| | |
| Ethyl (3*S*)-[3-(3-benzyl-1-cyclohexylmethyl-ureido)-6-fluoro-1,2,3,4-tetrahydro-9*H*-carbazol-9-yl]-acetate | C31H37N3O3F |
| | 519.655 |
| | |
| Ethyl (3*S*)-[3-(1,3-Dibenzyl-ureido)-6-fluoro-1,2,3,4-tetrahydro-9*H*-carbazol-9-yl]-acetate | C29H28FN303 |
| | 513.60 |

### 2) Reaction of intermediates of Structure 2a or 2b with chloroformates:

The appropriate chloroformate (neat) and a catalytical amount of DMAP is added to a 0°C cold solution of a hydrochloride of the appropriate intermediate of Structure **2a** or **2b** (0.132 mmol) and Et₃N (0.034 ml, 0.242 mmol) in DCM (2 ml). The reaction mixture is stirred at rt overnight. Then, a 1:4 mixture (1 ml) of sat. NaHCO₃ solution and H₂O is added. After phase separation, the aq. layer is extracted three times with DCM. The combined org. phases are washed with 10% citric acid. The solvent is evaporated and the pure (3-oxycarbonylamino-1,2,3,4-tetrahydro-9*H*-carbazol-9-yl)-acetate derivative of Structure 1 is obtained by preparative HPLC with 5 to 96% yield.

Listed in Table 3 below are ethyl (3-oxycarbonylamino-1,2,3,4-tetrahydro-9*H*-carbazol-9-yl)-acetate derivatives of Structure **1,** prepared according to the above mentioned method, with the corresponding compound of Structure **2a** or **2b** as starting material.

**Table 3**

| **Intermediates of Structure 1:** **Ethyl (3-oxycarbonylamino-1,2,3,4-tetrahydro-9*H*-carbazol-9-yl)-acetate derivatives** | **Formula MW** | **t_{R} [min] LC-MS Method** | **MS Data m/z** |
|---|---|---|---|
| Ethyl (3*R*)-(3-propoxycarbonylamino-1,2,3,4-tetrahydro-9*H*-carbazol-9-yl)-acetate | C20H26N204 | 1.14 | 380.98 |
| | 358.436 | LC-2 | [M+Na]⁺ |
| Ethyl (3*R*)-(3-isobutoxycarbonylamino-1,2,3,4-tetrahydro-9*H*-carbazol-9-yl)-acetate | C21H28N204 | 1.20 | 395.04 |
| | 372.463 | LC-2 | [M+Na]⁺ |
| Ethyl (3*R*)-(3-benzyloxycarbonylamino-1,2,3,4-tetrahydro-9*H*-carbazol-9-yl)-acetate | C24H26N204 | 1.17 | 407.02 |
| | 406.48 | LC-2 | [M+H]⁺ |
| Ethyl (3*R*)-[3-(2-benzyloxy-ethoxycarbonylamino)-1,2,3,4-tetrahydro-9*H*-carbazol-9-yl]-acetate | C26H30N205 | 1.17 | 473.01 |
| | 450.533 | LC-2 | [M+Na]⁺ |
| Ethyl (3*S*)-(3-propoxycarbonylamino-1,2,3,4-tetrahydro-9*H*-carbazol-9-yl)-acetate | C20H26N204 | 1.12 | 381.09 |
| | 358.436 | LC-2 | [M+Na]⁺ |
| Ethyl (3*S*)-(3-isobutoxycarbonylamino-1,2,3,4-tetrahydro-9*H*-carbazol-9-yl)-acetate | C21H28N204 | 1.15 | 373.13 |
| | 372.463 | LC-2 | [M+H]⁺ |
| Ethyl (3*S*)-(3-benzyloxycarbonylamino-1,2,3,4-tetrahydro-9*H*-carbazol-9-yl)-acetate | C24H26N204 | 1.16 | 407.2 |
| | 406.48 | LC-2 | [M+H]⁺ |
| Ethyl (3*S*)-[3-(2-benzyloxy-ethoxycarbonylamino)-1,2,3,4-tetrahydro-9*H*-carbazol-9-yl]-acetate | C26H30N205 | 1.17 | 473.14 |
| | 450.533 | LC-2 | [M+Na]⁺ |

Listed in Table 3a below are further ethyl (3-oxycarbonylamino-1,2,3,4-tetrahydro-9*H-*carbazol-9-yl)-acetate derivatives of Structure **1,** prepared according to the above mentioned method, with the corresponding intermediate of Structure **2a** or **2b** as starting material.

**Table 3a**

| **Intermediates of Structure 1:** **Ethyl (3-oxycarbonylamino-1,2,3,4-tetrahydro-9*H*-carbazol-9-yl)-acetate derivatives** | **Formula MW** |
|---|---|
| Ethyl (3*RS*)-(3-benzyloxycarbonylamino-6-trifluoromethyl-1,2,3,4-tetrahydro-9*H*-carbazol-9-yl)-acetate | C25H25N204F3 |
| | 474.478 |
| Ethyl (3*RS*)-(3-benzyloxycarbonylamino-8-bromo-6-fluoro-1,2,3,4-tetrahydro-9*H*-carbazol-9-yl)-acetate | C24H24N204BrF |
| | 503.367 |
| Ethyl (3*RS*)-(3-benzyloxycarbonylamino-6-fluoro-8-vinyl-1,2,3,4-tetrahydro-9*H*-carbazol-9-yl)-acetate | C26H27N204F |
| | 450.508 |
| Ethyl (3*RS*)-(3-benzyloxycarbonylamino-6-fluoro-8-methanesulfonyl-1,2,3,4-tetrahydro-9*H*-carbazol-9-yl)-acetate | C25H27N206FS |
| | 502.561 |
| Ethyl (3*S*)-(3-benzyloxycarbonylamino-6-fluoro-8-methyl-1,2,3,4-tetrahydro-9*H*-carbazol-9-yl) acetate | C25H27N204F |
| | 438.497 |
| Ethyl (3*S*)-(3-benzyloxycarbonylamino-7-chloro-6-fluoro-1,2,3,4-tetrahydro-9*H*-carbazol-9-yl)-acetate | C24H24ClFN2O4 |
| | 458.91 |
| Ethyl (3*S*)-(8-allyl-3-benzyloxycarbonylamino-6-fluoro-1,2,3,4-tetrahydro-9*H*-carbazol-9-yl)-acetate | C27H29N2O4F |
| | 464.535 |
| Ethyl (3*R*)-(3-benzyloxycarbonylamino-8-chloro-1,2,3,4-tetrahydro-9*H*-carbazol-9-yl)-acetate | C24H25N2O4Cl |
| | 440.926 |
| Ethyl (3*S*)-[3-(benzyloxycarbonyl-methyl-amino)-6-fluoro-1,2,3,4-tetrahydro-9*H*-carbazol-9-yl]-acetate | C25H27N204F |
| | 438.494 |
| Ethyl {(3*S*)-3-[(2-chloro-benzyloxycarbonyl)-methyl-amino]-6-fluoro-1,2,3,4-tetrahydro-9*H*-carbazol-9-yl}-acetate | C25H26N2O4ClF |
| | 472.939 |
| Ethyl (3*S*)-[3-(benzyloxycarbonyl-cyclopropylmethyl-amino)-6-fluoro-1,2,3,4-tetrahydro-9*H*-carbazol-9-yl]-acetate | C28H31N204F |
| | 478.558 |
| Ethyl (3*S*)-(3-{benzyloxycarbonyl-[2-(4-trifluoromethyl-phenoxy)-ethyl]-amino}-6-fluoro-1,2,3,4-tetrahydro-9*H*-carbazol-9-yl)-acetate | C33H32N205F4 |
| | 612.614 |
| Ethyl (3*S*)-{3-[benzyloxycarbonyl-(2-methoxy-ethyl)-amino]-6-fluoro-1,2,3,4-tetrahydro-9*H*-carbazol-9-yl}-acetate | C27H31N205F |
| | 482.546 |

### 3) N-Acylation of an intermediate of Structure 2a or 2b:

### Method (A)

The appropriate acid chloride (0.132 mmol) and a catalytical amount of DMAP are added to a stirred solution of a hydrochloride of the appropriate intermediate of Structure **2a** or **2b** (0.11 mmol) and Et₃N (0.034 ml, 0.242 mmol) in DCM (2 ml) at 0°C, and the resulting reaction mixture is kept stirring at rt overnight. Then, a (1:4) mixture (1 ml) of sat. NaHCO₃ and H₂O is added. After phase separation, the aq. layer is extracted three times with DCM, and the combined org. layers are washed with 10% citric acid to remove DMAP. The solvent is evaporated and the pure ethyl (3-acylamido-1,2,3,4-tetrahydro-9*H*-carbazol-9-yl)-acetate derivative of Structure **1** is isolated by preparative HPLC in 10 to 95 % yield.

### Method (B)

A solution of a hydrochloride of the appropriate intermediate of Structure **2a** or **2b** (0.075 mmol) and DIEA (0.15 mmol) in a 4:1 mixture (2 ml) of dry DMF and THF is added dropwise to a stirred solution of the appropriate carboxylic acid (0.113 mmol), HATU (0.15 mmol) and DIEA (0.15 mmol) in a 4:1 mixture (2 ml) of dry DMF and THF at 0°C. The mixture is stirred at rt for 1 h, or overnight, then sat. NaHCO₃ solution is added. After phase separation, the aq. layer is extracted three times with DCM. The combined org. phases are evaporated. The crude ethyl (3-acylamido-1,2,3,4-tetrahydro-9*H*-carbazol-9-yl)-acetate derivative of Structure **1** is obtained with >50% yield and is either used as such in the next step or purified by prepaprative HPLC to give the pure ethyl (3-acylamino-1,2,3,4-tetrahydro-9*H*-carbazol-9-yl)-acetate derivative of Structure **1** with 13 to 95% yield.

Listed in Table 4 below are crude ethyl (3-acylamino-1,2,3,4-tetrahydro-9*H*-carbazol-9-yl)-acetate derivatives of Structure **1,** prepared according to the above mentioned methods (A) or (B), with the corresponding intermediate of Structure **2a** or **2b** as starting material.

**Table 4**

| **Intermediates of Structure 1:** **Ethyl (3-acylamino-1,2,3,4-tetrahydro-9*H*-carbazol-9-yl)-acetate derivatives** | **Formula MW** | **t_{R} [min] LC-MS Method** | **MS Data m/z [M+H]⁺** |
|---|---|---|---|
| Ethyl (3*R*)-(3-benzoylamino-1,2,3,4-tetrahydro-9*H*-carbazol-9-yl)-acetate | C23H24N203 | 1.11 | 399.00 |
| | 376.455 | LC-2 | [M+Na]⁺ |
| Ethyl (3*R*)-[3-(2-phenoxy-acetylamino)-1,2,3,4-tetrahydro-9*H*-carbazol-9-yl]-acetate | C24H26N204 | 1.13 | 428.93 |
| | 406.48 | LC-2 | [M+Na]⁺ |
| Ethyl (3*R*)-(3-phenylacetylamino-1,2,3,4-tetrahydro-9*H*-carbazol-9-yl)-acetate | C24H26N203 | 1.11 | 412.97 |
| | 390.481 | LC-2 | [M+Na]⁺ |
| Ethyl (3*R*)-[3-(2-thiophen-2-yl-acetylamino)-1,2,3,4-tetrahydro-9*H*-carbazol-9-yl]-acetate | C22H24N203S | 1.09 | 396.98 |
| | 396.51 | LC-2 | |
| Ethyl (3*R*)-[3-(3-phenyl-propionylamino)-1,2,3,4-tetrahydro-9*H*-carbazol-9-yl]-acetate | C25H28N203 | 1.13 | 427.03 |
| | 404.508 | LC-2 | [M+Na]⁺ |
| Ethyl (3*R*)-[3-(2-benzyloxy-acetylamino)-1,2,3,4-tetrahydro-9*H*-carbazol-9-yl]-acetate | C25H28N204 | 1.14 | 421.07 |
| | 420.507 | LC-2 | |
| Ethyl (3*R*)-[3-(3-methyl-butyrylamino)-1,2,3,4-tetrahydro-9*H*-carbazol-9-yl]-acetate | C21H28N2O3 | 1.09 | 357.1 |
| | 356.464 | LC-2 | |
| Ethyl (3*R*)-[3-(3-cyclopentyl-propionylamino)-1,2,3,4-tetrahydro-9*H*-carbazol-9-yl]-acetate | C24H32N203 | 1.17 | 397.05 |
| | 396.529 | LC-2 | |
| Ethyl (3*R*)-(3-decanoylamino-1,2,3,4-tetrahydro-9*H*-carbazol-9-yl)-acetate | C26H38N203 | 1.28 | 449.01 |
| | 426.599 | LC-2 | [M+Na]⁺ |
| Ethyl (3*S*)-[3-(2-phenoxy-acetylamino)-1,2,3,4-tetrahydro-9*H*-carbazol-9-yl]-acetate | C24H26N204 | 1.03 | 407.23 |
| | 406.48 | LC-2 | |
| Ethyl (3*S*)-(3-phenylacetylamino-1,2,3,4-tetrahydro-9*H*-carbazol-9-yl)-acetate | C24H26N203 | 1.1 | 391.13 |
| | 390.481 | LC-2 | |
| Ethyl (3*S*)-[3-(2-thiophen-2-yl-acetylamino)-1,2,3,4-tetrahydro-9*H*-carbazol-9-yl]-acetate | C22H24N203S | 1.1 | 397 |
| | 396.51 | LC-2 | |
| Ethyl (3*S*)-[3-(3-phenyl-propionylamino)-1,2,3,4-tetrahydro-9*H*-carbazol-9-yl]-acetate | C25H28N203 | 1.13 | 405.12 |
| | 404.508 | LC-2 | |
| Ethyl (3*S*)-[3-(2-benzyloxy-acetylamino)-1,2,3,4-tetrahydro-9*H*-carbazol-9-yl]-acetate | C25H28N204 | 1.14 | 421.09 |
| | 420.507 | LC-2 | |
| Ethyl (3*S*)-[3-(3-methyl-butylamino)-1,2,3,4-tetrahydro-9*H*-carbazol-9-yl]-acetate | C21H28N203 | 1.1 | 357.13 |
| | 356.464 | LC-2 | |
| Ethyl (3*S*)-[3-(3-cyclopentyl-propionylamino)-1,2,3,4-tetrahydro-9*H*-carbazol-9-yl]-acetate | C24H32N203 | 1.18 | 397.16 |
| | 396.529 | LC-2 | |
| Ethyl (3*S*)-(3-decanoylamino-1,2,3,4-tetrahydro-9*H*-carbazol-9-yl)acetate | C26H38N203 | 1.15 | 427.32 |
| | 426.599 | LC-2 | |
| Ethyl (3*S*)-(3-butyrylamino-1,2,3,4-tetrahydro-9*H*-carbazol-9-yl)-acetate | C20H26N203 | 1.07 | 343.15 |
| | 342.437 | LC-2 | |
| Ethyl (3*S*)-(3-heptanoylamino-1,2,3,4-tetrahydro-9*H*-carbazol-9-yl)-acetate | C23H32N203 | 1.18 | 385.18 |
| | 384.518 | LC-2 | |
| Ethyl (3*R*)[6-fluoro-3-(2-phenoxy-acetylamino)-1,2,3,4-tetrahydro-9*H*-carbazol-9-yl]-acetate | C24H25N204F | 1.15 | 425.28 |
| | 424.471 | LC-2 | |
| Ethyl (3*R*)-(6-fluoro-3-phenylacetylamino-1,2,3,4-tetrahydro-9*H*-carbazol-9-yl)-acetate | C24H25N203F | 1.12 | 409.28 |
| | 408.472 | LC-2 | |
| Ethyl (3*R*)-[6-Fluoro-3-(3-phenyl-propionylamino)-1,2,3,4-tetrahydro-9*H*-carbazol-9-yl]-acetate | C25H27N203F | 1.15 | 423.27 |
| | 422.498 | LC-2 | |
| Ethyl (3*R*)-{3-[2-(4-Chloro-phenyl)acetylamino]-6-fluoro-1,2,3,4-tetrahydro-9*H*-carbazol-9-yl}-acetate | C24H24N2O3ClF | 1.18 | 443.21 |
| | 442.917 | LC-2 | |
| Ethyl (3*R*)-{6-fluoro-3-[2-(4-methoxy-phenyl)-acetylamino]-1,2,3,4-tetrahydro-9*H*-carbazol-9-yl}-acetate | C25H27N204F | 1.12 | 439.2 |
| | 438.497 | LC-2 | |
| Ethyl (3*R*)-[6-fluoro-3-(2-*p*-tolyl-acetylamino)-1,2,3,4-tetrahydro-9*H*-carbazol-9-yl]-acetate | C25H27N203F | 1.14 | 423.34 |
| | 422.498 | LC-2 | |
| Ethyl (3*R*)-{3-[2-(3,4-dichloro-phenyl)-acetylamino]-C 6-fluoro-1,2,3,4-tetrahydro-9*H*-carbazol-9-yl}-acetate | C24H23N2O3Cl2F | 1.18 | 477.22 |
| | 477.362 | LC-2 | |
| Ethyl (3*R*)-{3-[2-(3-chloro-phenyl)-acetylamino]-6-fluoro-1,2,3,4-tetrahydro-9*H*-carbazol-9-yl}-acetate | C24H24N2O3ClF | 1.16 | 443.28 |
| | 442.917 | LC-2 | |
| Ethyl (3*R*)-{6-fluoro-3-[2-(4-triftuoromethyl-phenyl)-acetylamino]-1,2,3,4-tetrahydro-9*H*-carbazol-9-yl}-acetate | C25H24N203F4 | 1.17 | 477.29 |
| | 476.469 | LC-2 | |
| Ethyl (3*R*)-{3-[2-(4-chloro-phenoxy)-acetylamino]-6-fluoro-1,2,3,4-tetrahydro-9*H*-carbazol-9-yl}-acetate | C24H24N2O4ClF | 1.18 | 459.28 |
| | 458.916 | LC-2 | |
| Ethyl (3*R*)-[6-fluoro-3-(2-*p*-tolyloxy-acetylamino)-1,2,3,4-tetrahydro-9*H*-carbazol-9-yl]-acetate | C25H27N204F | 1.18 | 439.27 |
| | 438.497 | LC-2 | |
| Ethyl (3*R*)-{3-[2-(2-chloro-phenoxy)-acetylamino]-6-fluoro-1,2,3,4-tetrahydro-9*H*-carbazol-9-yl}-acetate | C24H24N2O4ClF | 1.17 | 459.21 |
| | 458.916 | LC-2 | |
| Ethyl (3*R*)-{3-[2-(3-chloro-phenoxy)-acetylamino]-6-fluoro-1,2,3,4-tetrahydro-9*H*-carbazol-9-yl}-acetate | C24H24N2O4ClF | 1.18 | 459.21 |
| | 458.916 | LC-2 | |
| Ethyl (3*R*)-{6-fluoro-3-[3-(4-methoxy-phenyl)-propionylamino]-1,2,3,4-tetrahydro-9*H*-carbazol-9-yl}-acetate | C26H29N204F | 1.14 | 453.26 |
| | 452.524 | LC-2 | |
| Ethyl (3*R*)-[6-fluoro-3-(3-*p*-tolyl-propionylamino)-1,2,3,4-tetrahydro-9*H*-carbazol-9-yl]-acetate | C26H29N203F | 1.17 | 437.26 |
| | 436.525 | LC-2 | |
| Ethyl (3*R*)-{3-[3-(4-chloro-phenyl)-propionylamino]-6-fluoro-1,2,3,4-tetrahydro-9*H*-carbazol-9-yl}-acetate | C25H26N2O3ClF | 1.18 | 457.2 |
| | 456.943 | LC-2 | |
| Ethyl (3*R*)-{3-[3-(2-chloro-phenyl)-propionylamino]-6-fluoro-1,2,3,4-tetrahydro-9*H*-carbazol-9-yl}-acetate | C25H26N2O3ClF | 1.18 | 457.2 |
| | 456.943 | LC-2 | |
| Ethyl (3*R*)-{3-[3-(3-chloro-phenyl)-propionylamino]-6-fluoro-1,2,3,4-tetrahydro-9*H*-carbazol-9-yl}-acetate | C25H26N2O3ClF | 1.17 | 457.2 |
| | 456.943 | LC-2 | |
| Ethyl (3*S*)-{3-[2-(4-chloro-phenyl)-acetylamino]-6-fluoro-1,2,3,4-tetrahydro-9*H*-carbazol-9-yl}-acetate | C24H24N2O3ClF | 1.15 | 443.21 |
| | 442.917 | LC-2 | |
| Ethyl (3*S*)-{6-fluoro-3-[2-(4-methoxy-pheny)-acetylamino]-1,2,3,4-tetrahydro-9*H*-carbazol-9-yl}-acetate | C25H27N204F | 1.12 | 439.33 |
| | 438.497 | LC-2 | |
| Ethyl (3*S*)-[6-fluoro-3-(2-p-tolyl-acetylamino)-1,2,3,4-tetrahydro-9*H*-carbazol-9-yl]-acetate | C25H27N203F | 1.15 | 423.27 |
| | 422.498 | LC-2 | |
| Ethyl (3*S*)-[6-fluoro-3-(2-phenoxy-acetylamino)-1,2,3,4-tetrahydro-9*H*-carbazol-9-yl]-acetate | C24H25N204F | 1.15 | 425.28 |
| | 424.471 | LC-2 | |
| Ethyl (3*S*)-[6-fluoro-3-(3-phenyl-propionylamino)-1,2,3,4-tetrahydro-9*H*-carbazol-9-yl]-acetate | C25H27N203F | 1.14 | 423.27 |
| | 422.498 | LC-2 | |
| Ethyl (3*S*)-{3-[2-(4-chloro-phenoxy)-acetylamino]-6-fluoro-1,2,3,4-tetrahydro-9*H*-carbazol-9-yl}-acetate | C24H24N2O4ClF | 1.17 | 459.21 |
| | 458.916 | LC-2 | |
| Ethyl (3*S*)-[6-fluoro-3-(2-*p*-tolyloxy-acetylamino)-1,2,3,4-tetrahydro-9*H*-carbazol-9-yl]-acetate | C25H27N204F | 1.18 | 439.27 |
| | 438.497 | LC-2 | |
| Ethyl (3*S*)-{6-fluoro-3-[3-(4-methoxy-phenyl)-propionylamino]-1,2,3,4-tetrahydro-9*H*-carbazol-9-yl}-acetate | C26H29N204F | 1.14 | 453.33 |
| | 452.524 | LC-2 | |
| Ethyl (3*S*)-[6-fluoro-3-(3-p-tolyl-propionylamino)-1,2,3,4-tetrahydro-9*H*-carbazol-9-yl]-acetate | C26H29N203F | 1.17 | 437.33 |
| | 436.525 | LC-2 | |
| Ethyl (3*S*)-{3-[3-(4-chtoro-phenyl)-propionylamino]-6-fluoro-1,2,3,4-tetrahydro-9*H*-carbazol-9-yl}-acetate | C25H26N2O3ClF | 1.17 | 457.27 |
| | 456.943 | LC-2 | |
| Ethyl (3*S*)-{3-[3-(2-chloro-phenyl)-propionylamino]-6-fluoro-1,2,3,4-tetrahydro-9*H*-carbazol-9-yl}-acetate | C25H26N2O3ClF | 1.17 | 457.27 |
| | 456.943 | LC-2 | |
| Ethyl (3*S*)-{3-[3-(3-chloro-phenyl)-propionylamino]-6-fluoro-1,2,3,4-tetrahydro-9*H*-carbazol-9-yl}-acetate | C25H26N2O3ClF | 1.18 | 457.27 |
| | 456.943 | LC-2 | |
| Ethyl (3*S*)-{3-[3-(3,4-difluoro-phenyl)-propionylamino]-6-fluoro-1,2,3,4-tetrahydro-9*H*-carbazol-9-yl}-acetate | C25H25N203F3 | 1.17 | 459.28 |
| | 458.479 | LC-2 | |
| Ethyl (3*S*)-{6-fluoro-3-[3-(3-methoxy-phenyl)-propionylamino]-1,2,3,4-tetrahydro-9*H*-carbazol-9-yl}-acetate | C26H29N204F | 1.14 | 453.33 |
| | 452.524 | LC-2 | |
| Ethyl (3*S*)-{6-fluoro-3-[3-(2-methoxy-phenyl)-propionylamino]-1,2,3,4-tetrahydro-9*H*-carbazol-9-yl}-acetate | C26H29N204F | 1.16 | 453.33 |
| | 452.524 | LC-2 | |
| Ethyl (3*S*)-[3-(2,3-diphenyl-propionylamino)-6-fluoro-1,2,3,4-tetrahydro-9*H*-carbazol-9-yl]-acetate | C31H31N2O3F | 1.21 | 499.39 |
| | 498.596 | LC-2 | |
| Ethyl (3*S*)-[3-(3,3-diphenyl-propionylamino)-6-fluoro-1,2,3,4-tetrahydro-9*H*-carbazol-9-yl]-acetate | C31H31N2O3F | 1.2 | 499.39 |
| | 498.596 | LC-2 | |
| Ethyl (3*S*)-{3-[4-(4-bromo-phenyl)-4-oxo-butyrylamino]-6-fluoro-1,2,3,4-tetrahydro-9*H*-carbazol-9-yl}-acetate | C26H26N2O4BrF | 1.17 | 531.25 |
| | 529.404 | LC-2 | |
| Ethyl (3*S*)-[6-fluoro-3-(4-oxo-4-phenyl-butyrylamino)-1,2,3,4-tetrahydro-9*H*-carbazol-9-yl]-acetate | C26H27N204F | 1.12 | 451.25 |
| | 450.508 | LC-2 | |
| Ethyl (3*S*)-{6-Fluoro-3-[4-(4-methanesulfonyl-phenyl)-4-oxo-butyrylamino]-1,2,3,4-tetrahydro-9*H*-carbazol-9-yl}-acetate | C27H29N2O6FS | 1.07 | 529.31 |
| | 528.599 | LC-2 | |
| Ethyl (3S*)*-[6-fluoro-3-(2-indan-2-yl-acetylamino)-1,2,3,4-tetrahydro-9*H*-carbazol-9-yl]-acetate | C27H29N203F | 1.18 | 449.31 |
| | 448.536 | LC-2 | |
| Ethyl (3*S*)-{6-fluoro-3-[3-(4-hydroxy-3-methoxy-phenyl)-propionylamino]-1,2,3,4-tetrahydro-9*H*-carbazol-9-yl}-acetate | C26H29FN205 | 1.08 | 469.04 |
| | 468.52 | LC-3 | |
| Ethyl (3*S*)-{6-fluoro-3-[3-(4-hydroxy-phenyl)-propionylamino]-1,2,3,4-tetrahydro-9*H*-carbazol-9-yl}-acetate | C25H27FN204 | 1.07 | 439.06 |
| | 438.49 | LC-3 | |
| Ethyl (3*S*)-{6-fluoro-3-[3-(3-hydroxy-phenyl)-propionylamino]-1,2,3,4-tetrahydro-9*H*-carbazol-9-yl}-acetate | C25H27FN205 | 1.08 | 439.06 |
| | 439.49 | LC-3 | |
| Ethyl (3*S*)-{6-fluoro-3-[3-(2-hydroxy-phenyl)-propionylamino]-1,2,3,4-tetrahydro-9*H*-carbazol-9-yl}-acetate | C25H27FN206 | n. d. | n. d. |
| | 440.49 | LC-3 | |
| Ethyl (3*S*)-{3-[(2,3-dihydro-1*H*-indole-2-carbonyl)-amino]-6-fluoro-1,2,3,4-tetrahydro-9*H*-carbazol-9-yl}-acetate | C25H26FN303 | 1.14 | 436.08 |
| | 435.49 | LC-3 | |
| Ethyl (3*S*)-[6-fluoro-3-(3-1*H*-indol-3-yl-propionylamino)-1,2,3,4-tetrahydro-9*H*-carbazol-9-yl]-acetate | C27H28FN303 | 1.15 | 462.05 |
| | 461.53 | LC-3 | |
| Ethyl (3*S*)-[3-(3-1*H*-benzoimidazol-2-yl-propionylamino)-6-fluoro-1,2,3,4-tetrahydro-9*H*-carbazol-9-yl]-acetate | C26H27FN403 | 0.86 | 463.09 |
| | 462.52 | LC-3 | |
| Ethyl (3*S*)-[3-(3-benzo[1,3]dioxo-5-yl-propionylamino)-6-fluoro-1,2,3,4-tetrahydro-9*H*-carbazol-9-yl]-acetate | C26H27FN205 | 1.15 | 467.03 |
| | 466.5 | LC-3 | |
| Ethyl (3*S*)-{6-fluoro-3-[(1*H*-indole-2-carbonyl)-amino]-1,2,3,4-tetrahydro-9*H*-carbazol-9-yl}-acetate | C25H24FN303 | n. d. | n. d. |
| | 433.47 | LC-3 | |
| Ethyl (3*S*)-[6-fluoro-3-(3-pyridin-3-yl-propionylamino)-1,2,3,4-tetrahydro-9*H*-carbazol-9-yl]-acetate | C24H26FN303 | n. d. | n. d. |
| | 423.48 | LC-3. | |
| Ethyl (3*S*)-{3-[2-(4-*tert*-butyl-phenyl)-acetylamino]-6-fluoro-1,2,3,4-tetrahydro-9*H*-carbazol-9-yl}-acetate | C28H33FN203 | n. d. | n. d. |
| | 464.57 | LC-3 | |
| Ethyl (3*S*)-{6-fluoro-3-[2-(4-trifluoromethyl-phenyl)-acetylamino]-1,2,3,4-tetrahydro-9*H*-carbazol-9-yl}-acetate | C25H24F4N203 | n. d. | n. d. |
| | 476.46 | LC-3 | |
| Ethyl (3*S*)-{6-fluoro-3-[2-(3-trifluoromethyl-phenyl)-acetylamino]-1,2,3,4-tetrahydro-9*H*-carbazol-9-yl}-acetate | C25H24F4N203 | 1.26 | 477.04 |
| | 476.46 | LC-2 | |
| Ethyl (3*S*)-{6-fluoro-3-[2-(4-trifluoromethyl-phenoxy)-acetylamino]-1,2,3,4-tetrahydro-9*H*-carbazol-9-yl}-acetate | C25H24F4N204 | n. d. | n. d. |
| | 492.46 | LC-3. | |
| Ethyl (3*S*)-[6-fluoro-3-(3-naphthalen-2-yl-acryloylamino)-1,2,3,4-tetrahydro-9*H*-carbazol-9-yl]-acetate | C29H27FN203 | 1.29 | 471.05 |
| | 470.53 | LC-2 | |
| Ethyl (3*S*)-[6-fluoro-3-(3-naphthalen-2-yl-propionylamino)-1,2,3,4-tetrahydro-9*H*-carbazol-9-yl]-acetate | C29H29FN203 | 1.27 | 473.08 |
| | 472.55 | LC-2 | |
| Ethyl (3*S*)-{6-fluoro-3-[methyl-(3-phenyl-propionyl)-amino]-1,2,3,4-tetrahydro-9*H*-carbazol-9-yl}-acetate | C26H29FN203 | 1.08 | 437.28 |
| | 436.52 | LC-3 | |
| Ethyl (3*S*)-(3-{[2-(4-chloro-phenyl)-acetyl]-methyl-amino}-6-fluoro-1,2,3,4-tetrahydro-9*H*-carbazol-9-yl)-acetate | C25H26ClFN2O3 | 1.09 | 473.08 |
| | 456.94 | LC-3 | |
| Ethyl (3*S*)-{6-fluoro-3-[ethyl-(3-phenyl-propionyl)-amino]-1,2,3,4-tetrahydro-9*H*-carbazol-9-yl}-acetate | C27H31FN203 | 1.09 | 451.23 |
| | 450.55 | LC-3 | |
| Ethyl (3*S*)-(3-{[2-(4-chloro-phenyl)-acetyl]-ethyl-amino}-6-fluoro-1,2,3,4-tetrahydro-9*H*-carbazol-9-yl)-acetate | C26H28ClFN2O3 | 1.1 | 471.22 |
| | 470.96 | LC-3 | |
| Ethyl (3*S*)-{6-fluoro-3-[propyl-(3-phenyl-propionyl)-amino]-1,2,3,4-tetrahydro-9*H*-carbazol-9-yl}-acetate | C28H33FN203 | 1.11 | 465.23 |
| | 464.57 | LC-3 | |
| Ethyl (3*S*)-(3-{[2-(4-chloro-phenyl)-acetyl]-propyl-amino}-6-fluoro-1,2,3,4-tetrahydro-9*H*-carbazol-9-yl)-acetate | C27H30ClFN2O | 1.12 | 485.20 |
| | 484.99 | LC-3 | |

Listed in Table 4a below are further ethyl (3-acylamino-1,2,3,4-tetrahydro-9*H*-carbazol-9-yl)-acetate derivatives of Structure **1,** prepared according to the above mentioned methods (A) or (B), with the corresponding intermediate of Structure **2a** or **2b** as starting material.

**Table 4a**

| **Intermediates of Structure 1:** **Ethyl (3-acylamino-1,2,3,4-tetrahydro-9*H*-carbazol-9-yl)-acetate derivatives** | **Formula MW** |
|---|---|
| Ethyl (3*S*)-{3-[3-(2,4-dimethoxy-phenyl)-propionylamino]-6-fluoro-1,2,3,4-tetrahydro-9*H*-carbazol-9-yl}-acetate | C27H31 N205F |
| | 482.546 |
| Ethyl (3*S*)-[6-fluoro-3-(3-naphthalen-1-yl-propionylamino)-1,2,3,4-tetrahydro-9*H*-carbazol-9-yl]-acetate | C29H29N203F |
| | 472.555 |
| Ethyl (3*RS*)-{6-fluoro-3-[2-(2-methoxy-phenoxy)-acetylamino]-1,2,3,4-tetrahydro-9*H*-carbazol-9-yl}-acetate | C25H27N205F |
| | 454.493 |
| Ethyl (3*RS*)-{6-fluoro-3-[3-(2-methylphenyl)-propionylamino]-1,2,3,4-tetrahydro-9*H*-carbazol-9-yl}-acetate | C26H29N203F |
| | 436.522 |
| Ethyl (3*RS*)-{6-fluoro-3-[3-(3-methylphenyl)-propionylamino]-1,2,3,4-tetrahydro-9*H*-carbazol-9-yl}-acetate | C26H29N203F |
| | 436.522 |
| Ethyl (3*RS*)-{6-fluoro-3-[3-(3-methoxy-phenyl)-propionylamino]-1,2,3,4-tetrahydro-9*H*-carbazol-9-yl}-acetate | C26H29N204F |
| | 452.521 |
| Ethyl (3*RS*)-{6-fluoro-3-[2-(3-methoxy-phenoxy)-acetylamino]-1,2,3,4-tetrahydro-9*H*-carbazol-9-yl}-acetate | C25H27N205F |
| | 454.493 |
| Ethyl (3*RS*)-{6-fluoro-3-[2-(2-methylphenoxy)-acetylamino]-1,2,3,4-tetrahydro-9*H*-carbazol-9-yl}-acetate | C25H27N204F |
| | 438.494 |
| Ethyl (3*S*)-{3-[3-(2,5-dimethoxy-phenyl)-propionylamino]-6-fluoro-1,2,3,4-tetrahydro-9*H*-carbazol-9-yl}-acetate | C22H31 N205F |
| | 482.546 |
| Ethyl (3*S*)-{6-fluoro-3-[3-(4-trifluoromethyl-phenyl)-propionylamino]-1,2,3,4-tetrahydro-9*H*-carbazol-9-yl}-acetate | C26H26N203F4 |
| | 490.492 |
| Ethyl (3*S*)-{3-[3-(2,6-dichloro-phenyl)-propionylamino]-6-fluoro-1,2,3,4-tetrahydro-9*H*-carbazol-9-yl}-acetate | C25H25N2O3Cl2F |
| | 491.385 |
| Ethyl (3*S*)-{3-[3-(2,5-bis-trifluoromethyl-phenyl)-propionylamino]-6-fluoro-1,2,3,4-tetrahydro-9*H*-carbazol-9-yl}-acetate | C27H25N203F7 |
| | 558.489 |
| Ethyl (3*S*)-{6-fluoro-3-[3-(4-methylsulfanyl-phenyl)-propionylamino]-1,2,3,4-tetrahydro-9*H*-carbazol-9-yl}-acetate | C26H29N203FS |
| | 468.588 |
| Ethyl (3*S*)-{6-fluoro-3-[3-(4-iodo-phenyl)-propionylamino]-1,2,3,4-tetrahydro-9*H*-carbazol-9-yl}-acetate | C25H26N2O3FI |
| | 548.387 |
| Ethyl (3*S*)-{6-fluoro-3-[3-(4-isopropyl-phenyl)-propionylamino]-1,2,3,4-tetrahydro-9*H*-carbazol-9-yl}-acetate | C28H33N203F |
| | 464.575 |
| Ethyl (3*S*)-{6-fluoro-3-[3-(3-trifluoromethyl-phenyl)-propionylamino]-1,2,3,4-tetrahydro-9*H*-carbazol-9-yl}-acetate | C26H26N203F4 |
| | 490.492 |
| Ethyl (3*S*)-{3-[3-(2,4-dichloro-phenyl)-propionylamino]-6-fluoro-1,2,3,4-tetrahydro-9*H*-carbazol-9-yl}-acetate | C25H25N2O3Cl2F |
| | 491.385 |
| Ethyl (3*S*)-{6-fluoro-3-[3-(4-fluoro-phenyl)-propionylamino]-1,2,3,4-tetrahydro-9*H*-carbazol-9-yl}-acetate | C25H26N203F2 |
| | 440.485 |
| Ethyl (3*S*)-{3-[3-(3,5-bis-trifluoromethyl-phenyl)-propionylamino]-6-fluoro-1,2,3,4-tetrahydro-9*H*-carbazol-9-yl}-acetate | C27H25N203F7 |
| | 558.489 |
| Ethyl (3*S*)-{3-[3-(4-ethyl-phenyl)-propionylamino]-6-fluoro-1,2,3,4-tetrahydro-9*H*-carbazol-9-yl}-acetate | C27H31N2O3F |
| | 450.548 |
| Ethyl (3*S*)-{6-fluoro-3-[3-(3-iodo-phenyl)-propionylamino]-1,2,3,4-tetrahydro-9*H*-carbazol-9-yl}-acetate | C25H26N2O3FI |
| | 548.387 |
| Ethyl (3*S*)-{6-fluoro-3-[3-(4-methanesulfonyl-phenylypropionylamino]-1,2,3,4-tetrahydro-9*H*-carbazol-9-yl}-acetate | C26H29N205FS |
| | 500.586 |
| Ethyl (3*S*)-{3-[3-(2,3-dimethoxy-phenyl)-propionylamino]-6-fluoro-1,2,3,4-tetrahydro-9*H*-carbazol-9-yl}-acetate | C27H31 N205F |
| | 482.546 |
| Ethyl (3*S*)-{3-[3-(2-bromo-phenyl)-propionylamino]-6-fluoro-1,2,3,4-tetrahydro-9*H*-carbazol-9-yl}-acetate | C25H26N203BrF |
| | 501.391 |
| Ethyl (3*S*)-{6-fluoro-3-[3-(3-trifluoromethoxy-phenyl)-propionylamino]-1,2,3,4-tetrahydro-9*H*-carbazol-9-yl}-acetate | C26H26N204F4 |
| | 506.491 |
| Ethyl (3*S*)-{3-[3-(2,4-dimethyl-phenyl)-propionylamino]-6-fluoro-1,2,3,4-tetrahydro-9*H*-carbazol-9-yl}-acetate | C27H31N203F |
| | 450.548 |
| Ethyl (3*S*)-{3-[3-(3-bromo-phenyl)-propionylamino]-6-fluoro-1,2,3,4-tetrahydro-9*H*-carbazol-9-yl}-acetate | C25H26N203BrF |
| | 501.391 |
| Ethyl (3*S*)-{3-[3-(3-*tert*-butoxycarbonylamino-phenyl)-propionylamino]-6-fluoro-1,2,3,4-tetrahydro-9*H*-carbazol-9-yl}-acetate | C30H36N305F |
| | 537.626 |
| Ethyl (3*S*)-{6-fluoro-3-[(*S*)-3-(4-fluoro-phenyl)-2-phenyl-propionylamino]-1,2,3,4-tetrahydro-9*H*-carbazol-9-yl}-acetate | C31H30N203F2 |
| | 516.582 |
| Ethyl (3*S*)-{6-fluoro-3-[(*S*)-3-(4-methoxy-phenyl)-2-phenyl-propionylamino]-1,2,3,4-tetrahydro-9*H*-carbazol-9-yl}-acetate | C32H33N204F |
| | 528.618 |
| Ethyl (3*S*)-{6-fluoro-3-[3-(2-fluoro-phenyl)-propionylamino]-1,2,3,4-tetrahydro-9*H*-carbazol-9-yl}-acetate | C25H26N203F2 |
| | 440.485 |
| Ethyl (3*S*)-(6-fluoro-3-{[(2*RS*)-1,2,3,4-tetrahydro-naphthalene-2-carbonyl]-amino}-1,2,3,4-tetrahydro-9*H*-carbazol-9-yl)-acetate | C27H29N203F |
| | 448.533 |
| Ethyl (3*S*)-(6-fluoro-3-{[(2*RS*)-8-methoxy-1,2,3,4-tetrahydro-naphthalene-2-carbonyl]-amino}-1,2,3,4-tetrahydro-9*H*-carbazol-9-yl)-acetate | C28H31N2O4F |
| | 478.558 |
| Ethyl (3*S*)-(6-fluoro-3-{(2*RS*)-2-[(4-fluoro-phenylcarbamoyl)-methyl]-3-phenyl-propionylamino}-1,2,3,4-tetrahydro-9*H*-carbazol-9-yl)-acetate | C33H33N304F2 |
| | 573.634 |
| Ethyl (3*S*)-{3-[(2*R*S)-2-benzyl-3,3-dimethyl-butyrylamino]-6-fluoro-1,2,3,4-tetrahydro-9*H*-carbazol-9-yl}-acetate | C29H35N203F |
| | 478.602 |
| Ethyl (3*S*)-(3-{[(3*RS*)-chroman-3-carbonyl]-amino}-6-fluoro-1,2,3,4-tetrahydro-9*H*-carbazol-9-yl)-acetate | C26H27N204F |
| | 450.505 |
| Ethyl (3*S*)-{6-fluoro-3-[3-(3-fluoro-phenyl)-propionylamino]-1,2,3,4-tetrahydro-9*H*-carbazol-9-yl}-acetate | C25H26N203F2 |
| | 440.485 |
| Ethyl (3*S*)-(6-fluoro-3-{[(1*R*,2*R*)-2-phenyl-cyclopropanecarbonyl]-amino}-1,2,3,4-tetrahydro-9*H*-carbazol-9-yl)-acetate | C26H27N203F |
| | 434.506 |
| Ethyl(3*S*)-{6-fluoro-3-[(2*R*)-2-methyl-3-phenyl-propionylamino]-1,2,3,4-tetrahydro-9*H*-carbazol-9-yl}-acetate | C26H29N203F |
| | 436.522 |
| Ethyl (3*S*)-[3-(2,2-dimethyl-3-phenyl-propionylamino)-6-fluoro-1,2,3,4-tetrahydro-9*H*-carbazol-9-yl]-acetate | C27H31N2O3F |
| | 450.552 |
| | 450.548 |
| Ethyl (3*S*)-[6-fluoro-3-(3-methyl-3-phenyl-butyrylamino)-1,2,3,4-tetrahydro-9*H*-carbazol-9-yl]-acetate | C27H31 N203F |
| | 450.548 |
| Ethyl (3*S*)-{6-fluoro-3-[(3*S*)-3-phenyl-butyrylamino]-1,2,3,4-tetrahydro-9*H*-carbazol-9-yl}-acetate | C26H29N203F |
| | 436.522 |
| Ethyl (3*S*)-[3-(2-benzy)oxy-acetyamino)-6-fluoro-1,2,3,4-tetrahydro-9*H*-carbazol-9-yl]-acetate | C25H27N204F |
| | 438.494 |
| Ethyl (3*S*)-[6-fluoro-3-(4-phenyl-butyrylamino)-1,2,3,4-tetrahydro-9*H*-carbazol-9-yl]-acetate | C26H29N203F |
| | 436.522 |
| Ethyl (3*S*)-{3-[(2*R*,3*R*)-2,3-dihydroxy-3-(2-methoxy-phenylcarbamoyl)-propionylamino]-6-fluoro-1,2,3,4-tetrahydro-9*H*-carbazol-9-yl}-acetate | C27H30N307F |
| | 527.543 |
| Ethyl (3*RS*)-{8-chloro-6-fluoro-3-[3-(2-methylphenyl)-propionylamino]-1,2,3,4-tetrahydro-9*H*-carbazol-9-yl}-acetate | C26H28N2O3ClF |
| | 470.967 |
| Ethyl (3*RS*)-{8-chloro-6-fluoro-3-[2-(2-methoxy-phenoxy)-acetylamino]-1,2,3,4-tetrahydro-9*H*-carbazol-9-yl}-acetate | C25H26N2O5ClF |
| | 488.938 |
| Ethyl (3*RS*)-{8-chloro-6-fluoro-3-[3-(3-hydroxy-phenyl)-propionylamino]-1,2,3,4-tetrahydro-9*H*-carbazol-9-yl}-acetate | C25H26N2O4ClF |
| | 472.939 |
| Ethyl (3*RS*)-{8-chloro-6-fluoro-3-[3-(3-methoxy-phenyl)-propionylamino]-1,2,3,4-tetrahydro-9*H*-carbazol-9-yl}-acetate | C26H28N2O4ClF |
| | 486.966 |
| Ethyl (3*RS*)-{8-chloro-6-fluoro-3-[3-(3-methylphenyl)-propionylamino]-1,2,3,4-tetrahydro-9*H*-carbazol-9-yl}-acetate | C22H28N2O3ClF |
| | 470.967 |
| Ethyl (3*RS*)-{8-chloro-6-fluoro-3-[3-(2-hydroxy-phenyl)-propionylamino]-1,2,3,4-tetrahydro-9*H*-carbazol-9-yl}-acetate | C25H26N2O4ClF |
| | 472.939 |
| Ethyl (3*RS*)-[8-chloro-6-fluoro-3-(3-phenyl-propionylamino)-1,2,3,4-tetrahydro-9*H*-carbazol-9-yl]-acetate | C25H26N2O3ClF |
| | 456.94 |
| Ethyl (3*RS*)-{8-chloro-6-fluoro-3-[3-(2-methoxy-phenyl)-propionylamino]-1,2,3,4-tetrahydro-9*H*-carbazol-9-yl}-acetate | C26H28N2O4ClF |
| | 486.966 |
| Ethyl (3*RS*)-{8-chloro-3-[3-(3-chloro-phenyl)-propionylamino]-6-fluoro-1,2,3,4-tetrahydro-9*H*-carbazol-9-yl}-acetate | C25H25N2O3Cl2F |
| | 491.385 |
| Ethyl (3*RS*)-[8-chloro-6-fluoro-3-(3-*1H*-indol-3-yl-propionylamino)-1,2,3,4-tetrahydro-9*H*-carbazol-9-yl]-acetate | C27H27N3O3ClF |
| | 495.977 |
| Ethyl (3*RS*)-{8-chloro-3-[2-(2-chloro-phenoxy)-acetylamino]-6-fluoro-1,2,3,4-tetrahydro-9*H*-carbazol-9-yl}-acetate | C24H23N2O4Cl2F |
| | 493.357 |
| Ethyl (3*RS*)-{8-chloro-6-fluoro-3-[2-(2-methylphenyl)-oxy-acetylamino]-1,2,3,4-tetrahydro-9*H*-carbazol-9-yl}-acetate | C25H26N2O4ClF |
| | 472.939 |
| Ethyl (3*RS*)-[3-(3-benzo[1,3]dioxol-5-yl-propionylamino)-8-chloro-6-fluoro-1,2,3,4-tetrahydro-9*H*-carbazol-9-yl]-acetate | C26H26N2O5ClF |
| | 500.949 |
| Ethyl (3*RS*)-{8-chloro-6-fluoro-3-[2-(3-methoxy-phenoxy)-acetylamino]-1,2,3,4-tetrahydro-9*H*-carbazol-9-yl}-acetate | C25H26N2O5ClF |
| | 488.938 |
| Ethyl (3*RS*)-{8-chloro-3-[2-(3-chloro-phenoxy)-acetylamino]-6-fluoro-1,2,3,4-tetrahydro-9*H*-carbazol-9-yl}-acetate | C24H23N2O4Cl2F |
| | 493.357 |
| Ethyl (3*RS*)-{8-chloro-3-[3-(2-chloro-phenyl)-propionylamino]-6-fluoro-1,2,3,4-tetrahydro-9*H*-carbazol-9-yl}-acetate | C25H25N2O3Cl2F |
| | 491.385 |
| Ethyl (3*RS*)-[8-chloro-6-fluoro-3-(2-indan-2-yl-acetylamino)-1,2,3,4-tetrahydro-9*H*-carbazol-9-yl]-acetate | C27H28N2O3ClF |
| | 482.978 |
| Ethyl (3*S*)-(6-fluoro-3-{[2-(4-methoxy-phenyl)-acetyl]-methyl-amino}-1,2,3,4-tetrahydro-9*H*-carbazol-9-yl)-acetate | C26H29N204F |
| | 452.521 |
| Ethyl (3*S*)-(6-fluoro-3-{methyl-[2-(4-methylphenyl)-acetyl]-amino}-1,2,3,4-tetrahydro-9*H*-carbazol-9-yl)-acetate | C22H29N203F |
| | 436.522 |
| Ethyl (3*S*)-(6-fluoro-3-{[2-(2-methoxy-phenyl)-acetyl]-methyl-amino}-1,2,3,4-tetrahydro-9*H*-carbazol-9-yl)-acetate | C26H29N204F |
| | 452.521 |
| Ethyl (3*S*)-{6-fluoro-3-[(2-indan-2-yl-acetyl)-methyl-amino]-1 ,2,3,4-tetrahydro-9*H*-carbazol-9-yl}-acetate | C28H31 N203F |
| | 462.559 |
| Ethyl (3*S*)-(3-{[2-(3-chloro-phenyl)-acetyl]-methyl-amino}-6-fluoro-1,2,3,4-tetrahydro-9*H*-carbazol-9-yl)-acetate | C25H26N2O3ClF |
| | 456.94 |
| Ethyl (3*S*)-(6-fluoro-3-{methyl-[2-(3-methylphenyl)-acetyl]-amino}-1,2,3,4-tetrahydro-9*H*-carbazol-9-yl)-acetate | C26H29N203F |
| | 436.522 |
| Ethyl (3*S*)-(6-fluoro-3-{[2-(3-methoxy-phenyl)-acetyl]-methyl-amino}-1,2,3,4-tetrahydro-9*H*-carbazol-9-yl)-acetate | C26H29N204F |
| | 452.521 |
| Ethyl (3*S*)-(3-{[2-(2-chloro-phenoxy)-acetyl]-methyl-amino}-6-fluoro-1,2,3,4-tetrahydro-9*H*-carbazol-9-yl)-acetate | C25H26N2O4ClF |
| | 472.939 |
| Ethyl (3*S*)-(3-{[2-(4-chloro-phenoxy)-acetyl]-methyl-amino}-6-fluoro-1,2,3,4-tetrahydro-9*H*-carbazol-9-yl)-acetate | C25H26N2O4ClF |
| | 472.939 |
| Ethyl (3*S*)-(6-fluoro-3-{[3-(3-methoxy-phenyl)-propionyl]-methyl-amino}-1,2,3,4-tetrahydro-9*H*-carbazol-9-yl)-acetate | C27H31N2O4F |
| | 466.547 |
| Ethyl (3*S*)-(6-fluoro-3-{methyl-[2-(2-methylphenyl)-acetyl]-amino}-1,2,3,4-tetrahydro-9*H*-carbazol-9-yl)-acetate | C26H29N203F |
| | 436.522 |
| Ethyl (3*S*)-{3-[(3,3-diphenyl-propionyl)-methyl-amino]-6-fluoro-1,2,3,4-tetrahydro-9*H*-carbazol-9-yl}-acetate | C32H33N203F |
| | 512.619 |
| Ethyl (3*S*)-(6-fluoro-3-{[3-(2-methoxy-phenyl)-propionyl]-methyl-amino}-1,2,3,4-tetrahydro-9*H*-carbazol-9-yl)-acetate | C27H31N204F |
| | 466.547 |
| Ethyl (3*S*)-{6-fluoro-3-[(3-1H-indol-3-yl-propionyl)-methyl-amino]-1,2,3,4-tetrahydro-9*H*-carbazol-9-yl}-acetate | C28H30N303F |
| | 475.558 |
| Ethyl (3*S*)-{3-[(2-benzyloxy-acetyl)-methyl-amino]-6-fluoro-1,2,3,4-tetrahydro-9*H*-carbazol-9-yl}-acetate | C26H29N204F |
| | 452.521 |
| Ethyl (3*S*)-{3-[(2,3-diphenyl-propionylmethyl-amino]-6-fluoro-1,2,3,4-tetrahydro-9*H*-carbazol-9-yl}-acetate | C32H33N203F |
| | 512.619 |
| Ethyl (3*S*)-{6-fluoro-3-[[3-(2-methoxy-phenyl)-propionyl]-(3-phenyl-propyl)-amino]-1,2,3,4-tetrahydro-9*H*-carbazol-9-yl}-acetate | C35H39N204F |
| | 570.699 |
| Ethyl (3*S*)-{3-[acetyl-(3-phenyl-propyl)-amino]-6-fluoro-1,2,3,4-tetrahydro-9*H*-carbazol-9-yl}-acetate | C27H31N203F |
| | 450.548 |
| Ethyl (3*S*)-{3-[cyclopropylmethyl-(3-phenyl-propionyl)-amino]-6-fluoro-1,2,3,4-tetrahydro-9*H*-carbazol-9-yl}-acetate | C29H33N203F |
| | 476.586 |
| Ethyl (3*S*)-{3-[cyclopropylmethyl-((*S*)-2-methyl-3-phenyl-propionyl)-amino]-6-fluoro-1,2,3,4-tetrahydro-9*H*-carbazol-9-yl}-acetate | C30H35N2O3F |
| | 490.613 |
| Ethyl (3*S*)-(3-{cyclopropylmethyl-[3-(2-methoxy-phenyl)-propionyl]-amino}-6-fluoro-1,2,3,4-tetrahydro-9*H*-carbazol-9-yl)-acetate | C30H35N2O4F |
| | 506.612 |
| Ethyl (3*S*)-(3-{[2-(3-chloro-phenoxy)-acetyl]-cyclopropylmethyl-amino}-6-fluoro-1,2,3,4-tetrahydro-9*H*-carbazol-9-yl)-acetate | C28H30N2O4ClF |
| | 513.003 |
| Ethyl (3*S*)-{3-[cyclopropylmethyl-(3,3-diphenyl-propionyl)-amino]-6-fluoro-1,2,3,4-tetrahydro-9*H*-carbazol-9-yl}-acetate | C35H37N2O3F |
| | 552.684 |
| Ethyl (3*S*)-{3-[cyclopropylmethyl-(2-naphthalen-1-yl-acetyl}-amino]-6-fluoro-1,2,3,4-tetrahydro-9*H*-carbazol-9-yl}-acetate | C32H33N2O3F |
| | 512.619 |
| Ethyl(3*S*)-(3-{acetyl-[2-(4-trifluoromethyl-phenoxy)-ethyl]-amino}-6-fluoro-1,2,3,4-tetrahydro-9*H*-carbazol-9-yl)-acetate | C27H28N2O4F4 |
| | 520.518 |
| Ethyl (3*S*)-(6-fluoro-3-{propionyl-[2-(4-trifluoromethyl-phenoxy)-ethyl]-amino}-1,2,3,4-tetra hydro-9*H*-carbazol-9-yl )-acetate | C28H30N2O4F4 |
| | 534.544 |
| Ethyl (3*S*)-(6-fluoro-3-{(3-phenyl-propionyl)-[2-(4-trifluoromethyl-phenoxy)-ethyl]-amino}-1,2,3,4-tetrahydro-9*H*-carbazol-9-yl)-acetate | C34H34N2O4F4 |
| | 610.642 |
| Ethyl (3*S*)-(6-fluoro-3-{[3-(2-methoxy-phenyl)-propionyl]-[2-(4-trifluoromethyl-phenoxy)-ethyl]-amino}-1,2,3,4-tetrahydro-9*H*-carbazol-9-yl)-acetate | C35H36N2O5F4 |
| | 640.668 |
| Ethyl (3*S*)-{6-fluoro-3-[(2-phenoxy-ethyl)-(3-phenyl-propionyl)-amino]-1,2,3,4-tetrahydro-9*H*-carbazol-9-yl}-acetate | C33H35N2O4F |
| | 542.645 |
| Ethyl(3*S*)-{6-fluoro-3-[((*S*)-2-methyl-3-phenyl-propionyl)-(2-phenoxy-ethyl)-amino]-1,2,3,4-tetrahydro-9*H*-carbazol-9-yl}-acetate | C34H37N204F |
| | 556.672 |
| Ethyl (3*S*)-{6-fluoro-3-[[3-(2-methoxy-phenyl)-propionyl]-(2-phenoxy-ethyl)-amino]-1,2,3,4-tetrahydro-9*H*-carbazol-9-yl}-acetate | C34H37N2O5F |
| | 572.671 |
| Ethyl (3*S*)-{3-[acetyl-(2-phenoxy-ethyl)-amino]-6-fluoro-1,2,3,4-tetrahydro-9*H*-carbazol-9-yl}-acetate | C26H29N2O4F |
| | 452.521 |
| Ethyl (3*S*)-{6-fluoro-3-[(2-methoxy-ethyl)-(3-phenyl-propionyl)-amino]-1,2,3,4-tetrahydro-9*H*-carbazol-9-yl}-acetate | C28H33N2O4F |
| | 480.574 |
| Ethyl (3*S*)-{6-fluoro-3-[(2-methoxy-ethyl)-((S)-2-methyl-3-phenyl-propionyl)-amino]-1,2,3,4-tetra hydro-9*H*-carbazol-9-yl}-acetate | C29H35N2O4F |
| | 494.601 |
| Ethyl (3*S*)-(6-fluoro-3-{(2-methoxy-ethyl)-[3-(2-methoxy-phenyl)-propionyl]-amino}-1,2,3,4-tetrahydro-9*H*-carbazol-9-yl )-acetate | C29H35N2O5F |
| | 510.6 |
| Ethyl (3*S*)-{3-[[2-(3-chloro-phenoxy)-acetyl]-(2-methoxy-ethyl)-amino]-6-fluoro-1,2,3,4-tetrahydro-9*H*-carbazol-9-yl}-acetate | C27H30N2O5ClF |
| | 516.991 |
| Ethyl (3*S*)-{3-[(3,3-diphenyl-propionyl)-(2-methoxy-ethyl)-amino]-6-fluoro-1,2,3,4-tetrahydro-9*H*-carbazol-9-yl}-acetate | C34H37N2O4F |
| | 556.672 |
| Ethyl (3*S*)-{6-fluoro-3-[(2-methoxy-ethyl)-(2-naphthalen-1-yl-acetyl)-amino]-1,2,3,4-tetrahydro-9*H*-carbazol-9-yl}-acetate | C31H33N2O4F |
| | 516.607 |
| Ethyl (3*S*)-(6-fluoro-3-{[(2*S*)-2-methyl-3-phenyl-propionyl]-phenethyl-amino}-1,2,3,4-tetrahydro-9*H*-carbazol-9-yl)-acetate | C34H37N2O3F |
| | 540.673 |
| Ethyl (3*S*)-(6-fluoro-3-{[3-(2-methoxy-phenyl)-propionyl]-phenethyl-amino}-1,2,3,4-tetrahydro-9*H*-carbazol-9-yl)-acetate | C34H37N2O4F |
| | 556.672 |
| Ethyl (3*S*)-[3-(acetyl-phenethyl-amino)-6-fluoro-1,2,3,4-tetrahydro-9*H*-carbazol-9-yl]-acetate | C26H29N2O3F |
| | 436.522 |
| Ethyl (3*S*)-{6-fluoro-3-[(2-naphthalen-1-yl-acetyl)-phenethyl-amino]-1,2,3,4-tetrahydro-9*H*-carbazol-9-yl}-acetate | C36H35N2O3F |
| | 562.679 |
| Ethyl (3*S*)-{6-fluoro-3-[phenethyl-(3-phenyl-propionyl)-amino]-1,2,3,4-tetrahydro-9*H*-carbazol-9-yl}-acetate | C33H35N2O3F |
| | 526.646 |
| Ethyl (3*S*)-[3-(3-benzyl-1-naphthalen-1-ylmethyl-ureido)-6-fluoro-1,2,3,4-tetrahydro-9*H*-carbazol-9-yl]-acetate | C35H34N3O3F |
| | 563.667 |
| Ethyl (3*S*)-[3-(benzyloxycarbonyl-naphthalen-1-ylmethyl-amino)-6-fluoro-1,2,3,4-tetrahydro-9*H*-carbazol-9-yl]-acetate | C35H31N2O4F |
| | 564.651 |
| Ethyl (3*S*)-{6-fluoro-3-[naphthalen-1-ylmethyl-(3-phenyl-propionyl)-amino]-1,2,3,4-tetrahydro-9*H*-carbazol-9-yl}-acetate | C36H35N2O3F |
| | 562.679 |
| Ethyl (3*S*)-{6-fluoro-3-[((*S*)-2-methyl-3-phenyl-propionyl)-naphthalen-1-ylmethyl-amino]-1,2,3,4-tetrahydro-9*H*-carbazol-9-yl}-acetate | C37H37N2O3F |
| | 576.706 |
| Ethyl (3*S*)-(6-fluoro-3-{[3-(2-methoxy-phenyl)-propionyl]-naphthalen-1-ylmethyl-amino}-1,2,3,4-tetrahydro-9*H*-carbazol-9-yl)-acetate | C37H37N2O4F |
| | 592.705 |
| Ethyl (3*S*)-{3-[(3,3-diphenyl-propionyl)-naphthalen-1-ylmethyl-amino]-6-fluoro-1,2,3,4-tetrahydro-9*H*-carbazol-9-yl}-acetate | C42H39N2O3F |
| | 638.777 |
| Ethyl (3*S*)-[3-(acetyl-naphthalen-1-ylmethyl-amino)-6-fluoro-1,2,3,4-tetrahydro-9*H*-carbazol-9-yl]-acetate | C29H29N2O3F |
| | 472.555 |
| Ethyl (3*S*)-[6-fluoro-3-(naphthalen-1-ylmethyl-propionyl-amino)-1,2,3,4-tetrahydro-9H-carbazol-9-yl]-acetate | C30H31N2O3F |
| | 486.581 |
| Ethyl (3*S*)-{6-fluoro-3-[3-phenyl-4-([1,3,4]thiadiazol-2-ylcarbamoyl)-butyrylamino]-1,2,3,4-tetrahydro-9*H*-carbazol-9-yl}-acetate | C29H30FN5O4S |
| | 563.64 |
| Ethyl (3*S*)-[6-fluoro-3-(3-thiophen-2-yl-propionylamino)-1,2,3,4-tetrahydro-9*H*-carbazol-9-yl]-acetate | C23H25FN2O3S |
| | 428.012 |
| Ethyl (3*S*)-{3-[3-(3-chloro-isoxazol-5-yl)-propionylamino]-6-fluoro-1,2,3,4-tetrahydro-9*H*-carbazol-9-yl}-acetate | C22H23ClFN3O4 |
| | 447.88 |
| Ethyl (3*S*)-[6-Fluoro-3-(3-pyrimidin-2-yl-propionylamino)-1,2,3,4-tetrahydro-9*H*-carbazol-9-yl]-acetate | C23H25FN4O3 |
| | 424.46 |
| Ethyl (3*S*)-(3-{acetyl-[2-(2-fluoro-phenyl)-ethyl]-amino}-6-fluoro-1,2,3,4-tetrahydro-9*H*-carbazol-9-yl)-acetate | C26H28F2N2O3 |
| | 454.51 |
| Ethyl (3*S*)-(3-{acetyl-[2-(3-fluoro-phenyl)-ethyl]-amino}-6-fluoro-1,2,3,4-tetrahydro-9*H*-carbazol-9-yl)-acetate | C26H28F2N2O3 |
| | 454.51 |
| Ethyl (3*S*)-(3-{cyclohexylmethyl-[3-(2-methoxy-phenyl)-propionyl]-amino}-6-fluoro-1,2,3,4-tetrahydro-9*H*-carbazol-9-yl )-acetate | C33H41N2O4F |
| | 548.693 |

### General Method for the Preparation of Intermediates of Structure 1, wherein R⁴ represents C₁₋₅-alkyl or allyl

To a stirred and degassed solution of an appropriately protected ethyl (3-amino-8-bromo-1,2,3,4-tetrahydro-9*H*carbazol-9-yl)-acetate derivative of Structure **2c** (0.2 mmol) and Pd(PPh₃)₄ (0.02 mmol, 0.1 eq.) in dry DMF (1.5 ml) is added under inert atmosphere the appropriate tetraC₁₋₅-alkyltin or allyltrialkyltin, respectively (0.22 mmol, 1.1 eq.). The reaction mixture is allowed to stir overnight at 110°C. After cooling to rt, acetonitrile (1 ml) and heptane (1 ml) are added. The acetonitrile-DMF phase is washed three times with heptane. Water is then added and the resulting aq. phase is extracted twice with AcOEt. The combined org. phases arc washed with brine and dried over Na₂SO₄. Evaporation of the solvent *in vaccuo* yields the protected ethyl (3-amino-8-C₁₋₅-alkyl-1,2,3,4-tetrahydro-9*H-*carbazol-9-yl)-acetate derivative or ethyl (3-amino-8-allyl-1,2,3,4-tetrahydro-9*H*-carbazol-9-yl)-acetate derivative of Structure **1**, respectively.

Intermediates of Structure **1** wherein R⁴ represents C₁₋₅-alkyl or allyl:
Ethyl (3S)-(3-benzyloxycarbonylamino-6-fluoro-8-methyl-1,2,3,4-tetrahydro-9H-carbazol-9-yl)-acetate is obtained in quantative yield as a yellow oil. t_{R} = 1.09 min (LC-3), ESI-MS (pos.): m/z 439.15 [M+H]⁺.
Ethyl (3*S*)-(3-benzyloxycarbonylamino-6-fluoro-8-allyl-1,2,3,4-tetrahydro-9*H*-carbazol-9-yl)-acetate is obtained in quantative yield as a yellow oil. t_{R} = 1.11 min (LC-3), ESI-MS (pos.): m/z 465.22 [M+H]⁺.

### General Method for the Preparation of Intermediates of Structure 1 wherein R⁴ represents vinyl

A suspension of an appropriately protected ethyl (3-amino-8-bromo-1,2,3,4-tetrahydro-9*H-*carbazol-9-yl)-acetate derivative of Structure **2c** (0.4 mmol), vinylboronic anhydride pyridine complex (0.22 mmol, 0.56 eq.), Pd(PPh₃)₄ (23 mg, 0.02 mmol, 0.05 eq.), and K₂CO₃ (55 mg, 0.4 mmol, 1 eq.) in 1 ml (DME/ H₂O) is stirred for 4 h at reflux. Water is added and the resulting aq. phase is extracted three times with AcOEt. The combined org. phases are dried over Na₂SO₄, filtered and concentrated *in vaccuo.* The crude product is purified by FC (heptane / AcOEt, 3:1) to yield protected ethyl (3-amino-8-vinyl-1,2,3,4-tetrahydro-9H-carbazol-9-yl)-acetate derivatives of Structure **1**.

Intermediate of Structure **1** wherein **R⁴** represents vinyl:
Ethyl (3*S*)-(3-benzyloxycarbonylamino-6-fluoro-8-vinyl-1,2,3,4-tetrahydro-9H-carbazol-9-yl)-acetate is obtained in 87% yield as a white solid. t_{R} = 1.11 min (LC-3), ESI-MS (pos.): m/z 451.19 [M+H]⁺.

### General Method for the Preparation of Intermediates of Structure 1 wherein R⁴ represents C₁₋₆-alkylsulfonyl

A solution of an appropriately protected ethyl (3-amino-8-bromo-1,2,3,4-tetrahydro-9H-carbazol-9-yl)-acetate derivative of Structure 2c (0.238 mmol), cuprous iodide (204 mg, 1.073 mmol, 4.5 eq.) and sodium methanesulfinate (129 mg, 1.073 mmol, 4.5 eq.) in degassed NMP (5 ml) is heated under inert atmosphere at 140°C overnight. Then, the mixture is diluted with heptane (5 ml) and AcOEt (5 ml) and filtered over a pad of silica gel with AcOEt as eluent. The solvent is removed *in vaccuo* and the residue dissolved in AcOEt and H₂O. The phases are separated and the aq. phase is extracted twice with AcOEt. The combined org. phases are washed with brine and H₂O, dried over Na₂SO₄ and concentrated *in vacuuo* to yield the corresponding ethyl (3-amino-8-methanesulfonyl-1,2,3,4-tetrahydro-9*H*-carbazol-9-yl)-acetate derivative of Structure **1**.

Intermediate of Structure **1** wherein R⁴ represents C₁₋₆-alkylsulfonyl:
Ethyl (3*RS*)-(3-benzyloxycarbonylamino-6-fluoro-8-methanesulfonyl-1,2,3,4-tetrahydro-9*H*-carbazol-9-yl)-acetate is obtained in quantitative yield as a brown solid. t_{R} = 1.04 min (LC-3), ESI-MS (pos.): m/z 503.12 [M+H]⁺.

### General Methods for the Preparation of Intermediates of Structure 2a:

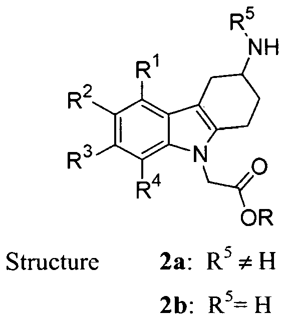

### Method (A)

### Step A) 4-Nitro-benzenesulfonylation of an intermediate of Structure 2b to yield an ethyl [3-(4-nitro-benzenesulfonylamino)-1,2,3,4-tetrahydro-9H-carbazol-9-yl]-acetate derivative of Structure 3a:

A catalytical amount of DMAP and p-nitrobenzenesulfonyl chloride (223 mg, 1.01 mmol) arc added to an ice cold stirred solution of the appropriate (3-amino-1,2,3,4-tetrahydro-9H carbazol-9-yl)-acetate derivative hydrochloride of Structure **2b** (0.92 mmol) and pyridine (0.96 ml, 11.9 mmol) in DCM. The reaction mixture is allowed to warm up to rt and is continued to stir overnight. The reaction is then quenched by addition of H₂O and sat. NaHCO₃ solution. After phase separation the aq. phase is extracted with DCM. The combined org. phases are dried over Na₂SO₄, filtered, and the solvent is evaporated to dryness. The crude product is filtered through a plug of silica gel (heptane/AcOEt, 2:1) to give the desired intermediate of Structure **3a**.

Intermediate of Structure **3a:** Ethyl (3S)-[6-fluoro-3-(4-nitro-benzenesulfonylamino)-1,2,3,4-tetrahydro-9*H*-carbazol-9-yl]-acetate is obtained in 71% yield as a yellow solid: t_{R} = 1.05 min (LC-3), ESI-MS (pos.): m/z 476.12 [M+H]⁺.

### Step B) N-Substitution of a 4-nitro-benzenesulfonamide intermediate of Structure 3a to yield intermediates of Structure 3b:

Following a procedure described in the literature (Peña, C. et al. Tetrahedron Lett. 2005, 46, 2783-2787), a stirred suspension of the appropriate intermediate of Structure **3a** (0.21 mmol), K₂CO₃ (291 mg, 2.1 mmol) and tetrabutylammonium bromide (6.78 mg, 0.021 mmol) in toluene (2 ml) is heated at 70°C for 30 min before adding the corresponding alkylating agent **R⁵**-L (0.841 mmol). The reaction mixture is continued to stir at 70°C overnight, cooled to rt, and treated with sat. NH₄Cl solution. After phase separation, the aq. layer is extracted three times with DCM. The combined org. phases are dried over Na₂SO₄, filtered, and the solvent evaporated to dryness, affording the corresponding intermediate of Structure **3b** in quantitative yield.

Listed in Table 5 below are intermediates of Structure **3b**, prepared according to the above-mentioned method.

**Table 5**

| **Intermediates of Structure 3b** | **Formula MW** | **t_{R} [min] Meth.** | **MS Data m/z [M+H]⁺** |
|---|---|---|---|
| Ethyl (3*S*)-{6-fluoro-3-[methyl-(4-nitro-benzenesulfonyl)-amino]-1,2,3,4-tetrahydro-9*H*-carbazol-9-yl}-acetate | C23H24N306F | 1.09 | 490.05 |
| | 489.52 | (LC-3) | |
| Ethyl (3*S*)-{6-fluoro-3-[ethyl-(4-nitro-benzenesulfonyl)-amino]-1,2,3,4-tetrahydro-9H-carbazol-9-yl}-acetate | C24H26N3O6FS | 1.11 | 504.15 |
| | 503.549 | LC-3 | |
| Ethyl (3*S*)-{6-fluoro-3-[propyl-(4-nitro-benzenesulfonyl)-amino]-1,2,3,4-tetrahydro-9*H*-carbazol-9-yl}-acetate | C25H28N3O6FS | 1.13 | 518.23 |
| | 517.576 | LC-3 | |

Listed in Table 5a below are further intermediates of Structure **3b**, prepared according to the above-mentioned method.

**Table 5a**

| **Intermediates of Structure 3b** | **Formula MW** | **t_{R} [min] Method** | **MS Data m/z [M+H]⁺** |
|---|---|---|---|
| Ethyl(3*S*)-{6-fluoro-3-[(4-nitro-benzenesulfonyl)-(3-phenyl-propyl)-amino]-1,2,3,4-tetrahydro-9*H*-carbazol-9-yl}-acetate | C31H32N3O6FS | 1.16 | 594.12 |
| | 593.674 | LC-3 | |
| Ethyl (3*S*){-3-[cyclopropylmethyl-(4-nitro-benzenesulfonyl)-amino]-6-fluoro-1,2,3,4-tetrahydro-9*H*-carbazol-9-yl}-acetate | C26H28N3O6FS | 1.12 | 530.02 |
| | 529.587 | LC-3 | |
| Ethyl (3*S*)-(6-fluoro-3-{(4-nitro-benzenesulfonyl)-[2-(3-trifluoromethyl-phenoxy)-ethyl]-amino}-1,2,3,4-tetrahydro-9*H*-carbazol-9-yl)-acetate | C31H29N3O7F4S | 1.17 | 664.17 |
| | 663.643 | LC-3 | |
| Ethyl (3*S*)-{6-fluoro-3-[(4-nitro-benzenesulfonyl)-(2-phenoxy-ethyl)-amino]-1,2,3,4-tetrahydro-9*H*-carbazol-9-yl}-acetate | C30H30N3O7FS | 1.14 | 596.18 |
| | 595.646 | LC-3 | |
| Ethyl (3*S*)-{6-fluoro-3-[(2-methoxy-ethyl)-(4-nitro-benzenesulfonyl)-amino]-1,2,3,4-tetrahydro-9*H*-carbazol-9-yl}-acetate | C25H28N3O7FS | 1.09 | 534.16 |
| | 533.575 | LC-3 | |

### Step C) Cleavage of the 4-nitro-benzenesulfonyl group to yield an intermediate of Structure 2a:

In analogy to the literature (Miller, S. C.; Scanlan, T. S. J. Am. Chem. Soc. 1997, 119, 2301-2302), mercaptoacetic acid (0.019 ml, 0.267 mmol) and DBU (0.081 ml, 0.53 mmol) are added to a stirred solution of an intermediate of Structure **3b** (0.179 mmol) in dry DMF (2 ml). The reaction mixture is allowed to stir overnight, then, at rt, sat. Na₂CO₃ solution, **H₂O** and DCM are added. After phase separation, the org. layer is extracted twice with sat. Na₂CO₃ solution, and twice with H₂O. The combined org. phases are washed with brine and dried over Na₂SO₄. After filtration, the solvent is evaporated and the residue is purified by preparative tlc on silica gel (DCM/MeOH/NH₄OH, 90:10:1) to give the desired intermediate of Structure **2a** in 30-40% yield.

Listed in Table 6 below are intermediates of Structure **2a**, prepared according to the above-mentioned method.

**Table 6**

| **Intermediates of Structure 2a** | **Formula MW** | **t_{R} [min] Method** | **MS Data m/z [M+H]⁺** |
|---|---|---|---|
| Ethyl (3*S*)-(6-fluoro-3-methylamino-1,2,3,4-tetrahydro-9*H*-carbazol-9-yl)-acetate | C17H21FN2O2 | 0.76 | 305.19 |
| | 304.36 | LC-3 | |
| Ethyl (3*S*)-(6-fluoro-3-ethylamino-1,2,3,4-tetrahydro-9*H*-carbazol-9-yl)-acetate | C18H23FN2O2 | 0.78 | 319.14 |
| | 318.39 | LC-3 | |
| Ethyl (3*S*)-(6-fluoro-3-propylamino-1,2,3,4-tetrahydro-9*H*-carbazol-9-yl}-acetate | C19H25FN2O2 | 0.81 | 333.15 |
| | 332.41 | LC-3 | |

Listed in Table 6a below are further intermediates of Structure **2a,** prepared according to the abovementioned method.

**Table 6a**

| **Intermediates of Structure 2a** | **Formula MW** | **t_{R} [min] LC-MS Method** | **MS Data m/z [M+H]⁺** |
|---|---|---|---|
| Ethyl (3*S*)-[6-fluoro-3-(3-phenyl-propylamino)-1,2,3,4-tetrahydro-9*H*-carbazol-9-yl]-acetate | C25H29N2O2F | 0.91 | 409.15 |
| | 408.515 | LC-3 | |
| Ethyl (3*S*)-[3-(cyclopropylmethyl-amino)-6-fluoro-1,2,3,4-tetrahydro-9*H*-carbazol-9-yl]-acetate | C20H25N2O2F | 0.82 | 345.18 |
| | 344.428 | LC-3 | |
| Ethyl (3*S*)-{6-fluoro-3-[2-(4-trifluoromethyl-phenoxy)-ethylamino]-1,2,3,4-tetrahydro-9*H*-carbazol-9-yl}-acetate | C25H26N2O3F4 | 0.95 | 479.07 |
| | 478.484 | LC-3 | |
| Ethyl (3*S*)-[6-fluoro-3-(2-phenoxy-ethylamino)-1,2,3,4-tetrahydro-9*H*-carbazol-9-yl)-acetate | C24H27N2O3F | 0.88 | 411.10 |
| | 410.487 | LC-3 | |
| Ethyl (3*S*)-[6-fluoro-3-(2-methoxy-ethylamino)-1,2,3,4-tetrahydro-9*H*-carbazol-9-yl]-acetate | C19H25N2O3F | 0.77 | 349.15 |
| | 348.417 | LC-3 | |

### Method (B)

To a stirred suspension of an appropriate ethyl (3-amino-1,2,3,4-tetrahydro-9H-carbazol-9-yl)-acetate derivative hydrochloride of Structure **2b** (0.73 mmol) and DIEA (0.769 mmol, 0.132 ml, 1.05 eq.) and the corresponding aldehyde (0.806 mmol, 1.1 eq.) in DCM (10 ml) is added NaBH(OAc)₃ (1.62 mmol, 2.2 eq.). The reaction mixture is stirred overnight and diluted with DCM and sat. NaHCO₃ solution. The resulting aq. phase is extracted three times with DCM. The combined org. phases are dried over Na₂SO₄, filtered, and the solvent is evaporated to dryness. The crude product is purified by flash-chromatography on silica gel (DCM/MeOH, 95:5) to give the desired intermediate of Structure **2a** in 66 to 95% yield.

Listed in Table 6b below are intermediates of Structure **2a**, prepared according to the abovementioned method.

**Table 6b**

| **Intermediates of Structure 2a** | **Formula MW** | **t_{R} [min] LC-MS Method** | **MS Data m/z [M+H]⁺** |
|---|---|---|---|
| Ethyl (3*S*)-(6-fluoro-3-phenethylamino-1,2,3,4-tetra hydro-9*H*-carbazol-9-yl )-acetate | C24H27N2O2F | 0.88 | 395.18 |
| | 394.488 | LC-3 | |
| Ethyl (3*S*)-{6-fluoro-3-[(naphthalen-1-ylmethyl)-amino]-1,2,3,4-tetrahydro-9*H*-carbazol-9-yl}-acetate | C27H27N2O2F | 0.91 | 431.22 |
| | 430.521 | LC-3 | |
| Ethyl (3*S*)-(3-benzylamino-6-fluoro-1,2,3,4-tetrahydro-9*H*-carbazol-9-yl)-acetate | C23H25N2O2F | 0.89 | 381.16 |
| | 380.461 | LC-3 | |
| Ethyl (3*S*)-(6-fluoro-3-[2-(2-fluoro-phenyl)-ethylamino]-1,2,3,4-tetrahydro-9*H*-carbazol-9-yl}-acetate | C24H26N2O2F2 | 0.88 | 413.14 |
| | 412.478 | LC-3 | |
| Ethyl (3*S*)-(6-fluoro-3-[2-(3-fluoro-phenyl)-ethylamino]-1,2,3,4-tetrahydro-9*H*-carbazol-9-yl}-acetate | C24H26N2O2F2 | 0.88 | 413.14 |
| | 412.478 | LC-3 | |
| Ethyl (3*S*)-[3-(cyclohexylmethyl-amino)-6-fluoro-1,2,3,4-tetrahydro-9*H*-carbazol-9-yl]-acetate | C23H31N2O2F | 0.88 | 387.20 |
| | 386.509 | LC-3 | |

### General Procedures for the Preparation of Intermediates of Structure 2b:

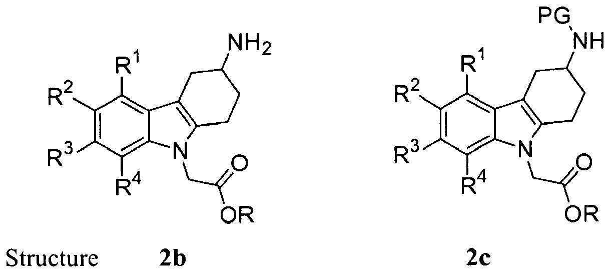

### Cleavage of PG= tert-butoxycarbonyl

To a stirred solution of an ethyl (3-tert-butoxycarbonylamino-1,2,3,4-tetrahydro-9H-carbazol-9-yl)-acetate derivative of Structure **2c** (1.61 mmol) in THF (4 ml) is added 2N HCl (2 ml) in diethylether, or in AcOEt. The reaction mixture is stirred overnight, and the formed precipitate is filtered off, rinsed with diethylether and dried to give the desired intermediate of Structure **2b** as a white solid in quantitative yield.

Listed in Table 7 below are intermediates of Structure **2b,** prepared according to the above-mentioned method, with corresponding intermediate of Structure **2c** as starting material.

**Table 7**

| **Intermediates of Structure 2b** | **Formula MW** | **t_{R} [min] Method** | **MS Data m/z [M+H]⁺ (parent)** |
|---|---|---|---|
| Ethyl (3*R*)-(3-amino-1,2,3,4-tetrahydro-9*H*-carbazol-9-yl)-acetate hydrochloride | C16H21N2O2Cl | 0.74 | 273.16 |
| | 308.807 | LC-2 | |
| Ethyl (3*S*)-(3-amino-1,2,3,4-tetrahydro-9*H*-carbazol-9-yl)-acetate hydrochloride | C16H21N2O2Cl | 0.74 | 273.16 |
| | 308.807 | LC-2 | |
| Ethyl (3*R*)-(3-amino-6-fluoro-1,2,3,4-tetra hydro-9*H*-carbazol-9-yl)-acetate hydrochloride | C16H20ClFN2O2 | 0.74 | 291.15 |
| | 326.79 | LC-2 | |
| Ethyl (3*S*)-(3-amino-6-fluoro-1,2,3,4-tetrahydro-9*H*-carbazol-9-yl)-acetate hydrochloride | C16H20N2O2ClF | 0.73 | 291.11 |
| | 326.79 | LC-2 | |

### Cleavage of PG= benzyloxycarbonyl

To a stirred solution of an ethyl (3-benzyloxycarbonylamino-1,2,3,4-tetrahydro-9H-carbazol-9-yl)-acetate derivative of Structure **2c** (7.58 mmol) in AcOH (85 ml) and EtOH (20 ml) is added Pd /C (806 mg, 0.76 mmol, 0.1 eq.). The reaction mixture is stirred for 1h under a H₂ atmosphere then diluted with DCM and filtered over a plug of celite. A solution of 4M HCl in dioxane (30 ml, 10 eq.) is added to the filtrate and the solvents are removed *in vaccuo* to give an intermediate of Structure **2b.**

Listed in Table **7a** below are further intermediates of Structure **2b,** prepared according to the above-mentioned methods, with the corresponding intermediate of Structure **2c** as starting material.

**Table 7a**

| **Intermediates of Structure 2b** | **Formula MW** | **t_{R} [min] LC-MS Method** | **MS Data m/z [M+H]⁺** |
|---|---|---|---|
| Ethyl (3*RS*)-(3-amino-8-chloro-6-fluoro-1,2,3,4-tetrahydro-carbazol-9-yl)-acetate hydrochloride | C16H19N2O2Cl2F | 0.78 | 325.05 |
| | 361.243 | LC-3 | |
| Ethyl (3*RS*)-(3-amino-6-fluoro-1,2,3,4-tetrahydro-carbazol-9-yl)-acetate hydrochloride | C20H19N2O2F | 1.02 | 339.12 |
| | 338.381 | LC-3 | |

### General Method for the Synthesis of Intermediates of Structure 2c:

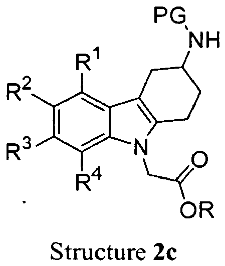

### Alkylation of an intermediate of Structure 4:

A solution of e.g. ethyl bromoacetate (1.25 ml, 11.25 mmol) in dry DMF (20 ml) is added dropwise to a heated (60°C) solution of an intermediate of Structure **4** (10.22 mmol) and Cs₂CO₃ (9.99 g, 30.67 mmol) in dry DMF (50 ml) over a period of 15 min. The resulting suspension is continued to stir at 60°C for 1 h, or overnight. After cooled to rt, the reaction mixture is filtered and washed with DCM. The DCM is evaporated and the residue is partitioned between AcOEt and H₂O. The aq. layer is extracted three times with AcOEt. The combined org. layers are washed with H₂O and brine, dried over MgSO₄ and filtered. The solvent is evaporated and the solid residue is purified by FC with a continuous gradient of eluents from AcOEt/ heptane 1:99 to 1:1 to give the desired intermediate of Structure **2c** in 40 to 80% yield.

Listed in Table below are intermediates of Structure **2c**, prepared according to the above-mentioned method, starting from corresponding intermediate of Structure **4.**

**Table 8**

| **Intermediates of Structure 2c** | **Formula MW** | **t_{R} [min] LC-MS Method** | **MS Data m/z [M+Na]⁺ or [M+H]⁺** |
|---|---|---|---|
| Ethyl (*3R*)*-*(*3-tert-*butoxycarbonylamino-1,2,3,4-tetrahydro-9H-carbazol-9-yl)-acetate | C21H28N2O4 | 1.15 | 394.95 |
| | 372.463 | LC-2 | |
| Ethyl (*3S*)*-*(3*-tert-*butoxycarbonylamino-1,2,3,4-tetrahydro-9*H*-carbazol-9-yl)-acetate | C21H28N2O4 | 1.15 | 395.15 |
| | 372.463 | LC-2 | |
| Ethyl (*3R*)*-*(3*-tert-*butoxycarbonylamino-6-fluoro-1,2,3,4-tetrahydro-9*H*-carbazol-9-yl)-acetate | C21H27FN2O4 | 1.15 | 413.09 |
| | 390.45 | LC-2 | |
| Ethyl (*3S*)*-*(3*-tert-*butoxycarbonylamino-6-fluoro-1,2,3,4-tetrahydro-9*H*-carbazol-9-yl)-acetate | C21H27FN2O4 | 1.16 | 413.09 |
| | 390.45 | LC-2 | |
| Ethyl (3*RS*)-(3-benzyloxycarbonylamino-8-chloro-5-fluoro-1,2,3,4-tetrahydro-9*H*-carbazol-9-yl)-acetate | C24H24N2O4ClF | 1.11 | 458.99 |
| | 458.916 | LC-3 | |
| Ethyl (3*RS*)-(3-benzyloxycarbonylamino-6-fluoro-1,2,3,4-tetrahydro-9H-carbazol-9-yl)-acetate | C24H25N2O4F | 1.06 | 425.22 |
| | 424.47 | LC-3 | |
| Ethyl (3*RS*)-(3-benzyloxycarbonylamino-8-chloro-6-fluoro-1,2,3,4-tetrahydro-9H-carbazol-9-yl)-acetate | C24H24N2O4ClF | 1.11 | 459.05 |
| | 458.916 | LC-3 | |

**Listed** in Table 8a below are further intermediates of Structure **2c,** prepared according to the above-mentioned method, starting from corresponding intermediate of Structure **4.**

**Table 8a**

| **Intermediates of Structure 2c** | **Formula MW** | **t_{R} [min] LC-MS Method** | **MS Data m/z [M+H]⁺** |
|---|---|---|---|
| Ethyl (3*RS*)-(3-benzyloxycarbonylamino-6-trifluoromethyl-1,2,3,4-tetrahydro-9H-carbazol-9-yl)-acetate | C25H25N2O4F3 | 1.11 | 475.14 |
| | 474.478 | LC-3 | |
| Ethyl (3*RS*)-(3-benzyloxycarbonylamino-8-bromo-6-fluoro-1,2,3,4-tetrahydro-9H-carbazol-9-yl)-acetate | C24H24N2O4BrF | 1.11 | 505.11 |
| | 503.367 | LC-3 | |
| Ethyl (3*S*)-(3-benzyloxycarbonylamino-7-chloro-6-fluoro-1,2,3,4-tetrahydro-9*H*-carbazol-9-yl)-acetate | C24H24ClFN2O4 | not determined | not determined |
| | 458.91 | | |
| Ethyl (3*R*)-(3-benzyloxycarbonylamino-8-chloro-1,2,3,4-tetrahydro-9*H*-carbazol-9-yl)-acetate | C24H25N2O4Cl | 1.1 | 441.07 |
| | 440.926 | LC-3 | |

Listed in Table 9 below are intermediates of Structure **4**, prepared in analogy to the procedure described in the literature (Ha, J. D. et al., Bulletin of the Korean Soc. Chem. 2004, 25, 1784-1790).

**Table 9**

| **Intermediates of Structure 4** | **Formula MW** | **t_{R} [min] Method** | **MS Data m/z [M+H]⁺** |
|---|---|---|---|
| (3RS)-(8-Chloro-5-fluoro-2,3,4,9-tetrahydro-1*H*-carbazol-3-yl)-carbamic acid benzyl ester | C20H18N2O2ClF | 1.07 | 373.03 |
| | 372.825 | LC-3 | |
| (3*R*S)-(8-Chloro-6-fluoro-2,3,4,9-tetrahydro-1*H*-carbazol-3-yl)-carbamic acid benzyl ester | C20H18N2O2ClF | 1.06 | 372.99 |
| | 372.825 | LC-3 | |
| (3*RS*)-(6-Fluoro-2,3,4,9-tetrahydro-1*H*-carbazol-3-yl)-carbamic acid benzyl ester | C20H19N2O2F | 1.02 | 339.12 |
| | 338.38 | LC-3 | |

Listed in Table 9a below are further intermediates of Structure **4**, prepared according to the abovementioned procedure.

**Table 9a**

| **Intermediates of Structure 4** | **Formula MW** | **t_{R} [min] Method** | **MS Data m/z [M+H]⁺** |
|---|---|---|---|
| (3*RS*)-(6-Trifluoromethyl-2,3,4,9-tetrahydro-1*H*-carbazol-3-yl)-carbamic acid benzyl ester | C21H19N2O2F3 | 1.07 | 389.08 |
| | 388.388 | LC-3 | |
| (3*RS*)-(8-Bromo-6-fluoro-2,3,4,9-tetrahydro-1*H*-carbazol-3-yl)-carbamic acid benzyl ester | C20H18N2O2BrF | 1.07 | 417.03 |
| | 417.277 | LC-3 | |
| (3*RS*)-(7-Chloro-6-fluoro-2,3,4,9-tetrahydro-1*H*-carbazol-3-yl)-carbamic acid benzyl ester | C20H18N2O2ClF | 1.05 | 373.07 |
| | 372.825 | LC-3 | |
| (3*R*)-(8-Chloro-2,3,4,9-tetrahydro-1 H-carbazol-3-yl)-carbamic acid benzyl ester | C20H19N2O2Cl | 1.05 | 355.11 |
| | 354.836 | LC-3 | |

### General Method for the Preparation of Intermediates of Structure 1 from intermediates of Structure 8a

To a solution of the respective amine (0.140 mmol, 1.5 eq.), HATU (0.140 mmol, 1.5eq.) and DIEA (0.048 ml, 0.280 mmol, 3 eq.) in 0.5 ml (DMF/THF 4:1) is added a solution of an intermediate of Structure **8a** in 0.5 ml (DMF/THF 4:1). The reaction mixture is stirred for 24h, then diluted with DCM and sat. NaHCO₃ solution. After stirring for an additional 1h, H₂O is added and the org. phase is separated. The aq. phase is extracted with DCM, the combined org. extracts are concentrated under a stream of air to yield the desired crude intermediate of Structure **1**.

Listed in Table **10** below are intermediates of Structure **1**, prepared according to the abovementioned method with corresponding intermediate of Structure **8a** as starting material.

**Table 10**

| **Intermediates of Structure 1 starting from intermediates of Structure 8a** | **Formula MW** | **t_{R} [min] LC-MS Method** | **MS Data m/z [M+H]⁺** |
|---|---|---|---|
| Ethyl (3*S*)-{3-[(*R*S)-2-benzyl-3-(2-methylbenzyl)-carbamoyl-propionylamino]-6-fluoro-1,2,3,4-tetrahydro-9*H*-carbazol-9-yl}-acetate | C34H36FN3O4 | 1.06 | 570.21 |
| | 569.67 | LC-3 | |
| Ethyl (3*S*)-{3-[(*RS*)-2-benzyl-3-(3-methoxy-phenylcarbamoyl)-propionylamino]-6-fluoro-1,2,3,4-tetrahydro-9*H*-carbazol-9-yl}-acetate | C34H36FN3O5 | 1.06 | 586.21 |
| | 585.67 | LC-3 | |
| Ethyl (3*S*)-{3-[(R*S*)-2-benzyl-3-(4-chloro-phenylcarbamoyl)-propionylamino]-6-fluoro-1,2,3,4-tetrahydro-9*H*-carbazol-9-yl}-acetate | C33H33ClFN3O4 | 1.09 | 590.2 |
| | 590.08 | LC-3 | |
| Ethyl (3*S*)-{3-[(*RS*)-2-benzyl-3-(4-fluoro-benzylcarbamoyl)-propionylamino]-6-fluoro-1,2,3,4-tetrahydro-9*H*-carbazol-9-yl}-acetate | C34H35F2N3O4 | 1.05 | 588.25 |
| | 587.66 | LC-3 | |
| Ethyl [(3*S*)-3-((*RS*)-2-benzyl-3-propylcarbamoyl-propionylamino)-6-fluoro-1,2,3,4-tetrahydro-9H-carbazol-9-yl]-acetate | C30H36FN3O4 | 1 | 522.26 |
| | 521.62 | LC-3 | |

### General Method for the Preparation of Intermediates of Structure 8a from intermediates of Structure 8b

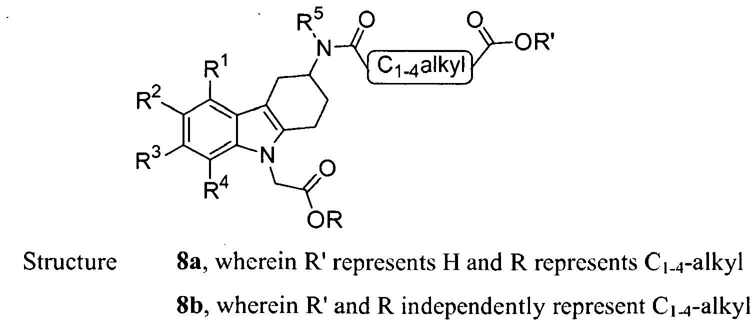

A solution of an intermediate of Structure **8b** (0.54 mmol) and TFA (0.8 ml, 10 mmol, 20 eq.) in DCM (8 ml) is stirred for 2.5h. The volatiles are removed under reduced pressure to yield an intermediate of Structure **8a.**

Intermediate of Structure **8a:** 3-benzyl-*N*-(9-ethoxycarbonylmethyl-6-fluoro-2,3,4,9-tetrahydro-1*H*-carbazol-3-yl)-succinamic acid is quantitative yield as light brown foam. t_{R} = 0.97 min (LC-3), ESI-MS (pos.): m/z 481.22 [M+H]⁺.

### General Method for the Preparation of Intermediates of Structure 8b from intermediates of Structure 2a or 2b

A solution of an appropriate intermediate of Structure **2a** or **2b** (2.16 mmol), an appropriate C₁₋₄alkanedicarboxylic acid mono-ester of Structure **9** (4.05 mmol, 1.9 eq.), DIEA (1.5 ml, 8.65 mmol, 4 eq.) and HATU (1.64 g, 4.32 mmol, 2 eq.) in 10 ml (DMF / THF, 4:1) is stirred overnight. The reaction mixture is diluted with AcOEt and sat. NaHCO₃. The aq. phase is extracted twice with AcOEt. The combined org. extracts are washed with brine, dried over MgSO₄, and concentrated *in vacuo.* The residue is purified by flash-chromatography on silica gel with a gradient of heptane/AcOEt to yield the desired intermediate of Structure **8b.**

### Intermediate of Structure 8b:

3-Benzyl-N-(9-ethoxycarbonylmethyl-6-fluoro-2,3,4,9-tetrahydro-*1H*-carbazol-3-yl)-succinamic acid *tert*-butyl ester is obtained in 25% yield as an orange oil: t_{R} = 1.10 min (LC-3), ESI-MS (pos.): m/z 537.28 [M+H]⁺.

### Starting materials:

### Starting materials of Structure 4:

(3*R*)-(2,3,4,9-tetrahydro-1*H*-carbazol-3-yl)-carbamic acid 1,1-dimethylethyl ester,
(3*S*)-(2,3,4,9-tetrahydro-1*H*-carbazol-3-yl)-carbamic acid 1,1-dimethylethyl ester,
(3*R*)-(6-fluoro-2,3,4,9-tetrahydro-1*H*-carbazol-3-yl)-carbamic acid 1,1-dimethylethyl ester, and
(3*S*)-(6-fluoro-2,3,4,9-tetrahydro-1*H*-carbazol-3-yl)-carbamic acid 1,1-dimethylethyl ester;
as well as

### Starting materials of Structure 7:

(3*R*)-(2,3,4,9-tetrahydro-1*H*-carbazol-3-ylamine),
(3*S*)-(2,3,4,9-tetrahydro-1*H*-carbazol-3-ylamine),
(3*R*)-(6-fluoro-2,3,4,9-tetrahydro-1*H*-carbazol-3-ylamine), and
(3*S*)-(6-fluoro-2,3,4,9-tetrahydro-1*H*-carbazol-3-ylamine),
are prepared according to literature procedures (Rosentreter U. et al., Arzneim.-Forsch. 1989, 39(12), 1519-1521); EP 0242518; Ha J. D. et al., Bulletin of the Korean Soc. Chem. 2004, 25, 1784-1790; WO 03/033099).

### Starting materials of Structure 9:

Starting materials of Structure **9** are commercially available or synthesized according to well known methods (see for example: J. Org. Chem. 1986, 51(6), 938-940).

NMR data of selected compounds are given in Table 11 below.

**Table 11**

| Compound | Chemical shifts (δ) in parts per million (ppm) | Solvent |
|---|---|---|
| (3*S*)-[3-(3-Cyclopentyl-propionylamino)-1,2,3,4-tetrahydro-9*H*-carbazol-9-yl]-acetic acid | 0.99 (*br.s,* 2H), 1.41-1.64 *(m,* 9H), 1.98 (*m,* 2H), 2.06 (*t*, 2H), 2.48-2.72 (*m*, 4H), 2.99 (*m,* 1H), 4.33 (*m*, 1H), 4.63 (*s*, 2H), 6.03 (*d,* 1H), 6.94-7.14 (*m*, 3H), 7.32 (*d*, 2H). | CDCl₃ |
| (3*RS*)-[3-(3-Benzyl-ureido)-6-fluoro-1,2,3,4-tetrahydro-9H-carbazol-9-yl]-acetic acid | 1.78 (*m*. 1 H), 2.02 (m, 1 H), 2.48 (m, 1 H), 2.69 (m, 2 H), 2.92 (dd, 1 H), 3.93 (m, 1 H), 4.23 (m, 2 H), 4.86 (d, 2 H), 6.09 (d, 1 H), 6.30 (t, 1 H), 6.88 (td, 1 H), 7.15 (dd, 1 H), 7.24 (m, 3 H), 7.32 (m, 3 H) | DMSO-*d₆* |
| (3*S*)- {6-Fluoro-3-[3-(4-isopropyl-phenyl)-propionylamino]-1,2,3,4-tetrahydro-9*H*-carbazol-9-yl}-acetic acid | 1.19 (d, 6 H), 1.73 (m, 1 H), 1.97 (m, 1 H), 2.38 (t, 2 H), 2.36-2.45 (m, 1H), 2.70 (m, 2 H), 2.80 (t, 2H), 2.82-2.90 (m, 2H), 4.01 (m, 1 H), 4.87 (m, 2 H), 6.88 (dt, 1 H), 7.01 (m, 4 H), 7.33 (dd, 1 H), 7.93 (d, 1 H), 12.90 (bs, 1H) | DMSO-*d₆* |
| (3*S*-(6-Fluoro-3- {(2*RS*)-2-[(4-fluoro-phenylcarbamoyl)-methyl]-3-phenyl-propionylamino}-1,2,3,4-tetrahydro-9*H* carbazol-9-yl)-acetic acid | 1.59 (m, 0.5 H), 1.74 (m, 1 H), 1.91 (m, 0.5 H), 2.32 (m, 2 H), 2.68 (m, 4 H), 2.90 (m, 1 H), 3.08 (m, 1 H), 3.57 (m, 1 H), 3.95 (m, 1 H), 4.84 (m, 2 H), 6.87 (m, 1 H), 6.97 (d, 0.5 H), 7.10 (m, 2.5 H), 7.21 (m, 3 H), 7.30 (m, 3 H), 7.60 (m, 2 H), 7.97 (t, 1 H), 9.99 (d, 1 H), 13.00 (br.s, 1H) | DMSO-*d₆* |
| (3*S*)-[3-(Benzyloxycarbonyl-cyclopropylmethyl-amino)-6-fluoro-1,2,3,4-tetrahydro-9*H*-carbazol-9-yl]-acetic acid | 0.26 (m, 2 H), 0.47 (d, 2 H), 1.07 (m, 1 H), 2.05 (m, 1 H), 2.18 (m, 1 H), 2.82 (m, 2H), 3.21 (d, 2 H), 4.10 (m, 1 H), 4.88 (s, 2 H), 5.14 (s, 2 H), 6.88 (t, 1 H), 7.19 (d, 1 H), 7.45 (m, 6 H), 12.90 (br.s, 1H). | DMSO-*d₆* |
| (3*S*)-{3-[Benzyloxycarbonyl-(2-methoxy-ethyl)-amino]-6-fluoro- 1,2,3,4-tetrahydro-9*H*-carbazol-9-yl} -acetic acid | 2.06 (m, 2 H), 2.77 (m, 6 H), 3.29 (m, 2 H), 3.45 (m, 3 H), 4.08 (dd, 1 H), 4.88 (m, 2 H), 5.13 (s, 2 H), 6.89 (m, 1 H), 7.16 (m, 1 H), 7.34 (m, 6 H), 13.00 (br. s, 1H). | DMSO-*d₆* |
| (3*S*)-{6-Fluoro-3-[[3-(2-methoxy-phenyl)-propionyl]-(2-phenoxy-ethyl)-amino]-1,2,3,4-tetrahydro-9*H*-carbazol-9-yl}-acetic acid | 1.93 (dd, 1 H), 2.09 (m, 1 H), 2.75 (m, 8 H), 3.66 (m, 3 H), 3.77 (m, 2 H), 4.13 (m, 3 H), 4.90 (m, 2 H), 6.90 (m, 6 H), 7.17 (m, 3 H), 7.33 (m, 3 H), 12.90 (br. s, 1H) | DMSO-d₆ |
| (3*S*)-{6-Fluoro-3-[naphthalen-1-ylmethyl-(3-phenyl-propionyl)-amino]-1,2,3,4-tetrahydro-9*H*-carbazol-9-yl}-acetic acid | 1.93 (m, 2 H), 2.70-3.08 (m, 8 H), 4.41 (m, 0.5 H), 4.86 (m, 2.5 H), 5.05 (m, 2 H), 6.87 (m, 1 H), 7.04 (m, 2 H), 7.19 (m, 3 H), 7.35 (m, 3 H), 7.56 (m, 3 H), 7.83 (m, 1 H), 7.97 (m, 1 H), 8.15 (m, 1 H), 13.00 (br.s, 1H). | DMSO-*d₆* |
| (3*RS*)-(3-Benzyloxycarbonylamino-6-fluoro-1,2,3,4-tetrahydro-9*H*-carbazol-9-yl)-acetic acid | 1.76 *(m,* 1H), 2.05 (m*,* 1H), 2.48 (*m,* 1H), 2.68 *(m,* 1H), 2.75 (*t*, 1H), 2.93 (*d,* 1H), 3.77 (*m*, 1H), 4.85 (s, 2H), 5.05 (s, 2H), 6.87 (*dt,* 1H), 7.14 (*dd,* 1H), 7.29-7.38 (*m*, 5H), 7.46 (*m*, 1H). | DMSO-*d₆* |
| (3*S*)-Ethyl [3-(3-butyl-ureido)-1,2,3,4-tetrahydro-9*H*-carbazol-9-yl]-acetate | 0.86 (*t*, 3H), 1.20 (*t,* 3H), 1.31 (*m*, 2H), 1.42 (*m,* 2H), 2.06 (*m*, 2H), 2.64 *(dd,* 1H), 2.70 (*m,* 2H), 3.03-3.10 (*m*, 3H), 4.18 (q, 2H), 4.28 (*m*, 1H), 4.71 (*s*, 2H), 7.04-7.21 (*m*, 3H), 7.45 (*d,* 1H). | DMSO-*d₆* |
| (3*S*)-Ethyl (3-propoxycarbonylamino-1,2,3,4-tetrahydro-9*H*-carbazol-9-yl)-acetate | 0.93 (t, 3H), 1.24 (*t*, 3H), 1.63 (*m*, 2H), 2.03-2.14 (*m,* 2H), 2.65 (*dd,* 1H), 2.77 (t, 2H), 3.12 *(dd,* 1H), 4.02 (*t*, 2H), 4.20 (*m,* 4H), 4.72 (s, 2H), 7.07-7.14 (*m*, 1H, H), 7.17 (*m,* 2H), 7.44 *(d,* 1H). | CDCl₃ |
| (3*S*)- Ethyl [3-(2-phenoxy-acetylamino)-1,2,3,4-tetrahydro-9*H*-carbazol-9-yl]-acetate | 1.24 (*t*, 3H), 2.04 (*m,* 1H), 2.17 (*m*, 1H), 2.60-2.86 (*m*, 3H), 3.15 (*dd,* 1H), 4.20 (q, 2H), 4.53 *(m,* 1H), 4.49 (s, 2H), 4.72 (s, 2H), 6.71 *(d,* 1H), 6.90 (*s*, 2H), 7.02-7.27 (m, 6H), 7.45 *(d,* 1H). | CDCl₃ |
| (3*R*)- Ethyl (3*-tert-*butoxycarbonylamino-1,2,3,4-tetrahydro-9H-carbazol-9-yl)-acetate | 1.25 (*t,* 3H), 1.45 (s, 9H), 1.99 *(m,* 1H), 2.12 (*m*, 1H), 2.63 *(dd,* 1H,), 2.75 (*m*, 2H), 3.09 (*dd,* 1H), 4.16 *(m,* 1H), 4.21 *(d,* 2H), 4.72 (*s*, 2H), 7.18 (*m*, 3H), 7.45 (*d*, 1H). | CDCl₃ |
| (3*R*)- Ethyl {6-fluoro-3-[2-(4-trifluoromethyl-phenyl)-acetylamino]-1,2,3,4-tetrahydro-9*H*-carbazol-9-yl}-acetate | 1.24 (*t,* 3H), 2.04 (*m*, 2H), 2.60 (*m*, 2H), 2.70 *(dt,* 1H), 3.01 (*dd,* 1H), 3.54 (*s*, 2H), 4.19 (q, 2H), 4.43 (*m,* 1H), 4.66 (*s*, 2H), 5.58 (*d*, 1H), 6.88 *(dt,* 1H), 7.06 (*m,* 2H), 7.27 (*m*, 1H), 7.39 *(d,* 2H), 7.54 (*d*, 2H). | CDCl₃ |
| (3*RS*)- Ethyl (3-benzyloxycarbonylamino-8-chloro-6-fluoro-1,2,3,4-tetrahydro-9*H*-carbazol-9-yl)-acetate | 1.18 (*t*, 3H), 1.76 (*m,* 1H), 2.03 (*m*, 1H), 2.46 (*m,* 1H), 2.63-2.80 (*m,* 2H), 2.90 (*dd,* 1H), 3.75 (*m,* 1H), 4.14 (*q*, 2H), 5.03 (*s*, 2H), 5.15 (*s*, 2H), 7.01 (*dd,* 1H), 7.19 (*dd,* 1H), 7.27-7.36 (*m,* 5), 7.45 (*d,* 1H). | DMSO-d₆ |
| (3*S*)- Ethyl [6-fluoro-3-(4-nitro-benzenesulfonylamino)-1,2,3,4-tetrahydro-9H-carbazol-9-yl]-acetate | 1.28 (t, 3H), 2.07 *(dd,* 2H), 2.54 *(dd,* 1H), 2.73 (*t*, 2H), 2.87 (*dd,* 1H), 3.94 (*m,* 1H), 4.21 (*d,* 2H), 4.68 (*s*, 2H), 4.90 (*d,* 1H), 6.85-6.94 (*m*, 2H), 7.06 (*dd,* 1H), 8.03 (*d,* 1H), 8.30 (*d,* 1H). | CDCl₃ |
| (3*S*)- Ethyl {6-fluoro-3-[methyl-(4-nitro-benzenesulfonyl)-amino]-1,2,3,4-tetrahydro-9*H*-carbazol-9-yl}-acetate | 1.27 (*t*, 3H), 1.85 (*m,* 1H), 2.01 (*m*, 1H), 2.65 (*dd,* 1H), 2.75-2.82 (*m*, 3H), 2.94 (s, 3H), 4.20 (*d,* 2H), 4.36 (*m,* 1H), 4.67 (*s*, 2H), 6.90 (*dt,* 1H), 6.97 (*dd*, 1H), 7.06 (*dd,* 1H), 8.03 *(d,* 1H), 8.37 (d, 1H). | CDCl₃ |
| (3*RS*)-(8-Chloro-6-fluoro-2,3,4,9-tetrahydro-1H carbazol-3-yl)-carbamic acid benzyl ester | 1.76 (*m*, 1H), 1.99 (*m*, 1H), 2.48 (*m*, 1H), 2.74-2.93 (*m*, 3H), 3.80 (*m*, 1H), 5.03 (*s*, 2H), 7.00 (*dd*, 1H), 7.10 (*dd*, 1H), 7.28-7.36 (*m*, 5H), 7.43 (*d*, 1H), 11. 10 (s, 1H). | DMSO-*d₆* |

### Biological assays:

### Preparation of hCRTH2 receptor membranes and radioligand binding assay:

Preparation of the membranes and radioligand binding assays are performed according to known procedures (*e.g.* Sawyer N. et al., Br. J. Pharmacol. 2002, 137, 1163-1172). A clonal HEK 293 cell line, expressing high level of recombinant hCRTH2 receptor, is selected for the preparation of membranes. Cells are detached from culture plates in 5 ml buffer A per plate (5 mM Tris, pH 7.4, 1 mM MgCl₂, 0.1 mM PMSF, 0.1 mM phenanthroline) using a police rubber and transferred into centrifugation tubes and frozen at -80°C. After thawing, the cells are centrifuged at 500 g for 5 min and then resuspended in buffer A. Cells are then fragmented by homogenization with a Polytron cell homogenizer for 30 s. The membrane fragments are collected by centrifugation at 3000 g for 40 min and resuspended in buffer B (50 mM Tris, pH 7.4, 25 mM MgCl₂, 250 mM saccharose) and aliquots are stored at -20°C.

Binding assay is performed in a total volume of 250 µl. In each well, 75 µl buffer C (50 mM Tris, pH 7.4, 100 mM NaCl, 1 mM EDTA, 0.1% BSA (protease free), 0.01 % NaN₃) is mixed with 50 µl {³H}-PGD₂ (2.5 nM, 220'000 dpm/well, Amersham Biosciences, TRK734), 100 µl CRTH2 membranes to give 80 µg per well, and 25 µl of test compound in buffer C containing 1% DMSO. For unspecific binding, PGD₂ is added to the reaction mixture at 1 µM final concentration. This binding assay mix is incubated at rt for 90 min and then filtered through a GF/C filter plate. The filter is washed three times with ice cold binding buffer C. Then, Microscint-40 (Packard, 40 µl/well) is added and the receptor bound radioactivity is quantified by scintillation counting in a "TopCount" benchtop microplate scintillation counter (Packard).

### Results for ligand binding to the hCRTH2 receptor:

Antagonistic activities (IC₅₀ values) of compounds of Formula I are in the range of 0.1 to 10000 nM with respect to the hCRTH2 receptor (preferred compounds: < 1000 nM, more preferred compounds: < 100 nM, most preferred compounds: < 10 nM). IC₅₀ values of 242 from 251 exemplified compounds (9 IC₅₀ values being not available) are in the range of 0.4-2050 nM with an average of 97 nM with respect to the hCRTH2 receptor. Antagonistic activities of selected compounds are displayed in Table 12.

**Table 12**

| **Compound** | **hCRTH2 Bdg** |
|---|---|
| | **IC₅₀ (nM)** |
| (3*S*)-[3-(3,3-Diphenyl-propionylamino)-6-fluoro-1,2,3,4-tetrahydro-9*H*-carbazol-9-yl]-acetic acid | 2 |
| (3*S*)-(3-{Acetyl-[2-(2-fluoro-phenyl)-ethyl]-amino}-6-fluoro-1,2,3,4-tetrahydro-9*H*-carbazol-9-yl)-acetic acid | 4 |
| (3*S*)-(6-Fluoro-3-{(2-methoxy-ethyl)-[3-(2-methoxy-phenyl)-propionyl]-amino}-1,2,3,4-tetrahydro-9*H*--carbazol-9-yl)-acetic acid | 6 |
| (3*S*)-{3-[(*RS*)-2-Benzyl-3-(3-methoxy-phenylcarbamoyl)-propionylamino]-6-fluoro-1,2,3,4-tetrahydro-9*H*-carbazol-9-yl}-acetic acid | 6 |
| (3*S*)-[3-(3-Benzyl-1-cyclohexylmethyl-ureido)-6-fluoro-1,2,3,4-tetrahydro-9*H*-carbazol-9-yl]-acetic acid | 6 |
| (3*S*)-{3-[2-(4-Chloro-phenoxy)-acetylamino]-6-fluoro-1,2,3,4-tetrahydro-9*H*-carbazol-9-yl}-acetic acid | 9 |
| (3*S*)-{3-[(2,3-Dihydro-1*H*-indole-2-carbonyl)-amino]-6-fluoro-1,2,3,4-tetrahydro-9*H*-carbazol-9-yl}-acetic acid | 11 |
| (3*S*)-{6-Fluoro-3-[3-(4-methylsulfanyl-phenyl)-propionylamino]-1,2,3,4-tetrahydro-9*H*-carbazol-9-yl}-acetic acid | 14 |
| (3*S*)-{3-[3-(2,5-Bis-trifluoromethyl-phenyl)-propionylamino]-6-fluoro-1,2,3,4-tetrahydro-9*H*-carbazol-9-yl}-acetic acid | 15 |
| (3*S*)-[3-(3-Benzo[1,3]dioxol-5-yl-propionylamino)-6-fluoro-1,2,3,4-tetrahydro-9*H*-carbazol-9-yl]-acetic acid | 15 |
| (3*S*)-{6-Fluoro-3-[ethyl-(3-phenyl-propionyl)-amino]-1,2,3,4-tetrahydro-9*H*-carbazol-9-yl}-acetic acid | 16 |
| (3*S*)-{3-[3-(2,4-Dichloro-phenyl)-propionylamino]-6-fluoro-1,2,3,4-tetrahydro-9*H*-carbazol-9-yl}-acetic acid | 16 |
| (3*S*)-(6-Fluoro-3-{[(2*RS*)-8-methoxy-1,2,3,4-tetrahydro-naphthalene-2-carbonyl]-amino}-1,2,3,4-tetrahydro-9*H*-carbazol-9-yl)-acetic acid | 18 |
| (3*S*)-{6-Fluoro-3-[((*S*)-2-methyl-3-phenyl-propionyl)-(2-phenoxy-ethyl)-amino]-1,2,3,4-tetrahydro-9*H*-carbazol-9-yl}-acetic acid | 21 |
| (3*RS*)-(3-Benzyloxycarbonylamino-6-trifluoromethyl-1,2,3,4-tetrahydro-9*H*-carbazol-9-yl)-acetic acid | 26 |
| (3*S*)-{6-Fluoro-3-[3-(2-hydroxy-phenyl)-propionylamino]-1,2,3,4-tetrahydro-9*H*-carbazol-9-yl}-acetic acid | 33 |
| (3*R*)-{3-[3-(3-Chloro-phenyl)-propionylamino]-6-fluoro-1,2,3,4-tetrahydro-9*H*-carbazol-9-yl}-acetic acid | 39 |
| (3*S*)-{6-Fluoro-3-[3-(3-trifluoromethoxy-phenyl)-propionylamino]-1,2,3,4-tetrahydro-9*H*-carbazol-9-yl}-acetic acid | 47 |
| (3*RS*)-[8-Chloro-6-fluoro-3-(3-phenyl-propionylamino)-1,2,3,4-tetrahydro-9*H*-carbazol-9-yl]-acetic acid | 57 |
| (3*S*)-{3-[(2-Benzyloxy-acetyl)-methyl-amino]-6-fluoro-1,2,3,4-tetrahydro-9*H*-carbazol-9-yl}-acetic acid | 60 |
| (3*R*)-[6-Fluoro-3-(3-*p*-tolyl-propionylamino)-1,2,3,4-tetrahydro-9*H*-carbazol-9-yl]-acetic acid | 61 |
| (3*S*)-{6-Fluoro-3-[3-(4-methanesulfonyl-phenyl)-propionylamino]-1,2,3,4-tetrahydro-9*H*-carbazol-9-yl}-acetic acid | 72 |
| (3*S*)-(3-Isobutoxycarbonylamino-1,2,3,4-tetrahydro-9*H*-carbazol-9-yl)-acetic acid | 84 |
| (3*S*)-{3-[3-(3-*tert*-Butoxycarbonylamino-phenyl)-propionylamino]-6-fluoro-1,2,3,4-tetrahydro-9*H* carbazol-9-yl}-acetic acid | 93 |
| (3*S*)-[3-(2-Benzyloxy-ethoxycarbonylamino)-1,2,3,4-tetrahydro-9*H*-carbazol-9-yl]-acetic acid | 117 |
| (3*R*)-[3-(3-Phenylsulfonyl-ureido)-1,2,3,4-tetrahydro-9*H*-carbazol-9-yl]-acetic acid | 147 |
| (3*R*)-[3-(3-Naphthalen-1-yl-ureido)-1,2,3,4-tetrahydro-9*H*-carbazol-9-yl]-acetic acid | 152 |
| (3*R*)-[3-(2-Thiophen-2-yl-acetylamino)-1,2,3,4-tetrahydro-9*H*-carbazol-9-yl]-acetic acid | 297 |
| (3*R*)-[3-(3-Cyclopentyl-propionylamino)-1,2,3,4-tetrahydro-9*H* carbazol-9-yl]-acetic acid | 400 |
| (3*R*)-(3-Benzoylamino-1,2,3,4-tetrahydro-9*H*-carbazol-9-yl)-acetic acid | 488 |
| (3*S*)-{6-Fluoro-3-[(1*H*-indole-2-carbonyl)-amino]-1,2,3,4-tetrahydro-9*H*-carbazol-9-yl} -acetic acid | 824 |
| (3*S*)-[3-(3-Butyl-ureido)-1,2,3,4-tetrahydro-9*H*-carbazol-9-yl]-acetic acid | 896 |

### Intracellular calcium mobilization assay (FLIPR):

Cells (HEK-293), stably expressing the hCRTH2 receptor under the control of the cytomegalovirus promotor from a single insertion of the expression vector pcDNA5 (Invitrogen), are grown to confluency in DMEM (low glucose, Gibco) medium supplemented with 10% fetal calf serum (Bioconcept, Switzerland) under standard mammalian cell culture conditions (37°C in a humidified atmosphere of 5% CO₂). Cells are detached from culture dishes using a dissociation buffer (0.02% EDTA in PBS, Gibco) for 1 min, and collected by centrifugation at 200 g at rt for 5 min in assay buffer (equal parts of Hank's BSS (HBSS, Bioconcept) and DMEM (low glucose, without phenol red, Gibco)). After incubation for 45 min (37°C and 5% CO₂) in the presence of 1 µM Fluo-4 and 0.04% Pluronic F-127 (both Molecular Probes), and 20 mM HEPES (Gibco) in assay buffer, the cells are washed with and resuspended in assay buffer, then seeded onto 384-well FLIPR assay plates (Greiner) at 50,000 cells in 66 µl per well, and sedimented by centrifugation. Stock solutions of test compounds are made up at a concentration of 10 mM in DMSO, and serially diluted in assay buffer to concentrations required for inhibition dose response curves. Prostaglandin D₂ (Biomol, Plymouth Meeting, PA) is used as an agonist.

A FLIPR384 instrument (Molecular Devices) is operated according to the manufacturer's standard instructions, adding 4 µl of test compound dissolved at 10 mM in DMSO and diluted prior to the experiment in assay buffer to obtain the desired final concentration. 10 µl of 80 nM prostaglandin D₂ (Biomol, Plymouth Meeting, PA) in assay buffer, supplemented with 0.8% bovine serum albumin (fatty acid content <0.02%, Sigma), is then added to obtain a final concentration of 10 nM and 0.1%, respectively. Changes in fluorescence are monitored before and after the addition of test compounds at λₑₓ=488 nm and λₑₘ=540 nm. Emission peak values above base level after prostaglandin D₂ addition are exported after base line subtraction. Values are normalized to high-level control (no test compound added) after subtraction of base line value (no prostaglandin D₂ added). The program XLlfit 3.0 (IDBS) is used to fit the data to a single site dose response curve of the equation (A+((B-A)/(1+((C/x)^D)))) and to calculate the IC₅₀ values.

## Claims

1. A compound of Formula **I**: 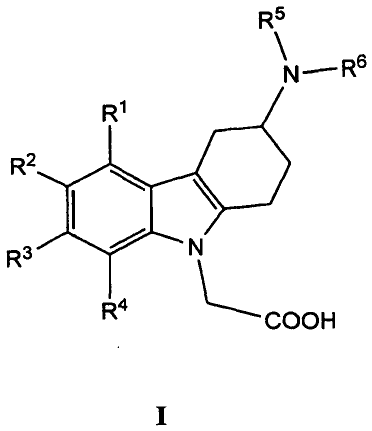 wherein
R¹, R², R³ and R⁴ independently represent hydrogen, C₁₋₅-alkyl, C₁₋₅-alkoxy, alkenyl, halogen, nitro, cyano, halo-C₁₋₆-alkoxy, halo-C₁₋₆-alkyl, C₁₋₆-alkylsulfonyl, or formyl;
R⁵ represents hydrogen, alkenyl, C₁₋₆-alkyl, cycloalkyl-C₁₋₄-alkyl, C₁₋₃-alkoxy-C₁₋₄-alkyl, aryl-C₁₋₄-alkyl, or aryloxy-C₁₋₄-alkyl;
wherein aryl is unsubstituted, mono- or di-substituted with a group independently selected from C₁₋₂-alkylendioxy, C₁₋₄-alkoxy, C₁₋₄-alkyl, halogen, trifluoromethyl, and trifluoromethoxy; and
R⁶ represents aryl-C₁₋₃-alkoxy-C₁₋₃-alkoxycarbonyl; aryl-C₁₋₃-alkoxycarbonyl; aryl-C₁₋₃-alkylaminocarbonyl; aryl-C₁₋₆-alkylcarbonyl; aryl-C₁₋₃-alkoxy-C₁₋₃-alkylcarbonyl; arylcarbonyl-C₁₋₄-alkylcarbonyl; aryloxy-C₁₋₃-alkylcarbonyl; cycloalkyl-C₁₋₃-alkylcarbonyl; diaryl-C₁₋₃-alkylcarbonyl; heteroaryl-C₁₋₃-alkylcarbonyl; aryl-C₃₋₆-cycloalkylcarbonyl; or R⁷-C₁₋₄-alkylcarbonyl, wherein the bridging C₁₋₄-alkyl group may additionally be mono-substituted with aryl, and R⁷ represents arylaminocarbonyl, heteroarylaminocarbonyl, C₁₋₆-alkylaminocarbonyl, or aryl-C₁₋₃-alkylaminocarbonyl;
wherein aryl is unsubstituted, mono- or di-substituted with a group independently selected from C₁₋₂-alkylendioxy, C₁₋₆-alkoxy, C₁₋₆-alkyl, C₁₋₆-alkylsulfonyl, halogen, hydroxy, halo-C₁₋₆-alkyl, halo-C₁₋₆-alkoxy, C₁₋₆-alkylthio, and C₁₋₄-alkoxycarbonylamino.
or a salt of such a compound.

2. A compound according to claim 1, wherein R¹, R³ and R⁴ represent hydrogen;
or a salt of such a compound.

3. A compound according to claim 1 or 2, wherein R² represents hydrogen, trifluoromethyl, or halogen;
or a salt of such a compound.

4. A compound according to any one of claims 1 to 3, wherein R⁵ represents hydrogen; C₁₋₃-alkyl; cyclopropylmethyl; 2-methoxyethyl; phenyt-C₂₋₃-alkyl; or phenoxyethyl, wherein the phenyl group is unsubstituted, or mono-substituted with a group selected from C₁₋₂-alkylendioxy, C₁₋₄-alkoxy, C₁₋₄-alkyl, halogen, trifluoromethyl, and trifluoromethoxy;
or a salt of such a compound.

5. A compound according to any one of claims 1 to 3, wherein
R⁵ represents hydrogen, methyl, ethyl, or *n*-propyl;
or a salt of such a compound.

6. A compound according to any one of claims 1 to 5, wherein R⁶ represents aryl-C₁₋₃-alkoxycarbonyl; aryl-C₁₋₃-alkylaminocarbonyl; aryl-C₁₋₆-alkylcarbonyl; aryl-C₁₋₃-alkoxy-C₁₋₃-alkylcarbonyl; arylcarbonyl-C₁₋₄-alkylcarbonyl; aryloxy-C₁₋₃-alkylcarbonyl; cycloalkyl-C₁₋₃-alkylcarbonyl; diaryl-C₁₋₃-alkylcarbonyl; aryl-C₃₋₆-cycloalkylcarbonyl; or R⁷-C₁₋₄-alkylcarbonyl, wherein the bridging C₁₋₄-alkyl group may additionally be mono-substituted with aryl, and R⁷ represents arylaminocarbonyl, heteroarylaminocarbonyl, C₁₋₆-alkylaminocarbonyl, or aryl-C₁₋₃-alkylaminocarbonyl;
wherein aryl is unsubstituted, mono- or di-substituted with a group independently selected from C₁₋₂-alkylendioxy, C₁₋₆-alkoxy, C₁₋₆-alkyl, C₁₋₆-alkylsulfonyl, halogen, hydroxy, halo-C₁₋₆-alkyl, halo-C₁₋₆-alkoxy, C₁₋₆-alkylthio, and C₁₋₄-alkoxycarbonylamino;
or a salt of such a compound.

7. A compound according to any one of claims 1 to 5, wherein R⁶ represents aryl-C₁₋₂-alkoxycarbonyl; aryl-C₁₋₂-alkylaminocarbonyl; aryl-C₁₋₄-alkylcarbonyl; aryloxy-C₁₋₂-alkylcarbonyl; or diaryl-C₂₋₃-alkylcarbonyl; or R⁷-C₂₋₄-alkylcarbonyl, wherein the bridging C₂₋₄-alkyl group may additionally be mono-substituted with aryl, and R⁷ represents arylaminocarbonyl, or C₁₋₄-alkylaminocarbonyl;
wherein aryl is unsubstituted, mono- or di-substituted with a group independently selected from C₁₋₂-alkylendioxy, C₁₋₆-alkoxy, C₁₋₆-alkyl, C₁₋₆-alkylsulfonyl, halogen, hydroxy, trifluoromethyl, and trifluoromethoxy;
or a salt of such a compound.

8. A compound according to any one of claims 1 to 5, wherein R⁶ represents aryl-C₂₋₄-alkylcarbonyl, wherein aryl is unsubstituted, mono- or di-substituted with a group independently selected from C₁₋₄-alkoxy, C₁₋₄-alkyl, halogen, and trifluoromethyl;
or a salt of such a compound.

9. A compound according to any one of claims 1 to 7, wherein, in case R⁶ represents a group which contains a carbonyl group and one or more aryl moieties, said group is such that it contains a bridging group between the carbonyl group and said aryl moiety (moieties) of said R⁶, wherein the carbonyl moiety and at least one of the aryl moieties are directly attached to different atoms of said bridging group;
or a salt of such a compound.

10. A compound according to claim 1 selected from the group consisting of:
(3*S*)-[3-(3,3-diphenyl-propionylamino)-6-fluoro-1,2,3,4-tetrahydro-9*H*-carbazol-9-yl]-acetic acid;
(3*R*)-{3-[2-(3-chloro-phenoxy)-acetylamino]-6-fluoro-1,2,3,4-tetrahydro-9*H*-carbazol-9-yl}-acetic acid;
(3*S*)-[6-fluoro-3 -(3-phenyl-propionylamino)-1,2,3,4-tetrahydro-9*H*-carbazol-9-yl]-acetic acid;
(3*R*)-{3-[2-(4-chloro-phenoxy)-acetylamino]-6-fluoro-1,2,3 ,4-tetrahydro-9*H*-carbazol-9-yl}-acetic acid;
(3*S*)-{3-[3-(2-chloro-phenyl)-propionylamino]-6-fluoro-1,2,3,4-tetrahydro-9*H*-carbazol-9-yl}-acetic acid;
(3*R*)-[6-fluoro-3-(3-phenyl-propionylamino)-1,2,3,4-tetrahydro-9*H*-carbazol-9-yl]-acetic acid;
(3*S*)-{3-[3-(4-chloro-phenyl)-propionylamino]-6-fluoro-1,2,3,4-tetrahydro-9*H*-carbazol-9-yl}-acetic acid;
(3*R*)-(3-benzyloxycarbonylamino-6-fluoro-1,2,3,4-tetrahydro-9*H*-carbazol-9-yl)-acetic acid;
(3*S*)-(3-benzyloxycarbonylamino-6-fluoro-1,2,3,4-tetrahydro-9*H*-carbazol-9-yl)-acetic acid;
(3*S*)-{3-[2-(4-chloro-phenoxy)-acetylamino]-6-fluoro-1,2,3,4-tetrahydro-9*H*-carbazol-9-yl}-acetic acid;
(3*R*)-{3-[2-(2-chloro-phenoxy)-acetylamino]-6-fluoro-1,2,3,4-tetrahydro-9*H*-carbazol-9-yl}-acetic acid;
(3*S*)-{3-[3-(3-chloro-phenyl)-propionylamino]-6-fluoro-1,2,3,4-tetrahydro-9*H*-carbazol-9-yl}-acetic acid;
(3*S*)-[6-fluoro-3-(4-oxo-4-phenyl-butyrylamino)-1,2,3,4-tetrahydro-9*H*-carbazol-9-yl]-acetic acid;
(3*S*)-[6-fluoro-3-(2-indan-2-yl-acetylamino)-1,2,3,4-tetrahydro-9*H*-carbazol-9-yl]-acetic acid;
(3*S*)-(3-{[2-(4-chloro-phenyl)-acetyl]-ethyl-amino}-6-fluoro-1,2,3,4-tetrahydro-9*H-*carbazol-9-yl)-acetic acid;
(3*R*)-[6-fluoro-3-(2-*p*-tolyloxy-acetylamino)-1,2,3,4-tetrahydro-9*H*-carbazol-9-yl]-acetic acid;
(3*S*)-{6-fluoro-3-[methyl-(3-phenyl-propionyl)-amino]-1,2,3,4-tetrahydro-9*H*-carbazol-9-yl}-acetic acid;
(3*S*)-[6-fluoro-3-(3-1*H*-indol-3-yl-propionylamino)-1,2,3,4-tetrahydro-9*H*-carbazol-9-yl]-acetic acid;
(3*S*)-[3-(3-benzo[1,3]dioxol-5-yl-propionylamino)-6-fluoro-1,2,3,4-tetrahydro-9*H*-carbazol-9-yl]-acetic acid;
(3*S*)-{6-fluoro-3-[ethyl-(3-phenyl-propionyl)-amino]-1,2,3,4-tetrahydro-9*H*-carbazol-9-yl}-acetic acid;
(3*S*)-{3-[2-(4-chloro-phenyl)-acetylamino]-6-fluoro-1,2,3,4-tetrahydro-9*H*-carbazol-9-yl}-acetic acid;
(3*S*)-[3-(2,3-diphenyl-propionylamino)-6-fluoro-1,2,3,4-tetrahydro-9*H*-carbazol-9-yl]-acetic acid;
(3*R*)-[6-fluoro-3-(2-phenoxy-acetylamino)-1,2,3,4-tetrahydro-9*H*-carbazol-9-yl]-acetic acid;
(3*S*)-{3-[3-(3,4-difluoro-phenyl)-propionylamino]-6-fluoro-1,2,3,4-tetrahydro-9*H*-carbazol-9-yl}-acetic acid;
(3*S*)-[3-(3-phenyl-propionylamino)-1,2,3,4-tetrahydro-9*H*-carbazol-9-yl]-acetic acid;
(3*R*)-[3-(2-benzyloxy-acetylamino)-1,2,3,4-tetrahydro-9*H*-carbazol-9-yl]-acetic acid;
(3*S*)-{6-fluoro-3-[3-(2-methoxy-phenyl)-propionylamino]-1,2,3,4-tetrahydro-9*H*-carbazol-9-yl}-acetic acid;
(3*S*)-{6-fluoro-3-[propyl-(3-phenyl-propionyl)-amino]-1,2,3,4-tetrahydro-9*H*-carbazol-9-yl}-acetic acid;
(3*S*)-[3-(2-benzyloxy-acetylamino)-1,2,3,4-tetrahydro-9*H*-carbazol-9-yl]-acetic acid;
(3*R*)-(3-benzyloxycarbonylamino-1,2,3,4-tetrahydro-9*H*-carbazol-9-yl)-acetic acid;
(3*R*)-{6-fluoro-3-[2-(4-methoxy-phenyl)-acetylamino]-1,2,3,4-tetrahydro-9*H*-carbazol-9-yl}-acetic acid;
(3*R*)-{3-[3-(4-chloro-phenyl)-propionylamino]-6-fluoro-1,2,3,4-tetrahydro-9*H*-carbazol-9-yl}-acetic acid;
(3*S*)-{3-[4-(4-bromo-phenyl)-4-oxo-butyrylamino]-6-fluoro-1,2,3,4-tetrahydro-9*H-*carbazol-9-yl}-acetic acid;
(3*S*)-(3-{[2-(4-chloro-phenyl)-acetyl]-propyl-amino}-6-fluoro-1,2,3,4-tetrahydro-9*H-*carbazol-9-yl)-acetic acid;
(3*R*)-(3-phenylacetylamino-1,2,3,4-tetrahydro-9*H*-carbazol-9-yl)-acetic acid;
(3*R*)-{3-[3-(2-chloro-phenyl)-propionylamino]-6-fluoro-1,2,3,4-tetrahydro-9*H*-carbazol-9-yl}-acetic acid;
(3*S*)-{6-fluoro-3-[2-(4-trifluoromethyl-phenoxy)-acetylamino]-1,2,3,4-tetrahydro-9*H-*carbazol-9-yl}-acetic acid;
(3*R*)-(6-fluoro-3-phenylacetylamino-1,2,3,4-tetrahydro-9*H*-carbazol-9-yl)-acetic acid;
(3*S*)-{6-fluoro-3-[3-(2-hydroxy-phenyl)-propionylamino]-1,2,3,4-tetrahydro-9*H*-carbazol-9-yl}-acetic acid;
(3*S*)-[3-(3-1H-benzoimidazol-2-yl-propionylamino)-6-fluoro-1,2,3,4-tetrahydro-9*H-*carbazol-9-yl]-acetic acid;
(3*S*)-{6-fluoro-3-[3-(4-hydroxy-phenyl)-propionylamino]-1,2,3,4-tetrahydro-9*H*-carbazol-9-yl}-acetic acid;
(3*S*)-[6-fluoro-3-(2-*p*-tolyloxy-acetylamino)-1,2,3,4-tetrahydro-9*H*-carbazol-9-yl]-acetic acid;
(3*R*)-[6-fluoro-3-(2-*p*-tolyl-acetylamino)-1,2,3,4-tetrahydro-9*H*-carbazol-9-yl]-acetic acid;
(3*R*)-{3-[3-(3-chloro-phenyl)-propionylamino]-6-fluoro-1,2,3,4-tetrahydro-9*H*-carbazol-9-yl}-acetic acid;
(3*R*)-[3-(2-phenoxy-acetylamino)-1,2,3,4-tetrahydro-9*H*-carbazol-9-yl]-acetic acid;
(3*S*)-[6-fluoro-3-(3-*p*-tolyl-propionylamino)-1,2,3,4-tetrahydro-9*H*-carbazol-9-yl]-acetic acid;
(3*S*)-(3-benzyloxycarbonylamino-1,2,3,4-tetrahydro-9*H*-carbazol-9-yl)-acetic acid;
(3*S*)-[6-fluoro-3-(2-*p*-tolyl-acetylamino)-1,2,3,4-tetrahydro-9*H*-carbazol-9-yl]-acetic acid;
(3*S*)-[3-(3-phenethyl-ureido)-1,2,3,4-tetrahydro-9*H*-carbazol-9-yl]-acetic acid;
(3*S*)-{6-fluoro-3-[3-(3-hydroxy-phenyl)-propionylamino]-1,2,3,4-tetrahydro-9*H*-carbazol-9-yl}-acetic acid;
(3*R*)-[3-(2-benzyloxy-ethoxycarbonylamino)-1,2,3,4-tetrahydro-9*H*-carbazol-9-yl]-acetic acid;
(3*S*)-[6-fluoro-3-(3-naphthalen-2-yl-propionylamino)-1,2,3,4-tetrahydro-9*H*-carbazol-9-yl]-acetic acid;
(3*S*)-{6-fluoro-3-[4-(4-methanesulfonyl-phenyl)-4-oxo-butyrylamino]-1,2,3,4-tetrahydro-9*H*-carbazol-9-yl}-acetic acid;
(3*S*)-(3-{[2-(4-chloro-phenyl)-acetyl]-methyl-amino}-6-fluoro-1,2,3,4-tetrahydro-9*H-*carbazol-9-yl)-acetic acid;
(3*S*)-{6-fluoro-3-[3-(4-methoxy-phenyl)-propionylamino]-1,2,3,4-tetrahydro-9*H*-carbazol-9-yl}-acetic acid;
(3*R*)-[3-(3-phenyl-propionylamino)-1,2,3,4-tetrahydro-9*H*-carbazol-9-yl]-acetic acid;
(3*R*)-{3-[2-(4-chloro-phenyl)-acetylamino]-6-fluoro-1,2,3,4-tetrahydro-9*H*-carbazol-9-yl}-acetic acid;
(3*R*)-[6-fluoro-3-(3-*p-*tolyl-propionylamino)-1,2,3,4-tetrahydro-9*H*-carbazol-9-yl]-acetic acid;
(3*R*)-{6-fluoro-3-[3-(4-methoxy-phenyl)-propionylamino]-1,2,3,4-tetrahydro-9*H*-carbazol-9-yl}-acetic acid;
(3*S*)-{6-fluoro-3-[3-(4-hydroxy-3-methoxy-phenyl)-propionylamino]-1,2,3,4-tetrahydro-9*H*-carbazol-9-yl}-acetic acid;
(3*S*)-{6-fluoro-3-[2-(3-trifluoromethyl-phenyl)-acetylamino]-1,2,3,4-tetrahydro-9*H-*carbazol-9-yl}-acetic acid;
(3*S*)-{6-fluoro-3-[2-(4-methoxy-phenyl)-acetylamino]-1,2,3,4-tetrahydro-9*H*-carbazol-9-yl}-acetic acid;
(3*R*)-{3-[2-(3-chloro-phenyl)-acetylamino]-6-fluoro-1,2,3,4-tetrahydro-9*H*-carbazol-9-yl}-acetic acid;
(3*S*)-{6-fluoro-3-[2-(4-trifluoromethyl-phenyl)-acetylamino]-1,2,3,4-tetrahydro-9*H-*carbazol-9-yl}-acetic acid;
(3*R*)-{3-[2-(3,4-dichloro-phenyl)-acetylamino]-6-fluoro-1,2,3,4-tetrahydro-9*H*-carbazol-9-yl}-acetic acid;
(3*S*)-{6-fluoro-3-[3-(3-methoxy-phenyl)-propionylamino]-1,2,3,4-tetrahydro-9*H*-carbazol-9-yl}-acetic acid;
(3*S*)-[6-fluoro-3-(2-phenoxy-acetylamino)-1,2,3,4-tetrahydro-9*H-*carbazol-9-yl]-acetic acid;
(3*R*)-[3-(3-phenethyl-ureido)-1,2,3,4-tetrahydro-9*H*-carbazol-9-yl]-acetic acid;
(3*S*)-[3-(2-benzyloxy-ethoxycarbonylamino)-1,2,3,4-tetrahydro-9*H*-carbazol-9-yl]-acetic acid;
(3*S*)-[3-(2-phenoxy-acetylamino)-1,2,3,4-tetrahydro-9*H*-carbazol-9-yl]-acetic acid;
(3*S*)-[3-(2-thiophen-2-yl-acetylamino)-1,2,3,4-tetrahydro-9*H*-carbazol-9-yl]-acetic acid;
(3*S*)-(3-phenylacetylamino-1,2,3,4-tetrahydro-9*H*-carbazol-9-yl)-acetic acid;
(3*S*)-[3-(3-benzyl-ureido)-1,2,3,4-tetrahydro-9*H*-carbazol-9-yl]-acetic acid;
(3*S*)-{3-[2-(4-*tert*-butyl-phenyl)-acetylamino]-6-fluoro-1,2,3,4-tetrahydro-9*H*-carbazol-9-yl}-acetic acid;
(3*R*)-(3-benzyloxycarbonylamino-8-chloro-6-fluoro-1,2,3,4-tetrahydro-9*H*-carbazol-9-yl)-acetic acid;
(3*S*)-(3-benzyloxycarbonylamino-8-chloro-6-fluoro-1,2,3,4-tetrahydro-9*H*-carbazol-9-yl)-acetic acid;
(3*S*)-[6-fluoro-3-(3-pyridin-3-yl-propionylamino)-1,2,3,4-tetrahydro-9*H*-carbazol-9-yl]-acetic acid;
(3*R*)-[3-(3-benzyl-ureido)-1,2,3,4-tetrahydro-9*H*-carbazol-9-yl]-acetic acid;
(3*R*)-{6-fluoro-3-[2-(4-trifluoromethyl-phenyl)-acetylamino]-1,2,3,4-tetrahydro-9*H-*carbazol-9-yl}-acetic acid;
(3*S*)-[3-(3-cyclopentyl-propionylamino)-1,2,3,4-tetrahydro-9*H*-carbazol-9-yl]-acetic acid;
(3*R*)-[3-(2-thiophen-2-yl-acetylamino)-1,2,3,4-tetrahydro-9*H*-carbazol-9-yl]-acetic acid;
(3*R*)-(3-benzyloxycarbonylamino-8-chloro-5-fluoro-1,2,3,4-tetrahydro-9*H*-carbazol-9-yl)-acetic acid;
(3*S*)-(3-benzyloxycarbonylamino-8-chloro-5-fluoro-1,2,3,4-tetrahydro-9*H*-carbazol-9-yl)-acetic acid; and
(3*R*)-[3-(3-cyclopentyl-propionylamino)-1,2,3,4-tetrahydro-9*H*-carbazol-9-yl]-acetic acid;
or a salt of such a compound.

11. A compound according to claim 1 selected from the group consisting of:
(3*R*)-[3-(3-Benzyl-ureido)-6-fluoro-1,2,3,4-tetrahydro-9*H*-carbazol-9-yl]-acetic acid;
(3*S*)-[3-(3-Benzyl-ureido)-6-fluoro-1,2,3,4-tetrahydro-9*H*-carbazol-9-yl]-acetic acid;
(3*S*)-[6-Fluoro-3-(3-phenethyl-ureido)-1,2,3,4-tetrahydro-9*H*-carbazol-9-yl]-acetic acid;
(3*R*)-[3-(3-Benzyl-ureido)-8-chloro-6-fluoro-1,2,3,4-tetrahydro-9*H*-carbazol-9-yl]-acetic acid;
(3*S*)-[3-(3-Benzyl-ureido)-8-chloro-6-fluoro-1,2,3,4-tetrahydro-9*H*-carbazol-9-yl]-acetic acid;
(3*R*)-[8-Chloro-6-fluoro-3-(3-phenethyl-ureido)-1,2,3,4-tetrahydro-9*H*-carbazol-9-yl]-acetic acid;
(3*S*)-[8-Chloro-6-fluoro-3-(3-phenethyl-ureido)-1,2,3,4-tetrahydro-9*H*-carbazol-9-yl]-acetic acid;
(3*R*)-(3-Benzyloxycarbonylamino-6-trifluoromethyl-1,2,3,4-tetrahydro-9*H*-carbazol-9-yl)-acetic acid;
(3*S*)-(3-Benzyloxycarbonylamino-6-trifluoromethyl-1,2,3,4-tetrahydro-9*H*-carbazol-9-yl)-acetic acid;
(3*R*)-(3-Benzyloxycarbonylamino-8-bromo-6-fluoro-1,2,3,4-tetrahydro-9*H*-carbazol-9-yl)-acetic acid;
(3*S*)-(3-Benzyloxycarbonylamino-8-bromo-6-fluoro-1,2,3,4-tetrahydro-9*H*-carbazol-9-yl)-acetic acid;
(3*R*)-(3-Benzyloxycarbonylamino-6-fluoro-8-vinyl-1,2,3,4-tetrahydro-9*H*-carbazol-9-yl)-acetic acid;
(3*S*)-(3-Benzyloxycarbonylamino-6-fluoro-8-vinyl-1,2,3,4-tetrahydro-9*H*-carbazol-9-yl)-acetic acid;
(3*R*)-(3-Benzyloxycarbonylamino-6-fluoro-8-methanesulfonyl-1,2,3,4-tetrahydro-9*H-*carbazol-9-yl)-acetic acid;
(3*S*)-(3-Benzyloxycarbonylamino-6-fluoro-8-methanesulfonyl-1,2,3,4-tetrahydro-9*H-*carbazol-9-yl)-acetic acid;
(3*S*)-(3-Benzyloxycarbonylamino-6-fluoro-8-methyl-1,2,3,4-tetrahydro-9*H*-carbazol-9-yl)-acetic acid;
(3*S*)-(3-Benzyloxycarbonylamino-7-chloro-6-fluoro-1,2,3,4-tetrahydro-9*H*-carbazol-9-yl)-acetic acid;
(3*S*)-(8-Allyl-3-benzyloxycarbonylamino-6-fluoro-1,2,3,4-tetrahydro-9*H*-carbazol-9-yl)-acetic acid;
(3*R*)-(3-Benzyloxycarbonylamino-8-chloro-1,2,3,4-tetrahydro-9*H*-carbazol-9-yl)-acetic acid;
(3*S*)-{3-[3-(2,4-Dimethoxy-phenyl)-propionylamino]-6-fluoro-1,2,3,4-tetrahydro-9*H-*carbazol-9-yl}-acetic acid;
(3*S*)-[6-Fluoro-3-(3-naphthalen-1-yl-propionylamino)-1,2,3,4-tetrahydro-9*H*-carbazol-9-yl]-acetic acid;
(3*R*)-{6-Fluoro-3-[2-(2-methoxy-phenoxy)-acetylamino]-1,2,3,4-tetrahydro-9*H*-carbazol-9-yl}-acetic acid;
(3*S*)-{6-Fluoro-3-[2-(2-methoxy-phenoxy)-acetylamino]-1,2,3,4-tetrahydro-9*H*-carbazol-9-yl}-acetic acid;
(3*R*)-{6-Fluoro-3-[3-(2-methylphenyl)-propionylamino]-1,2,3,4-tetrahydro-9*H*-carbazol-9-yl}-acetic acid;
(3*S*)-{6-Fluoro-3-[3-(2-methylphenyl)-propionylamino]-1,2,3,4-tetrahydro-9*H*-carbazol-9-yl}-acetic acid;
(3*R*)-{6-Fluoro-3-[3-(3-methylphenyl)-propionylamino]-1,2,3,4-tetrahydro-9*H*-carbazol-9-yl}-acetic acid;
(3*S*)-{6-Fluoro-3-[3-(3-methylphenyl)-propionylamino]-1,2,3,4-tetrahydro-9*H*-carbazol-9-yl}-acetic acid;
(3*R*)-{6-Fluoro-3-[3-(3-methoxy-phenyl)-propionylmnino]-1,2,3,4-tetrahydro-9*H*-carbazol-9-yl}-acetic acid;
(3*S*)-{6-Fluoro-3-[3-(3-methoxy-phenyl)-propionylamino]-1,2,3,4-tetrahydro-9*H*-carbazol-9-yl}-acetic acid;
(3*R*)-{6-Fluoro-3-[2-(3-methoxy-phenoxy)-acetylamino]-1,2,3,4-tetrahydro-9*H*-carbazol-9-yl}-acetic acid;
(3*S*)-{6-Fluoro-3-[2-(3-methoxy-phenoxy)-acetylamino]-1,2,3,4-tetrahydro-9*H*-carbazol-9-yl}-acetic acid;
(3*R*)-{6-Fluoro-3-[2-(2-methylphenoxy)-acetylamino]-1,2,3,4-tetrahydro-9*H*-carbazol-9-yl}-acetic acid;
(3*S*)-{6-Fluoro-3-[2-(2-methylphenoxy)-acetylamino]-1,2,3,4-tetrahydro-9*H*-carbazol-9-yl}-acetic acid;
(3*S*)-{3-[3-(2,5-Dimethoxy-phenyl)-propionylamino]-6-fluoro-1,2,3,4-tetrahydro-9*H-*carbazol-9-yl}-acetic acid;
(3*S*)-{6-Fluoro-3-[3-(4-trifluoromethyl-phenyl)-propionylamino]-1,2,3,4-tetrahydro-9*H-*carbazol-9-yl}-acetic acid;
(3*S*)-{3-[3-(2,6-Dichloro-phenyl)-propionylamino]-6-fluoro-1,2,3,4-tetrahydro-9*H-*carbazol-9-yl}-acetic acid;
(3*S*)-{3-[3-(2,5-Bis-trifluoromethyl-phenyl)-propionylamino]-6-fluoro-1,2,3,4-tetrahydro-9*H*-carbazol-9-yl}-acetic acid;
(3*S*)-{6-Fluoro-3-[3-(4-methylsulfanyl-phenyl)-propionylamino]-1,2,3,4-tetrahydro-9*H-*carbazol-9-yl}-acetic acid;
(3*S*)-{6-Fluoro-3-[3-(4-iodo-phenyl)-propionylamino]-1,2,3,4-tetrahydro-9*H*-carbazol-9-yl}-acetic acid;
(3*S*)-{6-Fluoro-3-[3-(4-isopropyl-phenyl)-propionylamino]-1,2,3,4-tetrahydro-9*H*-carbazol-9-yl}-acetic acid;
(3*S*)-{6-Fluoro-3-[3-(3-trifluoromethyl-phenyl)-propionylamino]-1,2,3,4-tetrahydro-9*H-*carbazol-9-yl}-acetic acid;
(3*S*)-{3-[3-(2,4-Dichloro-phenyl)-propionylamino]-6-fluoro-1,2,3,4-tetrahydro-9*H-*carbazol-9-yl}-acetic acid;
(3*S*)-{6-Fluoro-3-[3-(4-fluoro-phenyl)-propionylamino]-1,2,3,4-tetrahydro-9*H*-carbazol-9-yl}-acetic acid;
(3*S*)-{3-[3-(3,5-Bis-trifluoromethyl-phenyl)-propionylamino]-6-fluoro-1,2,3,4-tetrahydro-9*H*-carbazol-9-yl}-acetic acid;
(3*S*)-{3-[3-(4-Ethyl-phenyl)-propionylamino]-6-fluoro-1,2,3,4-tetrahydro-9*H*-carbazol-9-yl}-acetic acid;
(3*S*)-{6-Fluoro-3-[3-(3-iodo-phenyl)-propionylaminol-1,2,3,4-tetrahydro-9*H*-carbazol-9-yl}-acetic acid;
(3*S*)-{6-Fluoro-3-[3-(4-methanesulfonyl-phenyl)-propionylamino]-1,2,3,4-tetrahydro-9*H-*carbazol-9-yl}-acetic acid;
(3*S*)-{3-[3-(2,3-Dimethoxy-phenyl)-propionylamino]-6-fluoro-1,2,3,4-tetrahydro-9*H-*carbazol-9-yl}-acetic acid;
(3*S*)-{3-[3-(2-Bromo-phenyl)-propionylamino]-6-fluoro-1,2,3,4-tetrahydro-9*H*-carbazol-9-yl}-acetic acid;
(3*S*)-{6-Fluoro-3-[3-(3-trifluoromethoxy-phenyl)-propionylamino]-1,2,3,4-tetrahydro-9*H-*carbazol-9-yl}-acetic acid;
(3*S*)-{3-[3-(2,4-Dimethyl-phenyl)-propionylamino]-6-fluoro-1,2,3,4-tetrahydro-9*H-*carbazol-9-yl}-acetic acid;
(3*S*)-{3-[3-(3-Bromo-phenyl)-propionylamino]-6fluoro-1,2,3,4-tetrahydro-9*H*-carbazol-9-yl}-acetic acid;
(3*S*)-{3-[3-(3-*tert*-Butoxycarbonylamino-phenyl)-propionylamino]-6-fluoro-1,2,3,4-tetrahydro-9*H*-carbazol-9-yl}-acetic acid;
(3*S*)-{6-Fluoro-3-[(*S*)-3-(4-fluoro-phenyl)-2-phenyl-propionylamino]-1,2,3,4-tetrahydro-9*H*-carbazol-9-yl}-acetic acid;
(3*S*)-{6-Fluoro-3-[(*S*)-3-(4-methoxy-phenyl)-2-phenyl-propionylamino]-1,2,3,4-tetrahydro-9*H*-carbazol-9-yl}-acetic acid;
(3*S*)-{6-Fluoro-3-[3-(2-fluoro-phenyl)-propionylamino]-1,2,3,4-tetrahydro-9*H*-carbazol-9-yl}-acetic acid;
(3*S*)-(6-Fluoro-3-{(2*RS*)-2-[(4-fluoro-phenylcarbamoyl)-methyl]-3-phenyl-propionylamino}-1,2,3,4-tetrahydro-9*H*-carbazol-9-yl)-acetic acid;
(3*S*)-{3-[(2RS)-2-Benzyl-3,3-dimethyl-butyrylamino]-6-fluoro-1,2,3,4-tetrahydro-9*H-*carbazol-9-yl}-acetic acid;
(3*S*)-{6-Fluoro-3-[3-(3-fluoro-phenyl)-propionylamino]-1,2,3,4-tetrahydro-9*H*-carbazol-9-yl}-acetic acid;
(3*S*)-{6-Fluoro-3-[(2*R*)-2-methyl-3-phenyl-propionylamino]-1,2,3,4-tetrahydro-9*H-*carbazol-9-yl}-acetic acid;
(3*S*)-[3-(2,2-Dimethyl-3-phenyl-propionylamino)-6-fluoro-1,2,3,4-tetrahydro-9*H*-carbazol-9-yl]-acetic acid;
(3*S*)-[6-Fluoro-3-(3-methyl-3-phenyl-butyrylamino)-1,2,3,4-tetrahydro-9*H*-carbazol-9-yl]-acetic acid;
(3*S*)-{6-Fluoro-3-[(3*S*)-3-phenyl-butyrylamino]-1,2,3,4-tetrahydro-9*H*-carbazol-9-yl}-acetic acid;
(3*S*)-[3-(2-Benzyloxy-acetylamino)-6-fluoro-1,2,3,4-tetrahydro-9*H*-carbazol-9-yl]-acetic acid;
(3*S*)-[6-Fluoro-3-(4-phenyl-butyrylamino)-1,2,3,4-tetrahydro-9*H*-carbazol-9-yl]-acetic acid;
(3*R*)-{8-Chloro-6-fluoro-3-[3-(2-methylphenyl)-propionylamino]-1,2,3,4-tetrahydro-9*H-*carbazol-9-yl}-acetic acid;
(3*S*)-{8-Chloro-6-fluoro-3-[3-(2-methylphenyl)-propionylamino]-1,2,3,4-tetrahydro-9*H-*carbazol-9-yl}-acetic acid;
(3*R*)-{8-Chloro-6-fluoro-3-[2-(2-methoxy-phenoxy)-acetylamino]-1,2,3,4-tetrahydro-9*H-*carbazol-9-yl}-acetic acid;
(3*S*)-{8-Chloro-6-fluoro-3-[2-(2-methoxy-phenoxy)-acetylamino]-1,2,3,4-tetrahydro-9*H-*carbazol-9-yl}-acetic acid;
(3*R*)- {8-Chloro-6-fluoro-3-[3-(3-hydroxy-phenyl)-propionylamino]-1,2,3,4-tetrahydro-9*H-*carbazol-9-yl}-acetic acid;
(3*S*)-{8-Chloro-6-fluoro-3-[3-(3-hydroxy-phenyl)-propionylamino]-1,2,3,4-tetrahydro-9*H-*carbazol-9-yl}-acetic acid;
(3*R*)-{8-Chloro-6-fluoro-3-[3-(3-methoxy-phenyl)-propionylamino]-1,2,3,4-tetrahydro-9*H-*carbazol-9-yl}-acetic acid;
(3*S*)-{8-Chloro-6-fluoro-3-[3-(3-methoxy-phenyl)-propionylamino]-1,2,3,4-tetrahydro-9*H-*carbazol-9-yl}-acetic acid;
(3*R*)-{8-Chloro-6-fluoro-3-[3-(3-methylphenyl)-propionylamino]-1,2,3,4-tetrahydro-9*H-*carbazol-9-yl}-acetic acid;
(3*S*)-{8-Chloro-6-fluoro-3-[3-(3-methylphenyl)-propionylamino]-1,2,3,4-tetrahydro-9*H-*carbazol-9-yl}-acetic acid;
(3*R*)-{8-Chloro-6-fluoro-3-[3-(2-hydroxy-phenyl)-propionylamino]-1,2,3,4-tetrahydro-9*H-*carbazol-9-yl}-acetic acid;
(3*S*)-{8-Chloro-6-fluoro-3-[3-(2-hydroxy-phenyl)-propionylamino]-1,2,3,4-tetrahydro-9*H-*carbazol-9-yl}-acetic acid;
(3*R*)-[8-Chloro-6-fluoro-3-(3-phenyl-propionylamino)-1,2,3,4-tetrahydro-9*H*-carbazol-9-yl]-acetic acid;
(3*S*)-[8-Chloro-6-fluoro-3-(3-phenyl-propionylamino)-1,2,3,4-tetrahydro-9*H*-carbazol-9-yl]-acetic acid;
(3*R*)-{8-Chloro-6-fluoro-3-[3-(2-methoxy-phenyl)-propionylamino]-1,2,3,4-tetrahydro-9*H-*carbazol-9-yl}-acetic acid;
(3*S*)-{8-Chloro-6-fluoro-3-[3-(2-methoxy-phenyl)-propionylamino]-1,2,3,4-tetrahydro-9*H-*carbazol-9-yl}-acetic acid;
(3*R*)-{8-Chloro-3-[3-(3-chloro-phenyl)-propionylamino]-6-fluoro-1,2,3,4-tetrahydro-9*H-*carbazol-9-yl}-acetic acid;
(3*S*)-{8-Chloro-3-[3-(3-chloro-phenyl)-propionylamino]-6-fluoro-1,2,3,4-tetrahydro-9*H-*carbazol-9-yl}-acetic acid;
(3*R*)-[8-Chloro-6-fluoro-3-(3-1*H*-indol-3-yl-propionylamino)-1,2,3,4-tetrahydro-9*H-*carbazol-9-yl]-acetic acid;
(3*S*)-[8-Chloro-6-fluoro-3-(3-1*H*-indol-3-yl-propionylamino)-1,2,3,4-tetrahydro-9*H-*carbazol-9-yl]-acetic acid;
(3*R*)-{8-Chloro-3-[2-(2-chloro-phenoxy)-acetylamino]-6-fluoro-1,2,3,4-tetrahydro-9*H-*carbazol-9-yl}-acetic acid;
(3*S*)-{8-Chloro-3-[2-(2-chloro-phenoxy)-acetylamino]-6-fluoro-1,2,3,4-tetrahydro-9*H-*carbazol-9-yl}-acetic acid;
(3*R*)-{8-Chloro-6-fluoro-3-[2-(2-methylphenyl)-oxy-acetylamino]-1,2,3,4-tetrahydro-9*H-*carbazol-9-yl}-acetic acid;
(3*S*)-{8-Chloro-6-fluoro-3-[2-(2-methylphenyl)-oxy-acetylamino]-1,2,3,4-tetrahydro-9*H-*carbazol-9-yl}-acetic acid;
(3*R*)-[3-(3-Benzo[1,3]dioxol-5-yl-propionylamino)-8-chloro-6-fluoro-1,2,3,4-tetrahydro-9*H*-carbazol-9-yl]-acetic acid;
(3*S*)-[3-(3-Benzo[1,3]dioxol-5-yl-propionylamino)-8-chloro-6-fluoro-1,2,3,4-tetrahydro-9*H*-carbazol-9-yl]-acetic acid;
(3*R*)-{8-Chloro-6-fluoro-3-[2-(3-methoxy-phenoxy)-acetylamino]-1,2,3,4-tetrahydro-9*H-*carbazol-9-yl}-acetic acid;
(3*S*)-{8-Chloro-6-fluoro-3-[2-(3-methoxy-phenoxy)-acetylamino]-1,2,3,4-tetrahydro-9*H-*carbazol-9-yl}-acetic acid;
(3*R*)-{8-Chloro-3-[2-(3-chloro-phenoxy)-acetylamino]-6-fluoro-1,2,3,4-tetrahydro-9*H-*carbazol-9-yl}-acetic acid;
(3*S*)-{8-Chloro-3-[2-(3-chloro-phenoxy)-acetylamino]-6-fluoro-1,2,3,4-tetrahydro-9*H-*carbazol-9-yl}-acetic acid;
(3*R*)-{8-Chloro-3-[3-(2-chloro-phenyl)-propionylamino]-6-fluoro-1,2,3,4-tetrahydro-9*H-*carbazol-9-yl}-acetic acid;
(3*S*)-{8-Chloro-3-[3-(2-chloro-phenyl)-propionylamino]-6-fluoro-1,2,3,4-tetrahydro-9*H-*carbazol-9-yl}-acetic acid;
(3*R*)-[8-Chloro-6-fluoro-3-(2-indan-2-yl-acetylamino)-1,2,3,4-tetrahydro-9*H*-carbazol-9-yl]-acetic acid;
(3*S*)-[8-Chloro-6-fluoro-3-(2-indan-2-yl-acetylamino)-1,2,3,4-tetrahydro-9*H*-carbazol-9-yl]-acetic acid;
(3*S*)-[6-Fluoro-3-(1-methyl-3-phenethyl-ureido)-1,2,3,4-tetrahydro-9*H*-carbazol-9-yl]-acetic acid;
(3*S*)-{3-[3-(2-Chloro-benzyl)-1-methyl-ureido]-6-fluoro-1,2,3,4-tetrahydro-9*H*-carbazol-9-yl}-acetic acid;
(3*S*)-[3-(3-Benzyl-1-methyl-ureido)-6-fluoro-1,2,3,4-tetrahydro-9*H*-carbazol-9-yl]-acetic acid;
(3*S*)-[3-(Benzyloxycarbonyl-methyl-amino)-6-fluoro-1,2,3,4-tetrahydro-9*H*-carbazol-9-yl]-acetic acid;
{(3*S*)-3-[(2-Chloro-benzyloxycarbonyl)-methyl-amino]-6-fluoro-1,2,3,4-tetrahydro-9*H-*carbazol-9-yl}-acetic acid;
(3*S*)-(6-Fluoro-3-{[2-(4-methoxy-phenyl)-acetyl]-methyl-amino}-1,2,3,4-tetrahydro-9*H-*carbazol-9-yl)-acetic acid;
(3*S*)-(6-Fluoro-3-{methyl-[2-(4-methylphenyl)-acetyl]-amino}-1,2,3,4-tetrahydro-9*H-*carbazol-9-yl)-acetic acid;
(3*S*)-(6-Fluoro-3-{[2-(2-methoxy-phenyl)-acetyl]-methyl-amino}-1,2,3,4-tetrahydro-9*H-*carbazol-9-yl)-acetic acid;
(3*S*)-{6-Fluoro-3-[(2-indan-2-yl-acetyl)-methyl-amino]-1,2,3,4-tetrahydro-9H-carbazol-9-yl}-acetic acid;
(3*S*)-(3-{[2-(3-Chloro-phenyl)-acetyl]-methyl-amino}-6-fluoro-1,2,3,4-tetrahydro-9*H-*carbazol-9-yl)-acetic acid;
(3*S*)-(6-Fluoro-3-{methyl-[2-(3-methylphenyl)-acetyl]-amino}-1,2,3,4-tetrahydro-9*H-*carbazol-9-yl)-acetic acid;
(3*S*)-(6-Fluoro-3-{[2-(3-methoxy-phenyl)-acetyl]-methyl-amino}-1,2,3,4-tetrahydro-9*H-*carbazol-9-yl)-acetic acid;
(3*S*)-(3-{[2-(2-Chloro-phenoxy)-acetyl]-methyl-amino}-6-fluoro-1,2,3,4-tetrahydro-9*H-*carbazol-9-yl)-acetic acid;
(3*S*)-(3-{[2-(4-Chloro-phenoxy)-acetyl]-methyl-amino}-6-fluoro-1,2,3,4-tetrahydro-9*H-*carbazol-9-yl)-acetic acid;
(3*S*)-(6-Fluoro-3-{[3-(3-methoxy-phenyl)-propionyl]-methyl-amino}-1,2,3,4-tetrahydro-9*H-*carbazol-9-yl)-acetic acid;
(3*S*)-(6-Fluoro-3-{methyl-[2-(2-methylphenyl)-acetyl]-amino}-1,2,3,4-tetrahydro-9*H-*carbazol-9-yl)-acetic acid;
(3*S*)-{3-[(3,3-Diphenyl-propionyl)-methyl-amino]-6-fluoro-1,2,3,4-tetrahydro-9*H*-carbazol-9-yl}-acetic acid;
(3*S*)-(6-Fluoro-3-{[3-(2-methoxy-phenyl)-propionyl]-methyl-amino}-1,2,3,4-tetrahydro-9*H-*carbazol-9-yl)-acetic acid;
(3*S*)-{6-Fluoro-3-[(3-1*H*-indol-3-yl-propionyl)-methyl-amino]-1,2,3,4-tetrahydro-9*H-*carbazol-9-yl}-acetic acid;
(3*S*)-{3-[(2-Benzyloxy-acetyl)-methyl-amino]-6-fluoro-1,2,3,4-tetrahydro-9*H*-carbazol-9-yl}-acetic acid;
(3*S*)-{3-[(2,3-Diphenyl-propionyl)-methyl-aminol-6-fluoro-1,2,3,4-tetrahydro-9*H*-carbazol-9-yl}-acetic acid;
(3*S*)-{6-Fluoro-3-[[3-(2-methoxy-phenyl)-propionyl]-(3-phenyl-propyl)-amino]-1,2,3,4-tetrahydro-9*H*-carbazol-9-yl}-acetic acid;
(3*S*)-{3-[3-Benzyl-(1-cyclopropylmethyl)-ureido]-6-fluoro-1,2,3,4-tetrahydro-9*H*-carbazol-9-yl}-acetic acid;
(3*S*)-[3-(Benzyloxycarbonyl-cyclopropylmethyl-amino)-6-fluoro-1,2,3,4-tetrahydro-9*H-*carbazol-9-yl]-acetic acid;
(3*S*)-{3-[Cyclopropylmethyl-(3-phenyl-propionyl)-amino]-6-fluoro-1,2,3,4-tetrahydro-9*H-*carbazol-9-yl}-acetic acid;
(3*S*)-{3-[Cyclopropylmethyl-((S)-2-methyl-3-phenyl-propionyl)-amino]-6-fluoro-1,2,3,4-tetrahydro-9*H*-carbazol-9-yl} -acetic acid;
(3*S*)-(3-{Cyclopropylmethyl-[3-(2-methoxy-phenyl)-propionyl]-amino}-6-fluoro-1,2,3,4-tetrahydro-9*H*-carbazol-9-yl)-acetic acid;
(3*S*)-(3-{[2-(3-Chloro-phenoxy)-acetyl]-cyclopropylmethyl-amino}-6-fluoro-1,2,3,4-tetrahydro-9*H*-carbazol-9-yl)-acetic acid;
(3*S*)-{3-[Cyclopropylmethyl-(3,3-diphenyl-propionyl)-amino]-6-fluoro-1,2,3,4-tetrahydro-9*H*-carbazol-9-yl}-acetic acid;
(3*S*)-{3-[Cyclopropylmethyl-(2-naphthalen-1-yl-acetyl)-amino]-6-fluoro-1,2,3,4-tetrahydro-9*H*-carbazol-9-yl}-acetic acid;
(3*S*)-(3-{Benzyloxycarbonyl-[2-(4-trifluoromethyl-phenoxy)-ethyl]-amino}-6-fluoro-1,2,3,4-tetrahydro-9*H*-carbazol-9-yl)-acetic acid;
(3*S*)-(6-Fluoro-3-{(3-phenyl-propionyl)-[2-(4-trifluoromethyl-phenoxy)-ethyl]-amino}-1,2,3,4-tetrahydro-9*H*-carbazol-9-yl)-acetic acid;
(3*S*)-(6-Fluoro-3-{[3-(2-methoxy-phenyl)-propionyl]-[2-(4-trifluoromethyl-phenoxy)-ethyl]-amino}-1,2,3,4-tetrahydro-9*H*-carbazol-9-yl)-acetic acid;
(3*S*)-{6-Fluoro-3-[(2-phenoxy-ethyl)-(3-phenyl-propionyl)-amino]-1,2,3,4-tetrahydro-9*H-*carbazol-9-yl}-acetic acid;
(3*S*)-{6-Fluoro-3-[((*S*)-2-methyl-3-phenyl-propionyl)-(2-phenoxy-ethyl)amino]-1,2,3,4-tetrahydro-9*H*-carbazol-9-yl}-acetic acid;
(3*S*)-{6-Fluoro-3-[[3-(2-methoxy-phenyl)-propionyl]-(2-phenoxy-ethyl)-amino]-1,2,3,4-tetrahydro-9*H*-carbazol-9-yl}-acetic acid;
(3*S*)-{3-[3-Benzyl-1-(2-methoxy-ethyl)-ureido]-6-fluoro-1,2,3,4-tetrahydro-9*H*-carbazol-9-yl}-acetic acid;
(3*S*)-{3-[Benzyloxycarbonyl-(2-methoxy-ethyl)-amino]-6-fluoro-1,2,3,4-tetrahydro-9*H-*carbazol-9-yl}-acetic acid;
(3*S*)-{6-Fluoro-3-[(2-methoxy-ethyl)-(3-phenyl-propionyl)-amino]-1,2,3,4-tetrahydro-9*H-*carbazol-9-yl}-acetic acid;
(3*S*)-{6-Fluoro-3-[(2-methoxy-ethyl)-((S)-2-methyl-3-phenyl-propionyl)-amino]-1,2,3,4-tetrahydro-9*H*-carbazol-9-yl} -acetic acid;
(3*S*)-(6-Fluoro-3-{(2-methoxy-ethyl)-[3-(2-methoxy-phenyl)-propionyl]-amino}-1,2,3,4-tetrahydro-9*H*-carbazol-9-yl)-acetic acid;
(3*S*)-{3-[[2-(3-Chloro-phenoxy)-acetyl]-(2-methoxy-ethyl)-amino]-6-fluoro-1,2,3,4-tetrahydro-9*H*-carbazol-9-yl}-acetic acid;
(3*S*)-{3-[(3,3-Diphenyl-propionyl)-(2-methoxy-ethyl)-amino]-6-fluoro-1,2,3,4-tetrahydro-9*H*-carbazol-9-yl}-acetic acid;
(3*S*)-{6-Fluoro-3-[(2-methoxy-ethyl)-(2-naphthalen-1-yl-acetyl)-amino]-1,2,3,4-tetrahydro-9*H*-carbazol-9-yl}-acetic acid;
(3*S*)-(6-Fluoro-3-{[(2*S*)-2-methyl-3-phenyl-propionyl]-phenethyl-amino}-1,2,3,4-tetrahydro-9*H*-carbazol-9-yl)-acetic acid;
(3*S*)-(6-Fluoro-3-{[3-(2-methoxy-phenyl)-propionyl]-phenethyl-amino}-1,2,3,4-tetrahydro-9*H*-carbazol-9-yl)-acetic acid;
(3*S*)-{6-Fluoro-3-[(2-naphthalen-1-yl-acetyl)-phenethyl-amino]-1,2,3,4-tetrahydro-9*H-*carbazol-9-yl}-acetic acid;
(3*S*)-{6-Fluoro-3-[phenethyl-(3-phenyl-propionyl)-amino]-1,2,3,4-tetrahydro-9*H*-carbazol-9-yl}-acetic acid;
(3*S*)-[3-(3-Benzyl-1-naphthalen-1-ylmethyl-ureido)-6-fluoro-1,2,3,4-tetrahydro-9*H-*carbazol-9-yl]-acetic acid;
(3*S*)-[3-(Benzyloxycarbonyl-naphthalen-1-ylmethyl-amino)-6-fluoro-1,2,3,4-tetrahydro-9*H-*carbazol-9-yl]-acetic acid;
(3*S*)-{6-Fluoro-3-[naphthalen-1-ylmethyl-(3-phenyl-propionyl)-amino]-1,2,3,4-tetrahydro-9*H*-carbazol-9-yl}-acetic acid;
(3*S*)-{6-Fluoro-3-[((*S*)-2-methyl-3-phenyl-propionyl)-naphthalen-1-ylmethyl-amino]-1,2,3,4-tetrahydro-9*H*-carbazol-9-yl}-acetic acid;
(3*S*)-(6-Fluoro-3-{[3-(2-methoxy-phenyl)-propionyl]-naphthalen-1-ylmethyl-amino}-1,2,3,4-tetrahydro-9*H*-carbazol-9-yl)-acetic acid;
(3*S*)-{3-[(3,3-Diphenyl-propionyl)-naphthalen-1-ylmethyl-amino]-6-fluoro-1,2,3,4-tetrahydro-9*H*-carbazol-9-yl}-acetic acid;
(3*S*)-{3-[(*RS*)-2-Benzyl-3-(2-methylphenyl)-carbamoyl-propionylamino]-6-fluoro-1,2,3,4-tetrahydro-9*H*-carbazol-9-yl}-acetic acid;
(3*S*)-{-[(*RS*)-2-Benzyl-3-(3-methoxy-phenylcarbamoyl)-propionylamino]-6-fluoro-1,2,3,4-tetrahydro-9*H*-carbazol-9-yl}-acetic acid;
(3*S*)-{3-[(*RS*)-2-Benzyl-3-(4-chloro-phenylcarbamoyl)-propionylamino]-6-fluoro-1,2,3,4-tetrahydro-9*H*-carbazol-9-yl}-acetic acid;
(3*S*)-{3-[(*RS*)-2-Benzyl-3-(4-fluoro-benzylcarbamoyl)-propionylamino]-6-fluoro-1,2,3,4-tetrahydro-9*H*-carbazol-9-yl}-acetic acid;
[(3*S*)-3-((*RS*)-2-Benzyl-3-propylcarbamoyl-propionylamino)-6-fluoro-1,2,3,4-tetrahydro-9*H*-carbazol-9-yl]-acetic acid;
(3*S*)-[6-Fluoro-3-(3-thiophen-2-yl-propionylamino)-1,2,3,4-tetrahydro-9*H*-carbazol-9-yl]-acetic acid;
(3*S*)-{3-[3-(3-Chloro-isoxazol-5-yl)-propionylamino]-6-fluoro-1,2,3,4-tetrahydro-9*H-*carbazol-9-yl}-acetic acid;
(3*S*)-[6-Fluoro-3-(3-pyrimidin-2-yl-propionylamino)-1,2,3,4-tetrahydro-9*H*-carbazol-9-yl]-acetic acid;
(3*S*)-{6-Fluoro-3-[3-phenyl-4-([1,3,4]thiadiazol-2-ylcarbamoyl)-butyrylamino]-1,2,3,4-tetrahydro-9*H*-carbazol-9-yl}-acetic acid;
(3*S*)-[3-(1,3-Dibenzyl-ureido)-6-fluoro-1,2,3,4-tetrahydro-9*H*-carbazol-9-yl]-acetic acid;
(3*S*)-[3-(3-Benzyl-1-cyclohexylmethyl-ureido)-6-fluoro-1,2,3,4-tetrahydro-9*H*-carbazol-9-yl]-acetic acid; and
(3*S*)-(3-{Cyclohexylmethyl-[3-(2-methoxy-phenyl)-propionyl]-amino}-6-fluoro-1,2,3,4-tetrahydro-9*H*-carbazol-9-yl)-acetic acid;
or a salt of such a compound.

12. A pharmaceutical composition comprising a compound according to any one of claims 1 to 11, or a pharmaceutically acceptable salt thereof, and a pharmaceutically acceptable carrier.

13. A compound according to any one of claims 1 to 11, or a pharmaceutically acceptable salt thereof, or a pharmaceutical composition according to claim 11, for use as a medicament.

14. Use of a compound according to any one of claims 1 to 11, or a pharmaceutically acceptable salt thereof, for the preparation of a medicament for the prevention and/or treatment of chronic and acute allergic/immune diseases/disorders, comprising allergic asthma, rhinitis, allergic rhinitis, chronic obstructive pulmonary disease, dermatitis, inflammatory bowel disease, rheumatoid arthritis, allergic nephritis, conjunctivitis, atopic dermatitis, bronchial asthma, food allergy, systemic mast cell disorders, anaphylactic shock, urticaria, eczema, itching, inflammation, ischemia-reperfusion injury, cerebrovascular disorders, pleuritis, ulcerative colitis, eosinophil-related diseases comprising Churg-Strauss syndrome and sinusitis, and basophil-related diseases, comprising basophilic leukemia and basophilic leukocytosis.

## Patentansprüche

1. Verbindung der Formel **I**: 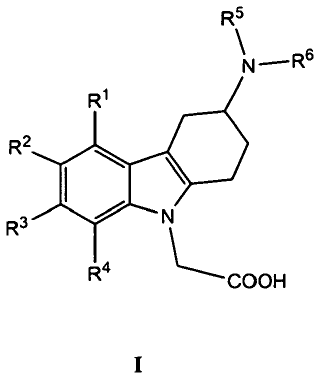 wobei
R¹, R², R³ und R⁴ unabhängig Wasserstoff, C₁₋₅-Alkyl, C₁₋₅-Alkoxy, Alkenyl, Halogen, Nitro, Cyano, Halo-C₁₋₆-alkoxy, Halo-C₁₋₆-alkyl, C₁₋₆-Alkylsulfonyl oder Formyl repräsentieren;
R⁵ Wasserstoff, Alkenyl, C₁₋₆-Alkyl, Cycloalkyl-C₁₋₄-alkyl, C₁₋₃-Alkoxy-C₁₋₄-alkyl, Aryl-C₁₋₄-alkyl oder Aryloxy-C₁₋₄-alkyl repräsentiert;
wobei Aryl unsubstituiert, mono- oder disubstituiert ist durch eine Gruppe, die unabhängig ausgewählt ist aus C₁₋₂-Alkylendioxy, C₁₋₄-Alkoxy, C₁₋₄-Alkyl, Halogen, Trifluormethyl und Trifluormethoxy; und
R⁶ Folgendes repräsentiert: Aryl-C₁₋₃-alkoxy-C₁₋₃-alkoxycarbonyl; Aryl-C₁₋₃-alkoxycarbonyl; Aryl-C₁₋₃-alkylaminocarbonyl; Aryl-C₁₋₆-alkylcarbonyl; Aryl-C₁₋₃-alkoxy-C₁₋₃-alkylcarbonyl; Arylcarbonyl-C₁₋₄-alkylcarbonyl; Aryloxy-C₁₋₃-alkylcarbonyl; Cycloalkyl-C₁₋₃-alkylcarbonyl; Diaryl-C₁₋₃-alkylcarbonyl; Heteroaryl-C₁₋₃-alkylcarbonyl; Aryl-C₃₋₆-cycloalkylcarbonyl; oder R⁷-C₁₋₄-Alkylcarbonyl, wobei die überbrückende C₁₋₄-Alkylgruppe zusätzlich durch Aryl monosubstituiert sein kann und R⁷ Arylaminocarbonyl, Heteroarylaminocarbonyl, C₁₋₆-Alkylaminocarbonyl oder Aryl-C₁₋₃-alkylaminocarbonyl repräsentiert;
wobei Aryl unsubstituiert, mono- oder disubstituiert ist durch eine Gruppe, die unabhängig ausgewählt ist aus C₁₋₂-Alkylendioxy, C₁₋₆-Alkoxy, C₁₋₆-Alkyl, C₁₋₆-Alkylsulfonyl, Halogen, Hydroxy, Halo-C₁₋₆-alkyl, Halo-C₁₋₆-alkoxy, C₁₋₆-Alkylthio und C₁₋₄-Alkoxycarbonylamino; oder ein Salz einer solchen Verbindung.

2. Verbindung nach Anspruch 1, wobei R¹, R³ und R⁴ Wasserstoff repräsentieren;
oder ein Salz einer solchen Verbindung.

3. Verbindung nach Anspruch 1 oder 2, wobei R² Wasserstoff, Trifluormethyl oder Halogen repräsentiert;
oder ein Salz einer solchen Verbindung.

4. Verbindung nach einem der Ansprüche 1 bis 3, wobei R⁵ Folgendes repräsentiert: Wasserstoff; C₁₋₃-Alkyl; Cyclopropylmethyl; 2-Methoxyethyl; Phenyl-C₂₋₃-alkyl; oder Phenoxyethyl, wobei die Phenylgruppe unsubstituiert oder monosubstituiert ist durch eine Gruppe, die ausgewählt ist aus C₁₋₂-Alkylendioxy, C₁₋₄-Alkoxy, C₁₋₄-Alkyl, Halogen, Trifluormethyl und Trifluormethoxy;
oder ein Salz einer solchen Verbindung.

5. Verbindung nach einem der Ansprüche 1 bis 3, wobei
R⁵ Wasserstoff, Methyl, Ethyl oder n-Propyl repräsentiert;
oder ein Salz einer solchen Verbindung.

6. Verbindung nach einem der Ansprüche 1 bis 5, wobei R⁶ Folgendes repräsentiert: Aryl-C₁₋₃-alkoxycarbonyl; Aryl-C₁₋₃-alkylaminocarbonyl; Aryl-C₁₋₆-alkylcarbonyl; Aryl-C₁₋₃-alkoxy-C₁₋₃-alkylcarbonyl; Arylcarbonyl-C₁₋₃-alkylcarbonyl; Aryloxy-C₁₋₃-alkylcarbonyl; Cycloalkyl-C₁₋₃-alkylcarbonyl; Diaryl-C₁₋₃-alkylcarbonyl; Aryl-C₃₋₆-cycloalkylcarbonyl; oder R⁷-C₁₋₄-Alkylcarbonyl, wobei die überbrückende C₁₋₄-Alkylgruppe zusätzlich durch Aryl monosubstituiert sein kann und R⁷ Arylaminocarbonyl, Heteroarylaminocarbonyl, C₁₋₆-Alkylaminocarbonyl oder Aryl-C₁₋₃-alkylaminocarbonyl repräsentiert;
wobei Aryl unsubstituiert, mono- oder disubstituiert ist durch eine Gruppe, die unabhängig ausgewählt ist aus C₁₋₂-Alkylendioxy, C₁₋₃-Alkoxy, C₁₋₆-Alkyl, C₁₋₆-Alkylsulfonyl, Halogen, Hydroxy, Halo-C₁₋₆-alkyl, Halo-C₁₋₆-alkoxy, C₁₋₆-Alkylthio und C₁₋₄-Alkoxycarbonylamino;
oder ein Salz einer solchen Verbindung.

7. Verbindung nach einem der Ansprüche 1 bis 5, wobei R⁶ Folgendes repräsentiert: Aryl-C₁₋₂-alkoxycarbonyl; Aryl-C₁₋₂-alkylaminocarbonyl; Aryl-C₁₋₄-alkylcarbonyl; Aryloxy-C₁₋₂-alkylcarbonyl; oder Diaryl-C₂₋₃-alkylcarbonyl; oder R⁷-C₂₋₄-Alkylcarbonyl, wobei die überbrückende C₂₋₄-Alkylgruppe zusätzlich durch Aryl monosubstituiert sein kann und R⁷ Arylaminocarbonyl oder C₁₋₄-Alkylaminocarbonyl repräsentiert;
wobei Aryl unsubstituiert, mono- oder disubstituiert ist durch eine Gruppe, die unabhängig ausgewählt ist aus C₁₋₂-Alkylendioxy, C₁₋₆-Alkoxy, C₁₋₆-Alkyl, C₁₋₆-Alkylsulfonyl, Halogen, Hydroxy, Trifluormethyl und Trifluormethoxy;
oder ein Salz einer solchen Verbindung.

8. Verbindung nach einem der Ansprüche 1 bis 5, wobei R⁶ Aryl-C₂₋₄-alkylcarbonyl repräsentiert, wobei Aryl unsubstituiert, mono- oder disubstituiert ist durch eine Gruppe, die unabhängig ausgewählt ist aus C₁₋₄-Alkoxy, C₁₋₄-Alkyl, Halogen und Trifluormethyl;
oder ein Salz einer solchen Verbindung.

9. Verbindung nach einem der Ansprüche 1 bis 7, wobei in dem Fall, dass R⁶ eine Gruppe repräsentiert, die eine Carbonylgruppe und einen oder mehrere Arylanteile enthält, die genannte Gruppe derart ist, dass sie eine Überbrückungsgruppe zwischen der Carbonylgruppe und dem (den) genannten Arylanteil (-anteilen) des genannten R⁶ enthält, wobei der Carbonylanteil und wenigstens einer der Arylanteile direkt an verschiedene Atome der genannten Überbrückungsgruppe angefügt sind;
oder ein Salz einer solchen Verbindung.

10. Verbindung nach Anspruch 1, die ausgewählt ist aus der Gruppe bestehend aus:
(3*S*)-[3-(3,3-Diphenyl-propionylamino)-6-fluor-1,2,3,4-tetrahydro-9*H*-carbazol-9-yl]-essigsäure;
(3*R*)-{3-[2-(3-Chlor-phenoxy)-acetylamino]-6-fluor-1,2,3,4-tetrahydro-9*H*-carbazol-9-yl}-essigsäure;
(3*S*)-[6-Fluor-3-(3-phenyl-propionylamino)-1,2,3,4-tetrahydro-9*H*-carbazol-9-yl]-essigsäure;
(3*R*)-(3-[2-(4-Chlor-phenoxy)-acetylamino]-6-fluor-1,2,3,4-tetrahydro-9*H*-carbazol-9-yl)-essigsäure;
(3*S*)-{3-[3-(2-Chlor-phenyl)-propionylamino]-6-fluor-1,2,3,4-tetrahydro-9*H*-carbazol-9-yl}-essigsäure;
(3*R*)-[6-Fluor-3-(3-phenyl-propionylamino)-1,2,3,4-tetrahydro-9*H*-carbazol-9-yl]-essigsäure;
(3*S*)-{3-[3-(4-Chlor-phenyl)-propionylamino]-6-fluor-1,2,3,4-tetrahydro-9*H*-carbazol-9-yl}-essigsäure;
(3*R*)-(3-Benzyloxycarbonylamino-6-fluor-1,2,3,4-tetrahydro-9*H*-carbazol-9-yl)-essigsäure;
(3*S*)-(3-Benzyloxycarbonylamino-6-fluor-1,2,3,4-tetrahydro-9*H*-carbazol-9-yl)-essigsäure;
(3*S*)-{3-[2-(4-Chlor-phenoxy)-acetylamino]-6-fluor-1,2,3,4-tetrahydro-9*H*-carbazol-9-yl}-essigsäure;
(3*R*)-{3-[2-(2-Chlor-phenoxy)-acetylamino]-6-fluor-1,2,3,4-tetrahydro-9*H-*carbazol-9-yl}-essigsäure;
(3*S*)-{3-[3-(3-Chlor-phenyl)-propionylamino]-6-fluor-1,2,3,4-tetrahydro-9*H*-carbazol-9-yl}-essigsäure;
(3*S*)-[6-Fluor-3-(4-oxo-4-phenyl-butyrylamino)-1,2,3,4-tetrahydro-9*H*-carbazol-9-yl]-essigsäure;
(3*S*)-[6-Fluor-3-(2-indan-2-yl-acetylamino)-1,2,3,4-tetrahydro-9*H*-carbazol-9-yl]-essigsäure;
(3*S*)-(3-([2-(4-Chlor-phenyl)-acetyl]-ethyl-amino)-6-fluor-1,2,3,4-tetrahydro-9*H*-carbazol-9-yl)-essigsäure;
(3*R*)-[6-Fluor-3-(2-p-tolyloxy-acetylamino)-1,2,3,4-tetrahydro-9*H*-carbazol-9-yl]-essigsäure;
(3*S*)-{6-Fluor-3-[methyl-(3-phenyl-propionyl)-amino]-1,2,3,4-tetrahydro-9*H*-carbazol-9-yl}-essigsäure;
(3*S*)-[6-Fluor-3-(3-1*H*-indol-3-yl-propionylamino)-1,2,3,4-tetrahydro-9*H*-carbazol-9-yl]-essigsäure;
(3*S*)-[3-(3-Benzo[1,3]dioxol-5-yl-propionylamino)-6-fluor-1,2,3,4-tetrahydro-9*H*-carbazol-9-yl]-essigsäure;
(3*S*)-{6-Fluor-3-[ethyl-(3-phenyl-propionyl)-amino]-1,2,3,4-tetrahydro-9*H*-carbazol-9-yl}-essigsäure;
(3*S*)-{3-[2-(4-Chlor-phenyl)-acetylamino]-6-fluor-1,2,3,4-tetrahydro-9*H*-carbazol-9-yl}-essigsäure;
(3*S*)-[3-(2,3-Diphenyl-propionylamino)-6-fluor-1,2,3,4-tetrahydro-9*H*-carbazol-9-yl]-essigsäure;
(3*R*)-[6-Fluor-3-(2-phenoxy-acetylamino)-1,2,3,4-tetrahydro-9*H*-carbazol-9-yl]-essigsäure;
(3*S*)-{3-[3-(3,4-Difluor-phenyl)-propionylamino]-6-fluor-1,2,3,4-tetrahydro-9*H*-carbazol-9-yl}-essigsäure;
(3*S*)-[3-(3-Phenyl-propionylamino)-1,2,3,4-tetrahydro-9*H*-carbazol-9-yl]-essigsäure;
(3*R*)-[3-(2-Benzyloxy-acetylamino)-1,2,3,4-tetrahydro-9*H*-carbazol-9-yl]-essigsäure;
(3*S*)-{6-Fluor-3-[3-(2-methoxy-phenyl)-propionylamino]-1,2,3,4-tetrahydro-9*H*-carbazol-9-yl}-essigsäure;
(3*S*)-{6-Fluor-3-[propyl-(3-phenyl-propionyl)-amino]-1,2,3,4-tetrahydro-9*H*-carbazol-9-yl}-essigsäure;
(3*S*)-[3-(2-Benzyloxy-acetylamino)-1,2,3,4-tetrahydro-9*H*-carbazol-9-yl]-essigsäure;
(3*R*)-(3-Benzyloxycarbonylamino-1,2,3,4-tetrahydro-9*H*-carbazol-9-yl)-essigsäure;
(3*R*)-{6-Fluor-3-[2-(4-methoxy-phenyl)-acetylamino]-1,2,3,4-tetrahydro-9*H*-carbazol-9-yl}-essigsäure;
(3*R*)-{3-[3-(4-Chlor-phenyl)-propionylamino]-6-fluor-1,2,3,4-tetrahydro-9*H*-carbazol-9-yl}-essigsäure;
(3*S*)-{3-[4-(4-Brom-phenyl)-4-oxo-butyrylamino]-6-fluor-1,2,3,4-tetrahydro-9*H*-carbazol-9-yl}-essigsäure;
(3*S*)-(3-{[2-(4-Chlor-phenyl)-acetyl]-propyl-amino}-6-fluor-1,2,3,4-tetrahydro-9*H-*carbazol-9-yl)-essigsäure;
(3*R*)-(3-Phenylacetylamino-1,2,3,4-tetrahydro-9*H*-carbazol-9-yl)-essigsäure;
(3*R*)-{3-[3-(2-Chlor-phenyl)-propionylamino]-6-fluor-1,2,3,4-tetrahydro-9*H*-carbazol-9-yl}-essigsäure;
(3*S*)-{6-Fluor-3-[2-(4-trifluormethyl-phenoxy)-acetylamino]-1,2,3,4-tetrahydro-9*H-*carbazol-9-yl}-essigsäure;
(3*R*)-(6-Fluor-3-phenylacetylamino-1,2,3,4-tetrahydro-9*H*-carbazol-9-yl)-essigsäure;
(3*S*)-{6-Fluor-3-[3-(2-hydroxy-phenyl)-propionylamino]-1,2,3,4-tetrahydro-9*H*-carbazol-9-yl}-essigsäure;
(3*S*)-[3-(3-1*H*-Benzoimidazol-2-yl-propionylamino)-6-fluor-1,2,3,4-tetrahydro-9*H-*carbazol-9-yl]-essigsäure;
(3*S*)-{6-Fluor-3-[3-(4-hydroxy-phenyl)-propionylamino]-1,2,3,4-tetrahydro-9*H*-carbazol-9-yl}-essigsäure;
(3*S*)-[6-Fluor-3-(2-*p*-tolyloxy-acetylamino)-1,2,3,4-tetrahydro-9*H*-carbazol-9-yl]-essigsäure;
(3*R*)-[6-Fluor-3-(2-*p*-tolyl-acetylamino)-1,2,3,4-tetrahydro-9*H*-carbazol-9-yl]-essigsäure;
(3*R*)-{3-[3-(3-Chlor-phenyl)-propionylamino]-6-fluor-1,2,3,4-tetrahydro-9*H*-carbazol-9-yl}-essigsäure;
(3*R*)-[3-(2-Phenoxy-acetylamino)-1,2,3,4-tetrahydro-9*H*-carbazol-9-yl]-essigsäure;
(3*S*)-[6-Fluor-3-(3-*p*-tolyl-propionylamino)-1,2,3,4-tetrahydro-9*H*-carbazol-9-yl]-essigsäure;
(3*S*)-(3-Benzyloxycarbonylamino-1,2,3,4-tetrahydro-9*H*-carbazol-9-yl)-essigsäure;
(3*S*)-[6-Fluor-3-(2-*p*-tolyl-acetylamino)-1,2,3,4-tetrahydro-9*H*-carbazol-9-yl]-essigsäure;
(3*S*)-[3-(3-Phenethyl-ureido)-1,2,3,4-tetrahydro-9*H*-carbazol-9-yl]-essigsäure;
(3*S*)-{6-Fluor-3-[3-(3-hydroxy-phenyl)-propionylamino]-1,2,3,4-tetrahydro-9*H*-carbazol-9-yl}-essigsäure;
(3*R*)-[3-(2-Benzyloxy-ethoxycarbonylamino)-1,2,3,4-tetrahydro-9*H*-carbazol-9-yl]-essigsäure;
(3*S*)-[6-Fluor-3-(3-naphthalen-2-yl-propionylamino)-1,2,3,4-tetrahydro-9*H*-carbazol-9-yl]-essigsäure;
(3*S*)-{6-Fluor-3-[4-(4-methansulfonyl-phenyl)-4-oxo-butyrylamino]-1,2,3,4-tetrahydro-9*H-*carbazol-9-yl}-essigsäure;
(3*S*)-(3-{[2-(4-Chlor-phenyl)-acetyl]-methyl-amino}-6-fluor-1,2,3,4-tetrahydro-9*H-*carbazol-9-yl)-essigsäure;
(3*S*)-{6-Fluor-3-[3-(4-methoxy-phenyl)-propionylamino]-1,2,3,4-tetrahydro-9*H*-carbazol-9-yl}-essigsäure;
(3*R*)-[3-(3-Phenyl-propionylamino)-1,2,3,4-tetrahydro-9*H*-carbazol-9-yl]-essigsäure;
(3*R*)-{3-[2-(4-Chlor-phenyl)-acetylamino]-6-fluor-1,2,3,4-tetrahydro-9*H*-carbazol-9-yl}-essigsäure;
(3R)-[6-Fluor-3-(3-p-tolyl-propionylamino)-1,2,3,4-tetrahydro-9*H*-carbazol-9-yl]-essigsäure;
(3*R*)-{6-Fluor-3-[3-(4-methoxy-phenyl)-propionylamino]-1,2,3,4-tetrahydro-9*H*-carbazol-9-yl}-essigsäure;
(3*S*)-{6-Fluor-3-[3-(4-hydroxy-3-methoxy-phenyl)-propionylamino]-1,2,3,4-tetrahydro-9*H-*carbazol-9-yl}-essigsäure;
(3*S*)-{6-Fluor-3-[2-(3-trifluormethyl-phenyl)-acetylamino]-1,2,3,4-tetrahydro-9*H*-carbazol-9-yl}-essigsäure;
(3*S*)-{6-Fluor-3-[2-(4-methoxy-phenyl)-acetylamino]-1,2,3,4-tetrahydro-9*H*-carbazol-9-yl}-essigsäure;
(3*R*)-{3-[2-(3-Chlor-phenyl)-acetylamino]-6-fluor-1,2,3,4-tetrahydro-9*H*-carbazol-9-yl}-essigsäure;
(3*S*)-{6-Fluor-3-[2-(4-trifluormethyl-phenyl)-acetylamino]-1,2,3,4-tetrahydro-9*H*-carbazol-9-yl}-essigsäure;
(3*R*)-{3-[2-(3,4-Dichlor-phenyl)-acetylamino]-6-fluor-1,2,3,4-tetrahydro-9*H*-carbazol-9-yl}-essigsäure;
(3*S*)-{6-Fluor-3-[3-(3-methoxy-phenyl)-propionylamino]-1,2,3,4-tetrahydro-9*H*-carbazol-9-yl}-essigsäure;
(3*S*)-[6-Fluor-3-(2-phenoxy-acetylamino)-1,2,3,4-tetrahydro-9*H*-carbazol-9-yl]-essigsäure;
(3*R*)-[3-(3-Phenethyl-ureido)-1,2,3,4-tetrahydro-9*H*-carbazol-9-yl]-essigsäure;
(3*S*)-[3-(2-Benzyloxy-ethoxycarbonylamino)-1,2,3,4-tetrahydro-9*H*-carbazol-9-yl]-essigsäure;
(3*S*)-[3-(2-Phenoxy-acetylamino)-1,2,3,4-tetrahydro-9*H*-carbazol-9-yl]-essigsäure;
(3*S*)-[3-(2-Thiophen-2-yl-acetylamino)-1,2,3,4-tetrahydro-9*H*-carbazol-9-yl]-essigsäure;
(3*S*)-(3-Phenylacetylamino-1,2,3,4-tetrahydro-9*H*-carbazol-9-yl)-essigsäure;
(3*S*)-[3-(3-Benzyl-ureido)-1,2,3,4-tetrahydro-9*H*-carbazol-9-yl]-essigsäure;
(3*S*)-{3-[2-(4-tert-Butyl-phenyl)-acetylamino]-6-fluor-1,2,3,4-tetrahydro-9*H*-carbazol-9-yl}-essigsäure;
(3*R*)-(3-Benzyloxycarbonylamino-8-chlor-6-fluor-1,2,3,4-tetrahydro-9*H*-carbazol-9-yl)-essigsäure;
(3*S*)-(3-Benzyloxycarbonylamino-8-chlor-6-fluor-1,2,3,4-tetrahydro-9*H*-carbazol-9-yl)-essigsäure;
(3*S*)-[6-Fluor-3-(3-pyridin-3-yl-propionylamino)-1,2,3,4-tetrahydro-9*H*-carbazol-9-yl]-essigsäure;
(3*R*)-[3-(3-Benzyl-ureido)-1,2,3,4-tetrahydro-9*H*-carbazol-9-yl]-essigsäure;
(3*R*)-{6-Fluor-3-[2-(4-trifluormethyl-phenyl)-acetylamino]-1,2,3,4-tetrahydro-9*H*-carbazol-9-yl}-essigsäure;
(3*S*)-[3-(3-Cyclopentyl-propionylamino)-1,2,3,4-tetrahydro-9*H*-carbazol-9-yl]-essigsäure;
(3*R*)-[3-(2-Thiophen-2-yl-acetylamino)-1,2,3,4-tetrahydro-9*H*-carbazol-9-yl]-essigsäure;
(3*R*)-(3-Benzyloxycarbonylamino-8-chlor-5-fluor-1,2,3,4-tetrahydro-9*H*-carbazol-9-yl)-essigsäure;
(3*S*)-(3-Benzyloxycarbonylamino-8-chlor-5-fluor-1,2,3,4-tetrahydro-9*H*-carbazol-9-yl)-essigsäure; und
(3*R*)-[3-(3-Cyclopentyl-propionylamino)-1,2,3,4-tetrahydro-9*H*-carbazol-9-yl]-essigsäure;
oder ein Salz einer solchen Verbindung.

11. Verbindung nach Anspruch 1, die ausgewählt ist aus der Gruppe bestehend aus:
(3*R*)-[3-(3-Benzyl-ureido)-6-fluor-1,2,3,4-tetrahydro-9*H*-carbazol-9-yl]-essigsäure;
(3*S*)-[3-(3-Benzyl-ureido)-6-fluor-1,2,3,4-tetrahydro-9*H*-carbazol-9-yl]-essigsäure;
(3*S*)-[6-Fluor-3-(3-phenethyl-ureido)-1,2,3,4-tetrahydro-9*H*-carbazol-9-yl]-essigsäure;
(3*R*)-[3-(3-Benzyl-ureido)-8-chlor-6-fluor-1,2,3,4-tetrahydro-9*H*-carbazol-9-yl]-essigsäure;
(3*S*)-[3-(3-Benzyl-ureido)-8-chlor-6-fluor-1,2,3,4-tetrahydro-9*H*-carbazol-9-yl]-essigsäure;
(3*R*)-[8-Chlor-6-fluor-3-(3-phenethyl-ureido)-1,2,3,4-tetrahydro-9*H*-carbazol-9-yl]-essigsäure;
(3*S*)-[8-Chlor-6-fluor-3-(3-phenethyl-ureido)-1,2,3,4-tetrahydro-9*H*-carbazol-9-yl]-essigsäure;
(3*R*)-(3-Benzyloxycarbonylamino-6-trifluormethyl-1,2,3,4-tetrahydro-9*H*-carbazol-9-yl)-essigsäure;
(3*S*)-(3-Benzyloxycarbonylamino-6-trifluormethyl-1,2,3,4-tetrahydro-9*H*-carbazol-9-yl)-essigsäure;
(3*R*)-(3-Benzyloxycarbonylamino-8-brom-6-fluor-1,2,3,4-tetrahydro-9*H*-carbazol-9-yl)-essigsäure;
(3*S*)-(3-Benzyloxycarbonylamino-8-brom-6-fluor-1,2,3,4-tetrahydro-9*H*-carbazol-9-yl)-essigsäure;
(3*R*)-(3-Benzyloxycarbonylamino-6-fluor-8-vinyl-1,2,3,4-tetrahydro-9*H*-carbazol-9-yl)-essigsäure;
(3*S*)-(3-Benzyloxycarbonylamino-6-fluor-8-vinyl-1,2,3,4-tetrahydro-9*H*-carbazol-9-yl)-essigsäure;
(3*R*)-(3-Benzyloxycarbonylamino-6-fluor-8-methansulfonyl-1,2,3,4-tetrahydro-9*H-*carbazol-9-yl)-essigsäure;
(3*S*)-(3-Benzyloxycarbonylamino-6-fluor-8-methansulfonyl-1,2,3,4-tetrahydro-9*H-*carbazol-9-yl)-essigsäure;
(3*S*)-(3-Benzyloxycarbonylamino-6-fluor-8-methyl-1,2,3,4-tetrahydro-9*H*-carbazol-9-yl)-essigsäure;
(3*S*)-(3-Benzyloxycarbonylamino-7-chlor-6-fluor-1,2,3,4-tetrahydro-9*H*-carbazol-9-yl)-essigsäure;
(3*S*)-(8-Allyl-3-benzyloxycarbonylamino-6-fluor-1,2,3,4-tetrahydro-9*H*-carbazol-9-yl)-essigsäure;
(3*R*)-(3-Benzyloxycarbonylamino-8-chlor-1,2,3,4-tetrahydro-9*H*-carbazol-9-yl)-essigsäure;
(3*S*)-{3-[3-(2,4-Dimethoxy-phenyl)-propionylamino]-6-fluor-1,2,3,4-tetrahydro-9*H-*carbazol-9-yl}-essigsäure;
(3*S*)-[6-Fluor-3-(3-naphthalen-1-yl-propionylamino)-1,2,3,4-tetrahydro-9*H*-carbazol-9-yl]-essigsäure;
(3*R*)-{6-Fluor-3-[2-(2-methoxy-phenoxy)-acetylamino]-1,2,3,4-tetrahydro-9*H*-carbazol-9-yl}-essigsäure;
(3*S*)-{6-Fluor-3-[2-(2-methoxy-phenoxy)-acetylamino]-1,2,3,4-tetrahydro-9*H*-carbazol-9-yl}-essigsäure;
(3*R*)-{6-Fluor-3-[3-(2-methylphenyl)-propionylamino]-1,2,3,4-tetrahydro-9*H*-carbazol-9-yl}-essigsäure;
(3*S*)-{6-Fluor-3-[3-(2-methylphenyl)-propionylamino]-1,2,3,4-tetrahydro-9*H*-carbazol-9-yl}-essigsäure;
(3*R*)-{6-Fluor-3-[3-(3-methylphenyl)-propionylamino]-1,2,3,4-tetrahydro-9*H*-carbazol-9-yl}-essigsäure;
(3*S*)-{6-Fluor-3-[3-(3-methylphenyl)-propionylamino]-1,2,3,4-tetrahydro-9*H*-carbazol-9-yl}-essigsäure;
(3*R*)-{6-Fluor-3-[3-(3-methoxy-phenyl)-propionylamino]-1,2,3,4-tetrahydro-9*H*-carbazol-9-yl}-essigsäure;
(3*S*)-{6-Fluor-3-[3-(3-methoxy-phenyl)-propionylamino]-1,2,3,4-tetrahydro-9*H*-carbazol-9-yl}-essigsäure;
(3*R*)-{6-Fluor-3-[2-(3-methoxy-phenoxy)-acetylamino]-1,2,3,4-tetrahydro-9*H*-carbazol-9-yl}-essigsäure;
(3*S*)-{6-Fluor-3-[2-(3-methoxy-phenoxy)-acetylamino]-1,2,3,4-tetrahydro-9*H*-carbazol-9-yl}-essigsäure;
(3*R*)-{6-Fluor-3-[2-(2-methylphenoxy)-acetylamino]-1,2,3,4-tetrahydro-9*H*-carbazol-9-yl}-essigsäure;
(3*S*)-{6-Fluor-3-[2-(2-methylphenoxy)-acetylamino]-1,2,3,4-tetrahydro-9*H*-carbazol-9-yl}-essigsäure;
(3*S*)-{3-[3-(2,5-Dimethoxy-phenyl)-propionylamino]-6-fluor-1,2,3,4-tetrahydro-9*H-*carbazol-9-yl}-essigsäure;
(3*S*)-{6-Fluor-3-[3-(4-trifluormethyl-phenyl)-propionylamino]-1,2,3,4-tetrahydro-9*H-*carbazol-9-yl}-essigsäure;
(3*S*)-{3-[3-(2,6-Dichlor-phenyl)-propionylamino]-6-fluor-1,2,3,4-tetrahydro-9*H*-carbazol-9-yl}-essigsäure;
(3*S*)-{3-[3-(2,5-Bis-trifluormethyl-phenyl)-propionylamino]-6-fluor-1,2,3,4-tetrahydro-9*H-*carbazol-9-yl}-essigsäure;
(3*S*)-{6-Fluor-3-[3-(4-methylsulfanyl-phenyl)-propionylamino]-1,2,3,4-tetrahydro-9*H-*carbazol-9-yl}-essigsäure;
(3*S*)-{6-Fluor-3-[3-(4-iod-phenyl)-propionylamino]-1,2,3,4-tetrahydro-9*H*-carbazol-9-yl}-essigsäure;
(3*S*)-{6-Fluor-3-[3-(4-isopropyl-phenyl)-propionylamino]-1,2,3,4-tetrahydro-9*H*-carbazol-9-yl}-essigsäure;
(3*S*)-{6-Fluor-3-[3-(3-trifluormethyl-phenyl)-propionylamino]-1,2,3,4-tetrahydro-9*H-*carbazol-9-yl}-essigsäure;
(3*S*)-{3-[3-(2,4-Dichlor-phenyl)-propionylamino]-6-fluor-1,2,3,4-tetrahydro-9*H*-carbazol-9-yl}-essigsäure;
(3*S*)-{6-Fluor-3-[3-(4-fluor-phenyl)-propionylamino]-1,2,3,4-tetrahydro-9*H*-carbazol-9-yl}-essigsäure;
(3*S*)-{3-[3-(3,5-Bis-trifluormethyl-phenyl)-propionylamino]-6-fluor-1,2,3,4-tetrahydro-9*H-*carbazol-9-yl}-essigsäure;
(3*S*)-{3-[3-(4-Ethyl-phenyl)-propionylamino]-6-fluor-1,2,3,4-tetrahydro-9*H*-carbazol-9-yl}-essigsäure;
(3*S*)-{6-Fluor-3-[3-(3-iod-phenyl)-propionylamino]-1,2,3,4-tetrahydro-9*H*-carbazol-9-yl}-essigsäure;
(3*S*)-{6-Fluor-3-[3-(4-methansulfonyl-phenyl)-propionylamino]-1,2,3,4-tetrahydro-9*H-*carbazol-9-yl}-essigsäure;
(3*S*)-{3-[3-(2,3-Dimethoxy-phenyl)-propionylamino]-6-fluor-1,2,3,4-tetrahydro-9*H-*carbazol-9-yl}-essigsäure;
(3*S*)-{3-[3-(2-Brom-phenyl)-propionylamino]-6-fluor-1,2,3,4-tetrahydro-9*H*-carbazol-9-yl}-essigsäure;
(3*S*)-{6-Fluor-3-[3-(3-trifluormethoxy-phenyl)-propionylamino]-1,2,3,4-tetrahydro-9*H-*carbazol-9-yl}-essigsäure;
(3*S*)-{3-[3-(2,4-Dimethyl-phenyl)-propionylamino]-6-fluor-1,2,3,4-tetrahydro-9*H*-carbazol-9-yl}-essigsäure;
(3*S*)-{3-[3-(3-Brom-phenyl)-propionylamino]-6-fluor-1,2,3,4-tetrahydro-9*H*-carbazol-9-yl}-essigsäure;
(3*S*)-{3-[3-(3-*tert*-Butoxycarbonylamino-phenyl)-propionylamino]-6-fluor-1,2,3,4-tetrahydro-9*H*-carbazol-9-yl}-essigsäure;
(3*S*)-{6-Fluor-3-[(*S*)-3-(4-fluor-phenyl)-2-phenyl-propionylamino]-1,2,3,4-tetrahydro-9*H-*carbazol-9-yl}-essigsäure;
(3*S*)-{6-Fluor-3-[(*S*)-3-(4-methoxy-phenyl)-2-phenyl-propionylamino]-1,2,3,4-tetrahydro-9*H*-carbazol-9-yl}-essigsäure;
(3*S*)-{6-Fluor-3-[3-(2-fluor-phenyl)-propionylamino]-1,2,3,4-tetrahydro-9*H*-carbazol-9-yl}-essigsäure;
(3*S*)-(6-Fluor-3-{(2*RS*)-2-[(4-fluor-phenylcarbamoyl)-methyl]-3-phenyl-propionylamino}-1,2,3,4-tetrahydro-9*H*-carbazol-9-yl)-essigsäure;
(3*S*)-{3-[(2*RS*)-2-Benzyl-3,3-dimethyl-butyrylamino]-6-fluor-1,2,3,4-tetrahydro-9*H-*carbazol-9-yl}-essigsäure;
(3*S*)-{6-Fluor-3-[3-(3-fluor-phenyl)-propionylamino]-1,2,3,4-tetrahydro-9*H*-carbazol-9-yl}-essigsäure;
(3*S*)-{6-Fluor-3-[(2*R*)-2-methyl-3-phenyl-propionylamino]-1,2,3,4-tetrahydro-9*H*-carbazol-9-yl}-essigsäure;
(3*S*)-[3-(2,2-Dimethyl-3-phenyl-propionylamino)-6-fluor-1,2,3,4-tetrahydro-9*H*-carbazol-9-yl]-essigsäure;
(3*S*)-[6-Fluor-3-(3-methyl-3-phenyl-butyrylamino)-1,2,3,4-tetrahydro-9*H*-carbazol-9-yl]-essigsäure;
(3*S*)-{6-Fluor-3-[(3*S*)-3-phenyl-butyrylamino]-1,2,3,4-tetrahydro-9*H*-carbazol-9-yl}-essigsäure;
(3*S*)-[3-(2-Benzyloxy-acetylamino)-6-fluor-1,2,3,4-tetrahydro-9*H*-carbazol-9-yl]-essigsäure;
(3*S*)-[6-Fluor-3-(4-phenyl-butyrylamino)-1,2,3,4-tetrahydro-9*H*-carbazol-9-yl]-essigsäure;
(3*R*)-{8-Chlor-6-fluor-3-[3-(2-methylphenyl)-propionylamino]-1,2,3,4-tetrahydro-9*H-*carbazol-9-yl}-essigsäure;
(3*S*)-{8-Chlor-6-fluor-3-[3-(2-methylphenyl)-propionylamino]-1,2,3,4-tetrahydro-9*H-*carbazol-9-yl}-essigsäure;
(3*R*)-{8-Chlor-6-fluor-3-[2-(2-methoxy-phenoxy)-acetylamino]-1,2,3,4-tetrahydro-9*H-*carbazol-9-yl}-essigsäure;
(3*S*)-{8-Chlor-6-fluor-3-[2-(2-methoxy-phenoxy)-acetylamino]-1,2,3,4-tetrahydro-9*H* carbazol-9-yl}-essigsäure;
(3*R*)-{8-Chlor-6-fluor-3-[3-(3-hydroxy-phenyl)-propionylamino]-1,2,3,4-tetrahydro-9*H-*carbazol-9-yl}-essigsäure;
(3*S*)-{8-Chlor-6-fluor-3-[3-(3-hydroxy-phenyl)-propionylamino]-1,2,3,4-tetrahydro-9*H-*carbazol-9-yl}-essigsäure;
(3*R*)-{8-Chlor-6-fluor-3-[3-(3-methoxy-phenyl)-propionylamino]-1,2,3,4-tetrahydro-9*H-*carbazol-9-yl}-essigsäure;
(3*S*)-{8-Chlor-6-fluor-3-[3-(3-methoxy-phenyl)-propionylamino]-1,2,3,4-tetrahydro-9*H-*carbazol-9-yl}-essigsäure;
(3*R*)-{8-Chlor-6-fluor-3-[3-(3-methylphenyl)-propionylamino]-1,2,3,4-tetrahydro-9*H-*carbazol-9-yl}-essigsäure;
(3*S*)-{8-Chlor-6-fluor-3-[3-(3-methylphenyl)-propionylamino]-1,2,3,4-tetrahydro-9*H-*carbazol-9-yl}-essigsäure;
(3*R*)-{8-Chlor-6-fluor-3-[3-(2-hydroxy-phenyl)-propionylamino]-1,2,3,4-tetrahydro-9*H-*carbazol-9-yl}-essigsäure;
(3*S*)-{8-Chlor-6-fluor-3-[3-(2-hydroxy-phenyl)-propionylamino]-1,2,3,4-tetrahydro-9*H-*carbazol-9-yl}-essigsäure;
(3*R*)-[8-Chlor-6-fluor-3-(3-phenyl-propionylamino)-1,2,3,4-tetrahydro-9*H*-carbazol-9-yl]-essigsäure;
(3*S*)-[8-Chlor-6-fluor-3-(3-phenyl-propionylamino)-1,2,3,4-tetrahydro-9*H*-carbazol-9-yl]-essigsäure;
(3*R*)-{8-Chlor-6-fluor-3-[3-(2-methoxy-phenyl)-propionylamino]-1,2,3,4-tetrahydro-9*H-*carbazol-9-yl}-essigsäure;
(3*S*)-{8-Chlor-6-fluor-3-[3-(2-methoxy-phenyl)-propionylamino]-1,2,3,4-tetrahydro-9*H-*carbazol-9-yl}-essigsäure;
(3*R*)-{8-Chlor-3-[3-(3-chlor-phenyl)-propionylamino]-6-fluor-1,2,3,4-tetrahydro-9*H-*carbazol-9-yl}-essigsäure;
(3*S*)-{8-Chlor-3-[3-(3-chlor-phenyl)-propionylamino]-6-fluor-1,2,3,4-tetrahydro-9*H-*carbazol-9-yl}-essigsäure;
(3*R*)-[8-Chlor-6-fluor-3-(3-*1H*-indol-3-yl-propionylamino)-1,2,3,4-tetrahydro-9*H*-carbazol-9-yl]-essigsäure;
(3*S*)-[8-Chlor-6-fluor-3-(3-*1H*-indol-3-yl-propionylamino)-1,2,3,4-tetrahydro-9*H*-carbazol-9-yl]-essigsäure;
(3*R*)-{8-Chlor-3-[2-(2-chlor-phenoxy)-acetylamino]-6-fluor-1,2,3,4-tetrahydro-9*H-*carbazol-9-yl} -essigsäure;
(3*S*)-{8-Chlor-3-[2-(2-chlor-phenoxy)-acetylamino]-6-fluor-1,2,3,4-tetrahydro-9*H*-carbazol-9-yl}-essigsäure;
(3*R*)-{8-Chlor-6-fluor-3-[2-(2-methylphenyl)-oxy-acetylamino]-1,2,3,4-tetrahydro-9*H* carbazol-9-yl}-essigsäure;
(3*S*)-{8-Chlor-6-fluor-3-[2-(2-methylphenyl)-oxy-acetylamino]-1,2,3,4-tetrahydro-9*H-*carbazol-9-yl}-essigsäure;
(3*R*)-[3-(3-Benzo[1,3]dioxol-5-yl-propionylamino)-8-chlor-6-fluor-1,2,3,4-tetrahydro-9*H-*carbazol-9-yl]-essigsäure;
(3*S*)-[3-(3-Benzo[1,3]dioxol-5-yl-propionylamino)-8-chlor-6-fluor-1,2,3,4-tetrahydro-9*H-*carbazol-9-yl]-essigsäure;
(3*R*)-{8-Chlor-6-fluor-3-[2-(3-methoxy-phenoxy)-acetylamino]-1,2,3,4-tetrahydro-9*H-*carbazol-9-yl}-essigsäure;
(3*S*)-{8-Chlor-6-fluor-3-[2-(3-methoxy-phenoxy)-acetylamino]-1,2,3,4-tetrahydro-9*H-*carbazol-9-yl}-essigsäure;
(3*R*)-{8-Chlor-3-[2-(3-chlor-phenoxy)-acetylamino]-6-fluor-1,2,3,4-tetrahydro-9*H-*carbazol-9-yl} -essigsäure;
(3*S*)-{8-Chlor-3-[2-(3-chlor-phenoxy)-acetylamino]-6-fluor-1,2,3,4-tetrahydro-9*H*-carbazol-9-yl}-essigsäure;
(3*R*)-{8-Chlor-3-[3-(2-chlor-phenyl)-propionylamino]-6-fluor-1,2,3,4-tetrahydro-9*H-*carbazol-9-yl}-essigsäure;
(3*S*)-{8-Chlor-3-[3-(2-chlor-phenyl)-propionylamino]-6-fluor-1,2,3,4-tetrahydro-9*H-*carbazol-9-yl}-essigsäure;
(3*R*)-[8-Chlor-6-fluor-3-(2-indan-2-yl-acetylamino)-1,2,3,4-tetrahydro-9*H*-carbazol-9-yl]-essigsäure;
(3*S*)-[8-Chlor-6-fluor-3-(2-indan-2-yl-acetylamino)-1,2,3,4-tetrahydro-9*H*-carbazol-9-yl]-essigsäure;
(3*S*)-[6-Fluor-3-(1-methyl-3-phenethyl-ureido)-1,2,3,4-tetrahydro-9*H*-carbazol-9-yl]-essigsäure;
(3*S*)-{3-[3-(2-Chlor-benzyl)-1-methyl-ureido]-6-fluor-1,2,3,4-tetrahydro-9*H*-carbazol-9-yl}-essigsäure;
(3*S*)-[3-(3-Benzyl-1-methyl-ureido)-6-fluor-1,2,3,4-tetrahydro-9*H*-carbazol-9-yl]-essigsäure;
(3*S*)-[3-(Benzyloxycarbonyl-methyl-amino)-6-fluor-1,2,3,4-tetrahydro-9*H*-carbazol-9-yl]-essigsäure;
{(3*S*)-3-[(2-Chlor-benzyloxycarbonyl)-methyl-amino]-6-fluor-1,2,3,4-tetrahydro-9*H-*carbazol-9-yl}-essigsäure;
(3*S*)-(6-Fluor-3-{[2-(4-methoxy-phenyl)-acetyl]-methyl-amino}-1,2,3,4-tetrahydro-9*H-*carbazol-9-yl)-essigsäure;
(3*S*)-(6-Fluor-3-{methyl-[2-(4-methylphenyl)-acetyl]-amino}-1,2,3,4-tetrahydro-9*H-*carbazol-9-yl)-essigsäure;
(3*S*)-(6-Fluor-3-{[2-(2-methoxy-phenyl)-acetyl]-methyl-amino}-1,2,3,4-tetrahydro-9*H-*carbazol-9-yl)-essigsäure;
(3*S*)-{6-Fluor-3-[(2-indan-2-yl-acetyl)-methyl-amino]-1,2,3,4-tetrahydro-9*H*-carbazol-9-yl}-essigsäure;
(3*S*)-(3-{[2-(3-Chlor-phenyl)-acetyl]-methyl-amino}-6-fluor-1,2,3,4-tetrahydro-9*H-*carbazol-9-yl)-essigsäure;
(3*S*)-(6-Fluor-3-{methyl-[2-(3-methylphenyl)-acetyl]-amino}-1,2,3,4-tetrahydro-9*H-*carbazol-9-yl)-essigsäure;
(3*S*)-(6-Fluor-3-{[2-(3-methoxy-phenyl)-acetyl]-methyl-amino}-1,2,3,4-tetrahydro-9*H-*carbazol-9-yl)-essigsäure;
(3*S*)-(3-{[2-(2-Chlor-phenoxy)-acetyl]-methyl-amino}-6-fluor-1,2,3,4-tetrahydro-9*H-*carbazol-9-yl)-essigsäure;
(3*S*)-(3-{[2-(4-Chlor-phenoxy)-acetyl]-methyl-amino}-6-fluor-1,2,3,4-tetrahydro-9*H-*carbazol-9-yl)-essigsäure;
(3*S*)-(6-Fluor-3-{[3-(3-methoxy-phenyl)-propionyl]-methyl-amino}-1,2,3,4-tetrahydro-9*H-*carbazol-9-yl)-essigsäure;
(3*S*)-(6-Fluor-3-{methyl-[2-(2-methylphenyl)-acetyl]-amino}-1,2,3,4-tetrahydro-9*H-*carbazol-9-yl)-essigsäure;
(3*S*)-{3-[(3,3-Diphenyl-propionyl)-methyl-amino]-6-fluor-1,2,3,4-tetrahydro-9*H*-carbazol-9-yl}-essigsäure;
(3*S*)-(6-Fluor-3-{[3-(2-methoxy-phenyl)-propionyl]-methyl-amino}-1,2,3,4-tetrahydro-9*H-*carbazol-9-yl)-essigsäure;
(3*S*)-{6-Fluor-3-[(3-1*H*-indol-3-yl-propionyl)-methyl-amino]-1,2,3,4-tetrahydro-9*H-*carbazol-9-yl}-essigsäure;
(3*S*)-{3-[(2-Benzyloxy-acetyl)-methyl-amino]-6-fluor-1,2,3,4-tetrahydro-9*H*-carbazol-9-yl}-essigsäure;
(3*S*)-{3-[(2,3-Diphenyl-propionyl)-methyl-amino]-6-fluor-1,2,3,4-tetrahydro-9*H*-carbazol-9-yl}-essigsäure;
(3*S*)-{6-Fluor-3-[[3-(2-methoxy-phenyl)-propionyl]-(3-phenyl-propyl)-amino]-1,2,3,4-tetrahydro-9*H*-carbazol-9-yl}-essigsäure;
(3*S*)-{3-[3-Benzyl-(1-cyclopropylmethyl)-ureido]-6-fluor-1,2,3,4-tetrahydro-9*H*-carbazol-9-yl}-essigsäure;
(3*S*)-[3-(Benzyloxycarbonyl-cyclopropylmethyl-amino)-6-fluor-1,2,3,4-tetrahydro-9*H-*carbazol-9-yl]-essigsäure;
(3*S*)-{3-[Cyclopropylmethyl-(3-phenyl-propionyl)-amino]-6-fluor-1,2,3,4-tetrahydro-9*H-*carbazol-9-yl}-essigsäure;
(3*S*)-{3-[Cydopropylmethyl-((S)-2-methyl-3-phenyl-propionyl)-amino]-6-fluor-1,2,3,4-tetrahydro-9*H*-carbazol-9-yl}-essigsäure;
(3*S*)-(3-{Cyclopropylmethyl-[3-(2-methoxy-phenyl)-propionyl]-amino}-6-fluor-1,2,3,4-tetrahydro-9*H*-carbazol-9-yl)-essigsäure;
(3*S*)-(3-{[2-(3-Chlor-phenoxy)-acetyl]-cyclopropylmethyl-amino}-6-fluor-1,2,3,4-tetrahydro-9*H*-carbazol-9-yl)-essigsäure;
(3*S*)-{3-[Cyclopropylmethyl-(3,3-diphenyl-propionyl)-amino]-6-fluor-1,2,3,4-tetrahydro-9*H*-carbazol-9-yl}-essigsäure;
(3*S*)-{3-[Cyclopropylmethyl-(2-naphthalen-1-yl-acetyl)-amino]-6-fluor-1,2,3,4-tetrahydro-9*H*-carbazol-9-yl}-essigsäure;
(3*S*)-(3-{Benzyloxycarbonyl-[2-(4-trifluormethyl-phenoxy)-ethyl]-amino}-6-fluor-1,2,3,4-tetrahydro-9*H*-carbazol-9-yl)-essigsäure;
(3*S*)-(6-Fluor-3-{(3-phenyl-propionyl)-[2-(4-trifluormethyl-phenoxy)-ethyl]-amino}-1,2,3,4-tetrahydro-9*H*-carbazol-9-yl)-essigsäure;
(3*S*)-(6-Fluor-3-{[3-(2-methoxy-phenyl)-propionyl]-[2-(4-trifluormethyl-phenoxy)-ethyl]-amino}-1,2,3,4-tetrahydro-9*H*-carbazol-9-yl)-essigsäure;
(3*S*)-{6-Fluor-3-[(2-phenoxy-ethyl)-(3-phenyl-propionyl)-amino]-1,2,3,4-tetrahydro-9*H-*carbazol-9-yl}-essigsäure;
(3*S*)-{6-Fluor-3-[((S)-2-methyl-3-phenyl-propionyl)-(2-phenoxy-ethyl)-amino]-1,2,3,4-tetrahydro-9*H*-carbazol-9-yl}-essigsäure;
(3*S*)-{6-Fluor-3-[[3-(2-methoxy-phenyl)-propionyl]-(2-phenoxy-ethyl)-amino]-1,2,3,4-tetrahydro-9*H*-carbazol-9-yl}-essigsäure;
(3*S*)-{3-[3-Benzyl-1-(2-methoxy-ethyl)-ureido]-6-fluor-1,2,3,4-tetrahydro-9*H*-carbazol-9-yl}-essigsäure;
(3*S*)-{3-[Benzyloxycarbonyl-(2-methoxy-ethyl)-amino]-6-fluor-1,2,3,4-tetrahydro-9*H-*carbazol-9-yl}-essigsäure;
(3*S*)-{6-Fluor-3-[(2-methoxy-ethyl)-(3-phenyl-propionyl)-amino]-1,2,3,4-tetrahydro-9*H-*carbazol-9-yl}-essigsäure;
(3*S*)-{6-Fluor-3-[(2-methoxy-ethyl)-((S)-2-methyl-3-phenyl-propionyl)-amino]-1,2,3,4-tetrahydro-9*H*-carbazol-9-yl}-essigsäure;
(3*S*)-(6-Fluor-3-{(2-methoxy-ethyl)-[3-(2-methoxy-phenyl)-propionyl]-amino}-1,2,3,4-tetrahydro-9*H*-carbazol-9-yl)-essigsäure;
(3*S*)-{3-[[2-(3-Chlor-phenoxy)-acetyl]-(2-methoxy-ethyl)-amino]-6-fluor-1,2,3,4-tetrahydro-9*H*-carbazol-9-yl}-essigsäure;
(3*S*)-{3-[(3,3-Diphenyl-propionyl)-(2-methoxy-ethyl)-amino]-6-fluor-1,2,3,4-tetrahydro-9*H*-carbazol-9-yl}-essigsäure;
(3*S*)-{6-Fluor-3-[(2-methoxy-ethyl)-(2-naphthalen-1-yl-acetyl)-amino]-1,2,3,4-tetrahydro-9*H*-carbazol-9-yl}-essigsäure;
(3*S*)-(6-Fluor-3-{[(2*S*)-2-methyl-3-phenyl-propionyl]-phenethyl-amino}-1,2,3,4-tetrahydro-9*H*-carbazol-9-yl)-essigsäure;
(3*S*)-(6-Fluor-3-{[3-(2-methoxy-phenyl)-propionyl]-phenethyl-amino}-1,2,3,4-tetrahydro-9*H*-carbazol-9-yl)-essigsäure;
(3*S*)-{6-Fluor-3-[(2-naphthalen-1-yl-acetyl)-phenethyl-amino]-1,2,3,4-tetrahydro-9*H-*carbazol-9-yl}-essigsäure;
(3*S*)-{6-Fluor-3-[phenethyl-(3-phenyl-propionyl)-amino]-1,2,3,4-tetrahydro-9*H*-carbazol-9-yl}-essigsäure;
(3*S*)-[3-(3-Benzyl-1-naphthalen-1-ylmethyl-ureido)-6-fluor-1,2,3,4-tetrahydro-9*H*-carbazol-9-yl]-essigsäure;
(3*S*)-[3-(Benzyloxycarbonyl-naphthalen-1-ylmethyl-amino)-6-fluor-1,2,3,4-tetrahydro-9*H-*carbazol-9-yl]-essigsäure;
(3*S*)-{6-Fluor-3-[naphthalen-1-ylmethyl-(3-phenyl-propionyl)-amino]-1,2,3,4-tetrahydro-9*H*-carbazol-9-yl}-essigsäure;
(3*S*)-{6-Fluor-3-[((*S*)-2-methyl-3-phenyl-propionyl)-naphthaten-1-ylmethyl-amino]-1,2,3,4-tetrahydro-9*H*-carbazol-9-yl}-essigsäure;
(3*S*)-(6-Fluor-3-{[3-(2-methoxy-phenyl)-propionyl]-naphthalen-1-ylmethyl-amino}-1,2,3,4-tetrahydro-9*H*-carbazol-9-yl)-essigsäure;
(3*S*)-{3-[(3,3-Diphenyl-propionyl)-naphthalen-1-ylmethyl-amino]-6-fluor-1,2,3,4-tetrahydro-9*H*-carbazol-9-yl}-essigsäure;
(3*S*)-{3-[(*RS*)-2-Benzyl-3-(2-methylphenyl)-carbamoyl-propionylamino]-6-fluor-1,2,3,4-tetrahydro-9*H*-carbazol-9-yl}-essigsäure;
(3*S*)-{3-[(*RS*)-2-Benzyl-3-(3-methoxy-phenylcarbamoyl)-propionylamino]-6-fluor-1,2,3,4-tetrahydro-9*H*-carbazol-9-yl}-essigsäure;
(3*S*)-{3-[(*RS*)-2-Benzyl-3-(4-chlor-phenylcarbamoyl)-propionylamino]-6-fluor-1,2,3,4-tetrahydro-9*H*-carbazol-9-yl}-essigsäure;
(3*S*)-{3-[(*RS*)-2-Benzyl-3-(4-fluor-benzylcarbamoyl)-propionylamino]-6-fluor-1,2,3,4-tetrahydro-9*H*-carbazol-9-yl}-essigsäure;
[(3*S*)-3-((*RS*)-2-Benzyl-3-propylcarbamoyl-propionylamino)-6-fluor-1,2,3,4-tetrahydro-9*H-*carbazol-9-yl]-essigsäure;
(3*S*)-[6-Fluor-3-(3-thiophen-2-yl-propionylamino)-1,2,3,4-tetrahydro-9*H*-carbazol-9-yl]-essigsäure;
(3*S*)-{3-[3-(3-Chlor-isoxazol-5-yl)-propionylamino]-6-fluor-1,2,3,4-tetrahydro-9*H-*carbazol-9-yl}-essigsäure;
(3*S*)-[6-Fluor-3-(3-pyrimidin-2-yl-propionylamino)-1,2,3,4-tetrahydro-9*H*-carbazol-9-yl]-essigsäure;
(3*S*)-{6-Fluor-3-[3-phenyl-4-([1,3,4]thiadiazol-2-ylcarbamoyl)-butyrylamino]-1,2,3,4-tetrahydro-9*H*-carbazol-9-yl}-essigsäure;
(3*S*)-[3-(1,3-Dibenzyl-ureido)-6-fluor-1,2,3,4-tetrahydro-9*H*-carbazol-9-yl]-essigsäure;
(3*S*)-[3-(3-Benzyl-1-cyclohexylmethyl-ureido)-6-fluor-1,2,3,4-tetrahydro-9*H*-carbazol-9-yl]-essigsäure; und
(3*S*)-(3-{Cyclohexylmethyl-[3-(2-methoxy-phenyl)-propionyl]-amino}-6-fluor-1,2,3,4-tetrahydro-9*H*-carbazol-9-yl)-essigsäure;
oder ein Salz einer solchen Verbindung.

12. Pharmazeutische Zusammensetzung, die eine Verbindung nach einem der Ansprüche 1 bis 11 oder ein pharmazeutisch akzeptables Salz davon und einen pharmazeutisch akzeptablen Träger umfasst.

13. Verbindung nach einem der Ansprüche 1 bis 11 oder ein pharmazeutisch akzeptables Salz davon oder eine pharmazeutische Zusammensetzung nach Anspruch 11 zur Verwendung als Medikament.

14. Verwendung einer Verbindung nach einem der Ansprüche 1 bis 11 oder eines pharmazeutisch akzeptablen Salzes davon zur Herstellung eines Medikaments zur Verhütung und/oder Behandlung von chronischen und akuten Allergie-/Immunerkrankungen/-störungen, umfassend allergisches Asthma, Rhinitis, allergische Rhinitis, chronische obstruktive Lungenerkrankung, Dermatitis, entzündliche Darmerkrankung, Rheumatoidarthritis, allergische Nephritis, Konjunktivitis, atopische Dermatitis, Bronchialasthma, Lebensmittelallergie, systemische Mastzellenstörungen, anaphylaktischen Schock, Urtikaria, Ekzem, Juckreiz, Inflammation, Ischämie-Reperfusionsverletzung, zerebrovaskuläre Störungen, Pleuritis, Colitis ulcerosa, Eosinophilen-bezogene Krankheiten, umfassend Churg-Strauss-Syndrom und Sinusitis, und Basophilen-bezogene Krankheiten, umfassend basophile Leukämie und basophile Leukozytose.

## Revendications

1. Composé de Formule I : 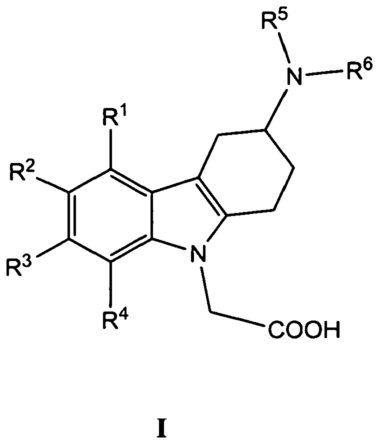 où
R¹, R², R³ et R⁴ représentent indépendamment un hydrogène, alkyle en C₁₋₅, alkoxy en C₁₋₅, alkényle, halogène, nitro, cyano, halo-alkoxy en C₁₋₆, halo-alkyle en C₁₋₆, alkyle en C₁₋₆-sulfonyle ou formyle ;
R⁵ représente un hydrogène, alkényle, alkyle en C₁₋₆, cycloalkyl-alkyle en C₁₋₄, alkoxy en C₁₋₃-alkyle en C₁₋₄, aryl-alkyle en C₁₋₄ ou aryloxy-alkyle en C₁₋₄;
où l'aryle est non substitué ou mono- ou disubstitué par un groupement indépendamment sélectionné parmi un alkylène en C₁₋₂-dioxy, alkoxy en C₁₋₄, alkyle en C₁₋₄, halogène, trifluorométhyle et trifluorométhoxy ; et
R⁶ représente un aryl-alkoxy en C₁₋₃-alkoxy en C₁₋₃-carbonyle ; aryl-alkoxy en C₁₋₃-carbonyle ; aryl-alkyle en C₁₋₃-aminocarbonyle ; aryl-alkyle en C₁₋₆-carbonyle ; aryl-alkoxy en C₁₋₃-alkyle en C₁₋₃-carbonyle ; arylcarbonyl-alkyle en C₁₋₄-carbonyle ; aryloxy-alkyle en C₁₋₃-carbonyle ; cycloalkyl-alkyle en C₁₋₃-carbonyle ; diaryl-alkyle en C₁₋₃-carbonyle ; hétéroaryl-alkyle en C₁₋₃-carbonyle ; aryl-cycloalkyle en C₃₋₆-carbonyle ; ou R⁷-alkyle en C₁₋₄-carbonyle, où le groupement alkyle en C₁₋₄ faisant pont peut de plus être monosubstitué par un aryle et où R⁷ représente un arylaminocarbonyle, hétéroarylaminocarbonyle, alkyle en C₁₋₆-aminocarbonyle ou aryl-alkyle en C₁₋₃-aminocarbonyle ;
où l'aryle est non substitué ou mono- ou disubstitué par un groupement indépendamment sélectionné parmi un alkylène en C₁₋₂-dioxy, alkoxy en C₁₋₆, alkyle en C₁₋₆, alkyle en C₁₋₆-sulfonyle, halogène, hydroxy, halo-alkyle en C₁₋₆, halo-alkoxy en C₁₋₆, alkyle en C₁₋₆-thio et alkoxy en C₁₋₄-carbonylamino ;
ou sel d'un tel composé.

2. Composé selon la revendication 1 où R¹, R³ et R⁴ représentent un hydrogène ;
ou sel d'un tel composé.

3. Composé selon la revendication 1 ou 2 où R² représente un hydrogène, trifluorométhyle ou halogène ;
ou sel d'un tel composé.

4. Composé selon l'une quelconque des revendications 1 à 3 où R⁵ représente un hydrogène ; alkyle en C₁₋₃ ; cyclopropylméthyle ; 2-méthoxyéthyle ; phényl-alkyle en C₂₋₃; ou phénoxyéthyle, où le groupement phényle est non substitué ou monosubstitué par un groupement sélectionné parmi un alkylène en C₁₋₂-dioxy, alkoxy en C₁₋₄, alkyle en C₁₋₄, halogène, trifluorométhyle et trifluorométhoxy ;
ou sel d'un tel composé.

5. Composé selon l'une quelconque des revendications 1 à 3 où
R⁵ représente un hydrogène, méthyle, éthyle ou *n*-propyle;
ou sel d'un tel composé.

6. Composé selon l'une quelconque des revendications 1 à 5 où R⁶ représente un aryl-alkoxy en C₁₋₃-carbonyle; aryl-alkyle en C₁₋₃-aminocarbonyle; aryl-alkyle en C₁₋₆-carbonyle ; aryl-alkoxy en C₁₋₃-alkyle en C₁₋₃-carbonyle; arylcarbonyl-alkyle en C₁₋₄-carbonyle ; aryloxy-alkyle en C₁₋₃-carbonyle; cycloalkyl-alkyle en C₁₋₃-carbonyle ; diaryl-alkyle en C₁₋₃-carbonyle ; aryl-cycloalkyle en C₃₋₆-carbonyle ; ou R⁷-alkyle en C₁₋₄-carbonyle, où le groupement alkyle en C₁₋₄ faisant pont peut de plus être monosubstitué par un aryle et où R⁷ représente un arylaminocarbonyle, hétéroarylaminocarbonyle, alkyle en C₁₋₆-aminocarbonyle ou aryl-alkyle en C₁₋₃-aminocarbonyle ;
où l'aryle est non substitué ou mono- ou disubstitué par un groupement indépendamment sélectionné parmi un alkylène en C₁₋₂-dioxy, alkoxy en C₁₋₆, alkyle en C₁₋₆, alkyle en C₁₋₆-sulfonyle, halogène, hydroxy, halo-alkyle en C₁₋₆, halo-alkoxy en C₁₋₆, alkyle en C₁₋₆-thio et alkoxy en C₁₋₄-carbonylamino ;
ou sel d'un tel composé.

7. Composé selon l'une quelconque des revendications 1 à 5 où R⁶ représente un aryl-alkoxy en C₁₋₂-carbonyle ; aryl-alkyle en C₁₋₂-aminocarbonyle ; aryl-alkyle en C₁₋₄-carbonyle ; aryloxy-alkyle en C₁₋₂-carbonyle ; ou diaryl-alkyle en C₂₋₃-carbonyle ; ou R⁷-alkyle en C₂₋₄-carbonyle, où le groupement alkyle en C₂₋₄ faisant pont peut de plus être monosubstitué par un aryle et où R⁷ représente un arylaminocarbonyle ou un alkyle en C₁₋₄-aminocarbonyle ;
où l'aryle est non substitué ou mono- ou disubstitué par un groupement indépendamment sélectionné parmi un alkylène en C₁₋₂-dioxy, alkoxy en C₁₋₆, alkyle en C₁₋₆, alkyle en C₁₋₆-sulfonyle, halogène, hydroxy, trifluorométhyle et trifluorométhoxy ;
ou sel d'un tel composé.

8. Composé selon l'une quelconque des revendications 1 à 5 où R⁶ représente un aryl-alkyle en C₂₋₄-carbonyle, où l'aryle est non substitué ou mono- ou disubstitué par un groupement indépendamment sélectionné parmi un alkoxy en C₁₋₄, alkyle en C₁₋₄, halogène et trifluorométhyle ;
ou sel d'un tel composé.

9. Composé selon l'une quelconque des revendications 1 à 7 où quand R⁶ représente un groupement qui contient un groupement carbonyle et un ou plusieurs fragments aryles, ledit groupement est tel qu'il contient un groupement faisant pont entre le groupement carbonyle et le(s)dit(s) fragment(s) aryle(s) dudit R⁶, où le fragment carbonyle et au moins un des fragments aryles sont directement rattachés à des atomes différents dudit groupement faisant pont ;
ou sel d'un tel composé.

10. Composé selon la revendication 1, sélectionné dans le groupe consistant en les suivants :
Acide (3*S*)-[3-(3,3-diphényl-propionylamino)-6-fluoro-1,2,3,4-tétrahydro-9*H-*carbazol-9-yl]-acétique ;
Acide (3*R*)-{3-[2-(3-chloro-phénoxy)-acétylamino]-6-fluoro-1,2,3,4-tétrahydro-9*H-*carbazol-9-yl}-acétique;
Acide (3*S*)-[6-fluoro-3-(3-phényl-propionylamino)-1,2,3,4-tétrahydro-9*H*-carbazol-9-yl]-acétique ;
Acide (3*R*)-{3-[2-(4-chloro-phénoxy)-acétylamino]-6-fluoro-1,2,3,4-tétrahydro-9*H-*carbazol-9-yl}-acétique ;
Acide (3*S*)-{3-[3-(2-chloro-phényl)-propionylamino]-6-fluoro-1,2,3,4-tétrahydro-9*H-*carbazol-9-yl}-acétique ;
Acide (3*R*)-[6-fluoro-3-(3-phényl-propionylamino)-1,2,3,4-tétrahydro-9*H*-carbazol-9-yl]-acétique ;
Acide (3*S*)-{3-[3-(4-chloro-phényl)-propionylamino]-6-fluoro-1,2,3,4-tétrahydro-9*H-*carbazol-9-yl}-acétique ;
Acide (3*R*)-(3-benzyloxycarbonylamino-6-fluoro-1,2,3,4-tétrahydro-9*H*-carbazol-9-yl)-acétique ;
Acide (3*S*)-(3-benzyloxycarbonylamino-6-fluoro-1,2,3,4-tétrahydro-9*H*-carbazol-9-yl)-acétique ;
Acide (3*S*)-{3-[2-(4-chloro-phénoxy)-acétylamino]-6-fluoro-1,2,3,4-tétrahydro-9*H-*carbazol-9-yl}-acétique ;
Acide (3*R*)-{3-[2-(2-chloro-phénoxy)-acétylamino]-6-fluoro-1,2,3,4-tétrahydro-9*H-*carbazol-9-yl}-acétique ;
Acide (3*S*)-{3-[3-(3-chloro-phényl)-propionylamino]-6-fluoro-1,2,3,4-tétrahydro-9*H-*carbazol-9-yl}-acétique ;
Acide (3*S*)-[6-fluoro-3-(4-oxo-4-phényl-butyrylamino)-1,2,3,4-tétrahydro-9*H*-carbazol-9-yl]-acétique ;
Acide (3*S*)-[6-fluoro-3-(2-indan-2-yl-acétylamino)-1,2,3,4-tétrahydro-9*H*-carbazol-9-yl]-acétique ;
Acide (3*S*)-(3-{[2-(4-chloro-phényl)-acétyl]-éthyl-amino}-6-fluoro-1,2,3,4-tétrahydro-9*H-*carbazol-9-yl)-acétique ;
Acide (3*R*)-[6-fluoro-3-(2-*p*-tolyloxy-acétylamino)-1,2,3,4-tétrahydro-9*H*-carbazol-9-yl]-acétique ;
Acide (3*S*)-{6-fluoro-3-[méthyl-(3-phényl-propionyl)-amino]-1,2,3,4-tétrahydro-9*H-*carbazol-9-yl}-acétique ;
Acide (3*S*)-[6-fluoro-3-(3-1*H*-indol-3-yl-propionylamino)-1,2,3,4-tétrahydro-9*H*-carbazol-9-yl]-acétique ;
Acide (3*S*)-[3-(3-benzo[1,3]dioxol-5-yl-propionylamino)-6-fluoro-1,2,3,4-tétrahydro-9*H-*carbazol-9-yl]-acétique ;
Acide (3*S*)-{6-fluoro-3-[éthyl-(3-phényl-propionyl)-amino]-1,2,3,4-tétrahydro-9*H*-carbazol-9-yl}-acétique ;
Acide (3*S*)-{3-[2-(4-chloro-phényl)-acétylamino]-6-fluoro-1,2,3,4-tétrahydro-9*H*-carbazol-9-yl}-acétique ;
Acide (3*S*)-[3-(2,3-diphényl-propionylainino)-6-fluoro-1,2,3,4-tétrahydro-9*H*-carbazol-9-yl]-acétique ;
Acide (3*R*)-[6-fluoro-3-(2-phénoxy-acétylamino)-1,2,3,4-tétrahydro-9*H*-carbazol-9-yl]-acétique ;
Acide (3*S*)-{3-[3-(3,4-difluoro-phényl)-propionylamino]-6-fluoro-1,2,3,4-tétrahydro-9*H-*carbazol-9-yl}-acétique ;
Acide (3*S*)-[3-(3-phényl-propionylamino)-1,2,3,4-tétrahydro-9*H*-carbazol-9-yl]-acétique;
Acide (3*R*)-[3-(2-benzyloxy-acétylamino)-1,2,3,4-tétrahydro-9*H*-carbazol-9-yl]-acétique;
Acide (3*S*)-{6-fluoro-3-[3-(2-méthoxy-phényl)-propionylamino]-1,2,3,4-tétrahydro-9*H-*carbazol-9-yl}-acétique ;
Acide (3*S*)-{6-fluoro-3-[propyl-(3-phényl-propionyl)-amino]-1,2,3,4-tétrahydro-9*H-*carbazol-9-yl}-acétique ;
Acide (3*S*)-[3-(2-benzyloxy-acétylamino)-1,2,3,4-tétrahydro-9*H*-carbazol-9-yl]-acétique ;
Acide (3*R*)-(3-benzyloxycarbonylamino-1,2,3,4-tétrahydro-9*H*-carbazol-9-yl)-acétique ;
Acide (3*R*)-{6-fluoro-3-[2-(4-méthoxy-phényl)-acétylamino]-1,2,3,4-tétrahydro-9*H-*carbazol-9-yl}-acétique ;
Acide (3*R*)-{3-[3-(4-chloro-phényl)-propionylamino]-6-fluoro-1,2,3,4-tétrahydro-9*H-*carbazol-9-yl}-acétique ;
Acide (3*S*)-{3-[4-(4-bromo-phényl)-4-oxo-butyrylamino]-6-fluoro-1,2,3,4-tétrahydro-9*H-*carbazol-9-yl}-acétique ;
Acide (3*S*)-(3-{[2-(4-chloro-phényl)-acétyl]-propyl-amino}-6-fluoro-1,2,3,4-tétrahydro-9*H-*carbazol-9-yl)-acétique ;
Acide (3*R*)-(3-phényl-acétylamino-1,2,3,4-tétrahydro-9*H*-carbazol-9-yl)-acétique ;
Acide (3*R*)-{3-[3-(2-chloro-phényl)-propionylamino]-6-fluoro-1,2,3,4-tétrahydro-9*H-*carbazol-9-yl}-acétique ;
Acide (3*S*)-{6-fluoro-3-[2-(4-trifluorométhyl-phénoxy)-acétylamino]-1,2,3,4-tétrahydro-9*H-*carbazol-9-yl}-acétique ;
Acide (3*R*)-(6-fluoro-3-phényl-acétylamino-1,2,3,4-tétrahydro-9*H*-carbazol-9-yl)-acétique ;
Acide (3*S*)-{6-fluoro-3-[3-(2-hydroxy-phényl)-propionylamino]-1,2,3,4-tétrahydro-9*H-*carbazol-9-yl}-acétique ;
Acide (3*S*)-[3-(3-1*H*-benzoimidazol-2-yl-propionylamino)-6-fluoro-1,2,3,4-tétrahydro-9*H-*carbazol-9-yl]-acétique ;
Acide (3*S*)-{6-fluoro-3-[3-(4-hydroxy-phényl)-propionylamino]-1,2,3,4-tétrahydro-9*H-*carbazol-9-yl}-acétique ;
Acide (3*S*)-[6-fluoro-3-(2*-p*-tolyloxy-acétylamino)-1,2,3,4-tétrahydro-9*H*-carbazol-9-yl]-acétique ;
Acide (3*R*)-[6-fluoro-3-(2-*p*-tolyl-acétylamino)-1,2,3,4-tétrahydro-9*H*-carbazol-9-yl]-acétique ;
Acide (3*R*)-{3-[3-(3-chloro-phényl)-propionylamino]-6-fluoro-1,2,3,4-tétrahydro-9*H-*carbazol-9-yl}-acétique ;
Acide (3*R*)-[3-(2-phénoxy-acétylamino)-1,2,3,4-tétrahydro-9*H*-carbazol-9-yl]-acétique;
Acide (3*S*)-[6-fluoro-3-(3-*p*-tolyl-propionylamino)-1,2,3,4-tétrahydro-9*H*-carbazol-9-yl]-acétique ;
Acide (3*S*)-(3-benzyloxycarbonylamino-1,2,3,4-tétrahydro-9*H*-carbazol-9-yl)-acétique ;
Acide (3*S*)-[6-fluoro-3-(2-*p*-tolyl-acétylamino)-1,2,3,4-tétrahydro-9*H*-carbazol-9-yl]-acétique ;
Acide (3*S*)-[3-(3-phenéthyl-uréido)-1,2,3,4-tétrahydro-9*H*-carbazol-9-yl]-acétique;
Acide (3*S*)-{6-fluoro-3-[3-(3-hydroxy-phényl)-propionylamino]-1,2,3,4-tétrahydro-9*H-*carbazol-9-yl}-acétique ;
Acide (3*R*)-[3-(2-benzyloxy-éthoxycarbonylamino)-1,2,3,4-tétrahydro-9*H*-carbazol-9-yl]-acétique ;
Acide (3*S*)-[6-fluoro-3-(3-naphtalén-2-yl-propionylamino)-1,2,3,4-tétrahydro-9*H*-carbazol-9-yl]-acétique ;
Acide (3*S*)-{6-fluoro-3-[4-(4-méthanesulfonyl-phényl)-4-oxo-butyrylamino]-1,2,3,4-tétrahydro-9*H*-carbazol-9-yl}-acétique ;
Acide (3*S*)-(3-{[2-(4-chloro-phényl)-acétyl]-méthyl-amino}-6-fluoro-1,2,3,4-tétrahydro-9*H-*carbazol-9-yl)-acétique ;
Acide (3*S*)-{6-fluoro-3-[3-(4-méthoxy-phényl)-propionylamino]-1,2,3,4-tétrahydro-9*H-*carbazol-9-yl}-acétique ;
Acide (3*R*)-[3-(3-phényl-propionylamino)-1,2,3,4-tétrahydro-9*H*-carbazol-9-yl]-acétique;
Acide (3*R*)-{3-[2-(4-chloro-phényl)-acétylamino]-6-fluoro-1,2,3,4-tétrahydro-9*H*-carbazol-9-yl}-acétique ;
Acide (3*R*)-[6-fluoro-3-(3-*p*-tolyl-propionylamino)-1,2,3,4-tétrahydro-9*H*-carbazol-9-yl]-acétique ;
Acide (3*R*)-{6-fluoro-3-[3-(4-méthoxy-phényl)-propionylamino]-1,2,3,4-tétrahydro-9*H-*carbazol-9-yl}-acétique ;
Acide (3*S*)-{6-fluoro-3-[3-(4-hydroxy-3-méthoxy-phényl)-propionylamino]-1,2,3,4-tétrahydro-9*H*-carbazol-9-yl}-acétique ;
Acide (3*S*)-{6-fluoro-3-[2-(3-trifluorométhyl-phényl)-acétylamino]-1,2,3,4-tétrahydro-9*H-*carbazol-9-yl}-acétique ;
Acide (3*S*)-{6-fluoro-3-[2-(4-méthoxy-phényl)-acétylamino]-1,2,3,4-tétrahydro-9**H-**carbazol-9-yl}-acétique ;
Acide (3*R*)-{3-[2-(3-chloro-phényl)-acétylamino]-6-fluoro-1,2,3,4-tétrahydro-9*H*-carbazol-9-yl}-acétique ;
Acide (3*S*)-{6-fluoro-3-[2-(4-trifluorométhyl-phényl)-acétylamino]-1,2,3,4-tétrahydro-9*H-*carbazol-9-yl}-acétique ;
Acide (3*R*)-{3-[2-(3,4-dichloro-phényl)-acétylamino]-6-fluoro-1,2,3,4-tétrahydro-9*H-*carbazol-9-yl}-acétique ;
Acide (3*S*)-{6-fluoro-3-[3-(3-méthoxy-phényl)-propionylamino]-1,2,3,4-tétrahydro-9*H-*carbazol-9-yl}-acétique ;
Acide (3*S*)-[6-fluoro-3-(2-phénoxy-acétylamino)-1,2,3,4-tétrahydro-9*H*-carbazol-9-yl]-acétique ;
Acide (3*R*)-[3-(3-phénéthyl-uréido)-1,2,3,4-tétrahydro-9*H*-carbazol-9-yl]-acétique ;
Acide (3*S*)-[3-(2-benzyloxy-éthoxycarbonylamino)-1,2,3,4-tétrahydro-9*H*-carbazol-9-yl]-acétique ;
Acide (3*S*)-[3-(2-phénoxy-acétylamino)-1,2,3,4-tétrahydro-9*H*-carbazol-9-yl]-acétique;
Acide (3*S*)-[3-(2-thiophén-2-yl-acétylamino)-1,2,3,4-tétrahydro-9*H*-carbazol-9-yl]-acétique ;
Acide (3*S*)-(3-phényl-acétylamino-1,2,3,4-tétrahydro-9*H*-carbazol-9-yl)-acétique ;
Acide (3*S*)-[3-(3-benzyl-uréido)-1,2,3,4-tétrahydro-9*H*-carbazol-9-yl]-acétique;
Acide (3*S*)-{3-[2-(4-*tert*-butyl-phényl)-acétylamino]-6-fluoro-1,2,3,4-tétrahydro-9*H-*carbazol-9-yl}-acétique ;
Acide (3*R*)-(3-benzyloxycarbonylamino-8-chloro-6-fluoro-1,2,3,4-tétrahydro-9*H*-carbazol-9-yl)-acétique ;
Acide (3*S*)-(3-benzyloxycarbonylamino-8-chloro-6-fluoro-1,2,3,4-tétrahydro-9*H*-carbazol-9-yl)-acétique ;
Acide (3*S*)-[6-fluoro-3-(3-pyridin-3-yl-propionylamino)-1,2,3,4-tétrahydro-9*H*-carbazol-9-yl]-acétique ;
Acide (3*R*)-[3-(3-benzyl-uréido)-1,2,3,4-tétrahydro-9H-carbazol-9-yl]-acétique;
Acide (3*R*)-{6-fluoro-3-[2-(4-trifluorométhyl-phényl)-acétylamino]-1,2,3,4-tétrahydro-9*H-*carbazol-9-yl}-acétique ;
Acide (3*S*)-[3-(3-cyclopentyl-propionylamino)-1,2,3,4-tétrahydro-9*H*-carbazol-9-yl]-acétique ;
Acide (3*R*)-[3-(2-thiophén-2-yl-acétylamino)-1,2,3,4-tétrahydro-9*H*-carbazol-9-yl]-acétique ;
Acide (3*R*)-(3-benzyloxycarbonylamino-8-chloro-5-fluoro-1,2,3,4-tétrahydro-9*H*-carbazol-9-yl)-acétique ;
Acide (3*S*)-(3-benzyloxycarbonylamino-8-chloro-5-fluoro-1,2,3,4-tétrahydro-9*H*-carbazol-9-yl)-acétique ; et
Acide (3*R*)-[3-(3-cyclopentyl-propionylamino)-1,2,3,4-tétrahydro-9*H*-carbazol-9-yl]-acétique ;
ou sel d'un tel composé.

11. Composé selon la revendication 1, sélectionné dans le groupe consistant en les suivants :
Acide (3*R*)-[3-(3-benzyl-uréido)-6-fluoro-1,2,3,4-tétrahydro-9*H*-carbazol-9-yl]-acétique ;
Acide (3*S*)-[3-(3-benzyl-uréido)-6-fluoro-1,2,3,4-tétrahydro-9*H*-carbazol-9-yl]-acétique ;
Acide (3*S*)-[6-fluoro-3-(3-phénéthyl-uréido)-1,2,3,4-tétrahydro-9*H*-carbazol-9-yl]-acétique;
Acide (3*R*)-[3-(3-benzyl-uréido)-8-chloro-6-fluoro-1,2,3,4-tétrahydro-9*H*-carbazol-9-yl]-acétique ;
Acide (3*S*)-[3-(3-benzyl-uréido)-8-chloro-6-fluoro-1,2,3,4-tétrahydro-9*H*-carbazol-9-yl]-acétique ;
Acide (3*R*)-[8-chloro-6-fluoro-3-(3-phénéthyl-uréido)-1,2,3,4-tétrahydro-9*H*-carbazol-9-yl]-acétique ;
Acide (3*S*)-[8-chloro-6-fluoro-3-(3-phénéthyl-uréido)-1,2,3,4-tétrahydro-9*H*-carbazol-9-yl]-acétique ;
Acide (3*R*)-(3-benzyloxycarbonylamino-6-trifluorométhyl-1,2,3,4-tétrahydro-9*H*-carbazol-9-yl)-acétique ;
Acide (3*S*)-(3-benzyloxycarbonylamino-6-trifluorométhyl-1,2,3,4-tétrahydro-9*H*-carbazol-9-yl)-acétique ;
Acide (3*R*)-(3-benzyloxycarbonylamino-8-bromo-6-fluoro-1,2,3,4-tétrahydro-9*H*-carbazol-9-yl)-acétique ;
Acide (3*S*)-(3-benzyloxycarbonylamino-8-bromo-6-fluoro-1,2,3,4-tétrahydro-9*H*-carbazol-9-yl)-acétique ;
Acide (3*R*)-(3-benzyloxycarbonylamino-6-fluoro-8-vinyl-1,2,3,4-tétrahydro-9*H*-carbazol-9-yl)-acétique ;
Acide (3*S*)-(3-benzyloxycarbonylamino-6-fluoro-8-vinyl-1,2,3,4-tétrahydro-9*H*-carbazol-9-yl)-acétique ;
Acide (3*R*)-(3-benzyloxycarbonylamino-6-fluoro-8-méthanesulfonyl-1,2,3,4-tétrahydro-9*H-*carbazol-9-yl)-acétique ;
Acide (3*S*)-(3-benzyloxycarbonylamino-6-fluoro-8-méthanesulfonyl-1,2,3,4-tétrahydro-9*H-*carbazol-9-yl)-acétique ;
Acide (3*S*)-(3-benzyloxycarbonylamino-6-fluoro-8-méthyl-1,2,3,4-tétrahydro-9*H*-carbazol-9-yl)-acétique ;
Acide (3*S*)-(3-benzyloxycarbonylamino-7-chloro-6-fluoro-1,2,3,4-tétrahydro-9*H*-carbazol-9-yl)-acétique ;
Acide (3*S*)-(8-allyl-3-benzyloxycarbonylamino-6-fluoro-1,2,3,4-tétrahydro-9*H*-carbazol-9-yl)-acétique ;
Acide (3*R*)-(3-benzyloxycarbonylamino-8-chloro-1,2,3,4-tétrahydro-9*H*-carbazol-9-yl)-acétique ;
Acide (3*S*)-{3-[3-(2,4-diméthoxy-phényl)-propionylamino]-6-fluoro-1,2,3,4-tétrahydro-9*H-*carbazol-9-yl}-acétique ;
Acide (3*S*)-[6-fluoro-3-(3-naphtalén-1-yl-propionylamino)-1,2,3,4-tétrahydro-9*H*-carbazol-9-yl]-acétique ;
Acide (3*R*)-{6-fluoro-3-[2-(2-méthoxy-phénoxy)-acétylamino]-1,2,3,4-tétrahydro-9*H-*carbazol-9-yl}-acétique ;
Acide (3*S*)-{6-fluoro-3-[2-(2-méthoxy-phénoxy)-acétylamino]-1,2,3,4-tétrahydro-9*H-*carbazol-9-yl}-acétique ;
Acide (3*R*)-{6-fluoro-3-[3-(2-méthylphényl)-propionylamino]-1,2,3,4-tétrahydro-9*H-*carbazol-9-yl}-acétique ;
Acide (3*S*)-{6-fluoro-3-[3-(2-méthylphényl)-propionylamino]-1,2,3,4-tétrahydro-9*H-*carbazol-9-yl}-acétique ;
Acide (3*R*)-{6-fluoro-3-[3-(3-méthylphényl)-propionylamino]-1,2,3,4-tétrahydro-9*H-*carbazol-9-yl}-acétique ;
Acide (3*S*)-{6-fluoro-3-[3-(3-méthylphényl)-propionylamino]-1,2,3,4-tétrahydro-9*H-*carbazol-9-yl}-acétique ;
Acide (3*R*)-{6-fluoro-3-[3-(3-méthoxy-phényl)-propionylamino]-1,2,3,4-tétrahydro-9*H-*carbazol-9-yl}-acétique ;
Acide (3*S*)-{6-fluoro-3-[3-(3-méthoxy-phényl)-propionylamino]-1,2,3,4-tétrahydro-9*H-*carbazol-9-yl}-acétique ;
Acide (3*R*)-{6-fluoro-3-[2-(3-méthoxy-phénoxy)-acétylamino]-1,2,3,4-tétrahydro-9*H-*carbazol-9-yl}-acétique ;
Acide (3*S*)-{6-fluoro-3-[2-(3-méthoxy-phénoxy)-acétylamino]-1,2,3,4-tétrahydro-9*H-*carbazol-9-yl}-acétique ;
Acide (3*R*)-{6-fluoro-3-[2-(2-méthylphénoxy)-acétylamino]-1,2,3,4-tétrahydro-9*H-*carbazol-9-yl}-acétique ;
Acide (3*S*)-{6-fluoro-3-[2-(2-méthylphénoxy)-acétylamino]-1,2,3,4-tétrahydro-9*H-*carbazol-9-yl}-acétique ;
Acide (3*S*)-{3-[3-(2,5-diméthoxy-phényl)-propionylamino]-6-fluoro-1,2,3,4-tétrahydro-9*H-*carbazol-9-yl}-acétique ;
Acide (3*S*)-{6-fluoro-3-[3-(4-trifluorométhyl-phényl)-propionylamino]-1,2,3,4-tétrahydro-9*H*-carbazol-9-yl}-acétique ;
Acide (3*S*)-{3-[3-(2,6-dichloro-phényl)-propionylamino]-6-fluoro-1,2,3,4-tétrahydro-9*H-*carbazol-9-yl}-acétique ;
Acide (3*S*)-{3-[3-(2,5-bis-trifluorométhyl-phényl)-propionylamino]-6-fluoro-1,2,3,4-tétrahydro-9*H*-carbazol-9-yl}-acétique ;
Acide (3*S*)-{6-fluoro-3-[3-(4-méthylsulfanyl-phényl)-propionylamino]-1,2,3,4-tétrahydro-9*H*-carbazol-9-yl}-acétique ;
Acide (3*S*)-{6-fluoro-3-[3-(4-iodo-phényl)-propionylamino]-1,2,3,4-tétrahydro-9*H-*carbazol-9-yl}-acétique ;
Acide (3*S*)-{6-fluoro-3-[3-(4-isopropyl-phényl)-propionylamino]-1,2,3,4-tétrahydro-9*H-*carbazol-9-yl}-acétique ;
Acide (3*S*)-{6-fluoro-3-[3-(3-trifluorométhyl-phényl)-propionylamino]-1,2,3,4-tétrahydro-9*H*-carbazol-9-yl}-acétique ;
Acide (3*S*)-{3-[3-(2,4-dichloro-phényl)-propionylamino]-6-fluoro-1,2,3,4-tétrahydro-9*H-*carbazol-9-yl}-acétique ;
Acide (3*S*)-{6-fluoro-3-[3-(4-fluoro-phényl)-propionylamino]-1,2,3,4-tétrahydro-9*H-*carbazol-9-yl}-acétique ;
Acide (3*S*)-{3-[3-(3,5-bis-trifluorométhyl-phényl)-propionylamino]-6-fluoro-1,2,3,4-tétrahydro-9*H*-carbazol-9-yl}-acétique ;
Acide (3*S*)-{3-[3-(4-éthyl-phényl)-propionylamino]-6-fluoro-1,2,3,4-tétrahydro-9*H-*carbazol-9-yl}-acétique ;
Acide (3*S*)-{6-fluoro-3-[3-(3-iodo-phényl)-propionylamino]-1,2,3,4-tétrahydro-9*H-*carbazol-9-yl}-acétique ;
Acide (3*S*)-{6-fluoro-3-[3-(4-méthanesulfonyl-phényl)-propionylamino]-1,2,3,4-tétrahydro-9*H*-carbazol-9-yl}-acétique ;
Acide (3*S*)-{3-[3-(2,3-diméthoxy-phényl)-propionylamino]-6-fluoro-1,2,3,4-tétrahydro-9*H-*carbazol-9-yl}-acétique ;
Acide (3*S*)-{3-[3-(2-bromo-phényl)-propionylamino]-6-fluoro-1,2,3,4-tétrahydro-9*H-*carbazol-9-yl}-acétique ;
Acide (3*S*)-{6-fluoro-3-[3-(3-trifluorométhoxy-phényl)-propionylamino]-1,2,3,4-tétrahydro-9*H*-carbazol-9-yl}-acétique ;
Acide (3*S*)-{3-[3-(2,4-diméthyl-phényl)-propionylamino]-6-fluoro-1,2,3,4-tétrahydro-9*H-*carbazol-9-yl}-acétique ;
Acide (3*S*)-{3-[3-(3-bromo-phényl)-propionylamino]-6-fluoro-1,2,3,4-tétrahydro-9*H-*carbazol-9-yl}-acétique ;
Acide (3*S*)-{3-[3-(3-tert-butoxycarbonylamino-phényl)-propionylamino]-6-fluoro-1,2,3,4-tétrahydro-9*H*-carbazol-9-yl}-acétique ;
Acide (3*S*)-{6-fluoro-3-[(*S*)-3-(4-fluoro-phényl)-2-phényl-propionylamino]-1,2,3,4-tétrahydro-9*H*-carbazol-9-yl}-acétique ;
Acide (3*S*)-{6-fluoro-3-[(*S*)-3-(4-méthoxy-phényl)-2-phényl-propionylamino]-1,2,3,4-tétrahydro-9*H*-carbazol-9-yl}-acétique ;
Acide (3*S*)-{6-fluoro-3-[3-(2-fluoro-phényl)-propionylamino]-1,2,3,4-tétrahydro-9*H-*carbazol-9-yl}-acétique ;
Acide (3*S*)-(6-fluoro-3-{(2*RS*)-2-[(4-fluoro-phénylcarbamoyl)-méthyl]-3-phényl-propionylamino}-1,2,3,4-tétrahydro-9*H*-carbazol-9-yl)-acétique ;
Acide (3*S*)-{3-[(2*RS*)-2-benzyl-3,3-diméthyl-butyrylamino]-6-fluoro-1,2,3,4-tétrahydro-9*H-*carbazol-9-yl}-acétique ;
Acide (3*S*)-{6-fluoro-3-[3-(3-fluoro-phényl)-propionylamino]-1,2,3,4-tétrahydro-9*H-*carbazol-9-yl}-acétique ;
Acide (3*S*)-{6-fluoro-3-[(2*R*)-2-méthyl-3-phényl-propionylamino]-1,2,3,4-tétrahydro-9*H-*carbazol-9-yl}-acétique ;
Acide (3*S*)-[3-(2,2-diméthyl-3-phényl-propionylamino)-6-fluoro-1,2,3,4-tétrahydro-9*H-*carbazol-9-yl]-acétique ;
Acide (3*S*)-[6-fluoro-3-(3-méthyl-3-phényl-butyrylamino)-1,2,3,4-tétrahydro-9*H*-carbazol-9-yl]-acétique ;
Acide (3*S*)-{6-fluoro-3-[(3*S*)-3-phényl-butyrylamino]-1,2,3,4-tétrahydro-9*H*-carbazol-9-yl}-acétique ;
Acide (3*S*)-[3-(2-benzyloxy-acétylamino)-6-fluoro-1,2,3,4-tétrahydro-9*H*-carbazol-9-yl]-acétique ;
Acide (3*S*)-[6-fluoro-3-(4-phényl-butyrylamino)-1,2,3,4-tétrahydro-9*H*-carbazol-9-yl]-acétique ;
Acide (3*R*)-{8-chloro-6-fluoro-3-[3-(2-méthylphényl)-propionylamino]-1,2,3,4-tétrahydro-9*H*-carbazol-9-yl}-acétique ;
Acide (3*S*)-{8-chloro-6-fluoro-3-[3-(2-méthylphényl)-propionylamino]-1,2,3,4-tétrahydro-9*H*-carbazol-9-yl}-acétique ;
Acide (3*R*)-{8-chloro-6-fluoro-3-[2-(2-méthoxy-phénoxy)-acétylamino]-1,2,3,4-tétrahydro-9*H*-carbazol-9-yl}-acétique ;
Acide (3*S*)-{8-chloro-6-fluoro-3-[2-(2-méthoxy-phénoxy)-acétylamino]-1,2,3,4-tétrahydro-9*H*-carbazol-9-yl}-acétique ;
Acide (3*R*)-{8-chloro-6-fluoro-3-[3-(3-hydroxy-phényl)-propionylamino]-1,2,3,4-tétrahydro-9*H*-carbazol-9-yl}-acétique ;
Acide (3*S*)-{8-chloro-6-fluoro-3-[3-(3-hydroxy-phényl)-propionylamino]-1,2,3,4-tétrahydro-9*H*-carbazol-9-yl}-acétique ;
Acide (3*R*)-{8-chloro-6-fluoro-3-[3-(3-méthoxy-phényl)-propionylamino]-1,2,3,4-tétrahydro-9*H*-carbazol-9-yl}-acétique ;
Acide (3*S*)-{8-chloro-6-fluoro-3-[3-(3-méthoxy-phényl)-propionylamino]-1,2,3,4-tétrahydro-9*H*-carbazol-9-yl}-acétique ;
Acide (3*R*)-{8-chloro-6-fluoro-3-[3-(3-méthylphényl)-propionylamino]-1,2,3,4-tétrahydro-9*H*-carbazol-9-yl}-acétique ;
Acide (3*S*)-{8-chloro-6-fluoro-3-[3-(3-méthylphényl)-propionylamino]-1,2,3,4-tétrahydro-9*H*-carbazol-9-yl}-acétique ;
Acide (3*R*)-{8-chloro-6-fluoro-3-[3-(2-hydroxy-phényl)-propionylamino]-1,2,3,4-tétrahydro-9*H*-carbazol-9-yl}-acétique ;
Acide (3*S*)-{8-chloro-6-fluoro-3-[3-(2-hydroxy-phényl)-propionylamino]-1,2,3,4-tétrahydro-9*H*-carbazol-9-yl}-acétique ;
Acide (3*R*)-[8-chloro-6-fluoro-3-(3-phényl-propionylamino)-1,2,3,4-tétrahydro-9*H-*carbazol-9-yl]-acétique ;
Acide (3*S*)-[8-chloro-6-fluoro-3-(3-phényl-propionylamino)-1,2,3,4-tétrahydro-9*H-*carbazol-9-yl]-acétique ;
Acide (3*R*)-{8-chloro-6-fluoro-3-[3-(2-méthoxy-phényl)-propionylamino]-1,2,3,4-tétrahydro-9*H*-carbazol-9-yl}-acétique ;
Acide (3*S*)-{8-chloro-6-fluoro-3-[3-(2-méthoxy-phényl)-propionylamino]-1,2,3,4-tétrahydro-9*H*-carbazol-9-yl}-acétique ;
Acide (3*R*)-{8-chloro-3-[3-(3-chloro-phényl)-propionylamino]-6-fluoro-1,2,3,4-tétrahydro-9*H*-carbazol-9-yl}-acétique ;
Acide (3*S*)-{8-chloro-3-[3-(3-chloro-phényl)-propionylamino]-6-fluoro-1,2,3,4-tétrahydro-9*H*-carbazol-9-yl}-acétique ;
Acide (3*R*)-[8-chloro-6-fluoro-3-(3-*1H*-indol-3-yl-propionylamino)-1,2,3,4-tétrahydro-9*H-*carbazol-9-yl]-acétique ;
Acide (3*S*)-[8-chloro-6-fluoro-3-(3-*1H*-indol-3-yl-propionylamino)-1,2,3,4-tétrahydro-9*H-*carbazol-9-yl]-acétique ;
Acide (3*R*)-{8-chloro-3-[2-(2-chloro-phénoxy)-acétylamino]-6-fluoro-1,2,3,4-tétrahydro-9*H*-carbazol-9-yl}-acétique ;
Acide (3*S*)-{8-chloro-3-[2-(2-chloro-phénoxy)-acétylamino]-6-fluoro-1,2,3,4-tétrahydro-9*H*-carbazol-9-yl}-acétique ;
Acide (3*R*)-{8-chloro-6-fluoro-3-[2-(2-méthylphényl)-oxy-acétylamino]-1,2,3,4-tétrahydro-9*H*-carbazol-9-yl}-acétique ;
Acide (3*S*)-{8-chloro-6-fluoro-3-[2-(2-méthylphényl)-oxy-acétylamino]-1,2,3,4-tétrahydro-9*H*-carbazol-9-yl}-acétique ;
Acide (3*R*)-[3-(3-benzo[1,3]dioxol-5-yl-propionylamino)-8-chloro-6-fluoro-1,2,3,4-tétrahydro-9*H*-carbazol-9-yl]-acétique ;
Acide (3*S*)-[3-(3-benzo[1,3]dioxol-5-yl-propionylamino)-8-chloro-6-fluoro-1,2,3,4-tétrahydro-9*H*-carbazol-9-yl]-acétique ;
Acide (3*R*)-{8-chloro-6-fluoro-3-[2-(3-méthoxy-phénoxy)-acétylamino]-1,2,3,4-tétrahydro-9*H*-carbazol-9-yl}-acétique ;
Acide (3*S*)-{8-chloro-6-fluoro-3-[2-(3-méthoxy-phénoxy)-acétylamino]-1,2,3,4-tétrahydro-9*H*-carbazol-9-yl}-acétique ;
Acide (3*R*)-{8-chloro-3-[2-(3-chloro-phénoxy)-acétylamino]-6-fluoro-1,2,3,4-tétrahydro-9*H*-carbazol-9-yl}-acétique;
Acide (3*S*)-{8-chloro-3-[2-(3-chloro-phénoxy)-acétylamino]-6-fluoro-1,2,3,4-tétrahydro-9*H*-carbazol-9-yl}-acétique ;
Acide (3*R*)-{8-chloro-3-[3-(2-chloro-phényl)-propionylamino]-6-fluoro-1,2,3,4-tétrahydro-9*H*-carbazol-9-yl}-acétique ;
Acide (3*S*)-{8-chloro-3-[3-(2-chloro-phényl)-propionylamino]-6-fluoro-1,2,3,4-tétrahydro-9*H*-carbazol-9-yl}-acétique ;
Acide (3*R*)-[8-chloro-6-fluoro-3-(2-indan-2-yl-acétylamino)-1,2,3,4-tétrahydro-9*H-*carbazol-9-yl]-acétique ;
Acide (3*S*)-[8-chloro-6-fluoro-3-(2-indan-2-yl-acétylamino)-1,2,3,4-tétrahydro-9*H-*carbazol-9-yl]-acétique ;
Acide (3*S*)-[6-fluoro-3-(1-méthyl-3-phénéthyl-uréido)-1,2,3,4-tétrahydro-9*H*-carbazol-9-yl]-acétique ;
Acide (3*S*)-{3-[3-(2-chloro-benzyl)-1-méthyl-uréido]-6-fluoro-1,2,3,4-tétrahydro-9*H-*carbazol-9-yl}-acétique ;
Acide (3*S*)-[3-(3-benzyl-1-méthyl-uréido)-6-fluoro-1,2,3,4-tétrahydro-9*H*-carbazol-9-yl]-acétique ;
Acide (3*S*)-[3-(benzyloxycarbonyl-méthyl-amino)-6-fluoro-1,2,3,4-tétrahydro-9*H*-carbazol-9-yl]-acétique ;
Acide {(3*S*)-3-[(2-chloro-benzyloxycarbonyl)-méthyl-amino]-6-fluoro-1,2,3,4-tétrahydro-9*H*-carbazol-9-yl}-acétique;
Acide (3*S*)-(6-fluoro-3-{[2-(4-méthoxy-phényl)-acétyl]-méthyl-amino}-1,2,3,4-tétrahydro-9*H*-carbazol-9-yl)-acétique ;
Acide (3*S*)-(6-fluoro-3-{méthyl-[2-(4-méthylphényl)-acétyl]-amino}-1,2,3,4-tétrahydro-9*H-*carbazol-9-yl)-acétique ;
Acide (3*S*)-(6-fluoro-3-{[2-(2-méthoxy-phényl)-acétyl]-méthyl-amino}-1,2,3,4-tétrahydro-9*H*-carbazol-9-yl)-acétique ;
Acide (3*S*)-{6-fluoro-3-[(2-indan-2-yl-acétyl)-méthyl-amino]-1,2,3,4-tétrahydro-9*H-*carbazol-9-yl}-acétique ;
Acide (3*S*)-(3-{[2-(3-chloro-phényl)-acétyl]-méthyl-amino}-6-fluoro-1,2,3,4-tétrahydro-9*H-*carbazol-9-yl)-acétique ;
Acide (3*S*)-(6-fluoro-3-{méthyl-[2-(3-méthylphényl)-acétyl]-amino}-1,2,3,4-tétrahydro-9*H-*carbazol-9-yl)-acétique ;
Acide (3*S*)-(6-fluoro-3-{[2-(3-méthoxy-phényl)-acétyl]-méthyl-amino}-1,2,3,4-tétrahydro-9*H*-carbazol-9-yl)-acétique ;
Acide (3*S*)-(3-{[2-(2-chloro-phénoxy)-acétyl]-méthyl-amino}-6-fluoro-1,2,3,4-tétrahydro-9*H*-carbazol-9-yl)-acétique ;
Acide (3*S*)-(3-{[2-(4-chloro-phénoxy)-acétyl]-méthyl-amino}-6-fluoro-1,2,3,4-tétrahydro-9*H*-carbazol-9-yl)-acétique ;
Acide (3*S*)-(6-fluoro-3-{[3-(3-méthoxy-phényl)-propionyl]-méthyl-amino}-1,2,3,4-tétrahydro-9*H*-carbazol-9-yl)-acétique ;
Acide (3*S*)-(6-fluoro-3-{méthyl-[2-(2-méthylphényl)-acétyl]-amino}-1,2,3,4-tétrahydro-9*H-*carbazol-9-yl)-acétique ;
Acide (3*S*)-{3-[(3,3-diphényl-propionyl)-méthyl-amino]-6-fluoro-1,2,3,4-tétrahydro-9*H-*carbazol-9-yl}-acétique ;
Acide (3*S*)-(6-fluoro-3-{[3-(2-méthoxy-phényl)-propionyl]-méthyl-amino}-1,2,3,4-tétrahydro-9*H*-carbazol-9-yl)-acétique ;
Acide (3*S*)-{6-fluoro-3-[(3-*1H*-indol-3-yl-propionyl)-méthyl-amino]-1,2,3,4-tétrahydro-9*H-*carbazol-9-yl}-acétique ;
Acide (3*S*)-{3-[(2-benzyloxy-acétyl)-méthyl-amino]-6-fluoro-1,2,3,4-tétrahydro-9*H-*carbazol-9-yl}-acétique ;
Acide (3*S*)-{3-[(2,3-diphényl-propionyl)-méthyl-amino]-6-fluoro-1,2,3,4-tétrahydro-9*H-*carbazol-9-yl}-acétique ;
Acide (3*S*)-{6-fluoro-3-[[3-(2-méthoxy-phényl)-propionyl]-(3-phényl-propyl)-amino]-1,2,3,4-tétrahydro-9*H*-carbazol-9-yl}-acétique ;
Acide (3*S*)-{3-[3-benzyl-(1-cyclopropylméthyl)-uréido]-6-fluoro-1,2,3,4-tétrahydro-9*H-*carbazol-9-yl}-acétique ;
Acide (3*S*)-[3-(benzyloxycarbonyl-cyclopropylméthyl-amino)-6-fluoro-1,2,3,4-tétrahydro-9*H*-carbazol-9-yl]-acétique ;
Acide (3*S*)-{3-[cyclopropylméthyl-(3-phényl-propionyl)-amino]-6-fluoro-1,2,3,4-tétrahydro-9*H*-carbazol-9-yl}-acétique ;
Acide (3*S*)-{3-[cyclopropylméthyl-((*S*)-2-méthyl-3-phényl-propionyl)-amino]-6-fluoro-1,2,3,4-tétrahydro-9*H*-carbazol-9-yl}-acétique ;
Acide (3*S*)-(3-{cyclopropylméthyl-[3-(2-méthoxy-phényl)-propionyl]-amino}-6-fluoro-1,2,3,4-tétrahydro-9*H*-carbazol-9-yl)-acétique ;
Acide (3*S*)-(3-{[2-(3-chloro-phénoxy)-acétyl]-cyclopropylméthyl-amino}-6-fluoro-1,2,3,4-tétrahydro-9*H*-carbazol-9-yl)-acétique ;
Acide (3*S*)-{3-[cyclopropylméthyl-(3,3-diphényl-propionyl)-amino]-6-fluoro-1,2,3,4-tétrahydro-9*H*-carbazol-9-yl}-acétique ;
Acide (3*S*)-{3-[cyclopropylméthyl-(2-naphtalén-1-yl-acétyl)-amino]-6-fluoro-1,2,3,4-tétrahydro-9*H*-carbazol-9-yl}-acétique ;
Acide (3*S*)-(3-{benzyloxycarbonyl-[2-(4-trifluorométhyl-phénoxy)-éthyl]-amino}-6-fluoro-1,2,3,4-tétrahydro-9*H*-carbazol-9-yl)-acétique ;
Acide (3*S*)-(6-fluoro-3-{(3-phényl-propionyl)-[2-(4-trifluorométhyl-phénoxy)-éthyl]-amino}-1,2,3,4-tétrahydro-9*H*-carbazol-9-yl)-acétique ;
Acide (3*S*)-(6-fluoro-3-{[3-(2-méthoxy-phényl)-propionyl]-[2-(4-trifluorométhyl-phénoxy)-éthyl]-amino}-1,2,3,4-tétrahydro-9*H*-carbazol-9-yl)-acétique ;
Acide (3*S*)-{6-fluoro-3-[(2-phénoxy-éthyl)-(3-phényl-propionyl)-amino]-1,2,3,4-tétrahydro-9*H*-carbazol-9-yl}-acétique ;
Acide (3*S*)-{6-fluoro-3-[((*S*)-2-méthyl-3-phényl-propionyl)-(2-phénoxy-éthyl)-amino]-1,2,3,4-tétrahydro-9*H*-carbazol-9-yl}-acétique ;
Acide (3*S*)-{6-fluoro-3-[[3-(2-méthoxy-phényl)-propionyl]-(2-phénoxy-éthyl)-amino]-1,2,3,4-tétrahydro-9*H*-carbazol-9-yl}-acétique ;
Acide (3*S*)-{3-[3-benzyl-1-(2-méthoxy-éthyl)-uréido]-6-fluoro-1,2,3,4-tétrahydro-9*H-*carbazol-9-yl}-acétique ;
Acide (3*S*)-{3-[benzyloxycarbonyl-(2-méthoxy-éthyl)-amino]-6-fluoro-1,2,3,4-tétrahydro-9*H*-carbazol-9-yl}-acétique;
Acide (3*S*)-{6-fluoro-3-[(2-méthoxy-éthyl)-(3-phényl-propionyl)-amino]-1,2,3,4-tétrahydro-9*H*-carbazol-9-yl}-acétique ;
Acide (3*S*)-{6-fluoro-3-[(2-méthoxy-éthyl)-((*S*)-2-méthyl-3-phényl-propionyl)-amino]-1,2,3,4-tétrahydro-9*H*-carbazol-9-yl}-acétique ;
Acide (3*S*)-(6-fluoro-3-{(2-méthoxy-éthyl)-[3-(2-méthoxy-phényl)-propionyl]-amino}-1,2,3,4-tétrahydro-9*H*-carbazol-9-yl)-acétique ;
Acide (3*S*)-{3-[[2-(3-chloro-phénoxy)-acétyl]-(2-méthoxy-éthyl)-amino]-6-fluoro-1,2,3,4-tétrahydro-9*H*-carbazol-9-yl}-acétique ;
Acide (3*S*)-{3-[(3,3-diphényl-propionyl)-(2-méthoxy-éthyl)-amino]-6-fluoro-1,2,3,4-tétrahydro-9*H*-carbazol-9-yl}-acétique ;
Acide (3*S*)-{6-fluoro-3-[(2-méthoxy-éthyl)-(2-naphtalén-1-yl-acétyl)-amino]-1,2,3,4-tétrahydro-9*H*-carbazol-9-yl}-acétique ;
Acide (3*S*)-(6-fluoro-3-{[(2*S*)-2-méthyl-3-phényl-propionyl]-phénéthyl-amino}-1,2,3,4-tétrahydro-9*H*-carbazol-9-yl)-acétique ;
Acide (3*S*)-(6-fluoro-3-{[3-(2-méthoxy-phényl)-propionyl]-phénéthyl-amino}-1,2,3,4-tétrahydro-9*H*-carbazol-9-yl)-acétique ;
Acide (3*S*)-{6-fluoro-3-[(2-naphtalén-1-yl-acétyl)-phénéthyl-amino]-1,2,3,4-tétrahydro-9*H-*carbazol-9-yl}-acétique ;
Acide (3*S*)-{6-fluoro-3-[phénéthyl-(3-phényl-propionyl)-amino]-1,2,3,4-tétrahydro-9*H-*carbazol-9-yl}-acétique ;
Acide (3*S*)-[3-(3-benzyl-1-naphtalén-1-ylméthyl-uréido)-6-fluoro-1,2,3,4-tétrahydro-9*H-*carbazol-9-yl]-acétique ;
Acide (3*S*)-[3-(benzyloxycarbonyl-naphtalén-1-ylméthyl-amino)-6-fluoro-1,2,3,4-tétrahydro-9*H*-carbazol-9-yl]-acétique ;
Acide (3*S*)-{6-fluoro-3-[naphtalén-1-ylméthyl-(3-phényl-propionyl)-amino]-1,2,3,4-tétrahydro-9*H*-carbazol-9-yl}-acétique ;
Acide (3*S*)-{6-fluoro-3-[((*S*)-2-méthyl-3-phényl-propionyl)-naphtalén-1-ylméthyl-amino]-1,2,3,4-tétrahydro-9*H*-carbazol-9-yl}-acétique ;
Acide (3*S*)-(6-fluoro-3-{[3-(2-méthoxy-phényl)-propionyl]-naphtalén-1-ylméthyl-amino}-1,2,3,4-tétrahydro-9*H*-carbazol-9-yl)-acétique ;
Acide (3*S*)-{3-[(3,3-diphényl-propionyl)-naphtalén-1-ylméthyl-amino]-6-fluoro-1,2,3,4-tétrahydro-9*H*-carbazol-9-yl}-acétique ;
Acide (3*S*)-{3-[(*RS*)-2-benzyl-3-(2-méthylphényl)-carbamoyl-propionylamino]-6-fluoro-1,2,3,4-tétrahydro-9*H-*carbazol-9-yl}-acétique ;
Acide (3*S*)-{3-[(*RS*)-2-benzyl-3-(3-méthoxy-phénylcarbamoyl)-propionylamino]-6-fluoro-1,2,3,4-tétrahydro-9*H*-carbazol-9-yl}-acétique ;
Acide (3*S*)-{3-[(*RS*)-2-benzyl-3-(4-chloro-phénylcarbamoyl)-propionylamino]-6-fluoro-1,2,3,4-tétrahydro-9*H*-carbazol-9-yl}-acétique ;
Acide (3*S*)-{3-[(*RS*)-2-benzyl-3-(4-fluoro-benzylcarbamoyl)-propionylamino]-6-fluoro-1,2,3,4-tétrahydro-9*H*-carbazol-9-yl}-acétique ;
Acide [(3*S*)-3-((*RS*)-2-benzyl-3-propylcarbamoyl-propionylamino)-6-fluoro-1,2,3,4-tétrahydro-9*H*-carbazol-9-yl]-acétique ;
Acide (3*S*)-[6-fluoro-3-(3-thiophén-2-yl-propionylamino)-1,2,3,4-tétrahydro-9*H*-carbazol-9-yl]-acétique ;
Acide (3*S*)-{3-[3-(3-chloro-isoxazol-5-yl)-propionylamino]-6-fluoro-1,2,3,4-tétrahydro-9*H-*carbazol-9-yl}-acétique ;
Acide (3*S*)-[6-fluoro-3-(3-pyrimidin-2-yl-propionylamino)-1,2,3,4-tétrahydro-9*H*-carbazol-9-yl]-acétique ;
Acide (3*S*)-{6-fluoro-3-[3-phényl-4-([1,3,4]thiadiazol-2-ylcarbamoyl)-butyrylamino]-1,2,3,4-tétrahydro-9*H*-carbazol-9-yl}-acétique ;
Acide (3*S*)-[3-(1,3-dibenzyl-uréido)-6-fluoro-1,2,3,4-tétrahydro-9*H*-carbazol-9-yl]-acétique ;
Acide (3*S*)-[3-(3-benzyl-1-cyclohexylméthyl-uréido)-6-fluoro-1,2,3,4-tétrahydro-9*H-*carbazol-9-yl]-acétique ; et
Acide (3*S*)-(3-{cyclohexylméthyl-[3-(2-méthoxy-phényl)-propionyl]-amino}-6-fluoro-1,2,3,4-tétrahydro-9*H*-carbazol-9-yl)-acétique ;
ou sel d'un tel composé.

12. Composition pharmaceutique qui comprend un composé selon l'une quelconque des revendications 1 à 11 ou un sel pharmaceutiquement acceptable de celui-ci, et un véhicule pharmaceutiquement acceptable.

13. Composé selon l'une quelconque des revendications 1 à 11 ou sel pharmaceutiquement acceptable de celui-ci, ou composition pharmaceutique selon la revendication 11, en vue d'une utilisation en tant que médicament.

14. Utilisation d'un composé selon l'une quelconque des revendications 1 à 11 ou d'un sel pharmaceutiquement acceptable de celui-ci dans la préparation d'un médicament pour la prévention et/ou le traitement de maladies/d'affections allergiques/immunitaires chroniques et aiguës, dont les suivantes : asthme allergique, rhinite, rhinite allergique, bronchopneumopathie chronique obstructive, dermatite, affection intestinale inflammatoire, polyarthrite rhumatoïde, néphrite allergique, conjonctivite, eczéma constitutionnel, asthme bronchique, allergie alimentaire, affection systémique à mastocytes, choc anaphylactique, urticaire, eczéma, démangeaisons, inflammation, lésions d'ischémie-reperfusion, maladies cérébro-vasculaires, pleurésie, rectocolite hémorragique, maladies liées aux polynucléaires éosinophiles, y compris syndrome de Churg et Strauss et sinusite, et affections à granulocytes basophiles, y compris leucémie à basophiles et granulocytose basophile.
